(19) Europäisches Patentamt
European Patent Office
Office européen des brevets

(11) EP 1 101 492 A1

(12) EUROPEAN PATENT APPLICATION
published in accordance with Art. 158(3) EPC

(43) Date of publication:
**23.05.2001 Bulletin 2001/21**

(21) Application number: **99929875.5**

(22) Date of filing: **16.07.1999**

(51) Int. Cl.$^7$: **A61K 31/16**, A61K 31/165, A61K 31/215, A61K 31/35, A61K 31/415, A61K 31/66, A61K 31/70

(86) International application number:
**PCT/JP99/03851**

(87) International publication number:
**WO 00/03703 (27.01.2000 Gazette 2000/04)**

(84) Designated Contracting States:
**AT BE CH CY DE DK ES FI FR GB GR IE IT LI LU MC NL PT SE**
Designated Extension States:
**AL LT LV MK RO SI**

(30) Priority: **17.07.1998 JP 21866298**

(71) Applicant:
**FUJI YAKUHIN KOGYO KABUSHIKI KAISHA**
**Takaoka-shi Toyama 933-8511 (JP)**

(72) Inventors:
- **IGETA, Katsuhiro-Fuji Yakuhin Kogyo Kabushiki Kais**
  **Toyama 933-8511 (JP)**
- **TOBETTO, Kenji-Maruho R+D Laboratories**
  **Kyoto 600-8815 (JP)**
- **SAIKI, Ikuo**
  **Toyama 939-2728 (JP)**
- **ODAKE, Shinjiro-Fuji Yakuhin Kogyo Kabushiki Kaish**
  **Toyama 933-8511 (JP)**
- **FUJISAWA, Tetsunori-Fuji Yakuhin Kogyo Kabushiki K**
  **Toyama 933-8511 (JP)**
- **MATSUO, Tetsu-Maruho Co., Ltd.**
  **Osaka 531-0071 (JP)**
- **OKU, Tohru-Fuji Yakuhin Kogyo Kabushiki Kaisha**
  **Toyama 933-8511 (JP)**

(74) Representative:
**von Kreisler, Alek, Dipl.-Chem. et al**
**Patentanwälte,**
**von Kreisler-Selting-Werner,**
**Bahnhofsvorplatz 1 (Deichmannhaus)**
**50667 Köln (DE)**

# (54) NOVEL REMEDIES FOR ALLERGIC DISEASES

(57) Disclosed are anti-inflammatory agents, antiallergic agents, particularly prophylactically and/or therapeutically effective drugs for type I and/or IV allergic conditions, and further pharmaceutical drugs for prophylactically and/or therapeutically treating bronchial asthma, atopic diseases, etc. The inventive drugs comprising a prophylactically and/or therapeutically effective amount of a hydroxamic acid derivative are active in the prophylaxis and/or therapy of allergy, especially type I and/or IV allergy, etc. The drugs are active in prophylactically and/or therapeutically treating inflammation, rhinitis, conjunctivitis, bronchial asthma, atopic diseases (including dermatitis, enteritis, etc.), or allergic gastroenterocolitis (allergic inflammation in digestive tract). The application of such drugs leads to (A) reduction of cells (such as lymphocytes, neutrophils, mast cells, eosinophils, basophils, macrophages and monocytes) at a diseased site, and/or (B) alleviation of inflammatory symptoms caused by migration, infiltration or accumulation of cells (such as lymphocytes, neutrophils, mast cells, eosinophils, basophils, macrophages and monocytes) to (or at) the diseased site, (C) inhibition of pathophysiological functions in cells (such as lymphocytes, neutrophils, mast cells, eosinophils, basophils, macrophages, monocytes, Langerhans cell and dendritic cells), and/or (D) reducing the blood level or inhibiting the production, of antibodies, especially IgE. Accordingly, the drugs are useful in the prophylaxis and/or therapy of diseases, disorders or ill conditions at the site.

EP 1 101 492 A1

## Description

### Field of the Invention

The present invention relates to antiallergic agents, anti-inflammatory agents, or prophylactic and/or therapeutic drugs for a disease selected from the group consisting of bronchial asthma, allergic gastroenterocolitis, allergic rhinitis, atopic diseases, allergic conjunctivitis, etc., which comprise each an effective amount of at least one member selected from the group consisting of a compound of the general formula (I) and a pharmaceutically acceptable salt or solvate thereof.

In another aspect, the present invention relates to

(i) a method for obtaining an antiallergic and/or anti-inflammatory action, and/or a normalizing action on physiological or biological responses related thereto, or

(ii) a method for prophylactically and/or therapeutically treating at least one disease selected from the group consisting of bronchial asthma, allergic gastroenterocolitis, allergic rhinitis, atopic diseases, allergic conjunctivitis, etc., wherein said method comprises the administration of a prophylactically and/or therapeutically effective amount of at least one member selected from the group consisting of a compound of the general formula (I) and a pharmaceutically acceptable salt or solvate thereof.

### Background of the Invention

Allergic reactions are classified according to their pathophysiological natures into four types: type I, type II, type III and type IV. Classically, the type I, type II and type III allergies are included in the immediate type hypersensitivity depending on the interaction of antigens with humoral antibodies and the type IV allergy is in the delayed type hypersensitivity, based on terms until the onset thereof after elicitation with antigen.

The type I allergy is a reaction in which IgE antibodies participate and also called anaphylaxis. Examples of type I allergic responses include bronchial asthma, atopic diseases (dermatitis, enteritis, etc.), allergic rhinitis such as pollinosis, allergic conjunctivitis, food allergy, etc.

The type II allergy is called the cytotoxic allergy or the stimulatory allergy. This type II allergy participates in the onset of diseases including tissue disorders caused by mismatched red cell transfusion, autoimmune hemolytic anemia, idiopathic thrombocytopenia, myasthenia gravis, Basedow's disease, etc. Thus, when IgG or IgM antibodies raised against extrinsic or intrinsic antigens are bound to the antigens on target cells, complements or phagocytes are activated and, as a result, the target cells are damaged. Or, when antibodies are bound to autoantigens such as receptors on a cell surface and the like, the antibodies are misunderstood as ligands for the receptors, or the antibodies disturb the binding of intrinsic specific ligands to the receptors whereby the biological functions of tissues are stimulated or suppressed and their homeostasis is made confused.

The type III allergy is called the immune complex-mediated or the Arthus type allergy. The major disorders of this type III allergy include serum sickness, acute glomerulonephritis, lupus nephritis, hypersensitivity pneumonitis, etc. IgG or IgM antibodies in blood are coupled with soluble antigens to form insoluble immune complexes which are subsequently deposited at fixed sites, etc., thereby giving rise to acute inflammatory reactions. Thus, the tissue damages are associated with activation of complements and phagocytes resulted by fixation of immune complexes in tissues, etc., said immune complexes being formed by coupling IgG or IgM antibodies in blood with soluble antigens.

The type IV allergy is called the delayed-type or cell-mediated allergy. It covers contact dermatitis, metal allergy, the tuberculin reaction, etc. Inflammatory symptoms characterized by erythema and induration become evident 24 to 72 hours after antigen exposure.

Inflammation has been clinically and basicly studied for many years but lots of matters have been left unsolved. With regard to its concept, definition and classification, there are a variety of diverse opinions. In terms of pathology, inflammation refers to diseased states accompanied by <u>rubor</u> (redness), <u>calor</u> (heat or warmth), <u>tumor</u> (swelling), <u>dolor</u> (pain) and <u>functio</u> <u>laesa</u> (inhibited or lost function). At present time, by inflammation is expressed the response by a host against an injury or surgical stress which damages cells and tissues, said response including a regeneration process against defective invasion, a repairing reaction to destructive stress and a defensive reaction to infectious stress. Such an invasion is a foreign matter or body to be discharged or excluded from the host irrespective of its origin (extrinsic and intrinsic).

Bronchial asthma is a chronic inflammatory illness of airways. Inflammation of the airway is accompanied by a bronchial hyperreactivity, thereby causing a reversible airway obstruction in response to various stimulants; as a result, signs or symptoms such as stridor and dyspnea occur. Further, the characteristic features of bronchial asthma reside in inflammation of airways histologically, presence of IgE antibodies to environmental antigens observed in a large number of patients immunologically, hyperresponsiveness of airways physiologically and paroxysmal dyspnea,

stridor and reversible airway contraction (bronchospasm) clinically. It is likely that type I allergy participates in its onset; however, there are some papers reporting that type IV allergy participates in the progress to chronic and intractable asthma during the stage of airway inflammation. Complete cure of bronchial asthma is fundamentally difficult. Furthermore, severe and dangerous processes are apt to happen upon asthma attacks. Accordingly, there has been a demand for effective therapeutic methods therefor.

Atopic dermatitis is a peculiar dermatitis, i.e., a disease which arises from an atopic diathesis as a background and proceeds chronically. There has been a paper reporting that atopic dermatitis is accompanied by a significantly high titer of blood IgE antibody similarly to allergic diseases including infantile allergic bronchial asthma, allergic rhinitis, urticaria, etc., thereby suggesting some possibility that type I allergy participates therein. However, it has been also said that atopic dermatitis cannot be explained by type I allergy only and so plenty of natures in atopic dermatitis are remaining to be solved. Pathohistological changes in atopic dermatitis skin are quite similar to those in allergic contact dermatitis which is a type IV allergy and, therefore, it is likely that atopic dermatitis is a disease where both type I and type IV allergic responses are complicately entangled. Its characteristic symptoms are that at face, head, auricle, etc. eczema-like rash of skin and at head and eyebrow sites erythema with yellowish white crust can be observed during an infantile period, etc. wherein in some cases erythema accompanied by desquamation may occur on the trunk and limbs; dry eczema on the trunk or rash of skin at the flexures of limbs and the like may also be observed. In childhood, two types of cardinal symptoms are mostly noted as follows: (a) dry eczema often generated on the trunk, particularly on the back and (b) lichenified eczema often generated on skin at the flexures of the limbs and neck. It is another characteristic that, at adolescent and adult stages, there are plenty of severe cases of this disease which have not been naturally cured.

Although the causes of those symptoms have not been fully clarified yet, it is believed that constitutional factors are mostly very responsible in patients. It is considered that their examples are (a) a disorder of skin barrier as a result of abnormal lipid metabolism in the skin tissues, (b) a great aptitude for producing IgE, and (c) an antibody production to allergens, related to the above.

When the skin at the diseased sites (lichenified eczema, dry eczema, etc.) of the patients suffering from atopic dermatitis is abraded with a hard tool having a dull edge, anemic white lines due to vasoconstriction are generated (white line dermatographism) but red dermatographic lines which are normally noted in healthy people are not resulted. In addition, when acetylcholine or methacholine is intradermally injected, the diseased sites of the patient generate pale spots but does not generate the erythema which normally occurs in healthy persons. Such an atopic dermatitis often progresses chronically and is intractable. In recent years, such a clinical case tends to increase. Therefore, there has been a demand for safe and effective remedy therefor.

Allergic conjunctivitis is an inflammatory disease of conjunctiva caused by an allergic reaction. Although it is mainly caused by a type I allergic reaction, it sometimes includes those which are caused by a type IV allergic reaction. There are plenty of cases where pollens, ticks, house dusts, fungi, animal hairs and furfures, etc. act as antigens, thereby leading to ocular itching of which patients complain, as well as other symptoms such as conjunctival hyperemia and ocular discharge. When an ectocorneal disorder is complicated therewith, various symptoms such as a feel of foreign substance, ocular pain, lacrimation and vision reduction may be resulted as well.

Allergic rhinitis has three cardinal signs: frequent sneezing onset, excessive watery nasal discharge and nasal obstruction, caused by an allergic reaction; in addition, it may be accompanied by systemic malaise and itching of nasopharynx, eye, etc. There are not a few cases combined with other atopic diseases such as asthma, atopic conjunctivitis and atopic dermatitis.

Allergic digestive tract inflammation (allergic gastroenterocolitis, allergic gastrointestinal inflammation or allergic bowel inflammation) is an allergic inflammatory disease on the surface of digestive tracts and/or a digestive morbid state as one of systemic allergic symptoms, induced to an antigen contained in food, etc. Raised symptoms of this type of diseases include stomachache, diarrhea, vomiting and inflammatory signs around or in the mouth, at the anal periphery, etc. For spending a normal daily life for those patients, it is necessary that each substance acting as an antigen should be specified and the antigen should also be removed from stuff taken per os such as food. Further, in other allergic diseases (such as atopic dermatitis) where an allergic digestive tract inflammation is manifested as one of the symptoms, it is also important to remove the specific antigen. However, it is quite difficult to completely remove the antigens from foods, environment, etc. Therefore, there has been a demand for an effective and highly safe therapeutic agent therefor.

Matrix metalloproteinases (MMPs) are a family of neutral proteases and have $Zn^{2+}$ at the active center. It has been already known that the MMPs play an important role in the degradation of extracellular matrices in the diseases accompanied by destruction of tissues. Up to now, 17 MMPs have been known. Destruction of extracellular matrices by the MMPs is one of the important causes of delaying the cure of intractable diseases accompanied by destruction of tissues. The MMP activity is controlled by (a) a regulated expression at a gene level, (b) adjustment in conversion from a latent proform to an active form by proteases and (c) regulation of enzymatic activity by intrinsic MMP inhibitors, i.e., tissue inhibitors of metalloproteinase (TIMPs). Thus, it has been believed that an increase in the MMP

activity as such is resulted from an imbalance of quantities between the MMPs and the intrinsic inhibitors. For example, it has been reported that, with regard to the destruction of the extracellular matrices by the MMPs, etc., such TIMPs participate in rheumatoid arthritis, arthrosis, skin ulcer, metastasis of tumor cells, etc.

However, there has been no paper reporting that inhibitors of the MMP and/or compounds and natural products (including fragments thereof) structurally similar to the MMP inhibitors are effective in allergic diseases, particularly in type I and/or type IV allergies, inflammation, atopic diseases such as atopic dermatitis and bronchial asthma.

Conventional anti-allergic agents, adrenocortico-steroids, antihistaminic agents, etc. are used for the drug therapy of allergic diseases. However, although prior art antiallergic agents, antihistaminic agents and the like are effective in the allergic reactions at the initial stage of type I allergy, mediated by chemical mediators such as histamine, they are not expected to be effective in the cell-mediated chronic inflammation phases due to their nature. In addition, although the adrenocorticosteroids are limited therapeutic agents for the cell-mediated allergic reaction phases, there are also plenty of prior art agents which are still resistant to such a therapeutic use. Further, when the adrenocorticosteroids are continuously administered, quite severe adverse reactions such as weakening (thinning) of the skin, immunosuppression and inhibition of in vivo adrenocortical hormone secretion are resulted or a sudden interruption or withdrawal of the therapy is troublesome due to a phenomenon of steroid dependency, etc. Accordingly, there are a large number of unfavorable aspects for clinical use. Patients (particularly adult patients) suffering from bronchial asthma, atopic diseases, allergic rhinitis, allergic conjunctivitis, food allergy and inflammatory skin diseases are increasing in recent years. Therefore, there has been a demand for new and safer drugs capable of broadly achieving the effects on acute, subacute and/or chronic inflammatory responses, particularly on type I and/or type IV allergic diseases.

With regard to metalloproteinase inhibitors, there have been reports on metastasis of tumor cells and arthropathy correlating with destruction of extracellular matrices by the MMPs, etc. However, there has been no paper reporting that the metalloproteinase inhibitor effectively acts on the diseases such as bronchial asthma and atopic diseases.

Bronchial asthma is a disease in which there is paroxysmal stridor and dyspnea. When an asthma attack becomes severe, the pulmonary function which is almost normal at the non-attack stage lowers in terms of a forced expiratory volume in one second or a ratio of forced expiratory volume in one second to forced vital capacity whereupon an obstructive ventilatory disorder is observed with an increase in airway resistance, a decrease in pulmonary compliance, a decrease in vital capacity, etc. Especially in the case of chronic bronchial asthma, airway resistance increases due to contraction of bronchial smooth muscles, inflammation, thickening, edema, muciferous promotion of airway mucosa, etc. In severe cases, death by suffocation (death by asthma) due to airway obstruction often happens. As a first-aid agent in such a case, a bronchodilator (β -adrenergic agent, theophylline, etc.) is used. With regard to metalloproteinase inhibitors, there has been no paper at all reporting that their therapeutic effect on inflammatory edema is specifically ascertained, as aforementioned. In addition to that, there has not been known at all that they are capable of preventing the death by suffocation caused by bronchial asthma attacks.

## Disclosure of the Invention

The present inventors have carried out an extensive investigation with paying their attention to the fact that metalloproteinase-inhibitory hydroxamic acid derivatives act on inflammatory edema in an inhibiting manner. As a result, the present inventors have succeeded in developing effective anti-allergic agents (such as prophylactic and/or therapeutic agents for type I and/or type IV allergies and, further, prophylactic and/or therapeutic agents for inflammation) utilizing the said hydroxamic acid derivatives. In addition, the present inventors have tested using metalloproteinase-inhibitory hydroxamic acid derivatives what pharmacological action the hydroxamic acid derivatives can actually achieve in various experimental inflammation animal models. As a result, the present inventors have ascertained that the metalloproteinase-inhibitory hydroxamic acid derivatives have potent antiinflammatory activities and excellent properties in terms of safety, etc. (i.e., no loss of body weight, no thinning of the skin and no rebound phenomenon after interruption and/or withdrawal of the drug administration are noted). Further, the present inventors have found that the metalloproteinase-inhibitory hydroxamic acid derivatives are clinically useful. Thus, the present invention is provided. The present inventors have also found that the hydroxamic acid derivatives of the present invention have excellent properties as the therapeutic agents to ventilatory disorders (which will sometimes result in death) caused by airway stenosis and airway obstruction frequently observed at the onset of acute and severe bronchial asthma. Thus, therapeutic methods for such diseases are provided.

An objective of the present invention is to provide a means for therapeutically treating cell-mediated inflammatory diseases and type I and/or type IV allergic diseases, for ameliorating morbid signs or symptoms of such diseases, and/or for preventing an onset of such clinical signs and symptoms, with a pharmaceutical composition which comprises an effective amount of the hydroxamic acid derivative according to the present invention.

A particular objective of the present invention is to provide the therapy of patients suffering from bronchial

asthma (including those who have become chronic), atopic dermatitis, hay fever, pollinosis, allergic rhinitis (including seasonal rhinitis), allergic conjunctivitis (including seasonal conjunctivitis), allergic digestive tract inflammation, food allergy, inflammatory skin diseases, etc, and the amelioration and/or prophylactic therapy of signs or symptoms experienced by the said patients, with a pharmaceutical composition comprising an effective amount of the hydroxamic acid derivative according to the present invention. In addition, the said pharmaceutical composition has various excellent characteristics in the therapy or amelioration of airway stenosis and airway obstruction often observed in acute, worsening bronchial asthma and further in the prophylaxis and/or therapy of morbid conditions thereof which have become chronic.

Thus, the present invention provides:

(1) a prophylactic and/or therapeutic drug for allergic diseases which comprises an effective amount of at least one member selected from the group consisting of a compound haying the following formula (I):

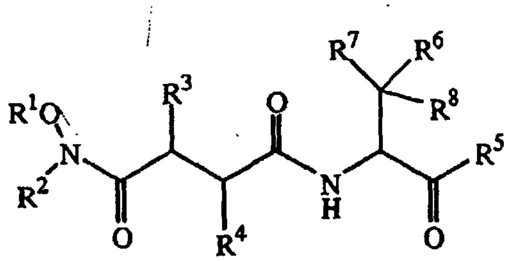

(I)

wherein $R^1$ is selected from the group consisting of hydrogen, unsubstituted or optionally substituted aralkyl, silyl which may optionally have three substituents, tetrahydropyranyl, unsubstituted or optionally substituted aralkyloxycarbonyl, unsubstituted or optionally substituted alkyloxycarbonyl, unsubstituted or optionally substituted alkyl, and a hydroxy-protecting group;
$R^2$ is selected from the group consisting of hydrogen, unsubstituted or optionally substituted aralkyloxycarbonyl, unsubstituted or optionally substituted alkyloxycarbonyl, 9-fluorenylmethyloxycarbonyl, and an amino-protecting group;
$R^3$ is selected from the group consisting of hydrogen, hydroxy, unsubstituted or optionally substituted alkyl, and unsubstituted or optionally substituted aralkyl;
$R^4$ is selected from the group consisting of unsubstituted or optionally substituted alkyl, and unsubstituted or optionally substituted aralkyl;
$R^5$ is $OR^9$ or

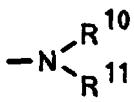

$R^9$ is selected from the group consisting of hydrogen, unsubstituted or optionally substituted alkyl, unsubstituted or optionally substituted aralkyl, and a carboxyl-protecting group;
$R^{10}$ and $R^{11}$, which may be identical or different, are each independently selected from the group consisting of hydrogen, unsubstituted or optionally substituted alkyl, unsubstituted or optionally substituted cycloalkyl, an unsubstituted or optionally substituted heterocyclic group, and an amino-protecting group, or $R^{10}$ and $R^{11}$ taken together with the nitrogen atom to which they are attached form an unsubstituted or optionally substituted heterocyclic group;
$R^6$ is selected from the group consisting of hydrogen, hydroxy, amino, and a group of the formula: -X-Y

wherein X is selected from the group consisting of oxygen, unsubstituted or optionally substituted ($C_1$-$C_6$)

alkylene, and unsubstituted or optionally substituted phenylene, and

Y is a group of the formula: -A-B or -B,

wherein A is selected from the group consisting of unsubstituted or optionally substituted ($C_1$-$C_6$) alkylene, oxygen, sulfur, imino, and unsubstituted or optionally substituted ($C_1$-$C_6$) alkyleneimino, and B is selected from the group consisting of hydrogen, amino, amidino, acylimidoyl, unsubstituted or optionally substituted imidazolyl, unsubstituted or optionally substituted hydantoin-1-yl, unprotected or optionally protected bis(phosphono)methyl, unprotected or optionally protected glucopyranosyl, and unprotected or optionally protected bis(phosphono)hydroxymethyl;

$R^7$ is selected from the group consisting of hydrogen, hydroxy, unsubstituted or optionally substituted alkyl, and unsubstituted or optionally substituted aralkyl;

$R^8$ is selected from the group consisting of hydrogen, hydroxy, unsubstituted or optionally substituted alkyl, and unsubstituted or optionally substituted aralkyl; and a pharmaceutically acceptable salt or solvate thereof;

(2) the drug according to the above (1) which exerts a prophylactic and/or therapeutic action on inflammation resulting from an allergic reaction;

(3) the drug according to the above (1) or (2) for the prophylactic and/or therapeutic treatment of type I allergic disorders;

(4) the drug according to the above (1) or (2) for the prophylactic and/or therapeutic treatment of type IV allergic disorders;

(5) the drug according to the above (1) or (2) for reducing a blood antibody titer or inhibiting the production of antibodies to prophylactically and/or therapeutically treat allergic diseases;

(6) the drug according to the above (1), (2) or (5) for reducing the blood level of IgE or inhibiting the production of IgE to prophylactically and/or therapeutically treat allergic diseases;

(7) a prophylactic and/or therapeutic drug for bronchial asthma which comprises an effective amount of at least one member selected from the group consisting of the aforementioned compound (I) wherein $R^1$ to $R^8$, all have the same meanings as defined in the above (1), and a pharmaceutically acceptable salt or solvate thereof;

(8) the drug according to the above (7) for the prophylactic and/or therapeutic treatment of chronic bronchial asthma;

(9) a prophylactic and/or therapeutic drug for allergic rhinitis which comprises an effective amount of at least one member selected from the group consisting of the aforementioned compound (I) wherein $R^1$ to $R^8$, all have the same meanings as defined in the above (1), and a pharmaceutically acceptable salt or solvate thereof;

(10) a prophylactic and/or therapeutic drug for atopic diseases which comprises an effective amount of at least one member selected from the group consisting of the aforementioned compound (I) wherein $R^1$ to $R^8$, all have the same meanings as defined in the above (1), and a pharmaceutically acceptable salt or solvate thereof;

(11) the drug according to the above (10) for the prophylactic and/or therapeutic treatment of atopic dermatitis;

(12) a prophylactic and/or therapeutic drug for allergic conjunctivitis which comprises an effective amount of at least one member selected from the group consisting of the aforementioned compound (I) wherein $R^1$ to $R^8$, all have the same meanings as defined in the above (1), and a pharmaceutically acceptable salt or solvate thereof;

(13) a prophylactic and/or therapeutic drug for immediate, late and/or very late allergic responses which comprises an effective amount of at least one member selected from the group consisting of the aforementioned compound (I) wherein $R^1$ to $R^8$, all have the same meanings as defined in the above (1), and a pharmaceutically acceptable salt or solvate thereof, said drug having the property of alleviating allergic symptoms wherein the allergic symptom is an immediate, late and/or very late response; and

(14) a prophylactic and/or therapeutic drug for allergic gastroenteritis (allergic inflammation in digestive tract) which

comprises an effective amount of at least one member selected from the group consisting of the aforementioned compound (I) wherein $R^1$ to $R^8$, all have the same meanings as defined in the above (1), and a pharmaceutically acceptable salt or solvate thereof.

In another aspect, the present invention provides:

(15) the drug according to any of the above (1) to (14) wherein, with respect to the aforementioned formula (I),

$R^1$ and $R^2$ are hydrogen;
$R^3$ is selected from the group consisting of hydrogen, ($C_1$-$C_9$) alkyl, ($C_3$-$C_7$) cycloalkyl-substituted lower ($C_1$-$C_4$) alkyl, hydroxy, amino-substituted ($C_1$-$C_6$) alkyl, phenyl-lower ($C_1$-$C_4$) alkyl, guanido-substituted phenyl-lower ($C_1$-$C_4$) alkyl, amino-substituted phenyl-lower ($C_1$-$C_4$) alkyl, carboxy-substituted phenyl-lower ($C_1$-$C_4$) alkyl, carbamoyl-substituted phenyl-lower ($C_1$-$C_4$) alkyl, hydroxy-substituted phenyl-lower ($C_1$-$C_4$) alkyl, guanido-substituted lower ($C_1$-$C_4$) alkyl-substituted phenyl-lower ($C_1$-$C_4$) alkyl, unprotected or optionally protected amino-substituted lower ($C_1$-$C_4$) alkyl-substituted phenyl-lower ($C_1$-$C_4$) alkyl, hydroxy-substituted lower ($C_1$-$C_4$) alkyl-substituted phenyl-lower ($C_1$-$C_4$) alkyl, lower ($C_1$-$C_4$) alkoxycarbonyl-substituted phenyl-lower ($C_1$-$C_4$) alkyl, lower ($C_1$-$C_4$) alkylimino-substituted ($C_1$-$C_6$) alkyl, lower ($C_1$-$C_4$) acylimidoylimino-substituted ($C_1$-$C_6$) alkyl, arylmethylimino-substituted ($C_1$-$C_6$) alkyl, nitrogen-containing heterocyclic radical-substituted lower ($C_1$-$C_4$) alkylimino-substituted ($C_1$-$C_6$) alkyl, nitrogen-containing heterocyclic radical-substituted lower ($C_1$-$C_4$) alkyl, oxygen-containing ($C_1$-$C_8$) straight chain or branched alkyl, arylsulfonamido-substituted lower ($C_1$-$C_4$) alkyl-substituted phenyl-lower ($C_1$-$C_4$) alkyl, alkylsulfonamido-substituted lower ($C_1$-$C_4$) alkyl-substituted phenyl-lower ($C_1$-$C_4$) alkyl, lower ($C_1$-$C_4$) alkylimino-substituted lower ($C_1$-$C_4$) alkyl-substituted phenyl-lower ($C_1$-$C_4$) alkyl, aryloxy-substituted lower ($C_1$-$C_4$) alkyl, and hydroxy-substituted ($C_1$-$C_8$) alkyl;
$R^4$ is selected from the group consisting of ($C_3$-$C_9$) alkyl, hydroxy-substituted ($C_3$-$C_8$) alkyl, and unsubstituted or optionally substituted aryl-lower ($C_1$-$C_4$) alkyl;
$R^5$ is $OR^9$ or

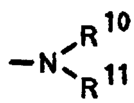

$R^9$ is selected from the group consisting of hydrogen, ($C_1$-$C_{16}$) alkyl, halogen-substituted ($C_1$-$C_{16}$) alkyl, hydroxy-substituted ($C_1$-$C_{16}$) alkyl, ($C_1$-$C_{10}$) alkoxy-substituted ($C_1$-$C_{10}$) alkyl, amino-substituted ($C_1$-$C_{16}$) alkyl, mono-($C_1$-$C_6$) alkylamino-substituted ($C_1$-$C_{16}$) alkyl, di-($C_1$-$C_6$) alkylamino-substituted ($C_1$-$C_{16}$) alkyl, ($C_3$-$C_{10}$) cycloalkyl, ($C_3$-$C_{10}$) cycloalkyl-substituted ($C_1$-$C_6$) alkyl, ($C_2$-$C_{16}$) alkenyl, ($C_2$-$C_{16}$) alkinyl, ($C_6$-$C_{10}$) aryl, ($C_6$-$C_{10}$) aryl-substituted ($C_1$-$C_6$) alkyl, ($C_1$-$C_6$) alkoxycarbonyloxy-substituted ($C_1$-$c_6$) alkyl, ($C_2$-$C_7$) alkanoyloxy-substituted ($C_1$-$C_6$) alkyl, silyl which may optionally have three substituents, and phthalimido-substituted ($C_1$-$C_6$) alkyl;
$R^{10}$ is selected from the group consisting of lower ($C_1$-$C_4$) alkyl, ($C_3$-$C_7$) cycloalkyl, mono- or di-lower ($C_1$-$C_4$) alkylamino-substituted lower ($C_1$-$C_4$) alkyl, carboxy-substituted lower ($C_1$-$C_4$) alkyl, hydroxy-substituted ($C_1$-$c_6$) alkyl, bis(phosphono)hydroxymethyl-substituted ($C_1$-$C_{11}$) alkyl, tetrabenzyl bis(phosphono)hydroxymethyl-substituted ($C_1$-$C_{11}$) alkyl, and a nitrogen-containing heterocyclic radical;
$R^{11}$ is hydrogen;
$R^6$ is selected from the group consisting of hydrogen, hydroxy, amino, and a group of the formula: -X-Y

wherein X is selected from the group consisting of oxygen, ($C_1$-$C_6$) alkylene and phenylene, and
Y is a group of the formula: -A-B or -B

wherein B is selected from the group consisting of hydrogen, amino, amidino, lower ($C_1$-$C_4$) acylimidoyl, unsubstituted or optionally substituted benzimidoyl, bis(phosphono)methyl, tetra-lower ($C_1$-$C_4$) alkyl bis(phosphono)methyl, tri-lower ($C_1$-$C_4$) alkyl bis(phosphono)methyl, bis(phosphono)hydroxymethyl, tetrabenzyl bis(phosphono)hydroxymethyl, lower ($C_1$-$C_4$) alkyl-substituted hydantoin-1-yl, ($C_1$-$C_4$) acyl-substituted glucopyranosyl, glucopyranosyl, and lower ($C_1$-$C_4$) alkyl-substituted imidazol-3-yl, and
A is selected from the group consisting of lower ($C_1$-$C_4$) alkylene, imino, and lower ($C_1$-$C_4$) alkylene-

imino;

$R^7$ is hydrogen or lower ($C_1$-$C_4$) alkyl; and
$R^8$ is hydrogen or lower ($C_1$-$C_4$) alkyl;

(16) the drug according to any of the above (1) to (15) wherein, with respect to the aforementioned formula (I),

$R^1$ and $R^2$ are hydrogen;
$R^3$ is selected from the group consisting of hydrogen, hydroxy, methyl, isobutyl, aminopropyl, phenylpropyl, guanidophenylpropyl, aminophenylpropyl, hydroxyphenylpropyl, carboxyphenylpropyl, carbamoylphenylpropyl, aminomethylphenylpropyl, guanidomethylphenylpropyl, hydroxymethylphenylpropyl, aminomethylbenzyl, toluenesulfonamidomethylbenzyl, methanesulfonamidomethylbenzyl, isobutyliminomethylbenzyl, phthalimidomethylbenzyl, phenoxyethyl, aminopentyl, acetimidoyliminopentyl, isobutyliminopentyl, pyridylmethyliminopentyl, methoxycarbonylphenylpropyl, ethoxyethoxyethyl, hydroxyoctyl, butoxyethyl, isobutyloxyethyl, morpholinopropyl, (3,4,4-trimethyl-2,5-dioxoimidazolidin-1-yl)propyl, cyclohexylpropyl, isopropyliminomethylbenzyl, isopropyliminopentyl, and piperidinopropyl;
$R^4$ is selected from the group consisting of naphthylmethyl, phenylpropyl, isobutyl, tert-butyl, isopropyl, and hydroxyoctyl;
$R^5$ is is $OR^9$ or

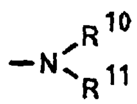

$R^9$ is selected from the group consisting of hydrogen, methyl, ethyl, n-propyl, iso-propyl, n-butyl, iso-butyl, sec-butyl, tert-butyl, n-hexyl, n-pentyl, neopentyl, heptyl, octyl, nonyl, decyl, undecyl, dodecyl, tridecyl, tetradecyl, pentadecyl, hexadecyl, adamantyl, 2,2,2-trichloroethyl, 2-chloroethyl, 2-hydroxyethyl, methoxymethyl, methoxyethyl, benzyloxymethyl, benzyloxyethyl, 2-methoxyethoxyethyl, 2-aminoethyl, 2-methylaminoethyl, 2-dimethylaminoethyl, cyclohexyl, cyclohexylmethyl, allyl, cinnamyl, 2-propenyl, phenyl, 4-methoxyphenyl, 4-bromophenyl, trityl, benzhydryl, acetoxymethyl, acetoxyethyl, triethylsilyl, tert-butyldimethylsilyl, tert-butyldiphenylsilyl, and phthalimidomethyl;
$R^{10}$ is selected from the group consisting of methyl, cyclopropyl, 2-(N,N-dimethylamino)ethyl, 2-carboxyethyl, 2-hydroxyethyl, 2-hydroxy-1,1-dimethylethyl, 2-hydroxy-1-methylethyl, 6,6-bis(phosphono)-6-hydroxyhexyl, tetrabenzyl 6,6-bis-(phosphono)-6-hydroxyhexyl, piperidyl, and morpholinyl;
$R^{11}$ is hydrogen;
$R^6$ is selected from the group consisting of hydrogen, hydroxy, amino, and a group of the formula: -X-Y

wherein X is selected from the group consisting of oxygen, methylene, ethylene, trimethylene, tetramethylene, pentamethylene, hexamethylene, and phenylene, and
Y is a group of the formula: -A-B or -B

wherein B is selected from the group consisting of amino, amidino, acetimidoyl, propionimidoyl, benzimidoyl, bis(phosphono)methyl, tetraethyl bis(phosphono)methyl, triethyl bis(phosphono)methyl, tetramethyl bis(phosphono)methyl, trimethyl bis(phosphono)methyl, bis(phosphono)hydroxymethyl, tetrabenzyl bis(phosphono)hydroxymethyl, 3,4,4-trimethyl-2,5-dioxo-imidazolidin-1-yl, tetraacetyl glucopyranosyl, glucopyranosyl, and 2-methyl-imidazol-3-yl, and
A is selected from the group consisting of imino, methyleneimino, and methylene;

$R^7$ is hydrogen or methyl; and
$R^8$ is hydrogen or methyl;

(17) the drug according to any of the above (1) to (16) wherein, with respect to the aforementioned formula (I) according to the above (1),

$R^1$ and $R^2$ are hydrogen;

$R^3$ is selected from the group consisting of hydrogen, hydroxy, methyl, isobutyl, aminopropyl, phenylpropyl, guanidophenylpropyl, carboxyphenylpropyl, carbamoylphenylpropyl, aminomethylphenylpropyl, guanidomethylphenylpropyl, aminomethylbenzyl, toluenesulfonamidomethylbenzyl, methanesulfonamidomethylbenzyl, isopropyliminomethylbenzyl, phthalimidomethylbenzyl, phenoxyethyl, acetimidoyliminopentyl, isopropyliminopentyl, pyridylmethyliminopentyl, methoxycarbonylphenylpropyl, and cyclohexylpropyl;
$R^4$ is selected from the group consisting of naphthylmethyl, isobutyl, and isopropyl;
$R^5$ is is $OR^9$ or

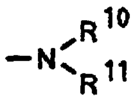

$R^9$ is hydrogen or methyl;
$R^{10}$ is selected from the group consisting of methyl, cyclopropyl, 2-(N,N-dimethylamino)ethyl, 2-hydroxyethyl, 2-hydroxy-1,1-dimethylethyl, 2-hydroxy-1-methylethyl, piperidyl, and morpholinyl;
$R^{11}$ is hydrogen;
$R^6$ is selected from the group consisting of hydrogen, amino, and a group of the formula: -X-Y

wherein X is selected from the group consisting of oxygen, methylene, ethylene, trimethylene, and phenylene, and
Y is a group of the formula: -A-B or -B

wherein B is selected from the group consisting of amino, amidino, acetimidoyl, propionimidoyl, tetraethyl bis(phosphono)methyl, triethyl bis(phosphono)methyl, tetramethyl bis(phosphono)methyl, trimethyl bis(phosphono)methyl, 3,4,4-trimethyl-2,5-dioxo-imidazolidin-1-yl, tetraacetyl glucopyranosyl, and glucopyranosyl, and
A is selected from the group consisting of imino, methyleneimino, and methylene;

$R^7$ and $R^8$ is hydrogen;

(18) the drug according to any of the above (1) to (17) wherein, with respect to the aforementioned formula (I) according to the above (1),

$R^1$ and $R^2$ are hydrogen;

$R^3$ is unsubstituted or optionally substituted aralkyl;

$R^4$ is unsubstituted or optionally substituted alkyl;

$R^5$ is $OR^9$ or

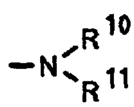

$R^9$ is selected from the group consisting of hydrogen, unsubstituted or optionally substituted alkyl, and unsubstituted or optionally substituted aralkyl;

$R^{10}$ is unsubstituted or optionally substituted alkyl;

$R^{11}$ is hydrogen;

$R^6$ is selected from the group consisting of unsubstituted or optionally substituted aralkyl, unsubstituted or optionally substituted aryl, and unsubstituted or optionally substituted alkyl; and

$R^7$ and $R^8$ is hydrogen;

(19) the drug according to any of the above (1) to (14) which comprises an effective amount of at least one member selected from the group consisting of the aforementioned compound (I) wherein

$R^1$ is hydrogen, or a hydroxy-protecting group;
$R^2$ is hydrogen, or an amino-protecting group;
$R^3$ is selected from the group consisting of hydrogen, hydroxy, unsubstituted or optionally substituted ($C_1$-$C_6$) alkyl, and unsubstituted or optionally substituted aryl-($C_1$-$C_6$) alkyl;
$R^4$ is selected from the group consisting of unsubstituted or optionally substituted ($C_1$-$C_6$) alkyl, and unsubstituted or optionally substituted aryl-($C_1$-$C_6$) alkyl;
$R^5$ is $OR^9$ or

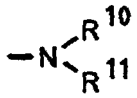

$R^9$ is selected from the group consisting of hydrogen, unsubstituted or optionally substituted ($C_1$-$C_{16}$) alkyl, and unsubstituted or optionally substituted aryl-($C_1$-$C_6$) alkyl;
$R^{10}$ and $R^{11}$, which may be identical or different, are each independently selected from the group consisting of hydrogen, unsubstituted or optionally substituted ($C_1$-$C_{11}$) alkyl, ($C_3$-$C_6$) cycloalkyl, and a heterocyclic group;
$R^6$ is selected from the group consisting of hydrogen, hydroxy, amino, and a group of the formula: -X-Y

wherein X is selected from the group consisting of oxygen, ($C_1$-$C_6$) alkylene, and phenylene, and
Y is a group of the formula: -A-B or -B,

wherein A is selected from the group consisting of ($C_1$-$C_6$) alkylene, imino, and ($C_1$-$C_6$) alkyleneimino, and
B is selected from the group consisting of hydrogen, amino, amidino, acylimidoyl, unsubstituted or optionally substituted imidazolyl, unsubstituted or optionally substituted hydantoin-1-yl, unprotected or optionally protected bis(phosphono)methyl, unprotected or optionally protected glucopyranosyl, and unprotected or optionally protected bis(phosphono)hydroxymethyl;

$R^7$ is selected from the group consisting of hydrogen, hydroxy, unsubstituted or optionally substituted ($C_1$-$C_6$) alkyl, and unsubstituted or optionally substituted aryl-($C_1$-$C_6$) alkyl;
$R^8$ is selected from the group consisting of hydrogen, hydroxy, unsubstituted or optionally substituted ($C_1$-$C_6$) alkyl, and unsubstituted or optionally substituted aryl-($C_1$-$C_6$) alkyl; a stereoisomer thereof, and a pharmaceutically acceptable salt or solvate thereof; and

(20) the drug according to the above (19) wherein, with respect to the aforementioned formula (I),

$R^1$ and $R^2$ are hydrogen;
$R^3$ is selected from the group consisting of hydrogen, hydroxy, methyl, isobutyl, aminopropyl, 3-phenylpropyl, p-guanidophenylpropyl, p-aminophenylpropyl, p-hydroxyphenylpropyl, p-carboxyphenylpropyl, m-carboxyphenylpropyl, p-aminomethylphenylpropyl, p-guanidomethylphenylpropyl, p-hydroxymethylphenylpropyl, p-aminomethylbenzyl, m-aminomethylbenzyl, o-aminomethylbenzyl, p-tolueaesulfonamidomethylbenzyl, p-methanesulfonamidomethylbenzyl, p-isobutyliminomethylbenzyl, p-phthalimidomethylbenzyl, phenoxyethyl, o-aminomethylphenylpropyl, aminopentyl, acetimidoyliminopentyl, isobutyliminopentyl, (pyridin-4-yl)methyliminopentyl, p-methoxycarbonylphenylpropyl, ethoxyethoxyethyl, 8-hydroxyoctyl, n-butoxyethyl, iso-butyloxyethyl, m-methoxycarbonylphenylpropyl, p-carbamoylphenylpropyl, morpholinopropyl, and piperidinopropyl;
$R^4$ is selected from the group consisting of isobutyl, tert-butyl, isopropyl, and 8-hydroxyoctyl;

$R^5$ is is $OR^9$ or

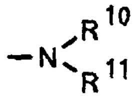

$R^9$ is selected from the group consisting of hydrogen, methyl, ethyl, n-propyl, iso-propyl, n-butyl, iso-butyl, sec-butyl, tert-butyl, n-hexyl, n-pentyl, neopentyl, heptyl, octyl, nonyl, decyl, undecyl, dodecyl, tridecyl, tetradecyl, pentadecyl, hexadecyl, adamantyl, 2,2,2-trichloroethyl, 2-chloroethyl, 2-hydroxyethyl, methoxymethyl, methoxyethyl, benzyloxymethyl, benzyloxyethyl, 2-methoxyethoxyethyl, 2-aminoethyl, 2-methylaminoethyl, 2-dimethylaminoethyl, cyclohexyl, cyclohexylmethyl, allyl, cinnamyl, 2-propenyl, phenyl, 4-methoxyphenyl, 4-bromophenyl, trityl, benzhydryl, acetoxymethyl, acetoxyethyl, triethylsilyl, tert-butyldimethylsilyl, tert-butyldiphenylsilyl, and phthalimidomethyl;
$R^{10}$ is selected from the group consisting of methyl, cyclopropyl, 2-(N,N-dimethylamino)ethyl, 2-carboxyethyl, 2-hydroxyethyl, 2-hydroxy-1,1-dimethylethyl, 2-hydroxy-1-methylethyl, 6,6-bis(phosphono)-6-hydroxyhexyl, tetrabenzyl 6,6-bis(phosphono)-6-hydroxyhexyl, piperidyl, and morpholinyl;
$R^{11}$ is hydrogen;
$R^6$ is selected from the group consisting of hydrogen, hydroxy, amino, and a group of the formula: -X-Y

wherein X is selected from the group consisting of oxygen, methylene, ethylene, trimethylene, tetramethylene, pentamethylene, hexamethylene, and phenylene, and
B which is included in Y or for Y is selected from the group consisting of amino, amidino, acetimidoyl, propionimidoyl, benzimidoyl, bis(phosphono)methyl, tetraethyl bis(phosphono)methyl, triethyl bis(phosphono)methyl, tetramethyl bis(phosphono)methyl, trimethyl bis(phosphono)methyl, bis(phosphono)hydroxymethyl, tetrabenzyl bis(phosphono)hydroxymethyl, 3,4,4-trimethyl-2,5-dioxoimidazolidin-1-yl, tetraacetyl glucopyranosyl, glucopyranosyl, and 2-methyl-imidazol-3-yl, and
when Y is -A-B, A is selected from the group consisting of imino, methyleneimino, and methylene;

$R^7$ is hydrogen or methyl; and
$R^8$ is hydrogen or methyl.

It is apparent that there are chiral carbon atoms designated with * in the compounds of the formula (I), as illustrated in the following formula:

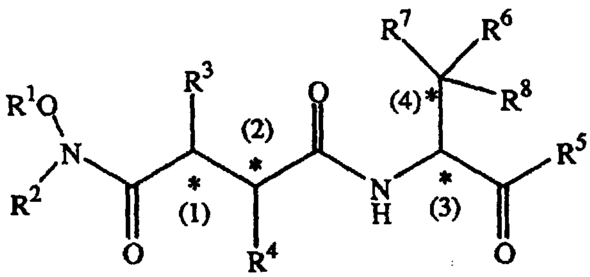

**(I')**

Thus, it should be understood that the compounds given in the above (1) may include not only geometric isomers, stereoisomers, each optically active isomer, racemates, and tautomers thereof, but also metabolite derivatives thereof. All such compounds, isomers, racemates, tautomers and derivatives thereof are intended to be within the scope of the present invention.

In a preferred embodiment of the present invention, the chiral carbon atoms designated with * in the above

compounds (I') include the carbon atoms (1) and (4) in the "R" or "S" configuration, the carbon atom (2) in the "R" configuration, and the carbon atom (3) in the "S" configuration.

In a further aspect, the present invention provides:

(21) a prophylactic and/or therapeutic drug for allergic diseases which comprises an effective amount of at least one member selected from the group consisting of a compound having the following formula (I-1):

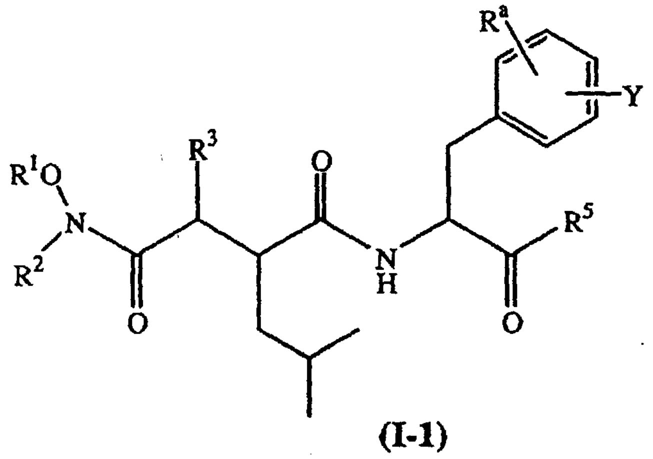

(I-1)

wherein $R^1$ to $R^3$, $R^5$ and Y, all have the same meanings as defined in the above (1); among them, $R^3$ is preferably hydrogen, hydroxy, or unsubstituted or optionally substituted aryl-($C_1$-$C_6$) alkyl;
when Y is a group of the formula: -A-B,

-A- is most preferably ($C_1$-$C_6$) alkylene, ($C_1$-$C_6$) alkyleneimino, or imino;
-B is most preferably hydrogen, amino, acetimidoyl, propionimidoyl, benzimidoyl, amidino, or bis(phosphono)-methyl;

when Y is a group of the formula: -B,

-B is most preferably acetimidoyl, propionimidoyl, benzimidoyl, or amidino;

$R^a$ is hydrogen, or has the same meaning as set forth for the substituent(s) on the "unsubstituted or optionally substituted phenylene" with regard to X in the above (1), or a pharmaceutically acceptable salt or solvate thereof;

(22) the drug according to the above (21) which exerts a prophylactic and/or therapeutic action on inflammation resulting from an allergic reaction;
(23) the drug according to the above (21) or (22) for the prophylactic and/or therapeutic treatment of type I allergic disorders;
(24) the drug according to the above (21) or (22) for the prophylactic and/or therapeutic treatment of type IV allergic disorders;
(25) the drug according to the above (21) or (22) for reducing a blood antibody titer or inhibiting the production of antibodies to prophylactically and/or therapeutically treat allergic diseases;
(26) the drug according to the above (21), (22) or (25) for reducing the blood level of IgE or inhibiting the production of IgE to prophylactically and/or therapeutically treat allergic diseases;
(27) a prophylactic and/or therapeutic drug for bronchial asthma which comprises an effective amount of at least one member selected from the group consisting of the aforementioned compound (I-1) wherein $R^1$ to $R^3$, $R^5$, Y and $R^a$, all have the same meanings as defined in the above (21), and a pharmaceutically acceptable salt or solvate thereof;
(28) the drug according to the above (27) for the prophylactic and/or therapeutic treatment of chronic bronchial asthma;
(29) a prophylactic and/or therapeutic drug for allergic rhinitis which comprises an effective amount of at least one

member selected from the group consisting of the aforementioned compound (I-1) wherein $R^1$ to $R^3$, $R^5$, Y and $R^a$, all have the same meanings as defined in the above (21), and a pharmaceutically acceptable salt or solvate thereof;

(30) a prophylactic and/or therapeutic drug for atopic diseases which comprises an effective amount of at least one member selected from the group consisting of the aforementioned compound (I-1) wherein $R^1$ to $R^3$, $R^5$, Y and $R^a$, all have the same meanings as defined in the above (21), and a pharmaceutically acceptable salt or solvate thereof;

(31) the drug according to the above (30) for the prophylactic and/or therapeutic treatment of atopic dermatitis;

(32) a prophylactic and/or therapeutic drug for allergic conjunctivitis which comprises an effective amount of at least one member selected from the group consisting of the aforementioned compound (I-1) wherein $R^1$ to $R^3$, $R^5$, Y and $R^a$, all have the same meanings as defined in the above (21), and a pharmaceutically acceptable salt or solvate thereof;

(33) a prophylactic and/or therapeutic drug for immediate, late and/or very late allergic responses which comprises an effective amount of at least one member selected from the group consisting of the aforementioned compound (I-1) wherein $R^1$ to $R^3$, $R^5$, Y and $R^a$, all have the same meanings as defined in the above (21), and a pharmaceutically acceptable salt or solvate thereof, said drug having the property of alleviating allergic symptoms wherein the allergic symptom is an immediate, late and/or very late response; and

(34) a prophylactic and/or therapeutic drug for allergic gastroenteritis (allergic inflammation in digestive tract) which comprises an effective amount of at least one member selected from the group consisting of the aforementioned compound (I-1) wherein $R^1$ to $R^3$, $R^5$, Y and $R^a$, all have the same meanings as defined in the above (21), and a pharmaceutically acceptable salt or solvate thereof.

In still another aspect, the present invention provides:

(35) a prophylactic and/or therapeutic drug for allergic diseases which comprises an effective amount of at least one member selected from the group consisting of a compound having the following formula (I-2):

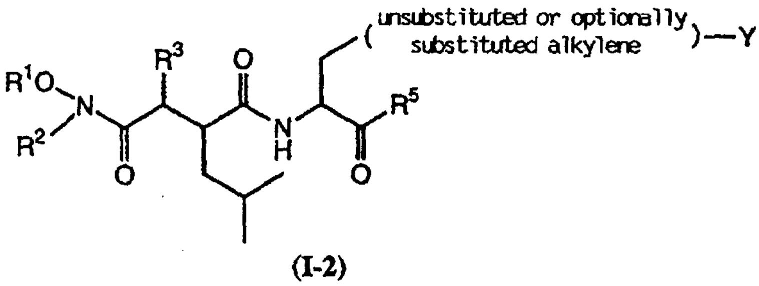

(I-2)

wherein $R^1$ to $R^3$, $R^5$ and Y, all have the same meanings as defined in the above (1);

the substituent on the "(unsubstituted or optionally substituted alkylene)" has the same meaning as set forth for the substituent(s) on the "unsubstituted or optionally substituted alkylene" with regard to X in the above (1), or a pharmaceutically acceptable salt or solvate thereof;

(36) the drug according to the above (35) which exerts a prophylactic and/or therapeutic action on inflammation resulting from an allergic reaction;

(37) the drug according to the above (35) or (36) for the prophylactic and/or therapeutic treatment of type I allergic disorders;

(38) the drug according to the above (35) or (36) for the prophylactic and/or therapeutic treatment of type IV allergic disorders;

(39) the drug according to the above (35) or (36) for reducing a blood antibody titer or inhibiting the production of antibodies to prophylactically and/or therapeutically treat allergic diseases;

(40) the drug according to the above (35), (36) or (39) for reducing the blood level of IgE or inhibiting the production of IgE to prophylactically and/or therapeutically treat allergic diseases;

(41) a prophylactic and/or therapeutic drug for bronchial asthma which comprises an effective amount of at least one member selected from the group consisting of the aforementioned compound (I-2) wherein $R^1$ to $R^3$, $R^5$, Y and the substituent on the "(unsubstituted or optionally substituted alkylene)", all have the same meanings as defined

in the above (35), and a pharmaceutically acceptable salt or solvate thereof;

(42) the drug according to the above (41) for the prophylactic and/or therapeutic treatment of chronic bronchial asthma;

(43) a prophylactic and/or therapeutic drug for allergic rhinitis which comprises an effective amount of at least one member selected from the group consisting of the aforementioned compound (I-2) wherein $R^1$ to $R^3$, $R^5$, Y and the substituent on the "(unsubstituted or optionally substituted alkylene)", all have the same meanings as defined in the above (35), and a pharmaceutically acceptable salt or solvate thereof;

(44) a prophylactic and/or therapeutic drug for atopic diseases which comprises an effective amount of at least one member selected from the group consisting of the aforementioned compound (I-2) wherein $R^1$ to $R^3$, $R^5$, Y and the substituent on the "(unsubstituted or optionally substituted alkylene)", all have the same meanings as defined in the above (35), and a pharmaceutically acceptable salt or solvate thereof;

(45) the drug according to the above (44) for the prophylactic and/or therapeutic treatment of atopic dermatitis;

(46) a prophylactic and/or therapeutic drug for allergic conjunctivitis which comprises an effective amount of at least one member selected from the group consisting of the aforementioned compound (I-2) wherein $R^1$ to $R^3$, $R^5$, Y and the substituent on the "(unsubstituted or optionally substituted alkylene)", all have the same meanings as defined in the above (35), and a pharmaceutically acceptable salt or solvate thereof;

(47) a prophylactic and/or therapeutic drug for immediate, late and/or very late allergic responses which comprises an effective amount of at least one member selected from the group consisting of the aforementioned compound (I-2) wherein $R^1$ to $R^3$, $R^5$, Y and the substituent on the "(unsubstituted or optionally substituted alkylene)", all have the same meanings as defined in the above (35), and a pharmaceutically acceptable salt or solvate thereof, said drug having the property of alleviating allergic symptoms wherein the allergic symptom is an immediate, late and/or very late response; and

(48) a prophylactic and/or therapeutic drug for allergic gastroenteritis (allergic inflammation in digestive tract) which comprises an effective amount of at least one member selected from the group consisting of the aforementioned compound (I-2) wherein $R^1$ to $R^3$, $R^5$, Y and the substituent on the "(unsubstituted or optionally substituted alkylene)", all have the same meanings as defined in the above (35), and a pharmaceutically acceptable salt or solvate thereof.

In yet another aspect, the present invention provides:

(49) the drug according to any of the above (1) to (48) for alleviating acute inflammatory symptoms;

(50) the drug according to any of the above (1) to (48) for alleviating subacute inflammatory symptoms;

(51) the drug according to any of the above (1) to (48) for alleviating chronic inflammatory symptoms;

(52) the drug according to any of the above (1) to (51) for ameliorating an inflammatory symptom selected from the group consisting of

(i) those caused by at least one member selected from the group consisting of lymphocyte, mast cell, eosinophil, basophil, neutrophil, macrophage, monocyte, histiocyte, Langerhans cell, etc.;

(ii) those characterized by migration, infiltration, and/or accumulation of at least one member selected from the group consisting of lymphocyte, mast cell, eosinophil, basophil, neutrophil, macrophage, monocyte, histiocyte, and Langerhans cell into diseased sites; and

(iii) those characterized by an increase in number or amount, or proliferation (growth & production)/destruction of at least one cellular or non-cellular member selected from the group consisting of lymphocyte, mast cell, eosinophil, basophil, neutrophil, macrophage, monocyte, histiocyte, Langerhans cell, and connective tissue components at diseased sites;

(53) the drug according to any of the above (1) to (51) for ameliorating an inflammatory symptom selected from the group consisting of

(i) those caused by at least one member selected from the group consisting of eosinophil, neutrophil and macrophage; and

(ii) those caused by at least one member selected from the group consisting of eosinophil and neutrophil at airway, nasal cavity, skin, conjunctiva and intestine;

(54) the drug according to any of the above (1) to (53) for ameliorating inflammatory edema;

(55) the drug according to any of the above (7), (8), (27), (28), (41) and (42) for ameliorating cyanosis by airway obstruction or narrowing of airways;

(56) the drug according to any of the above (7), (8), (27), (28), (41) and (42) for ameliorating cyanosis by muciparous promotion of airways or airway contraction;

(57) the drug according to any of the above (1) to (51) for applications comprising the therapy directed to extinction

of inflammatory symptoms or alleviation thereof or the preventive therapy for the onset of the said inflammatory symptoms;

(58) a pharmaceutical drug not only comprising an effective amount of at least one member selected from the group consisting of the aforementioned compound (I) wherein $R^1$ to $R^8$, all have the same meanings as defined in the above (1), and a pharmaceutically acceptable salt or solvate thereof, but also having at least one biological activity selected from the group consisting of

(i) inhibition of migration, infiltration, and/or accumulation of eosinophils and/or neutrophils into diseased sites;
(ii) extinction, alleviation or prophylaxis of bronchial asthma symptoms and/or atopic dermatitis symptoms;
(iii) extinction, alleviation or prophylaxis of cyanosis; and
(iv) remedy of deteriorated airway hyperresponsiveness due to bronchial asthma symptoms;

(59) a pharmaceutical drug not only comprising an effective amount of at least one member selected from the group consisting of the aforementioned compound (I-1) wherein $R^1$ to $R^3$, $R^5$, Y and $R^a$, all have the same meanings as defined in the above (21), and a pharmaceutically acceptable salt or solvate thereof, but also having at least one biological activity selected from the group consisting of

(i) inhibition of migration, infiltration, and/or accumulation of eosinophils and/or neutrophils into diseased sites;
(ii) extinction, alleviation or prophylaxis of bronchial asthma symptoms and/or atopic dermatitis symptoms;
(iii) extinction, alleviation or prophylaxis of cyanosis; and
(iv) remedy of deteriorated airway hyperresponsiveness due to bronchial asthma symptoms;

(60) a pharmaceutical drug not only comprising an effective amount of at least one member selected from the group consisting of the aforementioned compound (I-2) wherein $R^1$ to $R^3$, $R^5$, Y and the substituent on the "(unsubstituted or optionally substituted alkylene)", all have the same meanings as defined in the above (35), and a pharmaceutically acceptable salt or solvate thereof, but also having at least one biological activity selected from the group consisting of

(i) inhibition of migration, infiltration, and/or accumulation of eosinophils and/or neutrophils into diseased sites;
(ii) extinction, alleviation or prophylaxis of bronchial asthma symptoms and/or atopic dermatitis symptoms;
(iii) extinction, alleviation or prophylaxis of cyanosis; and
(iv) remedy of deteriorated airway hyperresponsiveness due to bronchial asthma symptoms;

(61) the drug according to any of the above (1), (21) and (35) for ameliorating an allergic symptom selected from the group consisting of

(i) those caused by at least one member selected from the group consisting of lymphocyte, mast cell, eosinophil, basophil, neutrophil, macrophage, monocyte, histiocyte, Langerhans cell, etc.;
(ii) those characterized by migration, infiltration, and/or accumulation of at least one member selected from the group consisting of lymphocyte, mast cell, eosinophil, basophil, neutrophil, macrophage, monocyte, histiocyte, and Langerhans cell into diseased sites; and
(iii) those characterized by an increase in number or amount, or proliferation (growth & production)/destruction of at least one cellular or non-cellular member selected from the group consisting of lymphocyte, mast cell, eosinophil, basophil, neutrophil, macrophage, monocyte, histiocyte, Langerhans cell, and connective tissue components at diseased sites;

(62) the drug according to any of the above (1), (21) and (35) for ameliorating an allergic symptom selected from the group consisting of

(i) those caused by eosinophil;
(ii) those caused by macrophage;
(iii) those caused by neutrophil;
(iv) those caused by lymphocyte;
(v) those caused by eosinophil and macrophage; and
(vi) those caused by eosinophil at airway, nasal cavity, skin, conjunctiva or intestine;

(63) the drug according to any of the above (1), (21) and (35) for ameliorating a chronic allergic symptom;
(64) the drug according to any of the above (1), (21) and (35) for ameliorating a very late allergic symptom (very

late phase allergic response);

(65) the drug according to any of the above (1), (21) and (35) for applications comprising the therapy directed to extinction of allergic symptoms or alleviation thereof in the treatment of allergy, or the preventive therapy for the onset of said allergic symptoms;

(66) a prophylactic and/or therapeutic drug for inflammation which comprises an effective amount of at least one member selected from the group consisting of the aforementioned compound (I) wherein $R^1$ to $R^8$, all have the same meanings as defined in the above (1), and a pharmaceutically acceptable salt or solvate thereof;

(67) a prophylactic and/or therapeutic drug for inflammation which comprises an effective amount of at least one member selected from the group consisting of the aforementioned compound (I-1) wherein $R^1$ to $R^3$, $R^5$, Y and $R^a$, all have the same meanings as defined in the above (21), and a pharmaceutically acceptable salt or solvate thereof;

(68) a prophylactic and/or therapeutic drug for inflammation which comprises an effective amount of at least one member selected from the group consisting of the aforementioned compound (I-2) wherein $R^1$ to $R^3$, $R^5$, Y and the substituent on the "(unsubstituted or optionally substituted alkylene)", all have the same meanings as defined in the above (35), and a pharmaceutically acceptable salt or solvate thereof;

(69) the drug according to any of the above (66) to (68) which acts on the prophylaxis and/or therapy of inflammation via allergic reactions;

(70) the drug according to any of the above (66) to (69) for alleviating an acute inflammatory symptom;

(71) the drug according to any of the above (66) to (69) for alleviating a subacute inflammatory symptom;

(72) the drug according to any of the above (66) to (69) for alleviating a chronic inflammatory symptom;

(73) the drug according to any of the above (66) to (72) for ameliorating an inflammatory symptom selected from the group consisting of

(i) those caused by at least one member selected from the group consisting of lymphocyte, mast cell, eosinophil, basophil, neutrophil, macrophage, monocyte, histiocyte, Langerhans cell, etc.;

(ii) those characterized by migration, infiltration, and/or accumulation of at least one member selected from the group consisting of lymphocyte, mast cell, eosinophil, basophil, neutrophil, macrophage, monocyte, histiocyte, and Langerhans cell into diseased sites; and

(iii) those characterized by an increase in number or amount, or proliferation (growth & production)/destruction of at least one cellular or non-cellular member selected from the group consisting of lymphocyte, mast cell, eosinophil, basophil, neutrophil, macrophage, monocyte, histiocyte, Langerhans cell, and connective tissue components at diseased sites;

(74) the drug according to any of the above (66) to (72) for ameliorating an inflammatory symptom selected from the group consisting of

(i) those caused by at least one member selected from the group consisting of eosinophil, neutrophil and macrophage; and

(ii) those caused by at least one member selected from the group consisting of eosinophil and neutrophil at airway, nasal cavity, skin, conjunctiva and intestine;

(75) the drug according to any of the above (66) to (69) for ameliorating inflammatory edema; and

(76) the drug according to any of the above (66) to (69) for applications comprising the therapy directed to extinction of inflammatory symptoms or alleviation thereof or the preventive therapy for the onset of said inflammatory symptoms.

In still another aspect, the present invention provides:

(77) the drug according to any of the above (1) to (76), wherein the drug is in a form of a pharmaceutical composition or formulation suitable for oral, topical, and/or parenteral application;

(78) the drug according to any of the above (1) to (77), suitable for administration or application by a route selected from the group consisting of oral, intracellular, intravenous, intramuscular, subcutaneous, intracutaneous, intraperitoneal, intrapleural and intraspinal routes, an intra-tissue route, a route by instillation, an enteral route, a per rectum route, routes by instillation into the ear, eye, or nose, and pasting or application on the skin or mucosa;

(79) the drug according to any of the above (1) to (78), which is a formulation selected from the group consisting of a pharmaceutical solution, pharmaceutical dispersion, semi-solid preparation, particulate preparation, shaped preparation, and extractive; and

(80) the drug according to any of the above (1) to (79), wherein its dosing form is selected from the group consisting of tablet, coated tablet, sugar coated tablet, pill, troche, hard capsule, soft capsule, microcapsule, implant, powder, pulvis, granule, fine granule, injection, liquid and solution, elixir, emulsion, irrigation, syrup, medicated water,

magma, milk, suspension, liniment, lotion, aerosol, spray, inhalation, nebula, ointment, plaster, patch, paste, cataplasm, creams, medicated oil, suppository (e.g., rectal suppository), tincture, dermatologic water, ophthalmic solution, collunarium, auristillae, paint, transfusion, powders for injection solution, lyophilized preparation, and conditioned gel.

The above objectives and other objectives, features, advantages, and aspects of the present invention are readily apparent to those skilled in the art from the following disclosures. It should be understood, however, that the description of the specification including the following best modes of carrying out the invention, examples, etc. is illustrating preferred embodiments of the present invention and given only for explanation thereof. It will become apparent to the skilled in the art that a great number of variations and/or alterations (or modifications) of this invention may be made based on knowledge from the disclosure in the following parts and other parts of the specification without departing from the spirit and scope thereof as disclosed herein. All of the patent publications and reference documents cited herein for illustrative purposes are hereby incorporated by reference into the present disclosure.

The term "and/or" used herein means the presence of both (i) a jointly connecting relation and (ii) a selectively connecting relation. For example, in the case of "prophylactically and/or therapeutically", it is used in such a sense that said expression covers both (i) "prophylactically and therapeutically" and (ii) "prophylactically or therapeutically". In other cases, the term "and/or" is used in the same sense that it covers both (i) a jointly connecting relation and (ii) a selectively connecting relation as well.

Brief Description of the Drawings

FIG. 1 is a graph showing the kinetics of the development of auricle edema in mouse experimental atopic dermatitis models.

FIG. 2 is a graph chronologically showing the cell types and the numbers of leukocytes infiltrated into inflamed sites of mouse experimental atopic dermatitis models.

FIG. 3 is a graph chronologically showing each auricle edema in both genetically mast cell (basophil)-deficient mutant mice and mice (each having an identical genetic background) having mast cells (basophils).

FIG. 4 is a graph chronologically showing each auricle edema in both genetically thymus-deficient mutant mice and mice (each having an identical genetic background) having thymus.

FIG. 5 is a graph chronologically showing kinetic changes in the development of auricle edema in response to the second elicitation after initial elicitation.

FIG. 6 is a graph chronologically showing inhibitory efficacies on the auricle edema development in a mouse experimental atopic dermatitis model.

FIG. 7 is a graph chronologically showing inhibitory efficacies on auricle edema in mouse experimental atopic dermatitis models.

FIG. 8 is a graph chronologically showing inhibitory efficacies on auricle edema in mouse experimental atopic dermatitis models.

FIG. 9 is a graph chronologically showing inhibitory efficacies on scratching behavior in mouse experimental atopic dermatitis models.

FIG. 10 is a graph chronologically showing changes in body weight in mouse experimental atopic dermatitis models.

FIG. 11 is a graph chronologically showing efficacies on allergic dermatitis of spontaneously diseased NC/Jic mice.

FIG. 12 is a graph showing efficacies on guinea pig experimental bronchial asthma models.

FIG. 13 is a graph showing efficacies on mouse contact-type (type IV) dermatitis models.

FIG. 14 is a graph showing inhibitory efficacies on infiltration of leukocytes in guinea pig allergic inflammation models (air pouch models).

FIG. 15 is a graph showing amelioratory efficacies on resistance within the nasal airway of guinea pig allergic rhinitis models.

Best Modes of Carrying out the Invention

The present invention is characterized in that a hydroxamic acid derivative having a metalloproteinase-inhibiting activity is used as an effective ingredient for pharmaceutical compositions. The present invention relates to pharmaceutical compositions used in the therapy and/or prophylaxis of allergy, particularly applied to patients suffering from allergic diseases including type I and/or type IV allergic diseases, or pharmaceutical compositions to the specific site of persons susceptible to allergy including type I and/or IV allergic responses. The present invention also relates to a

method for the therapy and/or prophylaxis of allergy which comprises applying the same. In specific embodiments, the pharmaceutical composition relates to an anti-allergic agent containing, as a main effective element, at least one member selected from the group consisting of the above-mentioned compounds of the formula (I) and pharmaceutically acceptable salts and solvates thereof. In another embodiment, it relates to pharmaceutical compositions capable of ameliorating all of immediate, late and very late allergic responses and symptoms which comprise each at least one member selected from the hydroxamic acid derivatives according to the present invention. Further, it relates to a method for the therapy and/or prophylaxis using the said composition.

The present invention relates to therapeutic and/or prophylactic agents for bronchial asthma which comprise each at least one member selected from the group consisting of the hydroxamic acid derivatives according to the present invention, particularly the above-mentioned compounds having the formula (I) and pharmaceutically acceptable salts and solvates thereof. Further, it also relates to a method for the therapy and/or prophylaxis of bronchial asthma which comprises administering the same to a subject. In another aspect, it relates to therapeutic and/or prophylactic agents for bronchial asthma which comprise each at least one member selected from the hydroxamic acid derivatives according to the present invention and also to a method for the therapy and/or prophylaxis of bronchial asthma which comprises administering the same to a subject.

Further, the present invention relates to therapeutic and/or prophylactic agents for bronchial asthma which comprise each an effective amount of at least one member selected from the group consisting of the hydroxamic acid derivatives of the present invention, particularly the aforementioned compounds of the formula (I) and pharmaceutically acceptable salts and solvates thereof and also to a method for the therapy and/or prophylaxis of bronchial asthma which comprises administering an effective amount of the same to a subject. Furthermore, the present invention relates to therapeutic and/or prophylactic agents for atopic diseases (including atopic dermatitis, atopic enteritis, etc.) which comprise each an effective amount of at least one member selected from the hydroxamic acid derivatives of the present invention and also to a method for the therapy and/or prophylaxis of atopic diseases which comprises administering an efective amount of the same to a subject. Still further, the present invention relates to therapeutic and/or prophylactic agents for allergic conjunctivitis which comprise each an effective amount of at least one member selected from the hydroxamic acid derivatives of the present invention and also to a method for the therapy and/or prophylaxis of allergic conjunctivitis which comprises administering an effective amount of the same to a subject. Still furthermore, the present invention relates to therapeutic and/or prophylactic agents for allergic digestive tract inflammation which comprise each an effective amount of at least one member selected from the hydroxamic acid derivatives of the present invention and also to a method for the therapy and/or prophylaxis of allergic digestive tract inflammation which comprises administering an effective amount of the same to a subject. In addition, the present invention relates to anti-inflammatory agents which comprise each an effective amount of at least one member selected from the group consisting of the hydroxamic acid derivatives of the present invention, particularly the above-mentioned compounds of the formula (I), and pharmaceutically acceptable salts and solvents thereof and also to a method for the therapy and/or prophylaxis of inflammation which comprises administering an effective amount of the same to a subject.

Moreover, the present invention provides pharmaceutical drugs for therapy or prophylaxis characterized in (A) suppressing the cell number (at diseased areas) of a cell member selected from the group consisting of lymphocytes, mast cells, eosinophils, basophils, neutrophils, monocytes, macrophages and the like and/or (B) suppressing the migration, infiltration or accumulation (into diseased areas) of a cell member selected from the group consisting of lymphocytes, mast cells, eosinophils, basophils, neutrophils, monocytes, macrophages and the like and/or (C) suppressing antibody production (particularly IgE production) mediated by a cell member selected from the group consisting of lymphocytes, mast cells, eosinophils, basophils, neutrophils, monocytes, macrophages, Langerhans cells, etc., and/or maintenance/increase of the blood content thereof and/or (D) removing, alleviating or preventing cyanosis caused by hypersecretion of airway mucus or by airway spasm, and/or (E) preventing asthmatic death, etc. The present invention also provides a method for the therapy and/or prophylaxis of diseases or morbid states which comprises administering an effective amount of the same to a subject.

The hydroxamic acid derivatives as used herein may include compounds (particularly synthetic compounds) each having an inhibitory activity against at least one member of the MMP family. Examples of such compounds are compounds of the formula (I) and a pharmaceutically acceptable salt and solvate thereof. At least one member selected selected from the group consisting of the compounds (I) and pharmaceutically acceptable salts and solvates thereof may be preferably used. Such a compound has plenty of excellent characteristics as a therapeutic agent for allergy, particularly as a therapeutic agent for type I and/or IV allergies, etc. More preferably, the hydroxamic acid derivative as used herein has lots of excellent characteristics as a therapeutic agent for bronchial asthma, allergic rhinitis (including seasonal rhinitis), atopic diseases (including atopic dermatitis, atopic enteritis, etc.), allergic conjunctivitis (including seasonal conjunctivitis), allergic digestive tract inflammation, allergic peritonitis, inflammation of various organs, etc. and has plenty of excellent characteristics as a prophylactic agent for such diseases.

Especially when allergic reactions take place in vivo, complicated multi-stage processes participate therein and various host cells and biologically active factors participate therein which are each different at a respective stage.

It should be understood that it is difficult to ascertain, under such a morbid condition that allergic reactions take place, what role a metalloproteinase inhibitor actually plays, what pharmacological or physiological activity it has and for what diseases it is effective.

In addition, with regard to an inflammation as a result of an IgE-mediated allergic response, it has been ascertained to appear in an immediate manner (inflammatory responses become evident within several minutes to about 60 minutes after re-elicitation with allergens) and in a delayed (late) manner (inflammatory responses become evident within about 24 to 72 hours after elicitation with allergens). However, the present inventors have found that, following the immediate and late responses (immediate and late phase responses; IPR and LPR), a very late response (very late phase response; vLPR) becomes manifest. The present inventors have ascertained that the hydroxamic acid derivatives according to the present invention are effective to inflammatory responses and symptoms in immediate inflammatory responses (IPR), late inflammatory responses (LPR) and/or very late inflammatory responses (vLPR).

Thus, with regard to allergic reactions in experimental atopic dermatitis models, it has been noted that, following the immediate and late responses, edemas as a result of the inflammatory responses, etc. are once relieved and then inflammatory responses are manifested again which are termed "very late phase response". Thus, in the experimental atopic dermatitis models, the immediate phase response becomes evident as auricle edemas which are an inflammatory symptom at the first hour after elicitation, and then the late phase response becomes evident as auricle edemas on day 1 after elicitation. Following that, edemas are once relieved and then auricle edemas start manifesting again from day 5 after re-elicitation as the very late phase response (vLPR) whereupon inflammatory symptoms worsen. In this experimental system, it has been ascertained that the peak of the very late phase response appears on Days 7 to 10 and the inflammatory symptoms are gradually ameliorated. With regard to the behavior of inflammatory cells, etc. in each response phase, it has been ascertained that eosinophils, neutrophils, macrophages, etc. increase noticeably in the late phase response. Further, it has been ascertained that an increase in eosinophil is very significant in the very late phase response and mast cells, lymphocytes and, especially, T cells also participate therein. It may be presumed that the alleviation of the allergic symptoms noted in this very late phase response is associated with an inhibition (suppression) of infiltrating or migrating eosinophils, etc. into inflammatory tissues and/or a decrease in blood IgE antibody titer. Under the circumstance where an effective agent for relieving the allergic symptoms has been demanded, it is believed that the hydroxamic acid derivatives according to the present invention are useful as preventive and/or therapeutic agents for not only suppressing the immediate and late inflammation but also especially relieving the very late (or post-late) inflammatory symptoms. In addition, the hydroxamic acid derivatives are believed to be useful in suppressing inflammation in allergic diseases which have become chronic and refractory. Further, the hydroxamic acid derivatives are believed to be useful as prophylactic agents and/or therapeutic agents for suppressing inflammation in allergic reactions at acute and subacute stages.

Furthermore, from the above viewpoint, when it is considered that the late phase response and the very late phase response show states similar to the onset mechanism of type IV allergy, it is believed that the hydroxamic acid derivatives according to the present invention are also useful as therapeutic agents for allergic responses in not only type I but also type IV allergies as well as analogues thereof.

Still further, as compared to prednisolone which is a known therapeutic agent for allergy, the hydroxamic acid derivatives of the present invention have a safer and more effective pharmacological effect in such a respect that they do not induce a decrease in body weight, a thinning of the skin and/or a rebound phenomenon after interruption and/or withdrawal of the steroid administration, etc.

In a more preferred embodiment, the present invention relates to a pharmacological agent selected from the group consisting of anti-allergic agents, anti-inflammatory agents, agents against bronchial asthma and agents against allergic rhinitis (including seasonal rhinitis), atopic diseases (including atopic dermatitis, atopic enteritis, etc.), allergic conjunctivitis (including seasonal conjunctivitis), allergic digestive tract inflammation, allergic peritonitis and/or inflammation of various organs which comprises an effective amount of at least one member selected from the group consisting of the above-mentioned compounds (I) and pharmaceutically acceptable salts and solvates thereof and also relates to a method for the therapy and/or prophylaxis of diseases or abnormal states which comprises administering the said pharmacological agent to a subject.

As used herein for $R^1$, $R^3$, $R^4$ and $R^7$ to $R^{11}$ in the compounds given above, specifically the compounds (I), the term "unsubstituted or optionally substituted alkyl" refers to a straight chain or branched alkyl moiety, preferably having from 1 to 20 carbon atoms, which can optionally be unsubstituted or substituted with one or more substituents which can be selected from the group given hereinbelow, including, for example, methyl, ethyl, propyl, isopropyl, butyl, isobutyl, sec-butyl, tert-butyl, n-pentyl, isopentyl, neopentyl, hexyl, heptyl, octyl, nonyl, decyl, undecyl, dodecyl, tridecyl, tetradecyl, pentadecyl, hexadecyl, adamantyl, etc.

As used herein for $R^1$, $R^3$, $R^4$ and $R^7$ to $R^9$ in the compounds given above, specifically the compounds (I), the term "unsubstituted or optionally substituted aralkyl" refers to a group having (a) an aryl moiety, preferably having from 6 to 10 carbon atoms, and more preferably 6 carbon atoms, and (b) a straight chain or branched alkylene moiety, preferably having from 1 to 8 carbon atoms, and more preferably from 1 to 4 carbon atoms, said aryl moiety and/or said

alkylene moiety which can optionally be unsubstituted or substituted with one or more substituents which can be selected from the group given hereinbelow. Representatives of said "aralkyl" include, for example, unsubstituted or optionally substituted phenyl-substituted lower ($C_1$-$C_4$) alkyl (also termed "phenyl-lower ($C_1$-$C_4$) alkylene"). Examples of said aralkyl are unsubstituted or optionally substituted benzyl such as trityl, diphenylmethyl, phenyl, 4-methoxyphenyl, allyl, cinnamyl, benzyl, 2- or 4-nitrobenzyl, 4-bromobenzyl and 4-methoxybenzyl, unsubstituted or optionally substituted phenethyl, unsubstituted or optionally substituted phenylpropyl (also termed "phenyl-trimethylene"), unsubstituted or optionally substituted naphthylpropyl (also termed "naphthyl-trimethylene") and the like.

As used herein for $R^1$ and $R^2$ in connection with the compounds (I), the term "unsubstituted or optionally substituted alkyloxycarbonyl" refers to a group having the "unsubstituted or optionally substituted alkyl moiety" as set forth for said "unsubstituted or optionally substituted alkyl" in connection with $R^1$, $R^3$, $R^4$ or any of $R^7$ to $R^{11}$. Said unsubstituted or optionally substituted alkyloxycarbonyl includes for example ($C_1$-$C_6$) alkyloxycarbonyl such as tert-butyloxycarbonyl.

As used herein for $R^1$ and $R^2$ in connection with the compounds (I), the term "unsubstituted or optionally substituted aralkyloxycarbonyl" refers to a group having the "unsubstituted or optionally substituted aralkyl moiety" as set forth for said "unsubstituted or optionally substituted aralkyl". Said "unsubstituted or optionally substituted aralkyloxycarbonyl" includes for example, unsubstituted or optionally substituted benzyloxycarbonyl, such as benzyloxycarbonyl, 2- or 4-nitro-benzyloxycarbonyl, and 4-methoxybenzyloxycarbonyl, unsubstituted or optionally substituted phenethyloxycarbonyl, and the like.

As used herein for $R^1$ in the above compounds (I), the term "silyl which may optionally have three substituents" refers to those silyl groups optionally having, as the substituent(s), unsubstituted or optionally substituted alkyl, and/or unsubstituted or optionally substituted aryl, and/or unsubstituted or optionally substituted aralkyl, wherein the "unsubstituted or optionally substituted aralkyl" as said substituent has the meaning as given above, the "unsubstituted or optionally substituted alkyl" has similarly the same meaning as set forth for the "unsubstituted or optionally substituted alkyl" in connection with $R^1$, $R^3$, $R^4$ and $R^7$ to $R^{11}$, and the "unsubstituted or optionally substituted aryl" as said substituent also has the same meaning as set forth for the "aryl moiety" of the "unsubstituted or optionally substituted aralkyl" in connection with $R^1$, $R^3$, $R^4$ and $R^7$ to $R^9$, including, for example, phenyl, naphthyl, etc. Representatives of the "silyl which may optionally have three substituents" are trialkylsilyl such as trimethylsilyl, triethylsilyl, and tert-butyldimethylsilyl, alkyldiarylsilyl such as tert-butyldiphenylsilyl, and the like.

As used herein for $R^{10}$ and $R^{11}$ in connection with the compounds (I), the term "unsubstituted or optionally substituted cycloalkyl" refers to a radical being monocyclic or having multiple condensed rings, such as a bicyclic radical, preferably from 3 to 10, more preferably from 3 to 7, and most preferably from 3 to 6 carbon atoms, which can optionally be unsubstituted or substituted with one or more substituents (said substituent is selected from those given hereinbelow), including for example, cyclopropyl, cyclobutyl, cyclopentyl, cyclohexyl, cycloheptyl, [2.2.1]bicycloheptyl, etc.

As used herein for $R^{10}$ and $R^{11}$ in connection with the compounds (I), the term "unsubstituted or optionally substituted heterocyclic group" refers to a saturated or unsaturated radical, being monocyclic or having multiple condensed rings, such as a bicyclic radical, containing one or more hetero atoms (which are identical or different) selected from nitrogen, oxygen, sulfur, etc., which can optionally be unsubstituted or substituted with one or more substituents (said substituent is selected from those given hereinbelow). Said unsubstituted or optionally substituted heterocyclic group includes for example, radicals having a 5-, 6- or 7-membered heterocyclic ring. Representatives of the "heterocyclic ring" include imidazole, pyrazole, imidazolirie, imidazolidine, pyridine, pyrimidine, benzimidazole, quinazoline, pteridine, purine, 1,3-azepine, aziridine, azetidine, pyrrole, pyrrolidine, tetrahydropyridine, piperidine, azepine, indole, quinoline, isoquinoline, morpholine, piperazine, etc.

When $R^{10}$ and $R^{11}$ taken together with the nitrogen atom to which they are attached form an "unsubstituted or optionally substituted heterocyclic group", said term "unsubstituted or optionally substituted heterocyclic group" refers to a saturated or unsaturated nitrogen-containing radical, being monocyclic or having multiple condensed rings, such as a bicyclic radical, wherein said heterocycle includes, for example, aziridine, azetidine, pyrrole, pyrrolidine, pyridine, tetrahydropyridine, piperidine, azepine, indole, quinoline, isoquinoline, morpholine, piperazine, etc.

As used herein for the term "hydroxy-protecting group" in connection with $R^1$ in the compound (I), suitable protecting groups are those known to artisans in the organic synthesis fields, for example, selected from those which have been employed in the technical fields including peptide synthesis, penicillin synthesis, cephalosporin synthesis, sugar synthesis, and the like. Said "hydroxy-protecting groups" include those removable by treatment with water, those removable by hydrogenolysis, those removable with Lewis acid catalysts such as $AlCl_3$, those removable with zinc/acetic acid, those removable with thiourea, those removable with acids or weak bases, and the like. Representative hydroxy-protecting groups include benzyl, 2,2,2-trichloroethoxycarbonyl, allyloxycarbonyl, 2-methoxyethoxymethyl, formyl, acetyl, chloroacetyl, dichloroacetyl, trityl, and the like. Such groups are any as long as they are capable of forming, or convertible into, a hydroxy group, chemically or under biological conditions, i.e., physiological conditions (for example, bioreactions such as oxidation, reduction, and hydrolysis, catalyzed by in vivo enzymes and the like). Said

"hydroxy-protecting groups" may also be selected from the group consisting of the above defined "silyl which may optionally have three substituents", "unsubstituted or optionally substituted aralkyl", tetrahydropyranyl, "unsubstituted or optionally substituted alkyloxycarbonyl", "unsubstituted or optionally substituted aralkyloxycarbonyl", and the like.

As used herein for $R^2$, $R^{10}$ and $R^{11}$ in the compound (I), the term "amino-protecting group" refers to protecting groups known to artisans in the organic synthesis fields, for example, selected from those which have been employed in the technical fields including peptide synthesis, penicillin synthesis, cephalosporin synthesis, sugar synthesis, and the like. Said "amino-protecting groups" include those removable by hydrogenolysis, those removable with fluorinated compounds, those removable with acids, and the like. Illustrative examples of such "amino-protecting groups" include benzyloxycarbonyl, p-nitro-benzyloxycarbonyl, trityl, tert-butoxycarbonyl; ($C_1$-$C_6$) aliphatic acyl which can optionally be unsubstituted or substituted with halogen, such as formyl, chloroacetyl, and dichloroacetyl; alkoxycarbonyl such as allyloxycarbonyl, 2-trimethylsilylethoxycarbonyl, and 2,2,2-trichloroethoxycarbonyl; 2-methoxyethoxymethyl, and the like. Such groups are any as long as they are capable of forming, or convertible into, a free or protonated amino group, chemically or under biological conditions, i.e., physiological conditions (for example, bioreactions such as oxidation, reduction, and hydrolysis, catalyzed by in vivo enzymes and the like). Said "amino-protecting groups" may also be selected from the group consisting of the above defined "unsubstituted or optionally substituted alkyloxycarbonyl", "unsubstituted or optionally substituted aralkyloxycarbonyl", 9-fluorenylmethyloxycarbonyl, and the like.

In connection with the above defined "unsubstituted or optionally substituted alkyl", "unsubstituted or optionally substituted aralkyl", "unsubstituted or optionally substituted aryl", "unsubstituted or optionally substituted cycloalkyl", and "unsubstituted or optionally substituted heterocyclic group", suitable substituents include the above defined alkyl, the above defined aryl, hydroxy, unsubstituted or optionally substituted amino (for example, amino, N-lower ($C_1$-$C_4$) alkylamino such as methylamino, ethylamino and isopropylamino, N-arylamino such as phenylamino, aralkylamino such as pyridylmethylamino and benzylamino, alicyclic amino such as morpholino, piperidino, piperazino and N-phenyl-piperazino, guanidino, and the like), amidino, acylimidoyl (for example, derivatives from ($C_2$-$C_5$) lower alkanoic acids, such as acetimidoyl and propionimidoyl, derivatives from ($C_7$-$C_{11}$) aromatic carboxylic acids, such as benzimidoyl, and the like), halogen (for example, F, Cl, Br, etc.), nitro, ($C_1$-$C_4$) lower alkoxy (for example, methoxy, ethoxy, etc.), ($C_1$-$C_4$) lower alkylthio (for example, methylthio, ethylthio, etc.), carboxyl, ($C_2$-$C_6$) lower alkanoyloxy, ($C_1$-$C_6$) lower alkoxycarbonyloxy, phosphono which can optionally be unprotected or protected at its hydroxy wherein said protecting group includes the above defined "hydroxy-protecting group", "unsubstituted or optionally substituted alkyl", "unsubstituted or optionally substituted aralkyl", "unsubstituted or optionally substituted aryl", "unsubstituted or optionally substituted alkyloxycarbonyl", "unsubstituted or optionally substituted aralkyloxycarbonyl", and the like.

As used herein for X, A and B in the above compound (I), in connection with the "unsubstituted or optionally substituted ($C_1$-$C_6$) alkylene", the "unsubstituted or optionally substituted phenylene", the "unsubstituted or optionally substituted ($C_1$-$C_6$) alkyleneimino", the "unsubstituted or optionally substituted imidazolyl", and the "unsubstituted or optionally substituted hydantoin-1-yl", suitable substituents may be selected from those listed for the above defined substituent in the "unsubstituted or optionally substituted alkyl", etc. As used herein for B in the above compound (I), in connection with the "unprotected or optionally protected bis(phosphono)methyl", the "unprotected or optionally protected glucopyranosyl", and the "unprotected or optionally protected bis(phosphono)-hydroxymethyl", suitable protecting groups may include the above defined "hydroxy-protecting group", "unsubstituted or optionally substituted alkyl", "unsubstituted or optionally substituted aralkyl", "unsubstituted or optionally substituted aryl", "unsubstituted or optionally substituted alkyloxycarbonyl", and "unsubstituted or optionally substituted aralkyloxycarbonyl".

As used herein for B in the above compound (I), the "acylimidoyl" includes a radical derived from a ($C_2$-$C_5$) lower alkanoic acid, such as acetimidoyl and propionimidoyl; a radical derived from a ($C_7$-$C_{11}$) aromatic carboxylic acid, such as benzimidoyl; and the like, as described hereinabove.

As used herein for $R^9$ in the above compound (I), the "carboxy-protecting group" may be selected from protecting groups known to artisans in the organic synthesis fields, for example, those which have been employed in the technical fields including peptide synthesis, penicillin synthesis, cephalosporin synthesis, sugar synthesis, and the like. Said "carboxy-protecting groups" have the same meanings as defined for the above "hydroxy-protecting group", including those removable by hydrogenolysis, those removable by treatment with acids or weak bases, and the like. Illustrative examples of such "carboxy-protecting groups" include the above defined "unsubstituted or optionally substituted alkyl", "unsubstituted or optionally substituted aralkyl", "unsubstituted or optionally substituted aryl", "unsubstituted or optionally substituted alkyloxycarbonyl", "unsubstituted or optionally substituted aralkyloxycarbonyl", and the like.

A preferred series of the aforementioned compounds (I) are compounds of the general formula (I) in which $R^1$ and $R^2$ is hydrogen; $R^3$ is unsubstituted or optionally substituted aralkyl; $R^4$ is unsubstituted or optionally substituted alkyl; $R^5$ is -$OR^9$ or

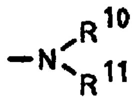

$R^9$ is hydrogen, unsubstituted or optionally substituted alkyl or unsubstituted or optionally substituted aralkyl; $R^{10}$ is unsubstituted or optionally substituted alkyl; $R^{11}$ is hydrogen; $R^6$ is unsubstituted or optionally substituted aralkyl, unsubstituted or optionally substituted aryl or unsubstituted or optionally substituted alkyl; and $R^7$ and $R^8$ is hydrogen; or a salt thereof.

The compounds (I) of the present invention may exist as alternative tautomers. There are several chiral centers in the compounds (I) of the present invention because of the presence of asymmetric carbon atoms. The presence of several asymmetric carbon atoms gives rise to a number of optical isomers with regard to each chiral center. All mixtures of such isomers and each individual optical isomer are intended to be within the scope of the present invention. The compounds of the present invention can also exist as separate enantiomers, as racemates, or as diastereomers. The compounds of the present invention can also be in the form of solvates or acid-addition salts. Further, the compounds of the present invention may be in the form of prodrugs, including those prodrugs of (i) compounds containing an acid residue or acidic group, such as a carboxyl radical and/or a hydroxy radical and/or a basic group, such as an optionally substituted or unsubstituted amino radical or (ii) derivatives thereof. The prodrugs of the compounds according to the present invention include those compounds which can be transformed in vivo, for example by metabolic processes, including hydrolysis, oxidation, reduction, trans-esterification and the like, to yield the parent compounds of the formula (I), etc. Representatives of such prodrugs are ester-, ether-, amide-, alcohol-, and amine-derivatives thereof.

The aforementioned compounds of formula (I) related to the present invention include compounds disclosed in the specifications and drawings attached to WO99/31052, WO96/33968 and JP patent application No. 11-136309 (filing date: May 17, 1999) and those compounds which can be manufactured or derived by methods or processes disclosed therein.

The compounds of general formula (I) can be prepared, for example, according to the following Scheme:

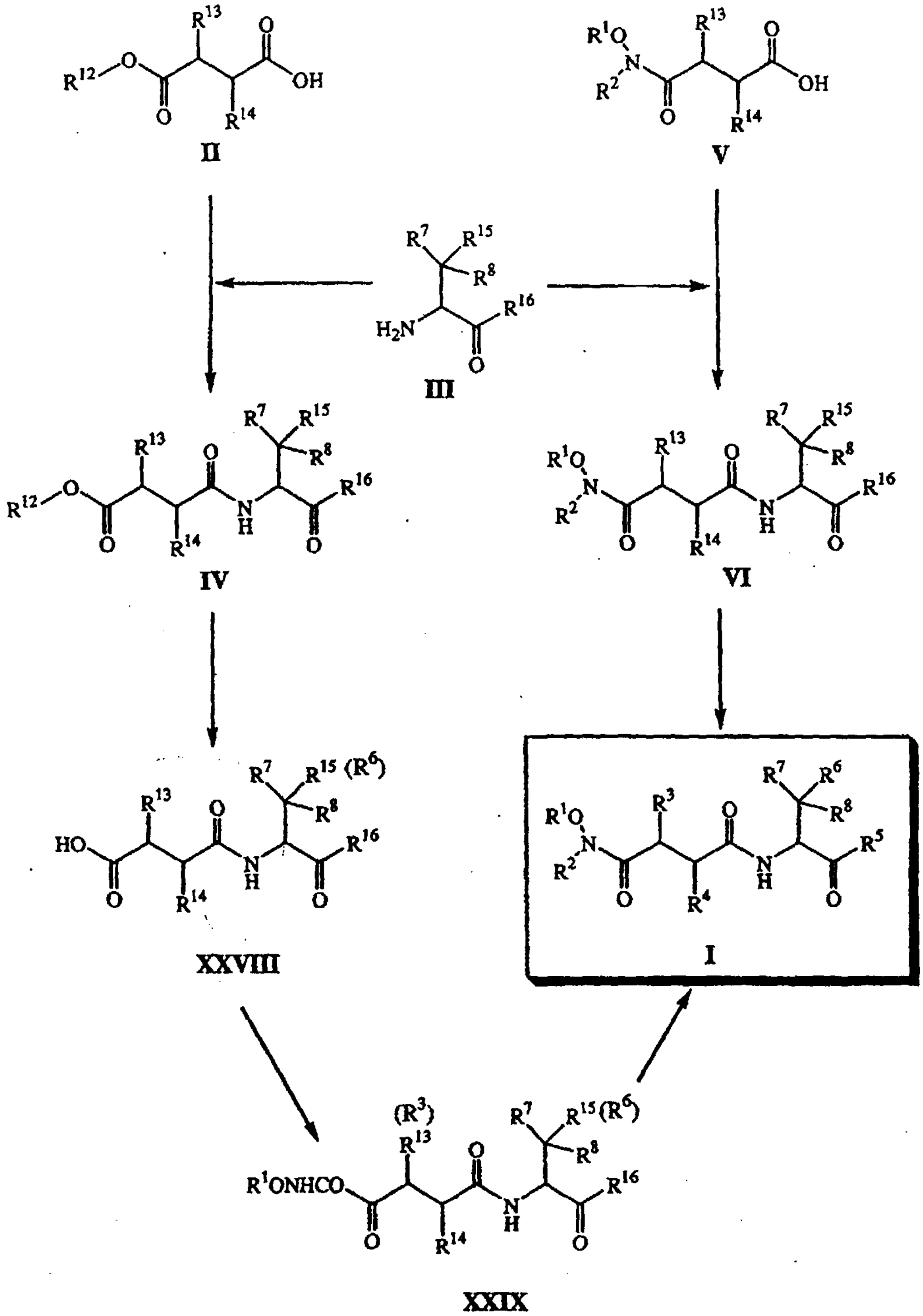

In the above Scheme, $R^1$ to $R^{11}$, all have the meanings as given above;

$R^{12}$ is selected from the group consisting of unsubstituted or optionally substituted alkyl, unsubstituted or optionally substituted aralkyl, and a carboxy-protecting group;

$R^{13}$ has the same meaning as defined for $R^3$, or is selected from the group consisting of protected hydroxy, protected guanido-substituted phenyl-lower ($C_1$-$C_4$) alkyl, protected amino-substituted phenyl-lower ($C_1$-$C_4$) alkyl,

nitro-substituted phenyl-lower ($C_1$-$C_4$) alkyl, protected amino-substituted ($C_1$-$C_6$) alkyl, nitro-substituted ($C_1$-$C_6$) alkyl, protected carboxy-substituted phenyl-lower ($C_1$-$C_4$) alkyl, protected hydroxy-substituted phenyl-lower ($C_1$-$C_4$) alkyl, protected guanido-substituted lower ($C_1$-$C_4$) alkyl-substituted phenyl-lower ($C_1$-$C_4$) alkyl, protected amino-substituted lower ($C_1$-$C_4$) alkyl-substituted phenyl-lower ($C_1$-$C_4$) alkyl, protected hydroxy-substituted lower ($C_1$-$C_4$) alkyl-substituted phenyl-lower ($C_1$-$C_4$) alkyl, protected carboxy-substituted lower ($C_1$-$C_4$) alkyl-substituted phenyl-lower ($C_1$-$C_4$) alkyl, protected hydroxy-containing ($C_1$-$C_8$) straight chain or branched alkyl, and cyano-substituted phenyl-lower ($C_1$-$C_4$) alkyl;

$R^{14}$ has the same meaning as defined for $R^4$, or is protected hydroxy-substituted ($C_1$-$C_8$) alkyl;

$R^{16}$ is $-OR^{17}$ or

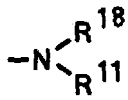

$R^{17}$ has the same meaning as defined for $R^9$, or is selected from the group consisting of protected hydroxy-substituted ($C_1$-$C_{10}$) alkyl, protected amino-substituted ($C_1$-$C_{16}$) alkyl, and protected mono-($C_1$-$C_6$) alkylamino-substituted ($C_1$-$C_{16}$) alkyl;

$R^{11}$ has the same meaning as as given above;

$R^{18}$ has the same meaning as defined for $R^{10}$, or is selected from the group consisting of protected carboxy-substituted lower ($C_1$-$C_4$) alkyl, protected hydroxy-substituted lower ($C_1$-$C_4$) alkyl, protected bis(phosphono)hydroxymethyl-substituted ($C_1$-$C_{11}$) alkyl, and a protected nitrogen-containing heterocyclic group; and

$R^{15}$ has the same meaning as defined for $R^6$, or is selected from the group consisting of protected amino, protected hydroxy, and a group of the formula: -X-E or -X-A-E

wherein X and A, both have the meanings as given above, and E is selected from the group consisting of nitro, cyano, amino, carboxyl, ($C_1$-$C_{11}$) hydroxyalkyl, protected amino, protected guanido, protected amidino, protected lower acylimidoyl, protected benzimidoyl, protected bis(phosphono)methyl, protected bis(phosphono)hydroxymethyl, and protected ($C_1$-$C_{11}$) alkyl-substituted imidazol-3-yl.

As used herein for the term "protected hydroxy" in connection with $R^{13}$ in the compound (IV), protecting groups are same as or similar to those for the "hydroxy-protecting group" in connection with $R^1$. As used herein for $R^{12}$ in the above compounds, particularly the compounds (IV), the "carboxy-protecting group" may be selected from protecting groups known to artisans in the organic synthesis fields, for example, those which have been employed in the technical fields including peptide synthesis, penicillin synthesis, cephalosporin synthesis, sugar synthesis, and the like. Said "carboxy-protecting groups" have the same meanings as defined for the above "hydroxy-protecting group", including those removable by hydrogenolysis, those removable by treatment with acids or weak bases, and the like. Illustrative examples of such "carboxy-protecting groups" include the above defined "unsubstituted or optionally substituted alkyl", "unsubstituted or optionally substituted aralkyl", "unsubstituted or optionally substituted aryl", "unsubstituted or optionally substituted alkyloxycarbonyl", "unsubstituted or optionally substituted aralkyloxycarbonyl", and the like.

As used herein for $R^{13}$, $R^{14}$, $R^{15}$, $R^{17}$, $R^{18}$ and E in the above compounds, particularly the compounds (IV), in connection with the "protected hydroxy", "protected guanido-substituted phenyl-lower ($C_1$-$C_4$) alkyl", "protected amino-substituted phenyl-lower ($C_1$-$C_4$) alkyl", "protected amino-substituted ($C_1$-$C_6$) alkyl", "protected carboxy-substituted phenyl-lower ($C_1$-$C_4$) alkyl", "protected hydroxy-substituted phenyl-lower ($C_1$-$C_4$) alkyl", "protected guanido-substituted lower ($C_1$-$C_4$) alkyl-substituted phenyl-lower ($C_1$-$C_4$) alkyl", "protected amino-substituted lower ($C_1$-$C_4$) alkyl-substituted phenyl-lower ($C_1$-$C_4$) alkyl", "protected hydroxy-substituted lower ($C_1$-$C_4$) alkyl-substituted phenyl-lower ($C_1$-$C_4$) alkyl", "protected carboxy-substituted lower ($C_1$-$C_4$) alkyl-substituted phenyl-lower ($C_1$-$C_4$) alkyl", "protected hydroxy-containing ($C_1$-$C_8$) straight chain or branched alkyl", "protected hydroxy-substituted ($C_1$-$C_8$) alkyl", "protected hydroxy-substituted ($C_1$-$C_{10}$) alkyl", "protected amino-substituted ($C_1$-$C_{16}$) alkyl", "protected mono-($C_1$-$C_6$) alkylamino-substituted ($C_1$-$C_{16}$) alkyl, "protected carboxy-substituted lower ($C_1$-$C_4$) alkyl", "protected hydroxy-substituted lower ($C_1$-$C_4$) alkyl", "protected bis(phosphono)hydroxymethyl-substituted ($C_1$-$C_{11}$) alkyl", "protected nitrogen-containing heterocyclic group", "protected amino", "protected guanido", "protected amidino", "protected lower acylimidoyl", "protected benzimidoyl", "protected bis(phosphono)methyl", "protected bis(phosphono)hydroxymethyl", and "protected ($C_1$-$C_{11}$) alkyl-substituted imidazol-3-yl", suitable protecting groups may include the above defined "hydroxy-protecting group", "amino-protecting group", and "carboxy-protecting group".

Compounds of the formula (IV) may be prepared by reacting the intermediate (II) with the intermediate (III)

according to conventional coupling techniques wherein $R^{12}$ has the meaning as given above, but is a carboxy-protecting group, including for example, unsubstituted or optionally substituted alkyl, unsubstituted or optionally substituted aralkyl, and the like; preferably tert-butyl, benzyl, substituted benzyl, phenacyl, or 2,2,2-trichloroethyl; and more preferably tert-butyl, or benzyl).

Coupling agents used in this reaction include N,N'-dicyclohexylcarbodiimide (DCC), 1-ethyl-3-(3-dimethylaminopropyl)-carbodiimide hydrochloride (EDC • HCl), N-ethoxycarbonyl-2-ethoxy-1,2-dihydroquinoline (EEDQ), 1-hydroxybenzotriazole (HOBT) derivatives, N-hydroxy-5-norbornene-2,3-dicarboximide (HONB) derivatives, N-hydroxysuccinimide (HOSu) derivatives, carbonate monoalkyl ester derivatives formed by treatment with isobutyloxycarbonyl chloride or ethyloxycarbonyl chloride, diphenylphosphorylazide (DPPA), and the like. A preferred coupling agent is EDC • HCl.

Suitable solvents for carrying out the reaction are not limited as long as they do not inhibit the process of reactions but are capable of dissolving starting materials. Illustrative examples of such solvents include amides (e.g., dimethylformamide (DMF), dimethylacetamide (DMAc), etc.), esters (e.g., ethyl acetate, etc.), ethers (e.g., diethyl ether, dioxane, etc.), halogenated hydrocarbons (e.g., dichloromethane, chloroform, etc.), and the like. The reaction temperature range is ordinarily from -20 to 50°C, and preferably from -15 to 30°C. The reaction time is ordinarily from 1 to 24 hours, and preferably from 2 to 15 hours.

Compounds of the formula (XXVIII) may be prepared by first de-esterification of the compound of the formula (IV) and, as required, then conversion of $R^{15}$ into the target functional group, $R^6$. For example, the de-esterification can be effected by treatment of the tert-butyl ester with a solution which is prepared by dissolving trifluoroacetic acid or hydrogen chloride in ethyl acetate or dioxane. The reaction temperature range is ordinarily from -10 to 20°C, and preferably from -5 to 5°C. The reaction time varies depending on the particular starting material, acid, reaction temperature, and the like employed, but it is ordinarily from 1 to 24 hours, and preferably from 1 to 15 hours.

Following the above process, conversion of $R^{15}$ into $R^6$ or of $R^{13}$ into $R^3$ may be carried out. For example, when $R^{15}$ is a radical containing a protected amino group and $R^6$ is an acetimidoylimino radical, the conversion can be accomplished by first removal of an amino group protection and then treatment with ethyl acetimidate. When the amino-protecting group is Boc, it is removed by treatment with TFA simultaneously with removal of the tert-butyl ester. Alternatively, when it is Z, the deprotection is accomplished by hydrogenolysis.

Suitable catalysts for the hydrogenation are those including palladium on carbon, platinum, etc., and preferably palladium on carbon. Suitable solvents for carrying out the reaction include inert organic solvents and the like as long as they do not poison the catalyst. Illustrative, examples of such solvents are alcohols (e.g., methanol, ethanol, etc.), ethers (e.g., THF, etc.), amides (e.g., DMF, DMAc, etc.), acetic acid, and water, and preferably methanol. The reaction temperature range is ordinarily from 0 to 50°C and preferably from 10 to 30°C. The reaction time varies depending on the particular starting material, solvent, reaction temperature, and the like employed, but it is ordinarily from 1 to 24 hours, and preferably from 1 to 15 hours.

Introduction of an acetimidoyl moiety into the deprotected amino radical may be accomplished by treatment with ethyl acetimidate in the presence of a member selected from customarily utilizable bases (inorganic bases such as sodium carbonate, potassium carbonate, sodium hydroxide and potassium hydroxide, and organic bases such as trialkylamine, N-methylmorpholine, pyridine and N,N-dimethylaminopyridine). Suitable solvents for carrying out the reaction are not limited as long as they do not inhibit the process of reactions but are capable of dissolving starting materials. Illustrative examples of such solvents include amides (e.g., DMF, DMAc, etc.), esters (e.g., ethyl acetate, etc.), ethers (e.g., diethyl ether, dioxane, etc.), halogenated hydrocarbons (e.g., dichloromethane, chloroform, etc.), and the like. A preferred solvent is DMF. The reaction temperature range is ordinarily from -20 to 50°C, and preferably from -15 to 30°C. The reaction time is ordinarily from 5 minutes to 24 hours, and preferably from 10 minutes to 15 hours.

Compounds of the formula (XXIX) may be prepared by reacting the carboxylic acid compound (XXVIII) with a protected hydroxy-containing hydroxyamine compound according to conventional coupling techniques wherein the hydroxy-protecting group is selected from those known to artisans in the art, but include for example, unsubstituted or substituted benzyl, trialkylsilyl, tert-butyldiphenylsilyl, tetrahydropyranyl, tert-butyl, and the like (preferably benzyl).

Coupling agents used in this reaction include DCC, EDC • HCl, EEDQ, HOBT derivatives, HONB derivatives, HOSu derivatives, carbonate monoalkyl ester derivatives formed by treatment with isobutyloxycarbonyl chloride or ethyloxycarbonyl chloride, DPPA, and the like. A preferred coupling agent is EDC • HCl. Suitable solvents for carrying out the reaction are not limited as long as they do not inhibit the process of reactions but are capable of dissolving starting materials. Illustrative examples of such solvents include amides (e.g., DMF, DMAc, etc.), esters (e.g., ethyl acetate, etc.), ethers (e.g., diethyl ether, dioxane, etc.), halogenated hydrocarbons (e.g., dichloromethane, chloroform, etc.), and the like. A preferred solvent is DMF. The reaction temperature range is ordinarily from -20 to 20°C, and preferably from -15 to 0°C. The reaction time is ordinarily from 1 to 72 hours, and preferably from 2 to 48 hours.

Compounds of the formula (VI) may be prepared by reacting the intermediate (III) with the intermediate (V) according to conventional coupling techniques wherein both $R^1$ and $R^2$ have the meanings as given above, but preferably $R^1$ is tert-butyl, benzyl, substituted benzyl, or tert-butyl-oxycarbonyl (Boc), more preferably benzyl, and $R^2$ is Boc

or benzyloxycarbonyl (Z).

Coupling agents used in this reaction include DCC, EDC • HCl, EEDQ, HOBT derivatives, HONB derivatives, HOSu derivatives, carbonate monoalkyl ester derivatives formed by treatment with isobutyloxycarbonyl chloride or ethyloxycarbonyl chloride, DPPA, and the like. A preferred coupling agent is EDC • HCl. Suitable solvents for carrying out the reaction are not limited as long as they do not inhibit the process of reactions but are capable of dissolving starting materials. Illustrative examples of such solvents include amides (e.g., DMF, DMAc, etc.), esters (e.g., ethyl acetate, etc.), ethers (e.g., diethyl ether, dioxane, etc.), halogenated hydrocarbons (e.g., dichloromethane, chloroform, etc.), and the like. The reaction temperature range is ordinarily from -20 to 20°C, and preferably from -15 to 0°C. The reaction time is ordinarily from 1 to 24 hours, and preferably from 2 to 15 hours.

The compounds of the formula (I) may be prepared by deprotection of the hydroxy- and/or amino-protecting group(s) on the compound (VI) or (XXIX), and, as required, optional conversion of $R^{13}$ into $R^3$, $R^{14}$ into $R^4$, $R^{16}$ into $R^5$, and/or $R^{15}$ into $R^6$.

When the hydroxy-protecting group is benzyl and the amino-protecting group is Z or (Cl-Z), the protecting groups are deprotected by hydrogenolysis simultaneously. For removal of benzyl by hydrogenolysis, suitable catalysts for the hydrogenation are those including palladium on carbon, platinum, etc., and preferably palladium on carbon. Suitable solvents for carrying out the reaction include inert organic solvents and the like as long as they do not poison the catalyst. Illustrative examples of such solvents are alcohols (e.g., methanol, ethanol, etc.), amides (e.g., DMF, DMAc, etc.), acetic acid, and water. A preferred solvent is methanol or acetic acid. The reaction temperature range is ordinarily from 0 to 100°C, and preferably from 10 to 50°C. The reaction time varies depending on the particular starting material, solvent, reaction temperature, and the like employed, but it is ordinarily from 1 to 24 hours, and preferably from 1 to 15 hours.

Following the above process, conversion of $R^{15}$ into $R^6$ may be carried out. For example, when $R^{15}$ is a radical containing a protected amino group and $R^6$ is an acetimidoylimino radical, the conversion can be accomplished by first removal of an amino group protection and then treatment with ethyl acetimidate.

Introduction of an acetimidoyl moiety into the deprotected amino radical may be accomplished by treatment with ethyl acetimidate in the presence of a member selected from customarily utilizable bases (inorganic bases such as sodium carbonate, potassium carbonate, sodium hydroxide and potassium hydroxide, and organic bases such as trialkylamine, N-methylmorpholine, pyridine and N,N-dimethylaminopyridine). Suitable solvents for carrying out the reaction are not limited as long as they do not inhibit the process of reactions but are capable of dissolving starting materials. Illustrative examples of such solvents include amides (e.g., DMF, DMAc, etc.), esters (e.g., ethyl acetate, etc.), ethers (e.g., diethyl ether, dioxane, etc.), halogenated hydrocarbons (e.g., dichloromethane, chloroform, etc.), and the like. A preferred solvent is DMF. The reaction temperature range is ordinarily from -20 to 50°C, and preferably from -5 to 30°C. The reaction time is ordinarily from 5 minutes to 24 hours, and preferably from 10 minutes to 15 hours.

The reaction products so prepared may be subjected to ordinarily separation and purification processes after completion of the reactions. The products can be readily isolated by methods including for example extraction with water or an organic solvent, concentration, neutralization, distillation, column chromatography and recrystallization.

The compounds (I) of the present invention may be in the form of solvates or salts (including acid addition salts). Further, the compounds (I) may include those salts derived from medicinally, pharmaceutically or physiologically acceptable acids and bases. These salts are not limited to, but include: those of inorganic acids such as hydrochloric acid, hydrobromic acid, hydroiodic acid, sulfuric acid, nitric acid, phosphoric acid, and perchloric acid; occasionally, those of organic acids such as acetic acid, propionic acid, oxalic acid, succinic acid, citric acid, ascorbic acid, lactic acid, p-toluenesulfonic acid, methanesulfonic acid, fumaric acid, tartaric acid, and maleic acid; those of inorganic bases including alkali or alkaline earth metals such as sodium, potassium, calcium, and magnesium, and ammonium, and those of organic bases including for example dialkylamines such as dimethylamine, and diethylamine, trialkylamines, dibenzylamine, ethanolamine, triethanolamine, morpholine, N-methylmorpholine, piperidine and the like.

These compounds (I) may be converted into salts of pharmaceutically or physiologically acceptable acids or bases by conventional methods. Examples of such salts are those of inorganic acids, including hydrochloride, sulfate, and nitrate; depending on the particular inventive compound, those of organic acids, including acetate, oxalate, succinate, and maleate; those of alkali metals, including a sodium salt, and a potassium salt; those of alkaline earth metals, including a a calcium salt; and the like.

For instance, the compounds of formula (I) related to the present invention (ex., Compounds as prepared in Preparation Examples 1 to 132 given below, especially Compound Nos. 22 to 107, 113 to 116, 121 to 122, 125 to 132, etc.) are active in inhibiting MMPs and remarkably improved in terms of oral bioavailability and/or therapeutic actions upon parenteral applications in comparison with conventional compounds. These compounds may be readily applicable to at least one member selected from the group consisting of diseases related to allergy and/or inflammation, bronchial asthma, allergic rhinitis, hay fever, pollinosis, atopic diseases, atopic enteritis or enterocolitis, allergic conjunctivitis, allergic peritonitis, allergic gastrointestinal inflammation, inflammatory diseases observed in various organs, allergy to food substances, urticaria, various contact-type dermatitides, graft-versus-host disease (GVH disease) occurred in organ transplantation, and the like, and have an aptitude for gaining therapeutic and/or prophylactic advantages.

The compounds (I) of the present invention can be used in combination with at least one member selected from the group consisting of antiallergic agents (antiallergics) and antiasthmatic agents known in the art. The antiallergic agents and antiasthmatic agents include, for example, sodium cromoglicate (1,3-bis(2-carboxychromon-5-yloxyl)-2-hydroxypropane disodium salt), tranilast (N-(3,4-dimethoxycinnamoyl)anthranilic acid), ketotifen fumarate, azelastin hydrochloride, oxatomide, amlexanox, terfenadine, repirinast, ibudilast, tazanolast, pemirolast potassium, gold sodium thiomalate, $\gamma$ -globulin preparations, MS-antigens, cortisone acetate, hydrocortisone, triamcinolone, dexamethasone, paramethasone acetate, prednisolone, methylprednisolone, beclomethasone dipropionate, cyproheptadine hydrochloride, homochlorcyclizine hydrochloride, tacrolimus, etc.

The compounds (I) as used herein can also be used in combination with at least one member selected from the group consisting of bronchodilators known in the art, including $\beta_2$ adrenaergic agonists such as ephedrine hydrochloride, methylephedrine hydrochloride, orciprenaline sulfate, clenbuterol hydrochloride, clorprenaline hydrochloride, salbutamol sulfate, fenoterol hydrobromide, tulobuterol, terbutaline sulfate, trimetoquinol hydrochloride, pirbuterol hydrochloride, formoterol fumarate, procaterol hydrochloride, hexoprenaline sulfate and mabuterol hydrochloride; xanthine drugs such as theophylline and proxyphylline; anticholinergic agents such as ipratropium bromide; $\alpha_2$ blockers; methoxyphenamine hydrochloride, etc.; anticonvulsants known in the art, including oxymethebanol, isoaminile citrate, oxeladin citrate, carbetapentane citrate, clofedanol hydrochloride, chloperastine hydrochloride, dextromethorphan hydrobromide, fominoben hydrochloride, dimemorfan phosphate, benproperine phosphate, etc.; expectorants known in the art, including lysozyme chloride, bromohexine hydrochloride, serapeptase, pronase, acetylcysteine, carbocysteine, ambroxol hydrochloride, ethyl L-cysteine hydrochloride, eprazinone hydrochloride, guaifenesin, tipepidine hibenzate, etc.; antihistaminic agents known in the art, including isothipendyl hydrochloride, diphenylpyraline, diphenhydramine hydrochloride, alimemazine tartrate, triprolidine hydrochloride, mbhydrolin napadisylate, clemastine fumarate, promethazine hydrochloride, chlorpheniramine maleate, mequitazine, etc.; antiinflammatory agants (antiphlogistics), antimicrobial agents and antibiotics known in the art.

When employed as pharmacological agents, the compounds (I) and salts thereof can be employed as pharmaceutical drugs usually in the form of a pharmaceutical composition or preparation alone or in admixture with a variety of pharmacologically acceptable aids. For example, the compound (I), a salt thereof, etc. can be administered alone or in a form of a pharmaceutical composition or preparation in admixture with any of various pharmaceutically acceptable aids. Preferably, it may be administered in the form of a convenient pharmaceutical composition or formulation suitable for oral, topical, parenteral application, or the like. Any of dosing forms (including those for inhalation and rectal administration) may be selected depending on purpose.

The parenteral administration includes topical, percutaneous, intravenous, intramuscular, subcutaneous, intracutaneous, and intraperitoneal routes. It is also possible to apply the drug directly to affected sites, and, in a certain case, the direct application is suitable. Preferably mammal animals including human can receive the drug orally or parenterally (e.g., intracellularly, intravenously, intramuscularly, subcutaneously, intracutaneously, intraperitoneally, intrapleurally, intraspinally, via an intra-tissue route, by instillation, enterally, per rectum, by instillation into the ear, eye, or nose, by pasting or application on the skin or mucosa, etc.). Specific dosing forms of the pharmaceutical preparations and formulations include pharmaceutical solutions, pharmaceutical dispersions, semisolid preparations, particulate preparations, shaped preparations, extractives, etc. Examples of the dosing forms are tablets, coated tablets, sugar coated tablets, pills, troches, hard capsules, soft capsules, microcapsules, implants, powders, pulvises, granules, fine granules, injections, liquids and solutions, elixirs, emulsions, irrigations, syrups, mixtures, suspensions, liniments, lotions, aerosols, sprays, inhalations, nebula, ointments, plasters, patches, pastes, cataplasms, creams, oleates, suppositories (e.g., rectal suppositories), tinctures, dermatologic waters, ophthalmic solutions, collunariums, auristillae, paints, transfusions, powders for injection solutions, lyophilized preparations, conditioned gels, etc.

The pharmaceutical compositions can be formulated in accordance with conventional techniques. For example, the pharmaceutical composition or formulation may comprise at least one of said compounds (I) of the present invention or a salt thereof alone or in admixture with physiologically acceptable carriers, pharmaceutically acceptable carriers, adjuvants, vehicles, excipients, diluents, etc. The compound (I) of the present invention or a salt thereof is usually admixed with a single member selected from the group consisting of physiologically allowable carriers, pharmaceutically acceptable carriers, adjuvants, vehicles, excipients, diluents, flavoring agents, perfuming agents, sweetening agents, expanders, antiseptics, stabilizers, binders, pH regulators, buffering agents, detergents (surfactants), bases, solvents, fillers, bulking agents, solution adjuvants, solubilizers, tonicity agents, emulsifiers, suspending agents, dispersers, viscosity-increasing agents, thickening agents, gelling agents, stiffening agents, absorbents, adhesives, elastomers, plasticizers, disintegrants, aerosol propellants, preservatives, antioxidants, opacifying agents, humectants, emollients, charge protectors, soothing agents, etc., or suitably in a combination thereof, depending on necessity, to give a unit dose form which is required for generally approved pharmaceutical practices.

The formulations suitable for oral application include solid compositions such as tablets, pills, capsules, powders, granules, and troches; fluid compositions such as solutions, syrups, and suspensions; etc.

The aforementioned solid compositions may be formulated in admixture with any of pharmaceutical aids. Such pharmaceutical aids include carriers such as dextran, agar, alginates, chitins, chitosans, pectins, gum tragacanth, gum arabic, gelatins, collagens, caseins, albumin, synthetic or semi-synthetic polymers, glycerides, lactose, crystalline cellulose, microcrystalline cellulose and shellac; binders such as dextrin, sucrose, crystalline cellulose, hydroxypropyl cellulose (HPC), carboxymethyl cellulose (CMC), gum arabic, gum tragacanth, calcium carbonate, gelatin, corn starch, polyvinyl pyrrolidone, water, ethanol, glucose syrup and starch syrup; excipients such as starch (ex., corn starch, etc.), lactose, sucrose, glucose, sodium chloride, calcium carbonate, carboxymethyl cellulose (CMC) and silicic acid; disintegrants such as starch (ex., corn starch, potato starch, etc.), sodium alginate, carboxymethyl cellulose, sodium carboxymethyl cellulose, gelatin, shellac, crystalline cellulose, calcium carbonate, sodium bicarbonate, gum tragacanth and calcium phosphate; lubricants such as salts (ex., Al, K, Na, Ca, Mg) of stearic acid (ex., magnesium stearate), light anhydrous silicic acid, synthetic aluminum silicate, talc and microcrystalline cellulose; surface active agents (surfactants) such as polyoxyethylene sorbitan fatty acid esters and alkyl sulfates; capsule bases such as gelatin; sweetening agents (sweeteners) such as sucrose, lactose and saccharin; flavoring agents (flavorings) such as peppermint, cinnamon oil, orange oil, spearmint, akamono oil and cherry; other flavors; disintegration inhibitors; absorption enhancers; stabilizers; preservatives such as parabens and sorbic acid; antioxidants such as ascorbic acid, $\alpha$ -tocopherol and cysteine; thickeners; etc. The tablets and pills can be prepared further by enteric coating. When the unit dosage form is a capsule, fluid carriers such as fats and oils can be contained in addition to the aforementioned materials.

Representatives of such formulations are tablets and capsules, each of which is in the form of a dose unit suitable for a single administration and may be manufactured by ordinary techniques wherein customarily acceptable additives as listed below are contained. The tablet may be coated by customarily known techniques for conventional pharmaceutical practices.

(A) ordinary vehicles which serve as binders, including syrup, acacia, gelatin, sorbitol, gum tragacanth, or polyvinylpyrrolidone;
(B) fillers, including lactose, sucrose, corn starch, calcium phosphate, sorbitol, and glycine;
(C) tablet lubricants, including for example magnesium stearate, talc, polyethylene glycol, and silica; and
(D) disintegrants such as potato starch or acceptable wetting agents such as sodium lauryl sulfate.

The fluid formulations may contain an inert diluent ordinarily used in the art, such as water. Representatives of the fluid formulations may be prepared in the form of a suspension, solution, emulsion, syrup, or elixir, wherein water or oil is contained as a component, or provided in the form of a dry product for reconstitution into a liquid drug by addition of water or a suitable vehicle just prior to use.

Such fluid formulations may also contain any of pharmaceutical aids, including for example suspending agents such as sorbitol, syrup, methylcellulose, carboxymethyl cellulose, glucose syrup, gelatin and hydrogenated dietary oils; emulsifiers such as lecithin, sorbitan monooleate and acacia; non-aqueous vehicles (including dietary oils) such as (i) vegetable oils including, for example, almond oil, fractionated cocoanut oil, etc. and (ii) oily esters including, for example, glycerin, propylene glycol, ethyl alcohol, etc.; customary additives including for example antiseptics (such as ethyl p-hydroxybenzoate, propyl p-hydroxybenzoate, and sorbic acid), absorption enhancers, stabilizers, preservatives, and, as required, ordinary condiments, sweetening agents (sweeteners), flavoring agents (flavorings), and/or coloring agents (colorants). In the formulations, the compounds of the present invention are applied in the form of compositions containing approximately from 0.1 to 95 percent by weight.

Formulations suitable for parenteral routes include aseptic solutions or suspensions containing at least one active component in admixture with water or other pharmaceutically acceptable media. Examples of such parenteral formulations are injections. Preferred liquid carriers for injection generally include water, saline, dextrose solution, other related saccharide solutions, ethanol, glycols such as propylene glycol and polyethylene glycol, etc. For the preparation of injections, the active component of the present invention is usually admixed with any of carriers such as distilled water, Ringer's solution, physiological saline, suitable dispersing agents, moistening agents, suspending agents, etc. to form injectable formulations including solutions, suspensions, emulsions, etc. by known techniques in the art.

Examples of aqueous liquids for the injection are a physiological saline and isotonic solutions containing glucose and other aids (e.g. D-sorbitol, D-mannitol, sodium chloride, etc.) wherein a suitable pharmaceutically acceptable auxiliary solubilizer such as alcohol (e.g. ethanol, etc.), polyalcohol (e.g. propylene glycol, polyethylene glycol, etc.), nonionic surface-active agent (e.g. Polysorbate 80™, HCO-50, etc.), etc. may be jointly used. The injectable oily liquids may include sesame oil, soybean oil, etc. wherein benzyl benzoate, benzyl alcohol, etc. may be jointly used as auxiliary solubilizers. In addition, buffers (e.g. phosphate buffer, sodium acetate buffer, etc.) or agents for osmoregulation, analgesic agents (e.g. benzalkonium chloride, procaine hydrochloride, etc.), stabilizers (e.g. human serum albumin, polyethylene glycol, etc.), preservatives (e.g. benzyl alcohol, phenol, etc.), antioxidants such as ascorbic acid, absorbefacients, etc. may be compounded therewith too. The prepared injection solution is usually filled in suitable

ampoules. Thus, not only safe but also less toxic preparations are manufactured which can be administered to warm-blooded animals (for example, human, etc.) and the like.

For parenteral administration, solution or suspension unit dosage forms are prepared in pharmaceutically acceptable sterile fluids such as water, ethanol, and oils, in admixture with or without detergent and other pharmaceutically acceptable aids. The oily vehicle and solvent used in the parenteral formulation may include natural, synthetic or semi-synthetic mono-, di-, or triglycerides; natural, semi-synthetic or synthetic fats and oils; and fatty acids. Examples of such oily vehicles and solvents are plant oils such as peanut oil, corn oil, soybean oil, and sesame oil. For example, this injection can usually be prepared to form unit doses each containing approximately from 0.1 to 10 wt% of the compound of the present invention.

The formulation suitable for topical use or rectal application, includes ophthalmic solutions (eye drops), inhalants, ointments (salves), suppositories, etc. The eye drops and other ophthalmic agents are prepared by conventional techniques, using pharmaceutically acceptable carriers. For the inhalants, the compound of the present invention can be applied to respiratory organs after dissolving alone or together with pharmaceutically acceptable inert carriers, in an aerosol or solution for nebulizers, or in the form of powders for inhalation. The ointments (salves) are prepared by conventional techniques, in admixture with conventionally employed pharmaceutical bases such as ointment bases (white petrolatum, paraffin, olive oil, macrogol 400, macrogol ointment, etc.). In the ointments, the compounds of the present invention are applied in the form of compositions containing approximately from 0.001 to 30 wt%.

The pharmaceutical drugs for topical application (including painting) to skin can be prepared in the form of a solution or suspension utilizing suitably sterilized water or non-aqueous vehicles. The additives used include buffering agents such as sodium bisulfite and disodium edetate; preservatives including antiseptic, antimicrobial and antifungal agents such as acetic acid, phenylmercuric nitrate, benzalkonium chloride and chlorhexidine; and thickeners such as hypromellose.

The suppositories can be prepared by conventional techniques utilizing carriers well known in the art, preferably suitable non-irritative excipients. Examples of the excipients are those which are solid at room temperature but liquid at rectal temperature wherein such substances melt in the rectum to deliver a drug, such as polyethylene glycols, lanolin, cacao butter, and fatty acid triglycerides. In the suppositories, the compounds of the present invention are applied in the form of compositions containing approximately from 0.1 to 95 wt%. The compound, depending on the vehicle and concentration used, can be either suspended or dissolved in the vehicle. Adjuvants such as a local anesthetic, preservative and buffering agent can be dissolved in the vehicle.

The aforementioned pharmaceutical preparations may be formulated in the form of compositions usually containing approximately from 0.001 to 95 wt% of the compound of the present invention.

Dose levels of said compound or a salt thereof may vary within a wide range. Specific dose levels and administration cycles for any particular patient will be employed depending upon a variety of factors including the activity of specific compounds employed, the sex, age, body weight, general health, diet, time of administration, route of administration, rate of excretion, drug combination, and the severity of the particular disease undergoing therapy.

Upon oral administration, actual dosage levels of the active ingredients in the pharmaceutical compositions of the present invention may be varied depending on the disease being treated, the severity of the symptom being treated, the general condition and prior medical history of a particular patient being treated, the route and cycle of administration, etc. When administered to a mammalian patient (for example, an adult person), a suitable daily dose will vary depending on the condition of the patient, but general dosage levels of about 0.01 to 500 mg (more preferably about 0.1 to 300 mg) of the compound (I) per kilogram of body weight per day are suitably administered.

A parenteral dose suitable for a single administration may be varied depending on the site being treated, the organ being treated, the severity of the symptom being treated, the general condition and prior medical history of a particular patient being treated, the route and cycle of administration, etc. For instance, it is generally advantageous that the drugs may be injected intravenously, for example to an adult person (taken as 60 kg), at a daily dose of from about 0.001 mg to 6 g (preferably from about 0.01 mg to 1 g) of the compound (I). For other animals, they can be given in terms of a dose per 60 kg.

A topical dose may also be varied depending on the size of a diseased site being treated. A typical dose for a single ophthalmic application (per eye) is ranging from 0.01 to 100mg of the compound (I).

With regard to therapeutic applications for inflammation, the active compound of the present invention can be administered orally or via injection to diseased sites.

For the manufacture of pharmaceutical compositions and preparations, the additives, etc., preparation methods and the like can be suitably selected from those disclosed in Nippon Yakkyokuho Kaisetsusho Henshu Iinkai (Ed.), "13th Ed. Nippon Yakkyokuho Kaisetsusho (Commentary on The Pharmacopoeia of Japan, 13th Ed.)", July 10, 1996, Hirokawa Pub. Co., Tokyo, Japan; Hisashi Ichibagade et al. (Ed.), "Pharmaceutical Research and Development (Ikuo Suzuki, chief editor), Volume 12 (Pharmaceutical Necessities 1)", October 15, 1990, Hirokawa Pub. Co., Tokyo, Japan; ibid., Volume 12 (Pharmaceutical Necessities 2), October 28, 1990, Hirokawa Pub. Co., Tokyo, Japan; etc., depending on necessity, and can be adapted by referring to the disclosures therein.

For terms (words) and/or abbreviations used in the specification and in the drawings, they are based on the meanings of the terms which are commonly used in the art.

EXAMPLES

Described below are examples, i.e., biological assay examples, formulation examples, and preparation examples, of the present invention which are provided only for illustrative purposes, and not to limit the scope of the present invention. It should be understood that numerous variations, equivalents and modifications that would be within the purview of one skilled in this art can be effected without departing from the true spirit and scope of the invention.

Biological Assay

Example 1

Auricle Edema in Experimental Atopic Dermatitis Model

Intravenous injection of an anti-2,4-dinitrophenol (DNP) antibody (anti-DNP Ab, monoclonal IgE Ab; its antibody titer was 1 : 512 to 1 : 1024 by a rat heterologous passive cutaneous anaphylaxis reaction using a Wister rat) into a tail vein of female Balb/c mice (7-week old, body weight: 17 to 20 g, purchased from JAPAN SLC, Inc.) at a dose of 1 mL per animal raised passive sensitization. Twenty four hours post-sensitization, 0.1% 2,4-dinitrofluorobenzene (DNFB; Nacalai Tesque, Inc.) in ethanol was painted onto both sides of a pair of mouse auricles at a dose of 10µL per each side for elicitation.

Ovalbumin (OVA) was conjugated with DNP according to the method specified by Eisen et al. (Eisen, H.N., et al., Journal of the American Chemical Society, 75: 4583-4585, 1953) and, as a result, approximately 3.5 DNP molecules were coupled to one OVA molecule. An aluminum hydroxide gel (Alum) was prepared according to the method specified by Levine et al. (Levine, B.B., et al., International Archives of Allergy, 39: 156-171, 1970). DNP-OVA (10µg) was well mixed with 1 mg of Alum and the resultant mixture was intraperitoneally injected into another set of mice, thereby inducing active sensitization. Two weeks later, 0.1% DNFB in ethanol was painted onto both sides of a pair of mouse ears for elicitation in the same manner as aforementioned.

Fresh unsensitized mice were painted with 0.1% DNFB in ethanol onto their ear in the same manner as aforementioned and served as a control group. Not only immediately prior to elicitation but also 1 hour, 5 hours, daily between Days 1 and 14, and on Day 16 post-elicitation, ear thickness was measured using a dial thickness gauge (Peacock Co.). Each degree of auricle edemas was calculated:

Auricular Edema (µm)=(Ear Thickness Post-Elicitation, µm) - (Ear Thickness immediately prior to Elicitation, µm)

The results are shown in FIG. 1 for the actively or passively sensitized mice and the unsensitized mice in terms of auricle edema (average± standard deviation, N=6). It has been observed that (a) one type of auricle edema peaks at 1 hour post-elicitation and bottoms at 5 hours post-elicitation and (b) another type of auricle edema restarts thereafter and repeaks on Day 1 post-elicitation. The former corresponds to "Immediate Phase Response (IPR)" and the latter to "Late Phase Response (LPR)", both of which have already been reported in the arts (Katayama, I., et al., Int. Arch. Allergy Appl. Immunol., 93: 148-154, 1990; Nagai, H., et al., Biol. Pharm. Bull., 18: 239-245, 1995; and Katayama, I., et al., Int. Arch. Allergy Immunol., 109: 390-397, 1996). Up to now, no auricle edemas other than biphasic have been reported. However, when the measurement of auricle thickness was continued after the late phase response in this Example, it was discovered that there was a novel auricle edema phase beginning on Day about 5, peaking on Days about 7 to 10, and lasting on Day 16 or later post-elicitation. This response is hereinafter referred to "very Late Phase Response (vLPR)". FIG. 1 indicates that, similarly to IPR and LPR, vLPR is an auricle edema dependent on either (1) active sensitization with an antigen or (2) passive sensitization by an intravenous injection of anti-DNP IgE Ab, as compared to auricle edemas in the unsensitized mouse group. It is shown that vLPR is observable irrespective of sensitizing modes (active sensitization and passive sensitization) and its intensity, pattern and time-course are equivalent.

Example 2

(1) Immunological Characteristics in Experimental Atopic Dermatitis

Mice were passively sensitized with anti-DNP IgE Ab in the same manner as in Example 1. Twenty four hours later, 0.1% DNFB in ethanol was painted onto both sides of a pair of mouse auricles at a dose of 10µL per each

side for elicitation. After elicitation, the mice were sacrificed under ether anesthesia successively (0, 1 and 5 hours, and 1, 4, 8 and 24 days later) and a pair of mouse auricles were surgically excised from each mouse. Ear tissues were fixed with 4% paraformaldehyde, paraffin embedded, sliced with a microtome, and then dehydrated with a series of alcohols. Thereafter, for assessing the difference in the numbers of leukocytes present in auricular tissues, the following staining was performed and cells discriminable by each staining of auricle slices with 5 mm in length were observed under a microscope and counted: for discrimination, staining with toluidine blue is used for mast cells and basophils; staining with naphthol AS-D chloroacetate (esterase staining) for neutrophils, macrophages, etc.; and staining with hematoxylin-eosin for eosinophils. The results are shown in FIG. 2 wherein the data are expressed in terms of average ± standard deviation (N=5).

It is disclosed that, one hour post-elicitation (IPR), both toluidine blue stain-positive and esterase-positive cells increased and eosinophils also did a little. It is observed that, following that, the numbers of toluidine blue stain-positive cells considered to be caused by degranulation gradually reduced 5 hours post-elicitation or thereafter. Further, esterase-positive cells and eosinophils which increased 1 hour post-elicitation (IPR) also reduced 5 hours post-elicitation. One day post-elicitation (LPR), esterase-positive cells and eosinophils increased again. It is disclosed that in this LPR the number of infiltrated eosinophils was markedly higher as compared to that in IPR. Four days post-elicitation, both toluidine blue stain-positive and esterase-positive cells were restored to levels immediately prior to elicitation.

Further, it is disclosed that, 8 days post-elicitation (vLPR), both toluidine blue stain-positive and esterase-positive cells increased a little but the number of eosinophils extremely increased as compared thereto or to that of eosinophils themselves prior to Day 8 post-elicitation. However, 24 days post-elicitation the numbers of toluidine blue stain-positive cells, esterase-positive cells and eosinophils became equivalent to those immediately prior to the elicitation.

Example 3

(2) Immunological Characteristics in Experimental Atopic Dermatitis

A role of mast cell in the development of auricle edemas (IPR, LPR and vLPR) was assessed using (1) a genetically mast cell-deficient mutant mouse ($WBB6F_1$ $W/W^v$) and (2) a mast cell-competent parent strain of mouse ($WBB6F_1$ +/+) having a genetic background identical therewith. In the same manner as in Example 1, mice were passively sensitized with anti-DNP IgE Ab and elicited with 0.1% DNFB in ethanol 24 hours later. Immediately prior to elicitation, 1 and 5 hours, daily between Days 1 to 15 and on Day 17 post-elicitation, auricle thickness was measured in the same manner as in Example 1. Each amount of auricle edemas was calculated. The results are shown in FIG. 3 wherein the data are expressed in terms of average ± standard deviation (N=6).

FIG. 3 indicates that auricle edemas 1 hour post-elicitation (IPR) were mast cell-dependent but auricle edemas on Day 1 post-elicitation (LPR) were mast cell-independent. However, it is disclosed that auricle edemas starting on Day 5 post-elicitation (vLPR) were partially mast cell-dependent. In view of another aspect, it is suggested that the auricle edema in vLPR is associated with a bioactive component(s)/cell(s) other than the mast cell.

Example 4

(3) Immunological Characteristics in Experimental Atopic Dermatitis

Next, a role of thymus in the development of auricle edemas (IPR, LPR and vLPR) was assessed using (1) a genetically thymus-deficient mutant mouse (Balb/c nu/nu) and (2) a mouse (Balb/c) having thymus and a genetic background identical therewith.

In the same manner as in Example 1, mice were passively sensitized with anti-DNP IgE Ab and then elicited with 0.1% DNFB in ethanol 24 hours later. Immediately prior to elicitation, at 1 and 5 hours, daily between Days 1 to 9 and on Days 11, 13 and 16 post elicitation, auricle thickness was measured in the same manner as in Example 1. Each amount of auricle edemas was calculated. The results are shown in FIG. 4 wherein the data are expressed in terms of average± standard deviation (N=6).

FIG. 4 clearly indicates that both auricle edemas at 1 hour post-elicitation (IPR) and on Day 1 post-elicitation (LPR) were less thymus-dependent but a part of auricle edemas beginning on Day 6 post-elicitation (vLPR) were thymus-dependent.

When the results in Examples 3 and 4 are summarized, it becomes apparent that (1) IPR is mast cell-dependent and less T cell-dependent, (2) LPR is T cell-dependent and mast cell-independent, and (3) vLPR is associated with T cell and/or mast cell.

Example 5

(4) Immunological Characteristics in Experimental Atopic Dermatitis

It was examined how DNFB-repainting prior to auricle edematization in vLPR would affect the development of auricle edema.

In the same manner as in Example 1, Balb/c mice were passively sensitized with anti-DNP IgE Ab and then elicited with 0.1% DNFB in ethanol 24 hours later. The mice were further painted onto both sides of a pair of their auricles with 0.001%, 0.01% or 0.1% DNFB in ethanol at a dose of 10 μL per each side 3 days later (on Day 3 post-elicitation). Immediately prior to elicitation, at 1 and 5 hours, and daily between Days 1 to 8 post elicitation, auricle thickness was measured in the same manner as in Example 1. Each amount of auricle edemas was calculated. Three days later from the first DNFB painting, a second DNFB painting was conducted, provided that auricle thickness was similarly measured just before the second painting, at 1 hour, and 5 hours after painting for calculation of each amount of auricle edemas.

It has been observed in FIG. 5 that, 1 hour (IPR) and 1 day (LPR) later, the second DNFB painting induces auricle edemas similarly to the first DNFB-elicitation. It is also apparent that the auricle edema development in vLPR on day 7 after the first DNFB-elicitation is enhanced dependently on the concentration of DNFB for the second painting. When consideration is given to its functional mechanism from another viewpoint, the following 3 points are deduced:

(1) the second DNFB paint merely acts as a chemotactic factor with regard to auricle edema development in vLPR, and the second DNFB painting induces more intensive accumulation of leukocytes such as eosinophils, thereby making auricle edemas worse;

(2) the second DNFB paint promotes degranulation from leukocytes such as eosinophils in the auricle edema at a stage of vLPR, thereby making auricle edemas worse; and/or

(3) interaction of the second DNFB paint with antibodies specifically reactive to DNFB, humoral factors such as components in the complement system activated by the initial DNFB elicitation, and/or cellular components such as eosinophils, mast cells, T cells, and antigen-presenting cells, leads to more accumulation of leukocytes such as eosinophils, thereby making auricle edemas in vLPR worse, and

(4) the second DNFB paint well interacts with antibodies specifically reactive to DNFB, liquid components such as components in the complement system activated by the initial DNFB elicitation, and/or cellular components such as eosinophils, T cells, mast cells, and antigen-presenting cells, thereby making auricle edemas in vLPR worse.

Example 6

(1) Efficacy against Experimental Atopic Dermatitis

Balb/c mice were passively sensitized with anti-DNP IgE Ab in the same manner as in Example 1. Twenty four hours later, 0.1% DNFB in ethanol was painted onto both sides of a pair of mouse auricles at a dose of 10 μL per each side for elicitation. Twenty four and 2 hours prior to elicitation, a solution of each test compound (Compound Nos. 22, 43, 44, 46, 91, 126, 127 and 130, respectively; 60mg/kg, once a day, subcutaneous administration) in physiological saline for injection or a suspension of prednisolone (10 mg/kg, once a day, intraperitoneal administration) in 0.5% aqueous methyl cellulose was administered to each animal (5 animals per group).

Immediately prior to elicitation, 1 hour and on Day 1 post-elicitation, auricle thickness was measured in the same manner as in Example 1. Each therapeutic efficacy is recorded as a percent inhibition wherein the "auricle edema (untreated group)" is used as a control.

$$\text{Auricle Edema Inhibition (\%)} = \left[ 1 - \frac{\text{Auricle Edema (Treated Group)}}{\text{Auricle Edema (Untreated Group)}} \right] \times 100\ (\%)$$

In Table 1, the data show that the test compounds have inhibitory actions on the development of auricle edema in IPR 1 hour and LPR on Day 1 post-elicitation. It is apparently shown that the percent inhibition (efficacy) of each Test Compound (Compound Nos. 22, 43, 44, 46, 91, 126, 127 and 130, respectively) is equivalent to or better than that of prednisolone.

TABLE 1

| (1) Efficacy on Experimental Atopic Dermatitis | | | |
|---|---|---|---|
| Compound No. (Name) | Treatment | % Inhibition | |
| | | IPR | LPR |
| Prednisolone | 24 & 2 Hours prior to DNFB-Elicitation | 36.8 | 29.9 |
| Compound No. 22 | | 52.7 | 33.1 |
| Compound No. 43 | | 38.2 | 39.2 |
| Compound No. 44 | | 43.9 | 43.6 |
| Compound No. 46 | | - | 46.7 |
| Compound No. 91 | | - | 43.0 |
| Compound No. 126 | | 38.2 | 39.2 |
| Compound No. 127 | | 47.8 | 41.1 |
| Compound No. 130 | | 64.4 | 51.3 |

Example 7

(2) Efficacy against Experimental Atopic Dermatitis Model

Mice were sensitized with anti-DNP IgE Ab and then elicited by painting 0.1% DNFB in ethanol onto both sides of a pair of mouse auricles at a dose of 10 μL per each side 24 hours post-sensitization in the same manner as in Example 1. At 24 and 2 hours pre-elicitation, and on Days 1, 2, 3, 4 and 5 post-elicitation, a solution of a test compound (Compound Nos. 44, 46, 90, 127 and 130, respectively; 60mg/kg, once a day, subcutaneous administration) in physiological saline for injection or a suspension of prednisolone (10 mg/kg, once a day, intraperitoneal administration) in 0.5% aqueous methyl cellulose was administered to each animal (5 animals per group). Immediately prior to elicitation, and on Day 7 post-elicitation, auricle thickness was similarly measured. Each therapeutic efficacy is recorded as a percent inhibition wherein the auricle edema in the untreated group is used as a control.

The results (therapeutic efficacies) are summarized in Table 2 wherein inhibitory actions on IgE-mediated auricle edema in the very Late Phase Response (vLPR; Day 7 post-elicitation) are shown. Consequently, it has been found that each Test Compound exerts an inhibitory action on the the development of auricle edema. It is concluded that the compounds according to the present invention are equivalent or superior to prednisolone in terms of edema inhibition and are excellently potent.

TABLE 2

| (2) Efficacy on Experimental Atopic Dermatitis | | |
|---|---|---|
| Compound No. (Name) | Treatment | % Inhibition |
| | | vLPR |
| Prednisolone | Daily from 1 Day prior to DNFB-Elicitation through 5 Days after DNFB-Elicitation | 24.8 |
| Compound No. 44 | | 22.0 |
| Compound No. 46 | | 27.5 |
| Compound No. 90 | | 20.6 |
| Compound No. 127 | | 46.7 |
| Compound No. 130 | | 79.0 |

Example 8

(3) Efficacy against Experimental Atopic Dermatitis Model

Mice were sensitized with anti-DNP IgE Ab and then elicited by painting 0.1% DNFB in ethanol onto both sides of a pair of mouse auricles at a dose of 10 µL per each side 24 hours post-sensitization in the same manner as in Example 1. On Days 3, 4, 5 and 6 post-elicitation, a solution of each test compound (Compound Nos. 22, 43, 44, 126, 127 and 130, respectively; 60mg/kg, once a day, subcutaneous administration) in physiological saline for injection or a suspension of prednisolone (10 mg/kg, once a day, intraperitoneal administration) in 0.5% aqueous methyl cellulose was administered to each animal (5 animals per group). Immediately prior to elicitation, and on Day 7 post-elicitation, auricle thickness was similarly measured. Each therapeutic efficacy is recorded as a percent inhibition wherein the auricle edema (untreated group) is used as a control.

The results (therapeutic efficacies) are summarized in Table 3 wherein inhibitory actions on IgE-mediated auricle edema in the very Late Phase Response (vLPR; Day 7 post-elicitation) are shown. Consequently, it has been found that each Test Compound exerts an inhibitory action on the development of auricle edema. It is concluded that Compounds according to the present invention are equivalent or superior to prednisolone in terms of edema inhibition percentage and excellently potent.

TABLE 3

| (3) Efficacy on Experimental Atopic Dermatitis | | |
|---|---|---|
| Compound No. (Name) | Treatment | % Inhibition |
| | | vLPR |
| Prednisolone | Daily from 3 to 6 Days after DNFB-Elicitation | 24.8 |
| Compound No. 22 | | 49.5 |
| Compound No. 43 | | 43.6 |
| Compound No. 44 | | 65.6 |
| Compound No. 126 | | 44.7 |
| Compound No. 127 | | 20.6 |
| Compound No. 130 | | 52.7 |

Example 9

(4) Efficacy against Experimental Atopic Dermatitis Model

Mice were sensitized with anti-DNP IgE Ab and then elicited by painting 0.1% DNFB in ethanol onto both sides of a pair of mouse auricles at a dose of 10 µL per each side 24 hours post-sensitization in the same manner as in Example 1. Twenty four hours and two hours prior to elicitation, a solution of a test compound (Compound No. 130; 60mg/kg, once a day, subcutaneous administration) in physiological saline for injection or a suspension of prednisolone (10 mg/kg, once a day, intraperitoneal administration) in 0.5% aqueous methyl cellulose was administered to each animal (5 animals per group). Immediately prior to elicitation, at 1 and 4 hours and on Days 1, 3 and 7 post-elicitation, auricle thickness was similarly measured. From each auricle edema (µm) based on the auricle thickness immediately prior to elicitation, especially a prophylactically therapeutic efficacy of the test compound was evaluated.

The results (therapeutic efficacies) are summarized in FIG. 6. The data indicate that the compound of the present invention has an inhibitory efficacy (average ± standard deviation) on IgE-mediated auricle edema in the Immediate Phase Response (IPR; 1 hour post-elicitation), the Late Phase Response (LPR; 1 day post-elicitation), and the very Late Phase Response (vLPR; 7 days post-elicitation). It is apparent that, as compared to prednisolone, the compound of the present invention (Compound No. 130) is endowed with an edema-inhibitory activity equivalent to that of prednisolone in IPR and slightly and markedly higher than that of prednisolone in LPR and vLPR, respectively, even where the drug is administered only at the pre-elicitation stage.

Example 10

(5) Efficacy against Experimental Atopic Dermatitis Model

Mice were sensitized with anti-DNP IgE Ab and then elicited by painting 0.1% DNFB in ethanol onto both sides of a pair of mouse auricles at a dose of 10 µL per each side 24 hours post-sensitization in the same manner as in Example 1. A solution of a test compound (Compound No. 130; 50 µg/ear/dose, twice a day) in ethanol was painted onto both sides of a pair of the mouse auricles 1 hour pre-elicitation, 0.25, 3, 4, 5 and 6 days post-elicitation (5 animals per group). Immediately prior to elicitation, at 0.5, 1 and 4 hours and on Days 1, 3 and 7 post-elicitation, auricle thickness was similarly measured. Each amount of auricle edemas was calculated (average± standard deviation).

The results (therapeutic efficacies) are summarized in FIG. 7. The data show inhibitory efficacies on IgE-mediated auricle edema in IPR (1 hour post-elicitation), LPR (1 day post-elicitation), and vLPR (7 days post-elicitation). It has been observed that the compound of the present invention (Compound No. 130) is endowed with an inhibitory activity on edemas in any of IPR, LPR, and vLPR. It has been found that it especially exerts an excellent inhibitory activity on the vLPR edema development.

Example 11

(6) Efficacy against Experimental Atopic Dermatitis Model

Balb/c mice were sensitized with anti-DNP IgE Ab and then elicited by painting 0.1% DNFB in ethanol onto both sides of a pair of mouse auricles at a dose of 10 µL per each side 24 hours post-sensitization in the same manner as in Example 1. A solution of a test compound (Compound No. 44; 150 mg/kg, once a day) in water for injection was administered orally 1 hour pre-sensitization, 4, 5 and 6 days post-sensitization (5 animals per group). Immediately prior to elicitation, at 1 and 4 hours and on Days 1, 4 and 7 post-elicitation, auricle thickness was similarly measured. In the same manner as in Example 1, each auricle edema (µm) was calculated and each therapeutic efficacy was evaluated.

The results (therapeutic efficacies) are summarized in FIG. 8. The data show inhibitory efficacies on IgE-mediated auricle edema in IPR (1 hour post-elicitation), LPR (1 day post-elicitation), and vLPR (7 days post-elicitation). As compared to the solvent control group, it is apparent that the compound of the present invention (Compound No. 44) is endowed with not only an inhibitory activity on the development of edemas in both IPR and LPR but also a remarkably strong inhibitory activity in vLPR.

Example 12

(7) Efficacy against Experimental Atopic Dermatitis Model

Mice were passively sensitized with anti-DNP IgE Ab and a pair of mouse auricles were then elicited with 0.1% DNFB in ethanol 24 hours post-sensitization in the same manner as in Example 1. A solution of each test compound (Compound Nos. 114, 122, 125, 129, and 132, respectively; 60 µg/ear/dose) in ethanol was painted onto both sides of a pair of the mouse auricles twice a day (morning and evening) 1 hour pre-elicitation, 6 hours, 1 to 6 days post-elicitation (5 animals per group). Each auricle edema was measured 1 hour, 1 day, and 7 or 8 days post-elicitation based on the auricle thickness just prior to elicitation. Each auricle edema inhibition percentage (%) was calculated in the same manner as in Example 6.

The results (therapeutic efficacies) are summarized in Table 4. The data show inhibitory efficacies on the auricle edema development in IPR (1 hour post-elicitation), LPR (1 day post-elicitation), and vLPR (7 or 8 days post-elicitation). It is apparent that the compounds of the present invention (Compound Nos. 114, 122, 125, 129, and 132) are biologically effective against the auricle edema development in IPR, LPR and vLPR.

TABLE 4

| (7) Efficacy on Experimental Atopic Dermatitis | | | |
|---|---|---|---|
| | % Inhibition | | |
| | IPR | LPR | vLPR |
| Compound No. 114 | 71 | 62 | 52 |
| Compound No. 122 | 31 | 22 | 35 |

TABLE 4 (continued)

| (7) Efficacy on Experimental Atopic Dermatitis | | | |
|---|---|---|---|
| | % Inhibition | | |
| | IPR | LPR | vLPR |
| Compound No. 125 | 56 | 58 | 31 |
| Compound No. 129 | 28 | 53 | 35 |
| Compound No. 132 | 20 | 39 | 46 |

Example 13

(1) Efficacy against Scratching Behavior in Experimental Atopic Dermatitis Model

Mice were passively sensitized with anti-DNP IgE Ab and then elicited by painting 0.1% DNFB in ethanol onto both sides of a pair of mouse auricles 24 hours post-elicitation in the same manner as in Example 1. Five minutes pre-elicitation, an ethanolic solution containing 0.3% of Compound No. 125 was painted onto both sides of a pair of the auricles at a dose of 10 µL per each side. Tacrolimus (Fujisawa Pharmaceutical Co., Ltd.) was used as a positive control drug. An ethanolic solution containing 0.1% of the positive control drug was prepared just prior to use and then painted onto the mouse auricles 1 hour pre-elicitation in the same manner as for the painting of Compound No. 125. A negative control group was painted with a solvent alone (6 animals per group).

From the point immediately after elicitation, each mouse was housed individually in a comparted acrylic cage and observed by taking a video without any attendant up to 2 hours after elicitation (Inagaki, N., et al., The European Journal of Pharmacology, 367: 361-371, 1999). After finishing such a video recording, scratching behavior by mouse hind paws to a pair of ears was visually examined for each mouse and integrated scratching behaviors were counted during 6 post-elicitation durations for 20 minutes each; 0 to 20 min., 20 to 40 min., 40 to 60 min., 60 to 80 min., 80 to 100 min., and 100 to 120 min. In this experiment, an action from a start to scratch the ear by the mouse hind paw to a release of the hind paw is counted as one scratching behavior.

As shown in FIG. 9, the results indicate the efficacy of the compounds of the present invention on the mouse scratching behavior. A mouse scratching behavior (average± standard deviation) was most noticeably observed in the 20 to 40 min post-elicitation duration. Compound No. 125 apparently inhibits mouse scratching behavior. For its intensity, the inhibitory efficacy thereof in the 20 to 40 min post-elicitation duration (wherein the scratching is most remarkable) is higher than that of tacrolimus. The results suggest that the compound of the present invention (Compound No. 125) inhibits itching due to an allergic reaction.

Example 14

(2) Efficacy against Scratching Behavior in Experimental Atopic Dermatitis Model

Mice were passively sensitized with anti-DNP IgE Ab and then elicited with 0.1% DNFB in ethanol 24 hours post-sensitization in the same manner as in Example 13. One hour pre-elicitation, a 0.1% solution of a compound of the present invention (Compound No. 114, 122, 125, 129, or 132) in ethanol was painted onto both sides of a pair of the auricles at a dose of 10 µL per each side. A negative control group was painted with a solvent alone. From the point immediately after the elicitation, each mouse was housed individually in a comparted cage and observed by taking a video without any attendant up to 2 hours thereafter (3 animals per group). In the same manner as in Example 13, scratching behaviors by mouse hind paws were counted for every 15 minutes: 8 post-elicitation durations; 0 to 15 min., 15 to 30 min., 30 to 45 min., 45 to 60 min., 60 to 75 min., 75 to 90 min., 90 to 105 min., and 105 to 120 min.

The results are summarized in Table 5. The data indicate the efficacy of the compounds according to the present invention on scratching responses associated with elicitation (counts for the 15 to 30 min and 30 to 45 min post-elicitation durations): average± standard deviation (solvent control groups) and percent inhibition (treated groups). It is apparent that all the compounds of the present invention (Compound Nos. 114, 122, 125, 129, and 132) highly alleviate mouse scratching behaviors in the experimental atopic dermatitis model.

TABLE 5

| (2) Efficacy on Scratching Behavior in Experimental Atopic Dermatitis Model | | | |
|---|---|---|---|
| | Conc. (%) | Scratching Behavior (Times) (Average± Deviation) | % Inhibition |
| Solvent Control | - | 209.0 ± 70.4 | - |
| Compound No. 114 | 0.1 | 345.7 ± 47.1 | 30.3 |
| Compound No. 122 | 0.1 | 96.0 ± 32.6 | 54.1 |
| Compound No. 125 | 0.1 | 124.0 ± 43.6 | 40.7 |
| Compound No. 129 | 0.1 | 144.0 ± 48.8 | 31.1 |
| Compound No. 132 | 0.1 | 104.7 ± 30.6 | 49.9 |

Example 15

Efficacy on Blood IgE Levels

Balb/c mice were sensitized with anti-DNP IgE Ab and then elicited by painting 0.1% DNFB in ethanol onto both sides of a mouse right auricle at a dose of 10 µL per each side 24 hours post-sensitization in the same manner as in Example 1. Ethanol alone was also painted onto both sides of a mouse left auricle. A solution of a test compound (Compound No. 44; 100 µg/ear, twice a day) in ethanol was painted onto both sides of a pair of the mouse auricles 1 hour pre-elicitation, 0.25, 4, 5 and 6 days post-elicitation (5 animals per group). Blood was also drawn from unsensitized healthy mice and untreated mice and treated to give serum samples. Seven days post-elicitation, blood samples were drawn by cardiac puncture using a syringe treated with heparin, and then centrifuged to give serum samples.

The samples were measured for serum IgE levels according to the Nagai et al. method (Nagai, H., et al., The Journal of Pharmacology and Experimental Therapy, 283: 321-327, 1997). A dilution of polyclonal goat anti-mouse IgE antibody (Bethyl Laboratories, Inc.; 500-fold dilution) in 0.1 M sodium carbonate-sodium bicarbonate buffer (pH 9.6) was added to each well of a 96-well microtest plate (MaxiSorp, Nunc, Inc.) at a rate of 100 µL/well and the resultant microtest plates were allowed to stand at about 4°C overnight in a refrigerator. Each well was rinsed 3 times with phosphate buffer (pH 7.4) containing 0.05% Tween 20. Next, 0.1 M sodium carbonate-sodium bicarbonate buffer (pH 9.6) containing 1 % bovine serum albumin was added to each well at a rate of 100 µL/well. The resultant microtest plates were allowed to stand at room temperature for 1 hour and washed 3 times. Thereafter, 2-fold serial dilutions of mouse serum samples were poured into each well of microtest plates at 100 µL/well, which was subjected to an antigen-antibody reaction at 4°C for 2 hours. Simultaneously, a mouse monoclonal anti-dinitrophenol antibody (IgE; Sigma) was used as a standard instead of the mouse serum sample. After washing 3 times, each well received alkaline phosphatase-labeled rat monoclonal anti-mouse IgE-specific antibody (Southern Biotechnology; 1,000-fold dilution) at a rate of 100 µL/well, and allowed to stand for 2 hours at room temperature. After washing 3 times, each well received 0.1% p-nitrophenyl phosphate (Kirkegaard & Perry Laboratories, Inc.) at a rate of 100 µL/well, and allowed to stand for 1 hour at room temperature. The resultant enzymatic reaction products were measured at 405 nm.

It is apparent from the results in Table 6 that the compounds of the present invention reduce serum IgE levels (average± standard deviation).

TABLE 6

| Efficacy on Serum IgE Level | | | |
|---|---|---|---|
| | Healthy Control Group | Untreated Control Group | Compound No. 44-Treated Group |
| Serum IgE Level (ng/mL) | 11.79 ± 2.35 | 34.55 ± 8.89 | 26.70 ± 10.88 |

Example 16

Efficacy on Antibody Production Induced by Peripheral Blood Mononuclear Cell from Healthy Person

Heparinized peripheral blood derived from a healthy person was diluted 2-fold with phosphate buffered saline (PBS) and applied to density-gradient centrifugation using Ficoll-Paque (Pharmacia Biotech) to give peripheral blood mononuclear cells (PBMC). After isolation, PBMC was rinsed with PBS, and suspended in RPMI-1640 medium (K.K. Nikken Seibutsu Igaku Kenkyujo) containing 10% heat-inactivated fetal calf serum ($1 \times 10^6$ cells/mL), followed by addition of 0.05% Staphylococcus aureus Cowan-I (SAC, CALBIOCHEM) and 10 units/mL interleukin-2 (IL-2, Genzyme) for stimulation, as well as by simultaneous addition of Compound No. 130 (final concentration: 0.1, 1 and 10 μM). The mixtures were incubated on a 96-well microtest plate at 37°C for 6 days in an atmosphere of 95% air and 5% $CO_2$. After incubation, supernatants were collected and quantitatively measured for IgG and IgM levels by an enzyme immunoassay (a sandwich immunoassay method using two antibodies).

IgG assay: A preparation containing 40 μg/mL anti-human IgG antibody ($F(ab')_2$, Cappel) in 50 mM sodium carbonate-sodium bicarbonate buffer (pH 9.6) was added to each well of a 96-well microtest plate (Nunc) at a rate of 100 μL/well. The plates were allowed to stand overnight at 4°C, and then rinsed 3 times with PBS containing 0.05% Tween 20. A 4-fold dilution of BlockAce (Snow Brand Milk Products Co., Ltd.) in PBS was added to the 96-well microtest plates (300 μL/well) which were then allowed to stand overnight at 4°C, followed by rinsing similarly. Next, a culture supernatant was serially diluted with a 4-fold dilution of BlockAce in PBS and a series of dilutions were added at a rate of 100 μL/well to the 96-well microtest plates which were then allowed to stand at 25°C for 1 hour, followed by rinsing similarly. Thereafter, a 2500-fold dilution of peroxidase-labeled mouse anti-human IgG antibody (Zymed) was added at a rate of 100 μL/well to the 96-well microtest plates which were then allowed to stand at 25°C for 1 hour, followed by rinsing similarly. A coloring kit for peroxidase (Sumitomo Bakelite) was added at a rate of 100 μL/well to each well which was subjected to an enzyme reaction for 15 to 30 minutes at room temperature, followed by addition of 1 N hydrochloric acid at a rate of 100 μL/well. The resultant colored product was measured for an optical density (OD) at 450 nm for a test wavelength and at 650 nm for a reference wavelength in a microplate reader. IgG levels are calculated using a calibration curve of a standard IgG.

IgM assay: The procedures for the IgG assay as aforementioned were repeated except that the anti-human IgG antibody ($F(ab')_2$) was replaced to anti-human IgM antibody ($F(ab')_2$, Cappel) and the peroxidase-labeled mouse anti-human IgG antibody also to peroxidase-labeled mouse anti-human IgM antibody (Zymed; 2,000-fold dilution). IgM levels are obtained in the same manner as for the IgG assay.

Compound No. 130 (final concentrations: 0.1, 1 and 10 μM) inhibited the IgG production of PBMC (percent inhibition: 84.5%, 96.1% and 96.0%). It also did the IgM production (percent inhibition: 32.9%, 68.1% and 63.4%).

Example 17

(1) Efficacy on Mouse Body Weight Gain

When drug therapy is applied, various side-effects are observed. It is considered that one of indexes therefor is a body weight change. Accordingly, for assessing the side-effect each of the following test compounds an action on body weight gains was examined.

A solution of each test compound (Compound No. 22, 44, 46, 91, 126, or 130; 60 mg/kg; subcutaneous injection) in physiological saline for injection or a suspension of prednisolone (10 mg/kg; intraperitoneal injection) in 0.5% aqueous methyl cellulose was administered to mice (5 animals per group) once a day for 6 days (Days 0, 1, 2, 3, 4, and 5). Next the mice were measured for their body weight. Each body weight gain was calculated on Days 5, 7 and 10 post-administration based on the body weight just prior to administration.

The changes in body weight (average ± standard deviation) are shown in Table 7. The body weight gains in mice to which the compounds of the present invention (Compound Nos. 22, 44, 46, 91, 126, and 130) were administered exceed those in the non-administered group, whereas the body weight in the prednisolone-administered group is below that of the non-administered group. Moreover, it fails to recover up to the level in the non-administered group even on Day 5 after the stop of administration (on Day 10 after the initiation of administration).

TABLE 7

| (1) Efficacy on Body Weight Gain | | | |
|---|---|---|---|
| Compound | Observation | | |
| | Day 5 | Day 7 | Day 10 |
| None | 0.50± 0.23 | 0.80± 0.48 | 1.07± 0.58 |
| Prednisolone | 0.00± 0.26 | 0.03± 0.57 | 0.97± 0.47 |
| Compound No. 22 | 0.97± 0.31 | 0.80± 0.39 | 1.20± 0.53 |
| Compound No. 44 | 0.80± 0.30 | 1.08± 0.57 | 1.23± 0.56 |
| Compound No. 46 | 1.08± 0.22 | 1.57± 0.33 | 1.33± 0.32 |
| Compound No. 91 | 1.28± 0.37 | 1.55± 0.48 | 1.30± 0.54 |
| Compound No. 126 | 1.08± 0.39 | 1.62± 0.26 | 1.40± 0.30 |
| Compound No. 130 | 0.57± 0.61 | 0.77± 0.55 | 1.20± 0.26 |

Example 18

(2) Efficacy on Body Weight Gain in Sensitized Mice

Experimental atopic dermatitis model mice sensitized in the same manner as in Example 1 were examined for influence on body weight gains.

A solution of a test compound (Compound No. 102; 50 µg/ear/dose, twice a day) in ethanol or a solution of prednisolone (5 µg/ear/dose, twice a day) in ethanol was administered to mice (5 animals per group) by painting onto both sides of a pair of the auricles, not only one hour prior to elicitation but also 0.25, 3, 4, 5 and 6 days post-elicitation. Each mouse was measured for its body weight not only immediately before the start in painting but also on Days 1, 4 and 7 after the start in painting. Each gain in body weight was calculated and considered to indicate an effect on body weight changes affected by said treatments.

The body weight changes (average± standard deviation) upon said treatments are shown in FIG. 10. It is apparent that the gain in body weight in the group receiving the compound of the present invention (Compound No. 130) is almost equivalent to that in the solvent control group (painting with ethanol), whereas the gain in body weight in the prednisolone-treated group is below that in the solvent control group.

Example 19

Efficacy on Spontaneously Manifested Allergic Dermatitis in NC/Jic Mice

Compound No. 130 was well blended with an equivalent mixture of polyethylene glycol 400 and polyethylene glycol 4000 to give an approximately homogeneous product. Thus, an ointment containing the test compound at a rate of 0.1% was prepared. For control groups, an ointment base alone was employed.

Male NC/Jic mice (body weight, 24.1 to 31.9 g; age, 12 to 24 weeks old; supplier, Clea Japan, Inc.) were checked for their health after purchase. Immediately after the health check, the mice were housed under conventional conditions until dermatitis occurred. Mouse heads surrounding auricle portions were observed by eye once daily during the preparatory housing and, after a start in painting with ointments, once daily prior to painting. The severity of spontaneous dermatitis was scored according to a pre-assigned sore table (Table 8) for the observation of dermatitis. Animals seriatim used in the experiment were selected from those showing signs of dermatitis with score 1 or 2 for 3 consecutive days or longer.

TABLE 8

| Score Table for Observation of Dermatitis | | |
|---|---|---|
| Score | Severity | Signs or symptoms |
| 0 | Normal | No manifestation of dermatitis, or complete cure at diseased sites |
| 1 | Mild | Very mild erythema, mild wound, or a cured state equivalent thereto |
| 2 | Moderate | Obvious erythema, wound, mild hemorrhage, or a cured state equivalent thereto Moderate to severe erythema, wound (partly |
| 3 | Severe | falling off of auricle), or a restricted area of ulcer, crust formation, or a cured state equivalent thereto |
| 4 | Falling off of auricle, etc. | Profuse hemorrhage, or falling off of auricle with hemorrhage & a wide area of ulcer |

The ointment was warmed to about 37°C prior to administration, and open-painted onto diseased sites at 100 mg/dosage/day. Seven weeks were set as a drug application period. A scratching behavior by fore paws or hind paws to portions afflicted with dermatitis or the neighborhood thereof was observed for 30 minutes before painting with the ointment in the last week (Days 47 to 48) during the application period, and a total of scratching behaviors were counted.

The results are shown in FIG. 11 and Table 9 (dermatitis score on Day 49). The dermatitis score is apparently low in NC/Jic mice painted with the Compound No. 130-containing ointments, as compared to that in mice painted with the ointment base only. Furthermore, scratching behaviors were also reduced (the ointment base group, 126, 138, 206 scratching actions (average: 157 scratching actions); the Compound No. 130-containing ointment groups, 19, 81, 177 scratching actions (average: 92 scratching actions)). These indicate that the drug inhibits the progression of spontaneously manifested dermatitis and alleviates effectively itching caused thereby.

TABLE 9

| Efficacy on Spontaneously Manifested Allergic Dermatitis in NC/Jic Mice | | | |
|---|---|---|---|
| | Content | Animal Number | Dermatitis Score |
| | | | (Average ± S. D.*) |
| Ointment Base | 0 % | 3 | 3.7 ± 0.6 |
| Compound No.130 | 0.1 % | 3 | 2.3 ± 1.5 |

* S. D.: Standard Deviation

<u>Example 20</u>

<u>(1) Efficacy on Experimental Asthma Model</u>

Guinea pigs (female, Std:Hartley, 3 weeks old, 200 to 250 g) were individually housed in a plastic chamber (width, 200 mm; depth, 300 mm; height, 167 mm). Aqueous ovalbumin (10 mg/mL) was administered to the guinea pigs once a day for sensitization by 10 min. inhalation with a nebulizer (5B, Nippon Shoji K.K.: flow rate, 6 L/min.) at a dose of 10 mg/mL during 8 cosecutive days. On Day 7 after the final sensitization, the guinea pigs were housed in a plastic chamber and inhaled using a nebulizer with a solution of ovalbumin (10 mg/mL) in physiological saline for 1 min. for elicitation (Matsumoto, T., et al., The Journal of Pharmacology and Experimental Therapeutics, 269: 1236-1244, 1994). The number of animals employed in the experiment was set to the extent there are at least 6 as individuals escaping asthmatic death. A solution of a test compound (Compound No. 130, 15 mg/kg) in water for injection was administered subcutaneously twice, i.e., 24 hours and 2 hours prior to elicitation. Similarly, the solvent alone was also administered to a negative control group. A suspension of prednisolone (10 mg/kg) in 0.5% aqueous methyl cellulose was administered orally twice, i.e., 24 hours and 2 hours prior to elicitation. Similarly, the solvent alone was also administered to another control group.

Since asthmatic death caused by elicitation with antigen-inhalation was frequently observed approximately 2 to 3 min. post-elicitation, the efficacy was evaluated using at least 6 representative examples selected from data among test animals. The evaluation for efficacies was conducted in terms of airway resistance. For this purpose, an apparatus dedicated to the measurement of airway resistance (K.K. Medical Agent) was employed. Measurement includes setting a guinea pig in a test chamber under a not-anesthetized condition, applying sinusoidal pressure waves of 30 Hz components to a part below the neck of the animal from the back side of the chamber, measuring respiratory waves of 30 Hz components leaking through the mouth (volume of 30 Hz air flow passing through the respiratory tract) in front of the neck, and calculating airway resistance mechanically. Measurement was carried out immediately prior to the first drug treatment (24 hours before elicitation), just prior to elicitation, 10 minutes, 1, 2, 4, 6, and 8 hours post-elicitation.

FIG. 12 shows the results obtained in the aforementioned test. When attention is given to each solvent control group, the airway resistance becomes maximal 1 and 6 hours post-elicitation, which are each called as "immediate airway response (IAR)" and "late airway response (LAR)", respectively. These two increases in airway resistance are caused by inflammation, hypertrophy, edema, mucus, etc. in the airway mucosa, indicating that there are bronchial asthmatic symptoms (narrowing of airways). Among them, the increased numbers of eosinophils and neutrophils are noticeable in LAR (Matsumoto, T., et al., The Journal of Pharmacology and Experimental Therapeutics, 269: 1236-1244, 1994). The data show that the subcutaneous administration of the compound of the present invention (Compound No. 130) leads to significant reduction of the increased airway resistance. The activity is approximately equal to or higher than that of prednisolone.

Example 21

(2) Efficacy on Experimental Asthma Model

There is also a period of increasing airway resistance within several minutes after elicitation (prior to the manifestation of IAR) in the aforementioned Example 20. It was visually observed by a person carrying out said test in terms, of the appearance of cyanosis or suffocative death (asthmatic death) due to respiratory obstruction. Percent mortality due to the occurrence of cyanosis after elicitation by antigen-inhalation was obtained using all the data in Example 20. The results are shown in Table 10. With regard to suffocative death due to cyanosis just after elicitation no animal is present in the group treated with a compound of the present invention (Compound No. 130).

TABLE 10

| (2) Efficacy on Experimental Bronchial Asthma Model | | |
|---|---|---|
| | $\frac{\text{Dead Animals}}{\text{TestedAnimals}}$ | % Mortality |
| 0.5% aq. Methyl Cellulose (Solvent Control) | 3/12 | 25.0 |
| Prednisolone | 2/9 | 22.2 |
| Physiological Saline for Injection (Solvent Control) | 2/10 | 20.0 |
| Compound No.130 | 0/10 | 0.0 |

Example 22

(3) Efficacy on Experimental Asthma Model

Guinea pigs were sensitized with ovalbumin and asthma was induced on Day 7 after final sensitization in the same manner as in Example 20. The number of animals employed in the experiment was set to the extent there are at least 6 as individuals escaping asthmatic death (8 to 11 animals/group). In the same manner as in Example 20, the animals were measured for airway resistance mechanically and each percent change (%) in airway resistance was calculated (each value just prior to elicitation was set as 100%). Each percent amelioration in airway resistance was calculated from the percent changes in airway resistance according to the following formula:

$$\text{Amelioration in Airway Resistance (\%)} = \left[\frac{\begin{pmatrix}\text{Percent Change in Airway Resistance}\\ \text{after Drug-Administration}\end{pmatrix} - \begin{pmatrix}\text{Value just prior to}\\ \text{Challenge (100)}\end{pmatrix}}{\begin{pmatrix}\text{Percent Change in Airway Resistance}\\ \text{after Solvent-Administration}\end{pmatrix} - \begin{pmatrix}\text{Value just prior to}\\ \text{Challenge (100)}\end{pmatrix}}\right] \times 100$$

The number of dead animals by asthmatic death was recorded which were observed within several minutes after elicitation in the same manner as in Example 21 and mortality rates (%, dead animal number/tested animal number $\times$ 100 ) were calculated.

A solution of a test compound of the present invention (Compound Nos. 51, 65, 114, 121, 127, and 128, respectively: 15 mg/kg or 30 mg/kg) in water for injection or the solvent alone as a negative control was administered, to each animal subcutaneously twice, i.e., 24 hours and 2 hours prior to elicitation. Similarly to Example 20, prednisolone was employed as a control drug while the solvent alone was also as a control for the prednisolone-group.

The results are shown in Table 11. The data show that either subcutaneous administration of the compounds of the present invention (Compound Nos. 51, 65, 114, 121, 127, and 128) or oral administration of prednisolone leads to amelioration on airway resistance immediately, 1 hour and 6 hours after elicitation. With regard to the amelioration by the compounds of the present invention, they all have an activity equal to or higher than prednisolone. For the compounds of the present invention, moderation in cyanosis is also observed. Regarding the number of dead animals associated with asthma-induction, as compared to prednisolone, it is demonstrated that the administration of the compounds of the present invention (Compound Nos. 51, 65, 114, 121, 127, and 128) leads to excellently good results.

Table 11

(3) Efficacy on Experimental Bronchial Asthma Models

| | Dose (mg/kg) | Animal Number | % Amelioration in Airway Resistance | | | % Survival |
|---|---|---|---|---|---|---|
| | | | Measurement Time after Elicitation | | | |
| | | | Immediately after Elicitation | 1 Hour | 6 Hours | |
| Water for Injection | 0 | 6 | 194.0 ± 19.7* | 170.8 ± 8.1* | 219.6 ± 26.4* | 54.5 |
| Compound No. 51 | 15 | 6 | 30.1 | 59.1 | 56.3 | 70.0 |
| Compound No. 65 | 15 | 6 | 29.9 | 22.3 | 63.6 | 87.5 |
| Compound No. 114 | 30 | 6 | 54.0 | 39.4 | 69.4 | 70.0 |
| Compound No. 121 | 15 | 6 | — | 56.4 | 102.2 | 88.9 |
| Compound No. 127 | 15 | 6 | 21.9 | 20.8 | 55.2 | 70.0 |
| Compound No. 128 | 15 | 6 | — | 53.2 | 49.4 | 72.7 |
| 0.5% Methyl Cellulose | 0 | 6 | 238.8 ± 21.4* | 173.9 ± 16.4* | 191.5 ± 15.9* | 66.7 |
| Prednisolone | 10 | 6 | 11.5 | 23.6 | 14.8 | 75.0 |

*: Airway Resistance in Solvent Control Groups
(% Value prior to Elicitation, Average ± Standard Error)

Example 23

Efficacy on Type IV Allergic Dermatitis

Mice (male, Balb/c, 6 weeks old, 20 to 25 g) were sensitized by painting a 3% solution of oxazolone (100 μL) abdominally. Six days after sensitization, a 0.5% solution of oxazolone in acetone was painted on both sides of a right ear at a dose of 10 μL to raise auricle edema. An untreated group was painted with the solvent (acetone) alone onto both sides of a left ear of the same mouse at 10 μL. At 24 hours after edema-induction, each mouse was sacrificed with ether and an equivalent each of right and left auricles was stamped out with a puncher (diameter: 6 mm). Both left and right auricle sections were measure for their weight and each percent edema was calculated (Kazuo Ouchi (Ed.), "Seibutsu Yakukagaku Jikken Koza, Ensho To Allergy (Inflammation and Allergy) I-1", 1993, Hirokawa Publishing Co., Japan)

$$\text{Percent Edema (\%)} = \frac{\text{(Right Auricular Segment Weight - Left Auricular Segment Weight)}}{\text{Left Auricular Segment Weight}} \times 100$$

A solution of a test compound of the present invention (Compound No. 130, 50 mg/mL) in ethanol was painted onto both sides of a right auricle at 10 μL per side twice, i.e., 0.5 hr prior to edema-induction and 6 hours after edema-induction. The solvent (ethanol) alone was painted on a left auricle at the same dose (treated group). Unsensitized mice were also treated for induction similarly and then treated with the compound of the present invention, only followed by examination for the potency of the compound of the present invention on auricles (unsensitized group). The number of animals used in the test was set 4 per group. The results are shown in FIG. 13, indicating the therapeutic potency (average ± standard deviation) on the development of auricle edemas. It is apparent that the compound of the present invention alone makes auricles almost free of swelling and/or thinning and the administration of the compound (Compound No. 130) leads to the amelioration of auricle edemas.

Example 24

Efficacy (1) on Allergic Peritonitis

Each efficacy on allergic peritonitis was examined according to methods as disclosed in Ouchi et al., "Seibutsu Yakukagaku Jikken Koza, Ensho To Allergy (Inflammation and Allergy) I-3", pp.139 to 151, Hirokawa Publishing Co., Japan and Spicer et al., The International Archives of Allergy and Applied Immunology, 81: 81 to 84, 1986.

A ragweed (Ambrosia artemisiaefolia) pollen extract (1:100, Torii Pharmaceutical Co., Ltd.) was diluted 10-fold with physiological saline and administered subcutaneously to male Balb/c mice (7 weeks old, 20 to 23 g, purchased from JAPAN SLC, Inc.) on Days 0 and 1 at a single dose of 0.1 mL and further on Days 6, 8 and 14 at a single dose of 0.2 mL for sensitization. On Day 20 after the initial sensitization, an antigen solution for induction (a mixture of the aforementioned 10-fold diluted ragweed pollen extract, 50 units/mL of penicillin G potassium, and 0.05 mg/mL of streptomycin sulfate) was administered intraperitoneally to each animal at a dose of 0.2 mL to raise allergic peritonitis. Compound No. 130 (30 mg/kg) or physiological saline (10 mL/kg) was administered intraperitoneally to each animal 5 minutes prior to induction, 3, 6 and 12 hours post-induction.

Twenty four hours after induction, mice were sacrificed under ether analgesia and each penitoneal cavity was lavaged twice or thrice with a washout medium (total volume: 5 mL, physiological saline containing 10 units/mL of heparin). Peritoneal exudate cells (PEC) were harvested in a siliconized glass tube. The retrieved washout medium was measured for its yield (mL) and the number of exudate cells therein by using Turk solution and counting on a haemocytometer and a total number of PEC was calculated. An aliquot thereof (an extent of approximately 20,000 cells of PEC) was taken. Specimens for staining were prepared using Cytospin 3 (CHANDON), and PEC was stained with Dif-Quick solution (International Reagents Corporation). After staining, PEC was subjected to fractional counts under a microscope with classification into eosinophils, neutrophils, lymphocytes/macrophages and basophils based on their form and stain nature (more than 500 PEC per specimen were randomly counted). Each cell ratio was as follows: 1.1% (eosinophil), 2.6% (neutrophil), 94.6% (lymphocyte/macrophage), and 1.7% (basophil) for unsensitized/physiological saline-administered mice; and 10.3% (eosinophil), 11.6% (neutrophil), 77.8% (lymphocyte/macrophage), and 0.3% (basophil) for sensitized/physiological saline-administered mice. Since basophil's occupation rate of PEC was extremely low and neutrophil's occupation rate of PEC scarcely varied, basophils and neutrophils were excluded from subjects to be examined. After counting, intraperitoneal exudation inhibition rates were calculated by the following formula and expressed as the therapeutic efficacies:

$$\text{Inhibition Rate (\%)} = \left[1 - \frac{\text{(PEC in Sensitized/Drug-Treated Mouse) - (PEC in Unsensitized/Saline-Treated Mouse)}}{\text{(PEC in Sensitized/Saline-Treated Mouse) - (PEC in Unsensitized/Saline-Treated Mouse)}}\right] \times 100$$

These results clearly show that when Compound No. 130 was administered it potently inhibited infiltration of eosinophils and lymphocytes/macrophages into the peritorieal cavity in response to challenge with ragweed pollen extracts (i.e., 57.2% and 219.0% inhibition, respectively). It is also apparent that it contributed a decrease in total PEC number (143.8%).

Example 25

Efficacy (2) on Allergic Peritonitis

A physiological saline solution containing ovalbumin (0.25 mg/mL) and an aluminum hydroxide gel (4 mg/mL) was administered subcutaneously to male Balb/c mice (7 weeks old, 20 to 23 g, purchased from JAPAN SLC, Inc.) twice at one week interval at a single dose of 0.4 mL for sensitization. On Day 14 after the initial sensitization, an antigen solution for induction (2.5 μg/mL) was administered intraperitoneally to each animal at a dose of 0.4 mL to raise allergic peritonitis. Compound No. 130 (30 mg/kg) or a negative control solvent, physiological saline (10 mL/kg), was administered intraperitoneally to each animal 5 minutes prior to induction, 3 and 6 hours post-induction.
Similarly to Example 24, the peritoneal cavities were lavaged to retrieve PEC. The drained liquids were measured for their yield and total PEC number therein. In the same manner as in Example 24, PEC was stained and subjected to fractional counting under a microscope based on their form. Each cell ratio was as follows: 1.5% (eosinophil), 1.1% (neutrophil), 97.3% (lymphocyte/macrophage), and 0.1% (basophil) for unsensitized/physiological saline-administered mice; and 24.3% (eosinophil), 7.4% (neutrophil), 68.2% (lymphocyte/macrophage), and 0.1% (basophil) for sensitized/physiological saline-administered mice. Similarly to Example 24, basophils and neutrophils were excluded from subjects to be examined. In the same manner as in Example 24, intraperitoneal exudation inhibition rates were calculated and expressed as the therapeutic efficacies.
It is clarified that Compound No. 130 markedly inhibits infiltration of eosinophils and lymphocytes/macrophages into the peritoneal cavity in response to ovalbumin challenge (56.3% and 74.9%, respectively). As a result, it intensively reduces total PEC numbers (70.7%).

Example 26

Efficacy (3) on Allergic Peritonitis

In the same manner as in Example 25, male Balb/c mice were sensitized with ovalbumin and an aluminum hydroxide gel, and, on Day 14 after the initial sensitization, treated with an antigen solution to raise allergic peritonitis. Compound No. 130 (30 mg/kg) or a negative control solvent, physiological saline (10 mL/kg), was administered subcutaneously to each animal 6 hours and 5 minutes prior to induction, respectively.
Similarly to Example 24, the peritoneal cavities were lavaged to retrieve PEC 24 hours after induction. The drained liquids were measured for their yield and total PEC number therein. In the same manner as in Example 24, PEC was stained and subjected to fractional counting under a microscope based on their form. Each cell ratio was as follows: 1.2% (eosinophil), 0.6% (neutrophil), 97.2% (lymphocyte/macrophage), and 1.0% (basophil) for unsensitized/physiological saline-administered mice; and 21.9% (eosinophil), 0.9% (neutrophil), 76.8% (lymphocyte/macrophage), and 0.4% (basophil) for sensitized/physiological saline-administered mice. Similarly to Example 24, basophils and neutrophils were excluded from subjects to be examined. In the same manner as in Example 24, intraperitoneal exudation inhibition rates were calculated and expressed as the therapeutic efficacies.
It is clarified that Compound No. 130 markedly inhibits infiltration of eosinophils and lymphocytes/macrophages into the peritoneal cavity in response to ovalbumin challenge (38.6% and 91.2%, respectively). As a result, it intensively reduces total PEC numbers (68.5%).
It is suggested by a line of the results obtained in Examples 24, 25 and 26 that the efficacies of Compound No. 130 are equivalent and significant even if the compound is administered topically to the inflammatory site (intraperitoneal route) or subcutaneously to tissues different from the inflammatory regions.

Example 27

Efficacy (4) on Allergic Peritonitis

In the same manner as in Example 25, mice were actively sensitized with ovalbumin and Alum, and, on Day 14 after the initial sensitization, elicited with an inducing antigen solution. A test compound of the present invention (Compound Nos. 114, 125, 129, and 132, respectively: 30 mg/kg) or a negative control solvent, physiological saline (10 mL/kg), was administered subcutaneously to each animal 6, 3 hours, and 5 minutes pre-induction, 3, and 6 hours post-induction. At 24 hours post-induction, a physiological saline (containing heparin) was injected intraperitoneally at 4 mL/mouse for peritoneal lavage. An aliquot of the retrieved washout medium was taken, and not only measured for a PEC density but also stained for PEC followed by fractional counting. Each effect of the compound on the total PEC number (PEC density× 4 mL), and eosinophils and lymphocytes/macrophages is shown in Table 12. Almost similarly to Examples 24 to 26, the total PEC number and the numbers of eosinophils and lymphocytes/macrophages in the peritoneal cavity are reduced.

TABLE 12

| Efficacy on Allergic Peritonitis in Model Animals | | | |
|---|---|---|---|
| | Leukocyte exudate inhibition rate (%) | | |
| | Total PEC Number | Eosinophils | Macrophages/Lymphocytes |
| Compound No. 114 | 33.4 | 37.5 | 29.0 |
| Compound No. 125 | 49.6 | 38.9 | 55.1 |
| Compound No. 129 | 35.1 | 30.2 | 37.7 |
| Compound No. 132 | 31.6 | 24.3 | 34.0 |

Example 28

Efficacy (5) on Allergic Peritonitis

Each efficacy on intraperitoneal exudation of neutrophils was examined according to the methods as disclosed in Ouchi et al., "Seibutsu Yakukagaku Jikken Koza, Ensho To Allergy (Inflammation and Allergy) I-3", pp.139 to 151, Hirokawa Publishing Co., Japan and Spicer et al., The International Archives of Allergy and Applied Immunology, 81: 81 to 84, 1986.

In the same manner as in Example 25, male Balb/c mice were sensitized with ovalbumin and an aluminum hydroxide gel, and, on Day 14 after the initial sensitization, elicited with an antigen solution to raise allergic peritonitis. Each test compound of the present invention (Compound Nos. 122, 125, and 129: 30 mg/kg) or a negative control solvent, physiological saline (10 mL/kg), was administered subcutaneously to each animal 1 hour prior to induction, and 3 hours post-induction. At 6 hours post-induction, peritoneal lavage was done similarly to Example 27, measured for a PEC density, and counted for the total PEC number. In the same manner as in Example 24, PEC was stained, and subjected to fractional counting under a microscope with classification into neutrophils, eosinophils, lymphocytes/macrophages and basophils based on their form and stain nature. Each cell ratio was as follows: 2.3% (neutrophil), 1.1% (eosinophil), 95.4% (lymphocyte/macrophage), and 1.2% (basophil) for unsensitized/physiological saline-administered mice; and 5.7% (neutrophil), 11.9% (eosinophil), 81.3% (lymphocyte/macrophage), and 1.1% (basophil) for sensitized/physiological saline-administered mice. By a similar line of reasoning in Example 24, basophils were excluded from subjects to be examined. In the same manner as in Example 24, intraperitoneal exudation inhibition rates were calculated and expressed as the therapeutic efficacies.

These results are summarized in Table 13. The data indicate that the compounds of the present invention (Compound Nos. 122, 125, and 129) not only potently inhibit infiltration of eosinophils; etc. but also markedly inhibit infiltration of neutrophils into the peritoneal cavity in response to ovalbumin challenge. As a result, it intensively reduces total PEC numbers.

TABLE 13

| Efficacy on Allergic Peritonitis in Model Animals | | | |
|---|---|---|---|
| | Leukocyte exudate inhibition rate (%) | | |
| | Total PEC Number | Eosinophils | Neutrophils |
| Compound No. 122 | 38.6 | - | 27.1 |
| Compound No. 125 | 40.9 | 38.4 | 41.4 |
| Compound No. 129 | 109.1 | 39.9 | 125.7 |

Example 29

Efficacy on Allergic Inflammation Model (Air Pouch Model)

Compound No. 130 was dissolved in physiological saline to make the final concentration 2.5 mg/mL (equal to 10 mg/kg) or 7.5 mg/mL (equal to 30 mg/kg) and administered into an air pouch of animals or intraperitoneally at a dose of 4 mL/kg. Dexamethasone was also dissolved in physiological saline to make the final concentration 25 μg/mL (equal to 0.1 mg/kg) and administered into an air pouch of animals at a dose of 4 mL/kg. Administration into such an air pouch was conducted simultaneously with the second challenge while the intraperitoneal administration was 30 minutes prior to the second challenge. Acetylated bovine serum albumin diazo-coupled with arsanilic acid (ABA-AcBSA) was prepared according to the Ouchi et al. and Tabachnick et al. methods as disclosed in Ouchi et al., "Seibutsu Yakukagaku Jikken Koza, Ensho To Allergy (Inflammation and Allergy) I-2", pp.194 to 206, Hirokawa Publishing Co., Japan and Tabachnick et al., The Journal of Biological Chemistry, 234: 1726- 1730, 1959, and mixed with a Freund's complete adjuvant (Difco) at an equal ratio. The mixture was treated using a connection-type glass syringe to form an emulsion which was used as a sensitizing solution. A 2% aqueous carboxylmethyl cellulose (CMC) containing 0.5 mg/mL ABA-AcBSA, 52 μg/mL polymyxin B sulfate (Sigma), 0.1 mg/mL streptomycin sulfate (Meiji Seika Kaisha, Ltd.) and 0.1 mg/mL penicillin G potassium (Meiji Seika Kaisha, Ltd.) was used as an initial challenge solution. A physiological saline containing 3 mg/mL ABA-AcBSA, 52 μg/mL polymyxin B sulfate, 0.1 mg/mL streptomycin sulfate and 0.1 mg/mL penicillin G potassium was used as a second challenge solution. Hair-shaved male rats (Crj: Sprague-Dawley line IGS, 5 weeks old) were treated by injecting intradermally the sensitizing solution at a dose of 0.1 mL (equivalent to 5mg of ABA-AcBSA) with a 1/4 subcutaneous needle into 5 different sites of the dorsal skin (2 sites for shoulder and 3 sites for lumbus). Nine days after the sensitization, 10 mL of air was subcutaneously injected with a 1/4 subcutaneous needle into the dorsal skin to form an oval air pouch. One day later, 4 mL of the initial challenge solution was injected with a 1/1 subcutaneous needle to raise allergic inflammation. Five days after the intial challenge, 1 mL of the second challenge solution was injected with a 1/3 subcutaneous needle to raise recrudescent inflammation. Eight hours after the second challenge, the rats were sacrificed by exsanguination to retrieve exudates in the air pouch. The retrieved exudates were measured by a dye-exclusion method using a haematocytometer and the number of exudated leukocytes were counted.

FIG. 14 show the results, demonstrating that infiltration of leukocytes into an air pouch was significantly inhibited via either direct inoculation into the air pouch or subcutaneous administration of Compound No. 130 (statistical analysis by a multi-comparison test:∗, significant with less than 5% risk; and ∗∗, significant with less than 1% risk). Further, the efficacy thereof is approximately equivalent to that of dexamethasone.

Example 30

Efficacy on Allergic Rhinitis

Guinea pigs (male, Std:Hartley, 3 weeks old) were housed and fed in a aluminum cage at a rate of up to 5 animals per cage. After acclimatization for 1 week, the guinea pigs were administered intravenously with an anti-ovalbumin antibody (Nihon Bioresearch Inc., Hajima Institute; antibody titer by a 3-hour homologous passive cutaneous anaphylaxis reaction, 1 : 8192) prepared according to the Orange & Moore method (Orange, R. P. and Moore, E. G., The Journal of Immunology 116: p.392-397, 1976) into their auricle vein at a dose of 0.3 mL/animal for passive sensitization. Twenty four to twenty six hours after sensitization under conditions of fast and free access to drinking water, the sensitized guinea pigs were anesthetized with urethane (1.2 g/kg, intraperitoneal administration), fixed in a supine position, and mediad excised cervically to expose tracheas. A polyethylene tube (No. 8, Hibiki) was inserted from the

excised portion of trachea to the nasal cavity side followed by ligation. A cotton ball impregnated with glycerin was packed into the oral cavity so as not to leak air from the nasal cavity to the oral cavity. To maintain breathing a polyethylene cannula (No. 8, Hibiki) was inserted at the pulmonary side and the polyethylene cannula inserted at the nasal cavity side was installed with a Y-type cannula connected to a three way cock. Animals were ventilated with 5 ml of air per ventilation at a rate of 60 beats/min via a Harvard Rodent Respirator (Model 683, Harvard) from an end of the Y-type cannula. A ventilatory overflow from another end of the Y-type cannula under isobaric loads (10 cm $H_2O$) was guided as an index for nasal airway resistance to a bronchospasm transducer (7020, Ugo basile). Variations of the ventilatory overflow were quantitatively recorded on a recticoder (RECTI-HORIZ-8K, Nippon Denki Sanei K.K.) for 20 minutes after antigen-challenge.

Wave height (cm) was measured on recorded papers and a nasal airway resistance value was calculated according to the following formula:

$$\text{Percent Increase (\%) in Nasal Airway Resistance} = \frac{\text{(Observed Value) - (Value prior to Administration)}}{\text{(Complete Obstruction)- (Value prior to Administration)}} \times 100$$

Compound No. 130 was dissolved in water for injection and injected subcutaneously one hour prior to rhinitis-induction: 15 mg/kg and 5 mL/kg (N=5). A solvent alone was administered to a control group (N=10).

The results (average± standard deviation) are shown in FIG. 15. It is apparent that the administration of Compound No. 130 markedly ameliorates an increase in nasal airway resistance due to antigen challenge.

Formulation Example 1

An ointment containing the following ingredients was prepared according to conventional techniques:

| Ingredients | Amount |
|---|---|
| White Petrolatum | 97 g |
| Liquid Paraffin | 2 g |
| Compound No. 132 | 1.0 g |
| Total Amount | 100 g |

Formulation Example 2

An ointment containing the following ingredients was prepared according to conventional techniques:

| Ingredients | Amount |
|---|---|
| White Petrolatum | 97.7 g |
| Purified Lanolin | 2 g |
| Compound No. 125 | 0.3 g |
| Total Amount | 100 g |

Formulation Example 3

An ointment containing the following ingredients was prepared according to conventional techniques:

| Ingredients | Amount |
|---|---|
| White Petrolatum | 97.9 g |
| Purified Lanolin | 2 g |
| Compound No. 130 | 0.1 g |
| Total Amount | 100 g |

Formulation Example 4

An ointment containing the following ingredients was prepared according to conventional techniques:

| Ingredients | Amount |
|---|---|
| White Petrolatum | 99.9 g |
| Compound No. 114 | 0.1 g |
| Total Amount | 100 g |

Formulation Example 5

An eyedrop containing the following ingredients was prepared according to conventional techniques:

| Ingredients | Amount |
|---|---|
| Compound No. 129 | 0.1 g |
| Sodium Chloride | 0.33 g |
| Sterile Purified Water | qs |
| Total Volume | 100 ml |

Formulation Example 6

An eyedrop containing the following ingredients was prepared according to conventional techniques:

| Ingredients | Amount |
|---|---|
| Compound No. 114 | 0.05 g |
| Sodium Chloride | 0.26 g |
| Sodium Dihydrogenphosphate anhydrous | 0.56 g |
| Disodium Phosphate anhydrous | 0.28 g |
| 0.002% Aqueous Benzalkonium Chloride | qs |
| Total Volume | 100 ml |

Formulation Example 7

An eyedrop containing the following ingredients was prepared according to conventional techniques:

| Ingredients | Amount |
|---|---|
| Compound No. 122 | 0.3 g |
| Sodium Chloride | 0.33 g |
| Sodium Sulfite Anhydrous | 0.10 g |
| Sterile Purified Water | qs |
| Total Volume | 100 ml |

Formulation Example 8

A preparation for injection containing the following ingredients was formulated according to conventional techniques:

| Ingredients | Amount |
|---|---|
| Compound No. 114 | 2.0 g |
| Distilled Water for Injection | qs |
| Total Volume | 100 ml |

Formulation Example 9

A preparation for injection containing the following ingredients was formulated according to conventional techniques:

| Ingredients | Amount |
|---|---|
| Compound No. 125 | 2.0 g |
| Sodium Chloride | 0.9 g |
| Distilled Water for Injection | qs |
| Total Volume | 100 ml |

Formulation Example 10

Preparation for Solutions

A formula for a single dose is:

| Ingredients | Amount |
|---|---|
| Compound No. 129 | 1.0 g |

(continued)

| Ingredients | Amount |
|---|---|
| Human Serum Albumin | 0.05 g |
| Lactose | 1 g |
| Physiological Saline for Injection | qs |
| Total Volume | 50 ml |

All the ingredients are mixed and dissolved to give a preparation for injection.

Formulation Example 11

Preparation for Ointments

A variety of ointment bases conventionally known and used in the art can be used in preparations for ointments.

For instance, Compound No. 114 can be formulated in admixture with a macrogol ointment according to the following processes:

To the macrogol ointment which is prepared by admixing macrogol 400 with macrogol 4000 equivalently is added ethyl p-hydroxybenzoate (5 mg) and Compound No. 114 (10 mg) to make the total amount 10 g. The mixture was blended thoroughly according to conventional techniques to provide an ointment.

Formulation Example 12

Preparation for Solutions

To a 0.005% benzalkonium chloride-containing physiological saline solution (0.15 M aqueous NaCl containing 0.005% benzalkonium chloride, pH 7.2) is added Compound No. 129 to make its final concentration 1.0%. The resultant solution is sterilized through a membrane filter with a pore size of 0.1 µm and refrigerated (4°C) until use.

Formulation Example 13

Preparation for Tablets

Tablets each containing Compound No. 44 (30.0 mg) are formulated according to conventional techniques. A formula for tablets is:

| Ingredients | Amount |
|---|---|
| Compound No. 44 | 30.0 mg |
| Hydroxypropyl Cellulose | 5.0 mg |
| Magnesium Stearate | 0.5 mg |
| Corn Starch | 24.5 mg |
| Lactose | qs |
| Total Amount | 150.0 mg |

Formulation Example 14

Preparation for Tablets

Tablets each containing Compound No. 114 (50.0 mg) are formulated according to conventional techniques.

A formula for tablets is:

| Ingredients | Amount |
|---|---|
| Compound No. 114 | 50.0 mg |
| Fine-powdered Cellulose | 25.0 mg |
| Corn Starch | 40.0 mg |
| Talc | 5.0 mg |
| Magnesium Stearate | 0.5 mg |
| Lactose | qs |
| Total Amount | 170.0 mg |

Formulation Example 15

Preparation for Inhalant Powders

Compound No. 129 (1.5 g) and lactose (60 g) are pulverized to form fine powders, respectively, and blended thoroughly under stirring to afford a homogeneous mixture as a powder. The resultant mixture is packed into capsules so that each capsule may contain 500 μg of Compound No. 129. Drugs are inhaled by using an insufflator wherein the capsule is set prior to use.

Formulation Example 16

Preparation for Inhalant Powders

A powder is formulated by pulverizing each ingredient to form a fine powder and then blending all the resulting powders thoroughly under stirring to afford a homogeneous mixture.
A formula for inhalant powders is:

| Ingredients | Amount |
|---|---|
| Compound No. 114 | 2.0 g |
| Starch | 30.0 g |
| Talc | 5.0 g |
| Magnesium Stearate | 1.0 g |
| Lactose | qs |
| Total Amount | 100.0 g |

PREPARATION EXAMPLES

In the examples (including the preparation examples, etc.) as well as elsewhere in the specification, the following abbreviations are intended to have the meanings set forth below.

DMF; N,N-dimethylformamide
EDC; 1-(3-dimethylaminopropyl)-3-ethylcarbodiimide hydrochloride
HOBT; 1-hydroxybenzotriazole
TEA; triethylamine
THF; tetrahydrofuran

Et; ethyl
Boc; tert-butyloxycarbonyl
Bn; benzyl
Me; methyl
Cl-Z; 2-chlorobenzyloxycarbonyl
Z; benzyloxycarbonyl
tBu; tert-butyl
Tce; 2,2,2-trichloroethyl
Na; sodium

Preparation Example 1

$N^a$-tert-Butyloxycarbonyl-$N^e$-benzyloxycarbonyl-L-lysine N-methylamide (Compound No. 1)

To a solution of $N^a$-tert-butyloxycarbonyl-$N^e$-benzyloxycarbonyl-L-lysine (10.0 g, 26.3 mmol) in DMF (50 ml) was added aqueous methylamine hydrochloride (2.13 g, 31.5 mmol/ 5 ml), HOBT (3.55 g, 26.3 mmol), EDC (6.04 g, 31.5 mmol), and TEA (4.39 ml, 31.5 mmol) sequentially with stirring at -15°C. The mixture was stirred for 1 hour at -15°C and for another 15 hours at room temperature, and evaporated under reduced pressure. AcOEt (200 ml) was added to the residue and the mixture was partitioned and washed successively with saturated aqueous sodium chloride, 1N hydrochloric acid, saturated aqueous $NaHCO_3$, and saturated aqueous sodium chloride (twice for each). The organic layer was dried over anhydrous $MgSO_4$, and evaporated under reduced pressure to give the title compound as a white solid (8.20 g, 20.8 mmol, yield 80%).

$^1$H-NMR ($CDCl_3$)δ ppm; 1.3-1.9 (15H, s+m, C$\underline{(CH_3)_3}$ + CH$\underline{(CH_2)_3}$), 2.73 (3H, d, J=4.7 Hz, NH-$\underline{CH_3}$), 3.30 (2H, m, -OC(=O)NH-$\underline{CH_2}$), 4.12 (1H, m, NH-$\underline{CH}$-CO), 5.08 (2H, s, O-$\underline{CH_2}$-Ph), 5.60 (1H, m, NH), 6.61 (1H, m, NH), 7.2-7.5 (5H, m, aromatic-H).

Preparation Example 2

$N^a$-tert-Butyloxycarbonyl-L-4'-cyanophenylalanine N-(morpholin-4-yl)amide (Compound No. 2)

The procedure of Preparation Example 1 except omitting the partition and washing with 1N hydrochloric acid was repeated using $N^a$-tert-butyloxycarbonyl-L-4'-cyanophenylalanine and 4-aminomorpholine to provide the title compound as a white solid (yield 50%), m.p.; 181-185°C, Rf value; 0.25 (chloroform:methanol= 20:1).

Preparation Example 3

$N^a$-Benzyloxycarbonyl-$N^e$-tert-butyloxycarbonyl-L-lysine N-(2-N',N'-dimethylaminoethyl)amide (Compound No. 3)

The procedure of Preparation Example 2 was repeated using $N^a$-benzyloxycarbonyl-$N^e$-tert-butyloxycarbonyl-L-lysine and N,N-dimethylethylenediamine to provide the title compound as a white solid (yield 50%), m.p.; 61°C, Rf value; 0.26 (chloroform:methanol= 10:1).

Preparation Example 4

$N^a$-Benzyloxycarbonyl-$N^e$-tert-butyloxycarbonyl-L-lysine N-(piperidin-1-yl)amide (Compound No. 4)

The procedure of Preparation Example 2 was repeated using $N^a$-benzyloxycarbonyl-$N^e$-tert-butyloxycarbonyl-L-lysine and 1-aminopiperidine to provide the title compound as a white solid (yield 83%), m.p.; 128-131°C, Rf value; 0.17 (chloroform;methanol= 20:1).

Preparation Example 5

$N^a$-tert-Butyloxycarbonyl-L-4'-cyanophenylalanine N-methylamide (Compound No. 5)

The procedure of Preparation Example 1 was repeated using $N^a$-tert-butyloxycarbonyl-L-4'-cyanophenylalanine and methylamine hydrochloride to provide the title compound as a white solid (yield 84%), m.p.; 165-167°C, Rf value; 0.55 (chloroform: methanol= 10:1).

Preparation Example 6

$N^a$-Benzyloxycarbony-$N^e$-tert-butyloxycarbonyl-L-lysine N-(2-hydroxy-1(RS)-methylethyl)amide (Compound No. 6)

The procedure of Preparation Example 1 was repeated using $N^a$-benzyloxycarbonyl-$N^e$-tert-butyloxycarbonyl-L-lysine and DL-2-amino-1-propanol to provide the title compound as a white solid (yield 87%), m.p.; 115-116°C, Rf value; 0.096 (chloroform:methanol= 20:1).

Preparation Example 7

$N^a$-tert-Butyloxycarbonyl-$N^d$-benzyloxycarbony-L-ornithine N-methylamide (Compound No. 7)

The procedure of Preparation Example 1 was repeated using $N^a$-tert-butyloxycarbonyl-$N^d$-benzyloxycarbonyl-L-ornithine and methylamine hydrochloride to provide the title compound as a white solid (yield 92%), m.p.; 141-143°C, Rf value; 0.22 (chloroform:methanol= 20:1).

Preparation Example 8

$N^a$-tert-Butyloxycarbonyl-$N^d$-benzyloxycarbonyl-L-ornithine N-cyclopropylamide (Compound No. 8)

The procedure of Preparation Example 1 was repeated using $N^a$-tert-butyloxycarbonyl-$N^d$-benzyloxycarbonyl-L-ornithine and cyclopropylamine to provide the title compound as a white solid (yield 95%), m.p.; 146-147°C, Rf value; 0.27 (chloroform:methanol 20:1).

Preparation Example 9

$N^a$-tert-Butyloxycarbonyl-$N^g$,$N^g$-dibenzyloxycarbonyl-L-arginine N-methylamide (Compound No. 9)

The procedure of Preparation Example 1 was repeated using $N^a$-tert-butyloxycarbonyl-$N^g$,$N^g$-dibenzyloxycarbonyl-L-arginine and methylamine to provide the title compound as a white solid (yield 96%), m.p.; 154-156°C, Rf value; 0.35 (chloroform: methanol= 20:1).

Preparation Example 10

2(S)-tert-Butyloxycarbonylamino-4-$N^1$,$N^2$-dibenzyloxycarbonylguanidinobutanoic acid N-methylamide (Compound No. 10)

To a solution of 4-amino-2(S)-tert-butyloxycarbonylaminobutanoic acid N-methylamide (15.2 g, 65.7 mmol) in DMF (300 ml) was added TEA (9.16 ml, 65.7 mmol) and 1H-pyrazole-N,N'-bis(benzyloxycarbonyl)carboxamidine (29.8 g, 78.8 mmol), and the mixture was stirred for 15 hours at room temperature. AcOEt (1000 ml) was added to the reaction mixture which was then partitioned and washed successively with saturated aqueous sodium chloride, 1N hydrochloric acid, saturated aqueous $NaHCO_3$, and saturated aqueous sodium chloride (twice for each). The organic layer was dried over anhydrous $MgSO_4$, and evaporated under reduced pressure. The resultant reaction mixture was purified by column chromatography (silica gel; 800 g, eluted with a mixture of benzene:AcOEt= 5:1 to 3:2) to give the title compound as a white solid (17.2 g, 48%), m.p.; 128°C, Rf value; 0.68 (chloroform:methanol= 10:1).

Preparation Example 11

$N^a$-Benzyloxycarbonyl-L-4'-[tetraethyl bis(phosphonyl)methylimino]phenylalanine N-methylamide (Compound No. 11)

A mixture of $N^a$-benzyloxycarbonyl-L-4'-aminophenylalanine N-methylamide (3.00 g, 9.16 mmol), ethyl orthoformate (1.83 ml, 11.0 mmol) and diethyl phosphite (4.72 ml, 36.6 mmol) was stirred for 2 hours at 140 to 160°C. The unreacted diethyl phosphite and EtOH produced during the reaction were removed by evaporation under reduced pressure and the reaction mixture was purified by column chromatography (silica gel; 500 g, eluted with a mixture of $CH_2Cl_2$:MeOH= 40:1 to 20:1) to give the title compound as a pale yellow solid (3.97 g, 71% ), m.p.; 126-128°C, Rf value; 0.44 (chloroform:methanol= 10:1).

Preparation Example 12

$N^a$-Benzyloxycarbonyl-L-4'-[tetramethyl bis(phosphonyl)methylimino]phenylalanine N-methylamide (Compound No. 12)

The procedure of Preparation Example 11 was repeated using $N^a$-benzyloxycarbonyl-L-4'-aminophenylalanine N-methylamide (115 g, 350 mmol), ethyl orthoformate (78 g, 530 mmol) and dimethyl phosphite (154 g, 1.40 mol) to provide the title compound as a pale yellow solid (157 g, 80% ), m.p.; 150-153°C, Rf value; 0.41 (chloroform:methanol= 10:1).

Preparation Example 13

$N^a$-tert-Butyloxycarbonyl-$N^e$-2-chlorobenzyloxycarbonyl-L-lysine N-methylamide (Compound No. 13)

The procedure of Preparation Example 1 was repeated using $N^a$-tert-butyloxycarbonyl-$N^e$-2-chlorobenzyloxycarbonyl-L-lysine and methylamine hydrochloride to provide the title compound as a white solid (yield 94%), m.p.; 109-110°C, Rf value; 0.48 (chloroform:methanol= 10:1).

Preparation Example 14

$N^a$-tert-Butyloxycarbonyl-L-4'-cyanophenylalanine N-(2-benzyloxyethyl)amide (Compound No. 14)

The procedure of Preparation Example 1 was repeated using $N^a$-tert-butyloxycarbonyl-L-4'-cyanophenylalanine and O-benzylaminoethanol to provide the title compound as a white solid (yield 90%), m.p.; 99-102°C, Rf value; 0.55 (chloroform: methanol= 20:1).

Preparation Example 15

$N^a$-tert-Butyloxycarbonyl-$N^e$-2-chlorobenzyloxycarbonyl-L-lysine N-(2-benzyloxyethyl)amide (Compound No. 15)

The procedure of Preparation Example 1 was repeated using $N^a$-tert-butyloxycarbonyl-$N^e$-2-chlorobenzyloxycarbonyl-L-lysine and O-benzylaminoethanol to provide the title compound as a white solid (yield 99%), m.p.; 69-71°C, Rf value; 0.60 (chloroform:methanol= 10:1).

Preparation Example 16

3(RS)-tert-Butyloxycarbonyl-6-phenyl-2(R)-isobutylhexanoic acid (Compound No. 16)

a) Benzyl 2(R)-bromo-4-methylpentanoate (Compound No. 16-a)

To a solution of 2(R)-bromo-4-methylpentanoic acid (28.5 g, 146 mmol), benzyl alcohol (18.1 ml, 175 mmol), and 4-dimethylaminopyridine (1.90 g, 14.6 mmol) in dichloromethane (140 ml) was added EDC (35.6 g, 175 mmol) with stirring while ice cooling. The mixture was stirred for 1 hour while ice cooling, and further overnight at room temperature. The resultant mixture was partitioned and washed successively with water, saturated aqueous sodium bicarbonate, and saturated aqueous sodium chloride (twice for each). The organic layer was dried over anhydrous magnesium sulfate, evaporated under reduced pressure, and purified by column chromatography (silica gel; 700 g, eluted with a mixture of n-hexane:AcOEt = 40:1) to give the title compound as a colorless oil (32.0 g, 77%), specific rotation $[\alpha]_D$ = +31.8° (c=1.0, MeOH), Rf value; 0.48 (AcOEt:n-hexane= 1:5).

b) Dibenzyl 3(RS)-tert-butyloxycarbonyl-2(R)-isobutylsuccinate (Compound No. 16-b)

To a solution of benzyl tert-butylmalonate (24.9 g, 99.6 mmol) in DMF (60 ml) was added potassium tert-butoxide (13.4 g, 120 mmol) portionwise with stirring at 0°C. The mixture was stirred for 1 hour at room temperature and re-cooled to 0°C. A solution of Compound No. 16-a (28.4 g, 99.6 mmol) in DMF (60 ml) was added dropwise to the cooled mixture over a period of 1 hour. After stirring for 15 hours at 5°C, AcOEt (2 L) was added to the reaction mixture which was then partitioned and washed successively with saturated aqueous sodium chloride, 1N hydrochloric acid, saturated aqueous sodium bicarbonate, and saturated aqueous sodium chloride (twice for each). The organic layer was dried over anhydrous magnesium sulfate, evaporated under reduced pressure, and purified by column chromatography

(silica gel; 750 g, eluted with a mixture of n-hexane:AcOEt= 20:1) to give the title compound as a colorless oil (40.0 g, 89%), specific rotation $[\alpha]_D$ = +16.7° (c=1.0, MeOH), Rf value; 0.56 (AcOEt:n-hexane= 1:5).

c) Dibenzyl 3(RS)-tert-butyloxycarbonyl-3-cinnamyl-2(R)-isobutylsuccinate (Compound No. 16-c)

To a solution of Compound No. 16-b (9.49 g, 20.9 mmol) in DMF (100 ml) was added 60% sodium hydride (1.0 g, 25.1 mmol) portionwise with stirring at room temperature. The mixture was stirred for 2 hours at room temperature and cooled to 0°C. Cinnamyl bromide (5.35 g, 27.2 mmol) was added portionwise to the cooled mixture which was then stirred for 15 hours at 5°C. The solvent was evaporated under reduced pressure, and AcOEt (500 ml) was added to the residue. The mixture was partitioned and washed successively with saturated aqueous sodium chloride, 1N hydrochloric acid, saturated aqueous sodium bicarbonate, and saturated aqueous sodium chloride (twice for each). The organic layer was dried over anhydrous magnesium sulfate, evaporated under reduced pressure, and purified by column chromatography (silica gel; 700 g, eluted with a mixture of n-hexane:AcOEt= 20:1) to give the title compound as a colorless oil (10.9 g, 91%), Rf value; 0.34 (AcOEt:n-hexane = 1:9).

d) 3(RS)-tert-Butyloxycarbonyl-6-phenyl-2(R)-isobutylhexanoic acid (Compound No. 16)

To a solution of Compound No. 16-c (4.2 g, 7.36 mmol) in ethanol (35 ml) was added 10% palladium on carbon (50% wet catalyst, 1.3 g), and the mixture was vigorously stirred under hydrogen atmosphere for 7 hours at room temperature. The catalyst was filtered off and then ethanol was evaporated under reduced pressure. To the residue was added N-methylmorpholine (0.81 ml, 7.36 mmol) and toluene (50 ml) and the mixture was refluxed for 2 hours. The reaction mixture was partitioned and washed successively with 1N hydrochloric acid, and saturated aqueous sodium chloride (twice for each), dried over anhydrous magnesium sulfate, evaporated under reduced pressure, and purified by column chromatography (silica gel; 150 g, eluted with a mixture of chloroform:methanol= 200:1) to give the title compound as a colorless oil (1.1 g, 43%), Rf value; 0.60 (chloroform:methanol:acetic acid= 95:5:3).

$^1$H-NMR ($CDCl_3$)δ ppm; 0.88 (6H, bd, J= 5.7Hz, CH($\underline{CH_3}$)$_2$), 1.0-2.0 (16H, m, $(CH_3)_2\underline{CH\text{-}CH_2}$, + C($\underline{CH_3}$)$_3$ + $\underline{CH_2}$-$\underline{CH_2}$-$CH_2$-Ph), 2.4-2.8 (4H, m, $\underline{CH}$-CO x 2 + $\underline{CH_2}$-Ph), 7.0-7.4 (5H, m, aromatic-H).

Preparation Example 17

3(RS)-tert-Butyloxycarbonyl-5-methyl-2(R)-isobutylhexanoic acid (Compound No. 17)

The procedures of Preparation Examples 16-c and d were repeated using Compound No. 16-b and methallyl iodide to provide the title compound as a pale yellow oil (overall yield 52%), Rf value; 0.23 (AcOEt:n-hexane= 1:4).

$^1$H-NMR ($CDCl_3$)δ ppm; 0.90 (12H, m, CH($\underline{CH_3}$)$_2$ x 2), 1.0-1.3 (2H, m, $\underline{CH}(CH_3)_2$ x 2), 1.4-1.8 (13H,S + m, $\underline{CH_2}$-$CH(CH_3)_2$ x 2 + C($\underline{CH_3}$)$_3$), 2.5-2.7 (2H, m, $\underline{CH}$-CO x 2).

Preparation Example 18

2(R)-[1(RS)-(tert-Butyloxycarbonyl)ethyl]-4-methylpentanoic acid (Compound No. 18)

The procedures of Preparation Examples 16-c and d were repeated using Compound No. 16-b and methyl iodide to provide the title compound as a pale yellow oil (overall yield 79%), Rf value; 0.28 (chloroform:methanol= 20:1).

$^1$H-NMR ($CDCl_3$)δ ppm; 0.91 (6H, m, CH($\underline{CH_3}$)$_2$), 1.1-1.3 (4H, m, $\underline{CH}(CH_3)_2$ + CO-CH-$\underline{CH_3}$), 1.4-1.8 (11H,S + m, $\underline{CH_2}$-$CH(CH_3)_2$ + C($\underline{CH_3}$)$_3$), 2.59 and 2.73 (2H, m, $\underline{CH}$-CO x 2).

Preparation Example 19

3(R)-Carboxy-5-methyl-2(RS)-(3-phenylpropyl)hexanoic acid-N-benzyloxy-N-benzyloxycarbonylamide (Compound No. 19)

a) 5-Methyl-2(RS)-(3-phenylpropyl)-3(R)-2,2,2-trichloroethyloxycarbonyl-hexanoic acid-N-benzyloxy-N-benzyloxycarbonylamide (Compound No. 19-a)

5-Methyl-2(RS)-(3-phenylpropyl)-3(R)-2,2,2-trichloroethyloxycarbonyl-hexanoic acid (1.00 g, 2.27 mmol)

was dissolved in $CH_2Cl_2$ (20 ml), and cooled to 0°C under nitrogen atmosphere. To the cooled solution was added DMF (5 drops), and oxalyl chloride (217 µl, 2.49 mmol) with a syringe and the mixture was stirred for 20 minutes at 0°C. The resultant solution was added to a solution of O-benzyl-N-benzyloxycarbonylhydroxylamine (640 mg, 2.49 mmol) and TEA (1.04 ml, 7.47 mmol) in $CH_2Cl_2$ (20 ml) dropwise under nitrogen atmosphere maintaining the temperature at 0°C. After stirring for 15 hours at room temperature, the reaction solution was partitioned and washed successively with 1N hydrochloric acid, saturated aqueous sodium bicarbonate, and saturated aqueous magnesium sulfate. The solvent was removed by evaporation under reduced pressure, and the residue was purified by column chromatography (silica gel; 40 g, eluted with a mixture of n-hexane:AcOEt= 10:1) to give the title compound as a colorless oil (1.1 g, 43%).

$^1$H-NMR ($CDCl_3$)δ ppm; 0.8-0.9 (6H, m, CH$\underline{(CH_3)_2}$), 1.1-1.3 (1H, m, $\underline{CH}(CH_3)_2$), 1.4-1.9 (6H, m, $\underline{CH_2}$-CH$(CH_3)_2$ + $\underline{CH_2}$-$\underline{CH_2}$-$CH_2$-Ph), 2.53 (2H, m, $CH_2$-$\underline{CH_2}$-Ph), 2.84 (1H, m, $\underline{CH}$-CO), 3.83 (1H, m, $\underline{CH}$-CO), 4.66 (2H, s, $\underline{CH_2}$-$CCl_3$), 4.88 (2H, s, O-$\underline{CH_2}$-Ph), 5.26 (2H, s, C(=O)O-$\underline{CH_2}$-Ph), 7.0-7.4 (15H, m, aromatic-H).

b) 3(R)-Carboxy-5-methyl-2(RS)-(3-phenylpropyl)hexanoic acid-N-benzyloxy-N-benzyloxycarbonylamide (Compound No. 19)

To a solution of Compound No. 19-a (800 mg, 1.21 mmol) in acetic acid (30 ml) was added Zn powders (2.80 g, 36.0mmol), and the mixture was stirred for 2.5 hours at room temperature. Zn powders were filtered off and then acetic acid was evaporated, followed by addition of AcOEt. The AcOEt solution was partitioned and washed successively with saturated aqueous sodium chloride, 1N hydrochloric acid, and saturated aqueous sodium chloride (twice for each), dried over anhydrous magnesium sulfate, evaporated under reduced pressure, and purified by column chromatography (silica gel; 50 g, eluted with a mixture of n-hexane:AcOEt= 5:1) to give the title compound as a colorless oil (430 mg, 68%), Rf value; 0.37 (AcOEt:n-hexane = 1 :2).

$^1$H-NMR ($CDCl_3$)δ ppm; 0.8-1.0 (6H, m, CH$\underline{(CH_3)_2}$), 1.1-1.3 (1H, m, $\underline{CH}(CH_3)_2$), 1.4-1.9 (6H, m, $\underline{CH_2}$-CH$(CH_3)_2$ + $\underline{CH_2}$-$\underline{CH_2}$-$CH_2$-Ph), 2.55 (2H, m, $CH_2$-$\underline{CH_2}$-Ph), 2.92 (1H, m, $\underline{CH}$-CO), 3.91 (1H, m, $\underline{CH}$-CO), 4.87 (2H, s, O-$\underline{CH_2}$-Ph), 5.26 (2H, s, C(=O)O-$\underline{CH_2}$-Ph), 7.0-7.4 (15H, m, aromatic-H).

<u>Preparation Example 20</u>

3(RS)-tert-Butoxycarbonyl-6-[4'-($N^1$,$N^2$-dibenzyloxycarbonylguanido)phenyl]-2(R)-isobutylhexanoic acid (Compound No. 20)

a) Dibenzyl 3(RS)-tert-butoxycarbonyl-3-(4'-nitro-cinnamyl)-2(R)-isobutylsuccinate (Compound No. 20-a)

The procedure of Preparation Example 16-c was repeated using Compound No. 16-b and 4-nitro-cinnamyl bromide to provide the title compound as a yellow oil (yield 71%).

b) 3(RS)-tert-Butoxycarbonyl-3-[3-(4'-aminophenyl)propyl]-2(R)-isobutylsuccinic acid (Compound No. 20-b)

To a solution of Compound No. 20-a (4.50 g, 7.31 mmol) in ethanol (100 ml) was added 5% palladium on carbon (50% wet catalyst, 2.5 g), and the mixture was vigorously stirred under hydrogen atmosphere for 2 hours at room temperature. The catalyst was filtered off and then ethanol was evaporated under reduced pressure to yield the title compound as a colorless oil (quantitative yield).

$^1$H-NMR ($CD_3OD$)δ ppm; 0.8-1.0 (6H, m, CH$\underline{(CH_3)_2}$), 1.1-2.0 (16H, m, $(CH_3)_2\underline{CH}$-$\underline{CH_2}$ + C$\underline{(CH_3)_3}$ + $\underline{CH_2}$-$\underline{CH_2}$-$CH_2$-$C_6H_4$), 2.49 (2H, m, $\underline{CH_2}$-$C_6H_4$), 2.7-3.5 (1H, m, $\underline{CH}$-CO), 6.7-7.1 (4H, m, aromatic-H).

c) 3(RS)-tert-Butoxycarbonyl-6-[4'-($N^1$,$N^2$-dibenzyloxycarbonylguanido)phenyl]-2(R)-isobutylhexanoic acid (Compound No. 20)

To a solution of Compound No. 20-b (2.98 g, 7.31 mmol) in DMF (30 ml) was added TEA (3.10 ml, 21.9 mmol) and 1H-pyrazole-N,N'-bis(benzyloxycarbonyl)carboxamidine (3.32 g, 8.77 mmol) and the mixture was stirred for 15 hours at 40°C. The reaction mixture was concentrated under reduced pressure, then dissolved in AcOEt (100 ml), partitioned and washed successively with 1N hydrochloric acid, and saturated aqueous sodium chloride (twice for each), dried over anhydrous magnesium sulfate, evaporated under reduced pressure, and purified by column chromatography (silica gel; 400 g, eluted with a mixture of $CHCl_3$:MeOH = 200:1) to give the title compound as a colorless oil (1.03 g, 21%).

$^1$H-NMR ($CDCl_3$)δ ppm; 0.8-1.0 (6H, m, CH($CH_3$)$_2$), 1.0-1.8 (16H, m, ($CH_3$)$_2$CH-$CH_2$ + C($CH_3$)$_3$ + $CH_2$-$CH_2$-$CH_2$-$C_6H_4$), 2.4-2.7 (4H, m, $CH_2$-$C_6H_4$ + CH-CO x 2), 5.1-5.3 (4H, m, O-$CH_2$-Ph x 2), 7.10 (2H, m, NH x 2), 7.2-7.5 (14H, m, aromatic-H).

Preparation Example 21

Methyl 3(R)-carboxy-2(S)-hydroxy-5-methylhexanoate (Compound No. 21)

Anhydrous trifluoroacetic acid (4 ml) was added to 3(R)-carboxy-2(S)-hydroxy-5-methylhexanoic acid (440 mg, 2.31 mmol) and the mixture was stirred for 4 hours at 0°C. The solvent was evaporated and methanol (4 ml) was added to the residue. The mixture was stirred for 2 hours at 0°C. After methanol was evaporated, the residue was purified by column chromatography (silica gel; 35 g, eluted with a mixture of chloroform:methanol= 20:1) to give the title compound as a colorless oil (344 mg, 73%).

$^1$H-NMR ($CDCl_3$)δ ppm; 0.94 (6H, d, J=5.0Hz, CH($CH_3$)$_2$), 1.3-2.0 (16H, m, ($CH_3$)$_2$CH-$CH_2$), 2.8-3.2 (1H, m, CH-$CO_2$H), 3.82 (3H, s, $OCH_3$), 4.29 (1H, d, J=3.5Hz, HO-CH), 6.6 (2H, brm, OH + $CO_2$H).

Preparation Example 22

N$^a$-[4-(Hydroxyamino)-2(R)-isobutyl-3(RS)-methylsuccinyl]-N$^e$-acetimidoyl-L-lysine N-methylamide • 1 acetate (Compound No. 22)

a) N$^e$-Benzyloxycarbony-L-lysine N-methylamide • 1 hydrochloride (Compound No. 22-a)

Compound No. 1 (8.20 g, 20.8 mmol) was dissolved in 4N HCl (AcOEt solution, 100 ml) under ice-cooling and the mixture was stirred for 45 minutes at the same temperature, and concentrated under reduced pressure. $Et_2O$ (100 ml) was added to the residue to yield precipitated crystals which were collected by filtration and dried under reduced pressure, giving the title compound as white crystals (quantitative yield).

$^1$H-NMR ($CD_3OD$)δ ppm; 1.3-1.8 (6H, m, CH($CH_2$)$_3$), 2.70 (3H, s, NH-$CH_3$), 3.27 (2H, m, -OC(=O)NH-$CH_2$), 4.25 (1H, m, NH-CH-CO), 5.05 (2H, s, O-$CH_2$-Ph), 7.2-7.5 (5H, m, aromatic-H).

b) N$^a$-[4-tert-Butoxy-2(R)-isobutyl-3(RS)-methylsuccinyl]-N$^e$-benzyloxycarbonyl-L-lysine N-methylamide (Compound No. 22-b)

To a solution of Compound No. 22-a (6.86 g, 20.8 mmol) in DMF (26 ml)-$CH_2Cl_2$ (60 ml) was added Compound No. 18 (4.60 g, 18.8 mmol), HOBT (3.40 g, 24.9 mmol), EDC (4.77 g, 24.9 mmol) and TEA (2.89 ml, 20.8 mmol) successively with stirring at -15°C, and the mixture was stirred for 1 hour at -15°C and for another 15 hours at room temperature. The solvent was evaporated under reduced pressure, and AcOEt (200 ml) was added to the residue. The mixture was partitioned and washed successively with saturated aqueous sodium chloride, 1N hydrochloric acid, saturated aqueous $NaHCO_3$, and saturated aqueous sodium chloride (twice for each). The organic layer was dried over anhydrous $MgSO_4$, and evaporated under reduced pressure. The resulting reaction mixture was purified by column chromatography (silica gel; 200 g, eluted with a mixture of chloroform:methanol= 40:1) to give the title compound as a white solid (6.87 g, 70%), Rf value; 0.55 (chloroform:methanol = 10:1).

$^1$H-NMR ($CDCl_3$)δ ppm; 0.8-0.9 (6H, m, CH($CH_3$)$_2$), 1.0-1.2 (4H, m, CO-CH-$CH_3$ + CH($CH_3$)$_2$), 1.3-1.9 (17H, s + m, $CH_2$-CH($CH_3$)$_2$ + ($CH_2$)$_3$-$CH_2$-NH + C($CH_3$)$_3$), 2.4-2.7 (5H, d + m, J=4.7Hz, NH-$CH_3$ + CH-CO x 2), 3.2-3.4 (2H, m, $CH_2$-NH), 4.25 (1H, m, NH-CH-CO), 5.10 (2H, s, O-$CH_2$-Ph), 6.5 (1H, m, NH), 7.0-7.2 (2H, m, NH x 2), 7.2-7.5 (5H, m, aromatic-H).

c) N$^a$-[4-Hydroxy-2(R)-isobutyl-3(RS)-methylsuccinyl]-N$^e$-benzyloxycarbonyl-L-lysine N-methylamide • 1 acetate (Compound No. 22-c)

To Compound No. 22-b (3.4 g, 6.54 mmol) was added ice-cooled 95% trifluoroacetic acid (containing 5% water, 20 ml) and the mixture was stirred for 4 hours at 5°C. The reaction mixture was concentrated under reduced pressure, and $Et_2O$ was added to the residue. The mixture was stirred for 1 hour at room temperature to precipitate solid products which were collected by filtration, and dried, yielding the title compound as colorless glassy materials (3.0 g, 99%).

$^1$H-NMR ($CDCl_3$)δ ppm; 0.8-1.0 (6H, m, CH(<u>$CH_3$</u>)<u>$_2$</u>), 1.0-1.3 (4H, m, CO-CH-<u>$CH_3$</u> + <u>CH</u>($CH_3$)$_2$), 1.3-1.8 (8H, m, <u>$CH_2$</u>-CH($CH_3$)$_2$ + CH-(<u>$CH_2$</u>)$_3$), 2.49 (1H, m, <u>CH</u>-CO), 2.75 (4H, m, NH-<u>$CH_3$</u> + <u>CH</u>-CO), 2.94 (2H, m, <u>$CH_2$</u>-NH), 4.30 (1H, m, NH-<u>CH</u>-CO), 5.12 (2H, s, O-<u>$CH_2$</u>-Ph), 6.5 (1H, m, NH), 7.0-7.1 (2H, m, NH x 2), 7.2-7.5 (5H, m, aromatic-H).

d) N$^a$-[4-(N-Benzyloxyamino)-2(R)-isobutyl-3(RS)-methylsuccinyl]-N$^e$-acetimidoy-L-lysine N-methylamide (Compound No. 22-d)

To a solution of Compound No. 22-c (2.0 g, 4.31 mmol) in methanol (40 ml) was added 5% palladium on carbon (50% wet catalyst, 1.0 g), and the mixture was vigorously stirred under hydrogen atmosphere for 3.5 hours at room temperature. The catalyst was filtered off and then the solvent was evaporated under reduced pressure. The resulting residue was dissolved in DMF (40 ml), and cooled to 0°C, followed by addition of TEA (1.93 ml, 13.8 mmol) and ethyl acetimidate hydrochloride (577 mg, 4.53 mmol).

The mixture was stirred for 15 hours at room temperature, and then cooled to 0°C, followed by successive addition of HOBT (612 mg, 24.9 mmol), O-benzylhydroxylamine hydrochloride (1.38 g, 8.62 mmol), EDC (1.65 g, 8.62 mmol) and TEA (1.20 ml, 8.62 mmol). The mixture was stirred for 15 hours at room temperature, and evaporated under reduced pressure. The reaction mixture was purified by DIAION HP-20 (Mitsubishi Chemical Corporation, Japan; 200 ml, eluted with 0-70% aqueous methanol) and column chromatography (silica gel; 70 g, eluted with a gradient mixture of chloroform:methanol:acetic acid= 10:2:1 to 5:2:1). The pertinent fractions were pooled, followed by addition of water (30 ml). Lyophilization of the resultant mixture yielded the title compound as amorphous white powders (1.0 g, 49%).

$^1$H-NMR ($CDCl_3$ + $CD_3Cl$)δ ppm; 0.7-0.9 (6H, m, CH(<u>$CH_3$</u>)<u>$_2$</u>), 0.9-1.1 (4H, m, CO-CH-<u>$CH_3$</u> + <u>CH</u>($CH_3$)$_2$), 1.2-1.9 (8H, m, <u>$CH_2$</u>-CH($CH_3$)$_2$ + CH-(<u>$CH_2$</u>)$_3$), 1.94 (3H, s, <u>$CH_3$</u>$CO_2$H), 2.18 (3H, s, C-<u>$CH_3$</u>), 2.55 (1H, m, <u>CH</u>-CO), 2.73 (4H, m, NH-<u>$CH_3$</u> + <u>CH</u>-CO), 3.0-3.4 (2H, m, <u>$CH_2$</u>-NH), 4.36 (1H, m, NH-<u>CH</u>-CO), 4.89 (2H, s, O-<u>$CH_2$</u>-Ph), 7.2-7.4 (5H, m, aromatic-H).

e) N$^a$-[4-(N-Hydroxyamlno)-2(R)-isobutyl-3(RS)-methylsuccinyl]-N$^e$-acetimidoyl-L-lysine N-methylamide • 1 acetate (Compound No. 22)

To a solution of Compound No. 22-d (1.0 g, 2.10 mmol) in acetic acid (10 ml) was added 5% palladium on carbon (50% wet catalyst, 1.0 g), and the mixture was vigorously stirred under hydrogen atmosphere for 2.0 hours at room temperature. The catalyst was filtered off and then acetic acid was evaporated under reduced pressure. $Et_2O$ (20 ml) was added to the resulting residue to precipitate crystals which were collected by filtration, yielding the title compound as white crystals (889 mg, 95%), m.p.; 161-165°C, Rf value; 0.15 (chloroform:methanol:acetic acid= 5:2:1), 0.43 (n-BuOH:AcOH: water= 4:1:1), FABMS ($M^+$ + 1): 386.

<u>Preparation Example 23</u>

N$^a$-[4-(Hydroxyamino)-2(R)-isobutyl-3(RS)-methylsuccinyl]-4'-aminomethyl-L-phenylalanine N-methylamide • 1 acetate (Compound No. 23)

a) N$^a$-[4-tert-Butoxy-2(R)-isobutyl-3(RS)-methylsuccinyl]-L-4'-cyanophenylalanine N-methylamide (Compound No. 23-a)

The procedures of Preparation Examples 22-a and b were repeated using Compound No. 5 and Compound No. 18 to provide the title compound as a white solid (yield 80%), m.p.; 160-164°C, Rf value; 0.60 (chloroform:methanol= 10:1).

b) N$^a$-[4-(N-Benzyloxyamino)-2(R)-isobutyl-3(RS)-methylsuccinyl]-L-4'-cyanophenylalanine N-methylamide (23-b)

To Compound No. 23-a (36.9 g, 85.9 mmol) was added ice-cooled 95% trifluoroacetic acid (containing 5% water, 200 ml) and the mixture was stirred for 2 hours at 5°C. The reaction mixture was concentrated under reduced pressure, and $Et_2O$ was added thereto. The mixture was stirred for 1 hour at room temperature to precipitate solid products which were collected by filtration, and dried.

The resultant solid materials were dissolved in DMF (300 ml), and cooled to -15°C, followed by successive addition of HOBT (13.9 g, 103 mmol), O-benzylhydroxylamine hydrochloride (20.6 g, 129 mmol), EDC (19.7 g, 103 mmol) and TEA (18.0 ml, 129 mmol). After stirred for 15 hours at room temperature, the reaction mixture was added dropwise to water (2 L) to precipitate crystals which were collected by filtration. The resultant crystals were washed successively with 1N hydrochloric acid, 10% aqueous $Na_2CO_3$, water, and $Et_2O$, and dried under reduced pressure to give the title compound as white crystals (39.2 g, 95%), Rf value; 0.42 and 0.47 (chloroform:methanol= 10:1).

c) $N^a$-[4-(Hydroxyamino)-2(R)-isobutyl-3(RS)-methylsuccinyl]-4'-aminomethyl-L-phenylalanine N-methylamide • 1 acetate (Compound No. 23)

To a solution of Compound No. 23-b (10.0 g, 20.8 mmol) in acetic acid (90 ml) was added 5% palladium on carbon (50% wet catalyst, 5 g), and the mixture was vigorously stirred under hydrogen atmosphere for 16 hours at room temperature. The catalyst was filtered off and then acetic acid was evaporated under reduced pressure. The resultant residue was dissolved in water (80 ml), and then lyophilized. The resulting crude product was purified by column reversed phase chromatography (500 g of Chromatorex ODS-1020T, Fuji Silysia Chemical, Japan; eluted with a gradient of 0% to 4% methanol/0.1% aqueous acetic acid), and then lyophilized to give the title compound as a white solid (6.7 g, 71%), m.p.; 223-226°C, Rf value; 0.14 (chloroform:methanol:acetic acid = 5:2:1), 0.52 and 0.60 (n-BuOH:AcOH:water= 4:1:1).

Preparation Example 24

$N^a$-[4-(Hydroxyamino)-2(R),3(R or S)-diisobutylsuccinyl]-L-arginine N-methylamide • 1 acetate (Compound No. 24)

a) $N^a$-[4-tert-Butoxy-2(R),3(RS)-diisobutylsuccinyl]-$N^g$,$N^g$-dibenzyloxycarbonyl-L-arginine N-methylamide (Compound No. 24-a)

The procedures of Preparation Examples 22-a and b were repeated using Compound No. 9 and Compound No. 17 to provide the title compound as a white solid (yield 66%).

$^1$H-NMR ($CDCl_3$)δ ppm; 0.8-0.9 (12H, m, CH$\underline{(CH_3)_2}$ x 2), 1.1-1.3 (2H, m, $\underline{CH}(CH_3)_2$ x 2), 1.4-2.0 (17H, s + m, $\underline{CH_2}$-CH$(CH_3)_2$ x 2 + $\underline{(CH_2)_2}$-$CH_2$-NH + C$\underline{(CH_3)_3}$), 2.4-2.6 (2H, m, $\underline{CH}$-CO x 2), 2.72 (3H, d, J=4.8Hz, NH-$\underline{CH_3}$), 3.6-3.9 (2H, m, $\underline{CH_2}$-NH), 4.32 (1H, m, NH-$\underline{CH}$-CO), 5.0-5.3 (4H, s x 2, O-$\underline{CH_2}$-Ph x2), 7.0 (1H, m, NH), 7.2-7.5 (11H, m, aromatic-H + NH), 8.3 and 8.5 (2H, m, NH x 2).

b) $N^a$-[4-(Hydroxyamino)-2(R),3(R or S)-diisobutylsuccinyl]-L-arginine N-methylamide • 1 acetate (Compound No. 24)

The procedures of Preparation Examples 23-b and c were repeated using Compound No. 24-a to provide the title compound as a white solid (overall yield 60%).

$^1$H-NMR ($CD_3OD$)δ ppm; 0.6-0.9 (12H, m, CH$\underline{(CH_3)_2}$ x 2), 1.0-1.2 (2H, m, $\underline{CH}(CH_3)_2$ x 2), 1.3-1.9 (8H, m, $\underline{CH_2}$-CH$(CH_3)_2$ x 2 + $\underline{(CH_2)_2}$-$CH_2$-NH), 1.96 (3H, s, $\underline{CH_3}CO_2H$), 2.4-2.6 (2H, m, $\underline{CH}$-CO x 2), 2.72 (3H, s, NH-$\underline{CH_3}$), 3.6-3.9 (2H, m, $\underline{CH_2}$-NH), 4.27 (1H, m, NH-$\underline{CH}$-CO).

Preparation Example 25

$N^a$-[4-(Hydroxyamino)-2(R)-isobutyl-3(RS)-methylsuccinyl]-L-arginine N-methylamide • 1 acetate (Compound No. 25)

a) $N^a$-[4-tert-Butoxy-2(R)-isobutyl-3(RS)-methylsuccinyl]-$N^g$,$N^g$-dibenzyloxycarbonyl-L-arginine N-methylamide (Compound No. 25-a)

The procedures of Preparation Examples 22-a and b were repeated using Compound No. 9 and Compound No. 18 to provide the title compound as a white solid (yield 57%).

$^1$H-NMR ($CDCl_3$)δ ppm; 0.7-1.0 (6H, m, CH$\underline{(CH_3)_2}$), 1.0-1.3 (4H, m, $\underline{CH}(CH_3)_2$ + CO-CH-$\underline{CH_3}$), 1.3-2.0 (15H, s + m, $\underline{CH_2}$-CH$(CH_3)_2$ + $\underline{(CH_2)_2}$-$CH_2$-NH + C$\underline{(CH_3)_3}$), 2.2-2.7 (5H, d + m, J=4.8Hz, NH-$\underline{CH_3}$ + $\underline{CH}$-CO x 2), 3.6-4.6 (3H, m, $\underline{CH_2}$-NH + NH-$\underline{CH}$-CO), 5.14 and 5.23 (4H, s x 2, O-$\underline{CH_2}$-Ph x2), 6.5-7.1 (3H, m, NH x 3), 7.2-7.5 (10H, m, aromatic-H), 9.5 (1H, m, NH).

b) $N^a$-[4-(Hydroxyamino)-2(R)-isobutyl-3(RS)-methylsuccinyl]-L-arginine N-methylamide • 1 acetate (Compound No. 25)

The procedures of Preparation Examples 23-b and c were repeated using Compound No. 25-a to provide

the title compound as amorphous white powders (overall yield 53%), Rf value; 0.15 (chloroform:methanol:acetic acid= 5:2:1), 0.47 (n-BuOH:AcOH:water= 4:1:1), FABMS ($M^+$ + 1): 373.

$^1$H-NMR ($CD_3OD$)δ ppm; 0.8-0.9 (6H, m, CH($\underline{CH_3}$)$_2$), 1.0-1.2 (4H, m, $\underline{CH}$($CH_3$)$_2$ + CO-CH-$\underline{CH_3}$), 1.3-1.9 (6H, m, $\underline{CH_2}$-CH($CH_3$)$_2$ + ($\underline{CH_2}$)$_2$-$CH_2$-NH), 1.94 (3H, s, $\underline{CH_3}CO_2H$), 2.2-2.6 (2H, m, $\underline{CH}$-CO x 2), 2.73 (3H, s, NH-$\underline{CH_3}$), 3.6-3.8 (2H, m, $\underline{CH_2}$-NH), 4.26 (1H, m, NH-$\underline{CH}$-CO).

Preparation Example 26

$N^4$-[3-Guanidino-1(S)-methylcarbamoylpropyl]-$N^1$-hydroxy-3(R)-isobutyl-2-(RS)-(3-phenylpropyl)succinamide • 1 acetate (Compound No. 26)

a) [4-tert-Butoxy-2(R)-isobutyl-3(RS)-(3-phenylpropyl)]-succinic acid N-[3-$N^1$,$N^2$-dibenzyloxycarbonylguanidino-1(S)-methylcarbamoyl-n-propyl]amide (Compound No. 26-a)

The procedures of Preparation Examples 22-a and b were repeated using Compound No. 10 and Compound No. 16 to provide the title compound as a white solid (yield 55%).

$^1$H-NMR ($CDCl_3$)δ ppm; 0.87 (6H, m, CH($\underline{CH_3}$)$_2$), 1.2-1.9 (18H, m, $\underline{CH_2}$-$\underline{CH}$($CH_3$)$_2$ + $\underline{CH_2}$-$CH_2$-NH + ($\underline{CH_2}$)$_2$-$CH_2$-Ph + C($\underline{CH_3}$)$_3$), 2.1-2.8 (7H, d + m, J=4.8Hz, NH-$\underline{CH_3}$ + $\underline{CH}$-CO x 2 + $\underline{CH_2}$-Ph), 3.6-3.8 (2H, m, $\underline{CH_2}$-NH), 4.45 (1H, m, + NH-$\underline{CH}$-CO), 5.0-5.3 (4H, m, O-$\underline{CH_2}$-Ph x 2), 7.0 (1H, m, NH), 7.2-7.5 (16H, m, aromatic-H + NH), 8.4 and 8.6 (2H, m, NH x 2).

b) $N^4$-[3-Guanidino-1(S)-methylcarbamoylpropyl]-$N^1$-hydroxy-3(R)-isobutyl-2-(RS)-(3-phenylpropyl)succinamide • 1 acetate (Compound No. 26)

The procedures of Preparation Examples 23-b and c were repeated using Compound No. 26-a to provide the title compound as a white solid (overall yield 51%), m.p.; 143-149°C, Rf value; 0.38 (chloroform:methanol:acetic acid= 5:2:1), 0.62 (n-BuOH:AcOH:water= 4:1:1), FABMS ($M^+$ +2): 464.

Preparation Example 27

$N^4$-[3-Guanidino-1(S)-methylcarbamoylpropyl]-$N^1$-hydroxy-3(R),2(RS)-diisobutylsuccinamide • 1 acetate (Compound No. 27)

a) [4-tert-Butoxy-2(R)-isobutyl-3(RS)-isobutyl]succinic acid-N-[3-$N^1$,$N^2$-dibenzyloxycarbonylguanidino-1(S)-methylcarbamoylpropyl]amide (Compound No. 27-a)

The procedures of Preparation Examples 22-a and b were repeated using Compound No. 10 and Compound No. 17 to provide the title compound as a white solid (yield 22%).

$^1$H-NMR ($CDCl_3$)δ ppm; 0.8-0.9 (12H, m, CH($\underline{CH_3}$)$_2$ x 2), 1.1-1.3 (2H, m, $\underline{CH}$($CH_3$)$_2$ x 2), 1.4-1.9 (15H, s + m, $\underline{CH_2}$-CH($CH_3$)$_2$ x 2 + $\underline{CH_2}$-$CH_2$-NH + C($\underline{CH_3}$)$_3$), 2.4-2.6 (2H, m, $\underline{CH}$-CO x 2), 2.71 (3H, d, J=4.8Hz, NH-$\underline{CH_3}$), 3.6-3.8 (2H, m, $\underline{CH_2}$-NH), 4.40 (1H, m, NH-$\underline{CH}$-CO), 5.0-5.3 (4H, m, O-$\underline{CH_2}$-Ph x2), 7.1 (1H, m, NH), 7.2-7.5 (11H, m, aromatic-H + NH), 8.4 and 8.6 (2H, m, NH x 2).

b) $N^4$-[3-Guanidino-1(S)-methylcarbamoylpropyl)-$N^1$-hydroxy-3(R),2(RS)-diisobutylsuccinamide • 1 acetate (Compound No. 27)

The procedures of Preparation Examples 23-b and c were repeated using Compound No. 27-a to provide the title compound as a white solid (overall yield 18%), m.p.; 165-167°C, Rf value; 0.27 (chloroform:methanol:acetic acid= 5:2:1), 0.66 (n-BuOH:AcOH:water= 4:1:1), FABMS ($M^+$ + 1): 401.

Preparation Example 28

$N^4$-[3-Guanidino-1(S)-methylcarbamoylpropyl]-$N^1$-hydroxy-3(R)-isobutyl-2(R or S)-methylsuccinamide • 1 acetate (Compound No. 28)

a) [4-tert-Butoxy-2(R)-isobutyl-3(R or S)-methyl]succinic acid N-[3-$N^1$,$N^2$-dibenzyloxycarbonylguanidino-1(S)-methylcarbamoylpropyl]amide (Compound No. 28-a)

The procedures of Preparation Examples 22-a and b were repeated using Compound No. 10 and Compound No. 18 to provide the title compound as a white solid (yield 25%).

$^1$H-NMR ($CDCl_3$)δ ppm; 0.7-1.0 (6H, m, $CH(\underline{CH_3})_2$), 1.0-1.2 (4H, m, $\underline{CH}(CH_3)_2$ + CO-CH-$\underline{CH_3}$), 1.3-2.1 (13H, s + m, $\underline{CH_2}$-$CH(CH_3)_2$ + $\underline{CH_2}$-$CH_2$-NH + $C(\underline{CH_3})_3$), 2.1-2.8 (5H, d + m, J=4.8Hz, $\underline{CH}$-CO x 2 + NH-$\underline{CH_3}$), 3.2 and 3.6 (1H each, m, $\underline{CH_2}$-NH), 4.45 (1H, m, NH-$\underline{CH}$-CO), 5.0-5.3 (4H, m, O-$\underline{CH_2}$-Ph x2), 7.1 (1H, m, NH), 7.2-7.5 (11H, m, aromatic-H + NH), 8.4 and 8.6 (2H, m, NH x 2).

b) $N^4$-[3-Guanidino-1(S)-methylcarbamoylpropyl]-$N^1$-hydroxy-3(R)-isobutyl-2(R or S)-methylsuccinamide • 1 acetate (Compound No. 28)

The procedures of Preparation Examples 23-b and c were repeated using Compound No. 28-a to provide the title compound as a white solid (overall yield 65%), m.p.; 135-139°C, Rf value; 0.15 (chloroform:methanol:acetic acid= 5:2:1), 0.49 (n-BuOH:AcOH:water= 4:1:1), FABMS ($M^+$ + 1): 359.

Preparation Example 29

$N^a$-[4-(Hydroxyamino)-2(R)-isobutyl-3(RS)-methylsuccinyl]-L-ornithine N-methylamide • 1 acetate (Compound No. 29)

a) $N^a$-[4-tert-Butoxy-2(R)-isobutyl-3(RS)-methylsuccinyl]-$N^d$-benzyloxycarbonyl-L-ornithine N-methylamide (Compound No. 29-a)

The procedures of Preparation Examples 22-a and b were repeated using Compound No. 7 and Compound No. 18 to provide the title compound as a colorless oil (yield 72%).

$^1$H-NMR ($CDCl_3$)δ ppm; 0.8-1.0 (6H, m, $CH(\underline{CH_3})_2$), 1.0-1.3 (4H, m, $\underline{CH}(CH_3)_2$ + CO-CH-$\underline{CH_3}$), 1.3-1.9 (15H, s + m, $\underline{CH_2}$-$CH(CH_3)_2$ + $(\underline{CH_2})_2$-$CH_2$-NH + $C(\underline{CH_3})_3$), 2.5-2.7 (2H, m, $\underline{CH}$-CO x 2), 2.72 (3H, d, J=4.8Hz, NH-$\underline{CH_3}$), 3.2-3.4 (2H, m, $\underline{CH_2}$-NH), 4.30 (1H, m, NH-$\underline{CH}$-CO), 5.09 (2H, s, O-$\underline{CH_2}$-Ph), 6.4-6.7 (1H, m, NH), 7.0-7.2 (2H, m, NH x 2), 7.2-7.5 (5H, m, aromatic-H).

b) $N^a$-[4-(Hydroxyamino)-2(R)-isobutyl-3(RS)-methylsuccinyl]-L-ornithine N-methylamide • 1 acetate (Compound No. 29)

The procedures of Preparation Examples 23-b and c were repeated using Compound No. 29-a to provide the title compound as amorphous white powders (overall yield 28%).

$^1$H-NMR ($CD_3OD$)δ ppm; 0.8-0.9 (6H, m, $CH(\underline{CH_3})_2$), 1.0-1.2 (4H, m, $\underline{CH}(CH_3)_2$ + CO-CH-$\underline{CH_3}$), 1.3-1.7 (6H, m, $\underline{CH_2}$-$CH(CH_3)_2$ + $(\underline{CH_2})_2$-$CH_3$-NH), 1.88 (3H, s, $\underline{CH_3}CO_2H$), 2.4-2.7 (5H, m, $\underline{CH}$-CO x 2 + NH-$\underline{CH_3}$), 2.85 (2H, m, $\underline{CH_2}$-NH), 4.27 (1H, m, NH-$\underline{CH}$-CO).

Preparation Example 30

$N^a$-[4-(Hydroxyamino)-2(R)-isobutyl-3(RS)-(3-phenylpropyl)succinyl]-$N^e$-acetimidoyl-L-lysine N-methylamide • 1 acetate (Compound No. 30)

a) $N^a$-[4-tert-Butoxy-2(R)-isobutyl-3(RS)-(3-phenylpropyl)succinyl]-$N^e$-2-chlorobenzyloxycarbonyl-L-lysine N-methylamide (Compound No. 30-a)

The procedures of Preparation Examples 22-a and b were repeated using Compound No. 13 and Compound No. 16 to provide the title compound as a colorless oil (yield 83%).

$^1H$-NMR ($CDCl_3$)δ ppm; 0.8-0.9 (6H, m, CH($CH_3$)$_2$), 1.08 (1H, m, CH($CH_3$)$_2$), 1.2-1.9 (21H, s + m, ($CH_2$)$_3$-$CH_2$-NH + ($CH_2$)$_2$-$CH_2$-Ph + C($CH_3$)$_3$ + $CH_2$CH($CH_3$)$_2$), 2.4-2.6 (4H, m, CH-CO x 2 + $CH_2$-Ph), 2.77 (3H, m, NH-$CH_3$), 3.18 (2H, m, $CH_2$-NH), 4.32 (1H, m,+ NH-CH-CO), 4.91 (1H, m, NH), 5.22 (2H, m, O-$CH_2$-$C_6H_4$), 6.28 and 6.4 (2H, m, NH x 2), 7.1-7.4 (9H, m, aromatic-H).

b) $N^4$-Benzyloxy-$N^4$-benzyloxycarbonyl-$N^1$-[5-benzyloxycarbonylamino-1(S)-methylcarbamoylpentyl]-2(R)-isobutyl-3(RS)-(3-phenylpropyl)succinamide (Compound No. 30-b)

The procedure of Preparation Example 22-a was repeated using Compound No. 1 and Compound No. 19 to provide the title compound as a white solid (yield 52%).

$^1H$-NMR ($CDCl_3$)δ ppm; 0.7-1.0 (6H, m, CH($CH_3$)$_2$), 1.0-1.2 (1H, m, CH($CH_3$)$_2$), 1.2-1.9 (12H, m, CH-($CH_2$)$_3$ + ($CH_2$)$_2$-$CH_2$-Ph + $CH_2$CH($CH_3$)$_2$), 2.4-2.6 (3H, m, CH-CO + $CH_2$-Ph ), 2.73 (3H, d, J=4.8Hz, NH-$CH_3$), 3.1-3.3 (2H, m, $CH_2$-NH), 3.90 (1H, m, CH-CO), 4.08 (1H, m, NH-CH-CO), 4.80 (2H, m, O-$CH_2$-Ph), 5.09 and 5.26 (4H, s x 2, C(=O)O-$CH_2$-Ph x 2, 6.5 (1H, m, NH), 7.0-7.2 (2H, m, NH x 2), 7.0-7.4 (20H, m, aromatic-H).

c) $N^a$-[4-(Hydroxyamino)-2(R)-isobutyl-3(RS)-(3-phenylpropyl)succinyl]-L-lysine N-methylamide (Compound No. 30-c)

The procedures of Preparation Examples 23-b and c except using MeOH for catalytic hydrogenation were repeated using Compound No. 30-a to provide the title compound as a white solid (overall yield 70%), m.p.; 181-182°C, Rf value; 0.46 (chloroform:methanol:acetic acid= 5:2:1), 0.56 (n-BuOH:AcOH: water= 4:1:1).

Similarly, the procedure of Preparation Example 23-c except using MeOH for catalytic hydrogenation was repeated using Compound No. 30-b to provide the title compound (yield 82%).

d) $N^a$-[4-(Hydroxyamino)-2(R)-isobutyl-3(RS)-(3-phenylpropyl)succinyl]-$N^e$-acetimidoyl-L-lysine N-methylamide • 1 acetate (Compound No. 30)

Compound No. 30-c (3.78 g, 8.42 mmol) was dissolved in DMF (100 ml), and cooled to 0°C, followed by addition of TEA (2.41 ml, 17.3 mmol) and ethyl acetimidate hydrochloride (1.13 g, 8.84 mmol). The mixture was stirred for 30 minutes at 0°C, and then for another 1 hour at room temperature. DMF was evaporated under reduced pressure and the residue was purified by column reversed phase chromatography (200 g of Chromatorex ODS-1020T, Fuji Silysia Chemical, Japan; eluted with a gradient of 4% to 20% methanol/0.1% aqueous acetic acid), and then lyophilized to give the title compound as a white solid (2.50 g, 54%), m.p.; 119-124°C, Rf value; 0.29 (chloroform:methanol: acetic acid= 5:2:1), 0.60 (n-BuOH:AcOH;water= 4:1:1), FABMS ($M^+$ +2): 492.

Preparation Example 31

$N^a$-[4-(Hydroxyamino)-2(R)-isobutyl-3(R or S)-methylsuccinyl]-4'-[tetramethyl bis(phosphono)methylimino]-L-phenylalanine N-methylamide (Compound No. 31)

a) $N^a$-[4-tert-Butoxy-2(R)-isobutyl-3(RS)-methylsuccinyl]-4'-[tetramethyl bis(phosphono)methylimino]-L-phenylalanine N-methylamide (Compound No. 31-a)

To a solution of Compound No. 12 (3.10 g, 5.56 mmol) in MeOH (100 ml) was added 10% palladium on carbon (50% wet catalyst, 3.1 g), and the mixture was vigorously stirred under hydrogen atmosphere for 1.5 hours at room temperature. The catalyst was filtered off and then the solvent was evaporated under reduced pressure. The resultant oily material was dissolved in DMF (60 ml), to which was added Compound No. 18 (1.36 g, 5.56 mmol), HOBT (900 mg, 6.67 mmol), EDC (1.28 g, 6.67 mmol) and TEA (775 μl, 5.58 mmol) successively with stirring at -15°C. The mixture was stirred for 1 hour at -15°C, and further for another 15 hours at room temperature, and evaporated under reduced pressure, followed by addition of AcOEt (200 ml). The resultant mixture was partitioned and washed successively with saturated aqueous sodium chloride, 1N hydrochloric acid, saturated aqueous $NaHCO_3$, and saturated aqueous sodium chloride (twice for each). The organic layer was dried over anhydrous $MgSO_4$, and evaporated under reduced pressure, and the residue was purified column chromatography (silica gel; 130 g, eluted with a mixture of chloroform:methanol= 20:1) to give the title compound as a pale yellow oil (1.49 g, 41%).

$^1H$-NMR ($CDCl_3$)δ ppm; 0.7-1.2 (9H, m, CH($CH_3$)$_2$ + CO-CH-$CH_3$), 1.3 (1H, m, CH($CH_3$)$_2$), 1.4-1.8 (11H, m, $CH_2$-CH($CH_3$)$_2$ + C($CH_3$)$_3$), 2.3-2.5 (2H, m, CH-CO x 2), 2.72 (3H, m, NH-$CH_3$), 2.95 (2H, m, $C_6H_4$-$CH_2$), 3.80 (12H, m, O-$CH_3$ x 4), 4.12 (1H, m, NH-CH-CO), 4.58 (1H, m, P-CH-P), 5.90 , 6.17 and 6.36 (3H, m, NH x 3), 6.61-7.07 (4H,

m, aromatic-H).

b) $N^a$-[4-(Hydroxyamino)-2(R)-isobutyl-3(R or S)-methylsuccinyl]-4'-[tetramethyl bis(phosphono)methylimino]-L-phenylalanine N-methylamide (Compound No. 31)

The procedures of Preparation Examples 23-b and c were repeated using Compound No. 31-a to provide the title compound as a white solid (overall yield 42%), m.p.; 204-208°C, Rf value; 0.52 (chloroform:methanol:acetic acid= 5:2:1), 0.63 (n-BuOH:AcOH:water= 4:1:1), FABMS ($M^+$ +2): 610.

Preparation Example 32

$N^a$-[4-(Hydroxyamino)-2(R)-isobutyl-3(R or S)-methylsuccinyl]-4'-[trimethyl bis(phosphono)methylimino]-L-phenylalanine N-methylamide • 1 sodium salt (Compound No. 32)

Compound No. 31-b (1.18 g, 1.94 mmol) was dissolved in MeOH (15 ml), and cooled to 0°C, followed by dropwise addition of 2N aqueous sodium hydroxide (9.7 ml). The mixture was stirred for 15 hours at room temperature, and then re-cooled to 0°C. The cooled reaction mixture was adjusted with 6N hydrochloric acid to pH 7, and evaporated. The resultant residue was purified by column reversed phase chromatography (50 g of Chromatorex ODS-1020T, Fuji Silysia Chemical, Japan; eluted with a gradient of 1 to 10% aqueous methanol), and then lyophilized to give the title compound as a white solid (263 mg, 22%), m.p.; 235-240°C, Rf value; 0.14 (chloroform: methanol:acetic acid= 5:2:1), 0.46 (n-BuOH:AcOH:water= 4:1:1).

Preparation Example 33

4'-[tetraethyl bis(phosphono)methylimino]-L-phenylalanine N-methylamide (Compound No. 33)

a) $N^a$-[4-tert-Butoxy-2(R)-isobutyl-3(RS)-methylsuccinyl]-4'-[tetraethyl bis(phosphono)methylimino]-L-phenylalanine N-methylamide (Compound No. 33-a)

The procedure of Preparation Example 31-a was repeated using Compound No. 11 and Compound No. 18 to provide the title compound as a yellow oil (yield 89%).

$^1$H-NMR ($CDCl_3$)δ ppm; 0.7-0.9 (6H, m, CH($\underline{CH_3}$)$_2$), 0.9-1.1 (4H, m, $\underline{CH}$($CH_3$)$_2$ + CO-CH-$\underline{CH_3}$), 1.2-1.35 (12H, m, $CH_2$-$\underline{CH_3}$ x 4), 1.43 (9H, s, C($\underline{CH_3}$)$_3$), 1.6-1.9 (2H, m, $\underline{CH_2}$-CH($CH_3$)$_2$), 2.3-2.5 (2H, m, $\underline{CH}$-CO x 2), 2.69 (3H, m, NH-$\underline{CH_3}$), 2.95-3.1 (2H, m, $C_6H_4$-$\underline{CH_2}$), 4.0-4.3 (9H, m, $\underline{CH_2}$-$CH_3$ x 4 + NH-$\underline{CH}$-CO), 4.55 (1H, m, P-$\underline{CH}$-P), 5.77 , 6.19 and 6.41 (1H each, m, NH x 3), 6.6-7.1 (4H, m, aromatic-H).

b) $N^a$-[4-(Hydroxyamino)-2(R)-isobutyl-3(RS)-methylsuccinyl]-4'-[tetraethyl bis(phosphono)methylimino]-L-phenylalanine N-methylamide

The procedures of Preparation Examples 23-b and c were repeated using Compound No. 33-a to provide the title compound as a white solid (overall yield 16%), m.p.; 185-189°C, Rf value; 0.68 (chloroform:methanol:acetic acid= 5:2:1), 0.83 (n-BuOH:AcOH:water= 4:1:1), FABMS ($M^+$ +2): 666.

Preparation Example 34

$N^a$-[4-(Hydroxyamino)-2(R)-isobutyl-3(RS)-methylsuccinyl-4'-[triethyl bis(phosphono)methylimino]-L-phenylalanine N-methylamide • 1 sodium salt (Compound No. 34)

The procedure of Preparation Example 32 was repeated using Compound No. 33-b (1.18 g, 1.94 mmol) to provide the title compound as a white solid (overall yield 32%), m.p.; 215-220°C, Rf value; 0.31 (chloroform:methanol:acetic acid= 5:2:1), 0.60 (n-BuOH:AcOH:water= 4:1:1).

Preparation Example 35

$N^a$-[4-(Hydroxyamino)-2(R),3(RS)-diisobutylsuccinyl]-4'-aminomethyl-L-phenylalanine N-(2-hydroxyethyl)amide • 1 acetate (Compound No. 35)

a) $N^a$-[4-tert-Butoxy-2(R),3(RS)-diisobutylsuccinyl]-L-4'-cyanophenylalanine N-(2-benzyloxyethyl)amide (Compound No. 35-a)

The procedure of Preparation Example 22-a was repeated using Compound No. 14 and Compound No. 17 to provide the title compound as a colorless glassy material (yield 58%), m.p.; 64-67°C, Rf value; 0.38 (chloroform:methanol= 20:1).

b) $N^a$-[4-(Hydroxyamino)-2(R),3(RS)-diisobutylsuccinyl]-4'-aminomethyl-L-phenylalanine N-(2-hydroxyethyl)amide • 1 acetate (Compound No. 35)

The procedures of Preparation Examples 23-b and c were repeated using Compound No. 35-a to provide the title compound as a white solid (overall yield 46%), m.p.; 196-198°C, Rf value; 0.45 (chloroform:methanol:acetic acid= 5:2:1), 0.49 (n-BuOH:AcOH:water= 4:1:1), FABMS ($M^+$ + 1): 465.

Preparation Example 36

$N^a$-[4-(Hydroxyamino)-2(R)-isobutyl-3(R or S)-(3-phenylpropyl)succinyl]-$N^e$-acetimidoyl-L-lysine N-(2-hydroxyethyl)amide • 1 acetate (Compound No. 36)

a) $N^a$-[4-tert-Butoxy-2(R)-isobutyl-3(RS)-(3-phenylpropyl)succinyl]-$N^e$-2-chlorobenzyloxycarbonyl-L-lysine N-(2-benzyloxyethyl)amide (Compound No. 36-a)

The procedure of Preparation Example 22-a was repeated using Compound No. 15 and Compound No. 16 to provide the title compound as a colorless waxy material (yield 88%).

$^1$H-NMR ($CDCl_3$)δ ppm; 0.84 (6H, m, CH$\underline{(CH_3)_2}$), 1.2-1.9 (22H, m, $\underline{(CH_2)_3}$-$CH_2$-NH + $\underline{(CH_2)_2}$-$CH_2$-Ph + C$\underline{(CH_3)_3}$ + $\underline{CH_2CH}(CH_3)_2$), 2.3-2.7 (4H, m, $\underline{CH}$-CO x 2 + $\underline{CH_2}$-Ph), 3.15 (2H, m, $\underline{CH_2}$-NH), 3.4-3.6 (4H, m, NH-$\underline{CH_2}$-$\underline{CH_2}$-O), 4.36 (1H, m, NH-$\underline{CH}$-CO), 4.51 (2H, m, O-$\underline{CH_2}$-Ph), 4.91 (1H, m, NH), 5.21 (3H, s + m, -C(=O)O-$\underline{CH_2}$-$C_6H_4$), 6.44 (1H, m, NH), 7.0-7.5 (14H, m, aromatic-H).

b) $N^a$-[4-(Benzyloxyamino)-2(R)-isobutyl-3(RS)-(3-phenylpropyl)succinyl]-$N^e$-2-chlorobenzyloxycarbonyl-L-lysine N-(2-benzyloxyethyl)amide (Compound No. 36-b)

The procedure of Preparation Example 23-b was repeated using Compound No. 36-a (3.83 g, 4.92 mmol) to provide the title compound as a white solid (yield 69%), m.p.; 158-165°C, Rf value; 0.67 (chloroform:methanol= 10:1).

C) $N^a$-[4-(Hydroxyamino)-2(R)-isobutyl-3(RS)-(3-phenylpropyl)succinyl]-L-lysine N-2-benzyloxyethylamide • 1 acetate (Compound No. 36-c)

To a solution of Compound No. 36-b (2.60 g, 3.10 mmol) in acetic acid (30 ml) was added 5% palladium on carbon (50% wet catalyst, 1.5 g), and the mixture was vigorously stirred under hydrogen atmosphere for 2.5 hours at room temperature. The catalyst was filtered off and then acetic acid was evaporated under reduced pressure. $Et_2O$ (50 ml) was added to the resulting residue to precipitate crystals which were collected by filtration, yielding the title compound as white crystals (1.44 g, 86%).

$^1$H-NMR ($CDCl_3$ + $CD_3OD$)δ ppm; 0.8-1.0 (6H, m, CH$\underline{(CH_3)_2}$), 1.0-1.2 (1H, m, $\underline{CH}(CH_3)_2$), 1.2-1.95 (12H, m, $\underline{(CH_2)_3}$-$CH_2$-NH + $\underline{(CH_2)_2}$-$CH_2$-Ph + $\underline{CH_2}CH(CH_3)_2$), 1.98 (3H, s, $\underline{CH_3}CO_2H$), 2.18 (1H, m, $\underline{CH}$-CO), 2.57 (3H, m, $\underline{CH}$-CO + $\underline{CH_2}$-Ph), 2.90 (2H, m, $\underline{CH_2}$-NH), 3.2-3.4 and 3.63 (4H, m, NH-$\underline{CH_2}$-$\underline{CH_2}$-O), 4.32 (1H, m, NH-$\underline{CH}$-CO), 7.0-7.3 (5H, m, aromatic-H).

d) $N^a$-[4-(Hydroxyamino)-2(R)-isobutyl-3(R or S)-(3-phenylpropyl)succinyl]-$N^e$-acetimidoyl-L-lysine N-(2-hydroxyethyl)amide • 1 acetate (Compound No. 36)

Compound No. 36-c (1.31 g, 2.43 mmol) was dissolved in DMF (50 ml), and cooled to 0°C, followed by addition of TEA (1.10 ml, 7.86 mmol) and ethyl acetimidate hydrochloride (360 mg, 2.92 mmol). The reaction mixture was stirred for 30 minutes at 0°C, and then evaporated. This reaction mixture was purified by column reversed phase chromatography (50 g of Chromatorex ODS-1012T, Fuji Silysia Chemical, Japan; eluted with a gradient of 0.1 to 7% methanol/0.1% aqueous acetic acid), and then lyophilized to give the title compound as a white solid (881 mg, 63%), m.p.; 118-121°C, Rf value; 0.22 (chloroform:methanol:acetic acid= 5:2:1), 0.48 (n-BuOH:AcOH: water= 4:1:1), FABMS ($M^+$ + 1); 520.

Preparation Example 37

$N^a$-[4-(Hydroxyamino)-2(R)-isobutyl-3(R or S)-methylsuccinyl]-L-ornithine N-cyclopropylamide • 1 acetate (Compound No. 37)

a) $N^a$-[4-tert-Butoxy-2(R)-isobutyl-3(RS)-methylsuccinyl]-$N^d$-benzyloxycarbonyl-L-ornithine N-methylamide (Compound No. 37-a)

The procedures of Preparation Examples 22-a and b were repeated using Compound No. 8 and Compound No. 18 to provide the title compound as a colorless oil (yield 54%).

$^1$H-NMR ($CDCl_3$)δ ppm; 0.48 (2H, m, $\underline{CH_2}$ of cyclopropyl), 0.7-0.9 (8H, m, $\underline{CH_2}$ of cyclopropyl + CH$\underline{(CH_3)_2}$), 1.0-1.2 (4H, m, $\underline{CH}(CH_3)_2$ + CO-CH-$\underline{CH_3}$), 1.3-1.9 (15H, m, $\underline{CH_2}$-CH$(CH_3)_2$ + $\underline{(CH_2)_2}$-$CH_2$-NH + C$\underline{(CH_3)_3}$), 2.4-2.7 (3H, m, $\underline{CH}$ of cyclopropyl + $\underline{CH}$-CO x 2), 3.2-3.4 (2H, m, $\underline{CH_2}$-NH), 4.31 (1H, m, NH-$\underline{CH}$-CO), 5.07 (2H, s, O-$\underline{CH_2}$-Ph), 6.4-6.7 (1H, m, NH), 7.0-7.2 (2H, m, NH x 2), 7.2-7.5 (5H, m, aromatic-H).

b) $N^a$-[4-(Hydroxyamino)-2(R)-isobutyl-3(R or S)-methylsuccinyl]-L-ornithine N-cyclopropylamide • 1 acetate

The procedures of Preparation Examples 23-b and c were repeated using Compound No. 37-a to provide the title compound as a white solid (overall yield 35%), m.p.; 185-192°C, Rf value; 0.16 (chloroform:methanol:acetic acid= 5:2:1), 0.46 (n-BuOH: AcOH:water= 4:1:1), FABMS ($M^+$ + 1): 357.

Preparation Example 38

$N^a$-[4-(Hydroxyamino)-2(R)-isobutyl-3(RS)-methylsuccinyl]-L-lysine N-methylamide • 1 acetate (Compound No. 38)

The procedures of Preparation Examples 36-b and c were repeated using Compound No. 22-b to provide the title compound as a white solid (overall yield 48%), m.p.; 173-177°C, Rf value; 0.14 (chloroform:methanol:acetic acid= 5:2:1), 0.42 (n-BuOH: AcOH:water= 4:1:1), FABMS ($M^+$ + 1): 345.

Preparation Example 39

$N^a$-[4-(Hydroxyamino)-2(R)-isobutyl-3(RS)-methylsuccinyl]-$N^e$-acetimidoyl-L-lysine N-[2-hydroxy-1(RS)-methylethyl]amide • 1 acetate (Compound No. 39)

a) $N^a$-[4-tert-Butoxy-2(R)-isobutyl-3(RS)-methylsuccinyl]-$N^e$-tert-butyloxycarbonyl-L-lysine N-[2-hydroxy-1(RS)-methylethyl]amide (Compound No. 39-a)

The procedures of Preparation Examples 22-a and b were repeated using Compound No. 6 and Compound No. 18 to provide the title compound as a colorless oil (yield 71%).

$^1$H-NMR ($CDCl_3$)δ ppm; 0.8-1.0 (6H, m, CH$\underline{(CH_3)_2}$), 1.0-1.2 (7H, m, $\underline{CH}(CH_3)_2$ + CH-$\underline{CH_3}$ x 2), 1.3-2.0 (26H, s x 2 + m, C$\underline{(CH_3)_3}$ x 2 + $\underline{CH_2}$-CH$(CH_3)_2$ + $\underline{(CH_2)_3}$-$CH_2$-NH), 2.4-2.6 (2H, m, $\underline{CH}$-CO x 2), 3.08 (2H, m, $\underline{CH_2}$-NH), 3.4-3.6 (3H, m, NH-$\underline{CH_2}$-$\underline{CH_2}$-OH), 4.04 (1H, m, NH-$\underline{CH}$-CO), 4.43 (1H, m, OH), 4.85 (1H, m, NH), 6.8-7.1 (2H, m, NH x 2).

b) $N^a$-[4-(Hydroxyamino)-2(R)-isobutyl-3(RS)-methylsuccinyl]-$N^e$-acetimidoyl-L-lysine N-[2-hydroxy-1(RS)-methylethyl]amide • 1 acetate (Compound No. 39)

To Compound No. 39-a (8.50 g, 16.0 mmol) was added ice-cooled 95% trifluoroacetic acid (containing 5% water, 70 ml) and the mixture was stirred for 30 minutes at 5°C, and for another 1 hour at room temperature, and concentrated under reduced pressure, followed by addition of $Et_2O$. The mixture was stirred for 1 hour at room temperature to precipitate solid products which were collected by filtration, and dried, yielding a white solid carboxylic acid compound (6.01 g, 79%).

This carboxylic acid compound was dissolved in DMF (60 ml), and cooled to 0°C. To the cooled solution was added TEA (6.90 ml, 49.7 mmol) and ethyl acetimidate hydrochloride (2.18 g, 17.6 mmol), and the mixture was stirred for 30 minutes at room temperature, and then re-cooled to 0°C, followed by successive addition of HOBT (2.38 g, 17.6 mmol), O-benzylhydroxylamine hydrochloride (5.38 g, 33.7 mmol), EDC (4.61 g, 24.1 mmol) and TEA (4.70 ml, 33.7 mmol). The mixture was stirred for 15 hours at room temperature, and then evaporated under reduced pressure. The reaction mixture was purified by DIAION HP-20 (Mitsubishi Chemical Corporation, Japan; 500 ml, eluted with 10 to 100% aqueous methanol) and column chromatography (silica gel; 150 g, eluted with a mixture of chloroform:methanol:acetic acid= 5:2:1 and a mixture of methanol:acetic acid= 1:1). The pertinent fractions were pooled, and concentrated under reduced pressure. The resulting residue was dissolved in acetic acid (100 ml), followed by addition of 5% palladium on carbon (50% wet catalyst, 3.0 g). The mixture was vigorously stirred under hydrogen atmosphere for 1.5 hours at room temperature. The catalyst was filtered off and then acetic acid was evaporated under reduced pressure. The resultant residue was dissolved in water, and lyophilized to give the title compound as a white solid (2.55 g, 32%), m.p.; 118-127°C, Rf value; 0.13 (chloroform:methanol:acetic acid= 5:2:1), 0.39 (n-BuOH:AcOH:water= 4:1:1), FABMS ($M^+$ + 1): 430.

Preparation Example 40

$N^a$-[4-(Hydroxyamino)-2(R)-isobutyl-3(RS)-methylsuccinyl]-$N^e$-acetimidoyl-L-lysine N-(piperidin-1-yl)amide • 1 acetate (Compound No. 40)

a) $N^a$-[4-tert-Butoxy-2(R)-isobutyl-3(RS)-methylsuccinyl]-$N^e$-tert-butyloxycarbonyl-L-lysine N-(piperidin-1-yl)amide (Compound No. 40-a)

The procedures of Preparation Examples 22-a and b were repeated using Compound No. 4 and Compound No. 18 to provide the title compound as a pale yellow oil (yield 71%).

$^1$H-NMR ($CDCl_3$)δ ppm; 0.8-1.0 (6H, m, $CH(\underline{CH_3})_2$), 1.0-1.2 (4H, m, $\underline{CH}(CH_3)_2$ + CO-CH-$\underline{CH_3}$), 1.3-1.9 (32H, s x 2 + m, $C(\underline{CH_3})_3$ x 2 + $\underline{CH_2}$-$CH(CH_3)_2$ + $\underline{(CH_2)_3}$-$CH_2$-NH + -$\underline{(CH_2)_3}$- of piperidine), 2.4-2.6 (2H, m, $\underline{CH}$-CO x 2), 2.6-3.1 (6H, m, $\underline{CH_2}$-N-$\underline{CH_2}$ + $\underline{CH_2}$-$NH_2$), 4.28 (1H, m, NH-$\underline{CH}$-CO), 5.13 (1H, m, NH), 6.41 and 6.57 (2H, m, NH x 2).

b) $N^a$-[4-(Hydroxyamlno)-2(R)-isobutyl-3(RS)-methylsuccinyl]-$N^e$-acetimidoyl-L-lysine N-(piperidin-1-yl)amide • 1 acetate (Compound No. 40)

The procedure of Preparation Example 39-b was repeated using Compound No. 40-a(3.80 g, 6.85 mmol) to provide the title compound as a white solid (overall yield 16%), m.p.; 155-162°C, Rf value; 0.21 (chloroform:methanol:acetic acid= 5:2:1), 0.48 (n-BuOH:AcOH:water= 4:1:1), FABMS ($M^+$ + 1): 455.

Preparation Example 41

$N^a$-[4-(Hydroxyamino)-2(R)-isobutyl-3(R or S)-methylsuccinyl]-4'-acetimidoyliminomethyl-L-phenylalanine N-methylamide • 1 acetate (Compound No. 41)

The procedures of Preparation Examples 22-c, d and e were repeated using Compound No. 23-a to provide the title compound as a white solid (overall yield 10%), m.p.; 144-151°C, Rf value; 0.15 (chloroform:methanol:acetic acid= 5:2:1), 0.46 (n-BuOH: AcOH:water= 4:1:1), FABMS ($M^+$ + 1): 434.

Preparation Example 42

$N^a$-[4-(Hydroxyamino)-2(R)-isobutyl-3(R or S)-methylsuccinyl]-4'-acetimidoyliminomethyl-L-phenylalanine N-(morpholin-4-yl)amide • 1 acetate (Compound No. 42)

a) $N^a$-[4-tert-Butoxy-2(R)-isobutyl-3(RS)-methylsuccinyl]-L-4'-cyanophenylalanine N-(morpholin-4-yl)amide (Compound No. 42-a)

The procedures of Preparation Examples 22-a and b were repeated using Compound No. 2 and Compound No. 18 to provide the title compound as a white solid (yield 64%).

$^1$H-NMR ($CDCl_3$)δ ppm; 0.7-0.9 (6H, m, CH$(\underline{CH_3})_2$), 0.9-1.2 (3H, m, CO-CH-$\underline{CH_3}$), 1.3-1.7 (12H, s + m, C$(\underline{CH_3})_3$ + $\underline{CH_2}$-$\underline{CH}$$(CH_3)_2$), 2.3-2.6 (2H, m, $\underline{CH}$-CO x 2), 2.6-2.8 (4H, m, $\underline{CH_2}$-N-$\underline{CH_2}$), 2.9-3.3 (2H, m, $C_6H_4$-$\underline{CH_2}$), 3.77 (4H, m, $\underline{CH_2}$-O-$\underline{CH_2}$), 4.62 (1H, m, NH-$\underline{CH}$-CO), 6.47 (2H, m, NH x 2), 7.3-7.6 (4H, m, aromatic-H).

b) $N^a$-[4-(Hydroxyamino)-2(R)-isobutyl-3(R or S)-methylsuccinyl]-4'-acetimidoyliminomethyl-L-phenylalanine N-(morpholin-4-yl)amide • 1 acetate (Compound No. 42)

The procedures of Preparation Examples 22-c, d and e were repeated using Compound No. 42-a to provide the title compound as amorphous white powders (overall yield 10%).

FABMS ($M^+$ + 1): 505
$^1$H-NMR ($CD_3OD$)δ ppm; 0.7-0.9 (6H, m, CH$(\underline{CH_3})_2$), 0.9-1.2 (4H, m, $\underline{CH}$$(CH_3)_2$ + CO-CH-$\underline{CH_3}$), 1.3-1.7 (2H, m, $\underline{CH_2}$-CH$(CH_3)_2$), 1.90 (3H, s, $\underline{CH_3}CO_2H$), 2.25 (3H, s, CH-$\underline{CH_3}$), 2.5-2.8 (6H, m, $\underline{CH}$-CO x 2 + $\underline{CH_2}$-N-$\underline{CH_2}$), 2.9-3.1 (2H, m, $C_6H_4$-$\underline{CH_2}$), 3.75 (4H, m, $\underline{CH_2}$-O-$\underline{CH_2}$), 4.02 (2H, m, NH-$\underline{CH_2}$), 4.52 (1H, m, NH-$\underline{CH}$-CO), 7.1-7.3 (4H, m, aromatic-H).

Preparation Example 43

$N^a$-[4-(Hydroxyamino)-2(R)-isobutyl-3(S)-hydroxysuccinyl]-4'-acetimidoyliminomethyl-L-phenylalanine N-methylamide • 1 acetate (Compound No. 43)

a) $N^a$-[3(S)-Hydroxy-2(R)-isobutyl-4-methoxysuccinyl]-L-4'-cyanophenylalanine N-methylamide (Compound No. 43-a)

The procedures of Preparation Examples 22-a and b were repeated using Compound No. 5 and Compound No. 21. The resultant product was re-solidified from chloroform-n-hexane to provide the title compound as a white solid (1.36 g, 50%).

$^1$H-NMR ($CDCl_3$)δ ppm; 0.7-1.1 (6H, m, CH$(\underline{CH_3})_2$), 1.0-1.6 (3H, m, $\underline{CH_2}$-$\underline{CH}$$(CH_3)_2$), 2.4-3.5 (6H, d + m, J = 4.8Hz, NH-$\underline{CH_3}$ + $\underline{CH}$-CO + $\underline{CH_2}$-$C_6H_4$), 3.80 (3H, s, O$\underline{CH_3}$), 4.24 (1H, m, NH-$\underline{CH}$-CO), 4.4-4.7 (1H, m, HO-$\underline{CH}$-CO), 6.35 (1H, m, NH), 6.76 (1H, m, OH), 7.4-7.6 (5H, m, aromatic-H + NH).

b) $N^a$-[4,3(S)-Dihydroxy-2(R)-isobutylsuccinyl]-L-4'-cyanophenylalanine N-methylamide (Compound No. 43-b)

To a solution of Compound No. 43-a (1.95 g, 5.0 mmol) in methanol (20 ml) was added 2N aqueous sodium hydroxide (10 ml, 20 mmol) with stirring at 0°C, and the mixture was stirred for 2 hours. After methanol was evaporated under reduced pressure, the reaction solution was adjusted with 6N hydrochloric acid to pH 2. The target product was extracted with AcOEt (10 ml x 2), and washed with saturated aqueous sodium chloride. The organic layer was dried over anhydrous $MgSO_4$, and evaporated under reduced pressure. The resulting residue was purified by column chromatography (silica gel; 30 g, eluted with a mixture of chloroform:methanol= 5:1 to 2:1) to give the title compound as a white solid (1.61 g, 86%), m.p.; 172-173°C, Rf value; 0.52 (chloroform:methanol:acetic acid= 5:2:1).

c) $N^a$-[4,3(S)-Dihydroxy-2(R)-isobutylsuccinyl]-4'-aminomethyl-L-phenylalanine N-methylamide • 1 hydrochloride (Compound No. 43-c)

To a solution of Compound No. 43-b (500 mg, 1.33mmol) in a mixture of DMF (10 ml) and conc. hydrochloric acid (550 μl, 6.66 mmol) was added 5% palladium on carbon (50% wet catalyst, 500 mg), and the mixture was vigorously stirred under hydrogen atmosphere for 15 hours at room temperature. The catalyst was filtered off and then DMF

was evaporated under reduced pressure. To the residue was added AcOEt to solidify a product which was collected by filtration to give the title compound as a white solid (479 mg, 82%).

$^1$H-NMR ($D_2O$)δ ppm; 0.64 (6H, m, CH(<u>$CH_3$</u>)$_2$), 0.9-1.4 (3H, m, <u>$CH_2$</u>-<u>CH</u>($CH_3$)$_2$), 2.4-2.6 (4H, s + m, NH-<u>$CH_3$</u> + <u>CH</u>-CO), 2.89 and 3.05 (1H each, m, $C_6H_4$-<u>$CH_2$</u>), 4.01 (2H, s, <u>$CH_2$</u>-$NH_2$), 4.4-4.7 (2H, m, HO-<u>CH</u>-CO + NH-<u>CH</u>-CO), 7.1-7.3 (4H, m, aromatic-H).

d) N$^a$-[4-(Hydroxyamino)-2(R)-isobutyl-3(S)-hydroxysuccinyl]-4'-acetimidoyliminomethyl-L-phenylalanine N-methylamide • 1 acetate (Compound No. 43)

Compound No. 43-c (330 mg, 0.79 mmol) was dissolved in DMF (10 ml), and cooled to 0°C, followed by addition of TEA (342 µl, 2.45 mmol) and ethyl acetimidate hydrochloride (110 mg, 0.87 mmol). The mixture was stirred for 30 minutes at 0°C and for another 30 minutes at room temperature, to which was HOBT (120 mg, 0.87 mmol), O-benzylhydroxylamine hydrochloride (260 mg, 1.66 mmol), EDC (230 mg, 1.19 mmol) and TEA (230 µl, 1.66 mmol) successively at 0°C. The resultant mixture was stirred for 2 hours at 4°C, and then evaporated under reduced pressure. The residue was purified by column chromatography (silica gel; 20 g, eluted with from a mixture of chloroform:methanol:acetic acid= 80:10:5 to a mixture of methanol:acetic acid= 1:1).

The purified product was dissolved in acetic acid (20 ml), followed by addition of 5% palladium on carbon (50% wet catalyst, 300 mg). The mixture was vigorously stirred under hydrogen atmosphere for 2 hours at room temperature. The catalyst was filtered off and then acetic acid was evaporated under reduced pressure. AcOEt (20 ml) was added to the residue, leading to precipitation of solids. The resulting solid was collected by filtration to give the title compound as a white solid (250 mg, 64%), Rf value; 0.16 (chloroform:methanol:acetic acid = 5:2:1), 0.50 (n-BuOH:AcOH: water= 4:1:1), FABMS ($M^+$ + 1): 436.

$^1$H-NMR ($CD_3OD$)δ ppm; 0.83 (6H, m, CH(<u>$CH_3$</u>)$_2$), 1.1-1.6 (3H, m, <u>$CH_2$</u>-<u>CH</u>($CH_3$)$_2$), 1.97 (3H, s, <u>$CH_3$</u>$CO_2$H), 2.25 (3H, s, C-<u>$CH_3$</u>), 2.6-2.8 (4H, s + m, NH-<u>$CH_3$</u> + <u>CH</u>-CO), 2.9-3.3 (2H, m, $C_6H_4$-<u>$CH_2$</u>), 4.03 (2H, s, <u>$CH_2$</u>-NH), 4.4-4.6 (2H, m, NH-<u>CH</u>-CO + HO-<u>CH</u>-CO), 7.28 (4H, m, aromatic-H).

<u>Preparation Example 44</u>

N$^a$-[4-(Hydroxyamino)-2(R)-isobutyl-3(RS)-(3-phenylpropyl)succinyl]-N$^e$-acetimidoyl-L-lysine N-(2-N',N'-dimethylaminoethyl)amide • 2 acetate (Compound No. 44)

a) N$^a$-[4-tert-Butoxy-2(R)-isobutyl-3(RS)-(3-phenylpropyl)succinyl]-N$^e$-tert-butyloxycarbonyl-L-lysine N-(2-N',N'-dimethylaminoethyl)amide (Compound No. 44-a)

The procedure of Preparation Example 31-a was repeated using Compound No. 3 and Compound No. 16 to provide the title compound as a white solid (yield 69%), m.p.; 102-105°C, Rf value; 0.38 (chloroform:methanol= 10:1).

b) N$^a$-[4-(Hydroxyamino)-2(R)-isobutyl-3(RS)-(3-phenylpropy)]succinyl]-N$^e$-acetimidoyl-L-lysine N-(2-N',N'-dimethylaminoethyl)amide • 2 acetate (Compound No. 44)

The procedure of Preparation Example 39-b was repeated using Compound No. 44-a (2.21 g, 3.41 mmol) to provide the title compound as amorphous white powders (overall yield 12%), m.p.; 95-101°C, Rf value; 0.082 (chloroform:methanol:acetic acid= 5:2:1), 0.14 (n-BuOH:AcOH:water= 4:1:1), FABMS ($M^+$ +2): 548.

<u>Preparation Example 45</u>

N$^a$-[4-(Hydroxyamino)-2(R)-isobutyl-3(RS)-methylsuccinyl]-N$^e$-propionimidoyl-L-lysine N-methylamide • 1 acetate (Compound No. 45)

The procedures of Preparation Examples 22-c, d and e except that ethyl acetimidate hydrochloride was replaced with ethyl propionimidate hydrochloride were repeated using Compound No. 22-b to provide the title compound as a white solid (overall yield 42%), m.p.; 113-119°C, Rf value; 0.15 (chloroform:methanol:acetic acid= 5:2:1), 0.47 (n-BuOH:AcOH: water= 4:1:1), FABMS ($M^+$ + 1): 400.

Preparation Example 46

$N^a$-[4-(Hydroxyamino)-2(R)-isobutyl-3(RS)-[3-(4'-guanidinophenyl)propyl]succinyl]-L-ornithine-N-methylamide • 2 acetate (Compound No. 46)

a) $N^a$-[4-tert-Butoxy-2(R)-isobutyl-3(RS)-[3-[p-($N^1$,$N^2$-dibenzyloxycarbonylguanidino)phenyl]propyl]succinyl]-$N^e$-benzyloxycarbonyl-L-lysine N-methylamide (Compound No. 46-a)

The procedures of Preparation Examples 22-a and b were repeated using Compound No. 7 and Compound No. 20 to provide the title compound as a colorless oil (yield 93%).

$^1$H-NMR ($CDCl_3$)δ ppm; 0.86 (6H, m, CH$\underline{(CH_3)_2}$), 1.0-1.9 (20H, s + m, $\underline{(CH_2)_2}$-$CH_2$-NH + $\underline{(CH_2)_2}$-$CH_2$-$C_6H_4$ + C$\underline{(CH_3)_3}$ + $\underline{CH_2CH}(CH_3)_2$), 2.2-2.8 (7H, m, $\underline{CH_2}$-$C_6H_4$ + $\underline{CH}$-CO x 2 + NH-$\underline{CH_3}$), 2.9-3.5 (2H, m, $\underline{CH_2}$-NH), 4.47 (1H, m, NH-$\underline{CH}$-CO), 5.0-5.3 (6H, m, $\underline{CH_2}$-Ph x 3), 6.4-6.7 (2H, m, NH x 2), 7.0-7.2 (2H, m, NH x 2), 7.2-7.5 (16H, m, aromatic-H + NH).

b) $N^a$-[4-(Hydroxyamino)-2(R)-isobutyl-3(RS)-[3-(4'-guanidinophenyl)propyl]succinyl]-L-ornithine-N-methylamide • 2 acetate (Compound No. 46)

The procedures of Preparation Examples 23-b and c were repeated using Compound No. 46-a to provide the title compound as amorphous white powders (overall yield 25%), Rf value; 0.04 (chloroform:methanol:acetic acid= 5:2:1), 0.34 (n-BuOH:AcOH; water= 4:1:1), FABMS ($M^+$ + 1): 492.

Preparation Example 47

$N^a$-[4-(Hydroxyamino)-2(R)-isobutyl-3(R or S)-(3-phenylpropyl)succinyl]-4'-aminomethyl-L-phenylalanine N-methylamide • 1 acetate (Compound No. 47)

The procedure of Preparation Example 23 was repeated using Compound No. 5 and Compound No. 16 to provide the title compound as a white solid.

FABMS ($M^+$ + 1):497.
$^1$H-NMR ($CD_3OD$)δ ppm; 0.8-1.1 (7H, m, $\underline{CH(CH_3)_2}$), 1.3-1.6 (6H, m, CH-$\underline{(CH_2)_2}$ + $\underline{CH_2}$-CH($CH_3$)$_2$), 1.89 (3H, s, $\underline{CH_3}CO_2H$), 2.10 (1H, m, $\underline{CH}$-CO), 2.3-2.5 (3H, m, $\underline{CH_2}$-Ph + $\underline{CH}$-CO), 2.63 (3H, s, NH-$\underline{CH_3}$), 2.8-3.1 (2H, m, $C_6H_4$-$\underline{CH_2}$), 3.93 (2H, m, $\underline{CH_2}$-$NH_2$), 4.58 (1H, m, NH-$\underline{CH}$-CO), 7.0-7.4 (9H, m, aromatic-H).

Preparation Example 48

$N^a$-[4-(Hydroxyamino)-2(R)-isobutyl-3(R or S)-(p-aminomethylbenzyl)succinyl]-L-ornithine N-methylamide • 2 acetate (Compound No. 48)

The procedure of Preparation Example 23 was repeated using 4-(p-benzyloxycarbonylaminomethylpheny)-3(RS)-tert-butyloxycarbonyl-2(R)-isobutylbutanoic acid and Compound No. 7 to provide the title compound as a white solid.

FABMS ($M^+$): 436.
$^1$H-NMR ($CD_3OD$)δ ppm; 0.7-1.0 (6H, m, CH$\underline{(CH_3)_2}$), 1.08 (1H, m, $\underline{CH}$($CH_3$)$_2$), 1.3-1.9 (6H, m, CH-$\underline{(CH_2)_2}$ + $\underline{CH_2}$-CH($CH_3$)$_2$), 1.84 (6H, s, $\underline{CH_3}CO_2H\times$ 2), 2.41 (1H, m, $\underline{CH}$-CO), 2.5-3.0 (8H, m, NH-$\underline{CH_3}$ + $\underline{CH_2}$-$NH_2$ + $\underline{CH}$-CO + $C_6H_4$-$\underline{CH_2}$), 3.95(2H, m, $C_6H_4$-$\underline{CH_2}$-$NH_2$), 4.34 (1H, m, NH-$\underline{CH}$-CO), 7.1-7.4 (4H, m, aromatic-H).

Preparation Example 49

$N^a$-[4-(Hydroxyamino)-2(R)-isobutyl-3(R or S)-(p-aminomethylbenzyl)succinyl]-4'-aminomethyl-L-phenylalanine N-methylamide • 2 acetate (Compound No. 49)

The procedure of Preparation Example 23 was repeated using 4-(p-benzyloxycarbonylaminomethylphenyl)-3(RS)-tert-butyloxycarbonyl-2(R)-isobutylbutanoic acid and Compound No. 5 to provide the title compound as a white solid.

FABMS ($M^+$ + 1): 498.

$^1$H-NMR ($CD_3OD$)δ ppm; 0.7-0.85 (6H, m, $CH(\underline{CH_3})_2$), 0.90 (1H, m, $\underline{CH}(CH_3)_2$), 1.2-1.4 (2H, m, $\underline{CH_2}$-$CH(CH_3)_2$), 1.84 (6H, s, $\underline{CH_3}CO_2H \times 2$), 2.07 (1H, m, $\underline{CH}$-CO), 2.41 (1H, m, $\underline{CH}$-CO), 2.64 (3H, m, NH-$\underline{CH_3}$), 2.81 and 3.06 (1H each, m, $C_6H_4$-$\underline{CH_2}$), 3.2 (m, $C_6H_4$-$\underline{CH_2}$), 3.93 (2H, m, $\underline{CH_2}$-$NH_2$), 4.77 (1H, m, NH-$\underline{CH}$-CO), 6.84 , 7.05 and 7.1-7.3 (8H, m, aromatic-H).

Preparation Example 50

$N^a$-[4-(Hydroxyamino)-2(R)-isobutyl-3(R or S)-[p-(p-toluenesulfonamidomethyl)benzyl]succinyl]-L-arginine N-methylamide • 1 acetate (Compound No. 50)

The procedure of Preparation Example 23 was repeated using 4-[p-(p-toluenesulfonamidomethyl)phenyl]-3(RS)-tert-butyloxycarbonyl-2(R)-isobutylbutanoic acid and Compound No. 9 to provide the title compound as a white solid.

FABMS ($M^+$ ): 632.

$^1$H-NMR ($CD_3OD$)δ ppm; 0.8-1.0 (6H, m, $CH(\underline{CH_3})_2$), 1.0-1.1 (1H, m, $\underline{CH}(CH_3)_2$), 1.4-1.8 (6H, m, CH-$\underline{(CH_2)_2}$ + $\underline{CH_2}$-$CH(CH_3)_2$), 1.89 (3H, s, $\underline{CH_3}CO_2H$), 2.39 (1H, m, $\underline{CH}$-CO), 2.43 (3H, s, $C_6H_4$-$\underline{CH_3}$), 2.5-2.8 (6H, m, $\underline{CH}$-CO + NH-$\underline{CH_3}$ + $C_6H_4$-$\underline{CH_2}$), 3.18 (2H, m, $\underline{CH_2}$-NH), 3.93 (2H, s, $C_6H_4$-$\underline{CH_2}$-NH), 4.38 (1H, m, NH-$\underline{CH}$-CO), 7.01 , 7.21 , 7.36 and 7.71 (2H each, m, aromatic-H).

Preparation Example 51

$N^a$-[4-(Hydroxyamino)-2(R)-isobutyl-3(R or S)-(p-phthalimidomethylbenzyl)succinyl]-L-arginine N-methylamide • 1 acetate (Compound No. 51)

The procedure of Preparation Example 23 was repeated using 4-(p-phthalimidomethylphenyl)-3(RS)-tert-butyloxycarbonyl-2(R)-isobutylbutanoic acid and Compound No. 9 to provide the title compound as a white solid.

FABMS ($M^+$ ): 608.

$^1$H-NMR ($CD_3OD$)δ ppm; 0.8-0.9 (6H, m, $CH(\underline{CH_3})_2$), 1.06 (1H, m, $\underline{CH}(CH_3)_2$), 1.4-1.85 (6H, m, CH-$\underline{(CH_2)_2}$ + $\underline{CH_2}$-$CH(CH_3)_2$), 1.90 (3H, s, $\underline{CH_3}CO_2H$), 2.39 (1H, m, $\underline{CH}$-CO), 2.5-2.8 (6H, m, $\underline{CH}$-CO + NH-$\underline{CH_3}$ + $C_6H_4$-$\underline{CH_2}$), 3.16 (2H, m, $\underline{CH_2}$-NH), 4.39 (1H, m, NH-$\underline{CH}$-CO), 4.75 (2H, s, $C_6H_4$-$\underline{CH_2}$-N), 7.05 and 7.23 (2H each, m, aromatic-H), 7.7-7.9 (4H, m, aromatic-H).

Preparation Example 52

$N^a$-[4-(Hydroxyamino)-2(R)-isobutyl-3(R or S)-(p-methanesulfonamidomethylbenzyl)succinyl]-L-arginine N-methylamide • 1 acetate (Compound No. 52)

The procedure of Preparation Example 23 was repeated using 4-(p-methanesulfonamidomethylphenyl)-3(RS)-tert-butyloxycarbonyl-2(R)-isobutylbutanoic acid and Compound No. 9 to provide the title compound as a white solid.

$^1$H-NMR ($CD_3OD$)δ ppm; 0.8-0.95 (6H, m, $CH(\underline{CH_3})_2$), 1.08 (1H, m, $\underline{CH}(CH_3)_2$), 1.4-1.85 (6H, m, CH-$\underline{(CH_2)_2}$ + $\underline{CH_2}$-$CH(CH_3)_2$), 1.89 (3H, s, $\underline{CH_3}CO_2H$), 1.96 (3H, s, $\underline{CH_3}$-$SO_2$), 2.41 (1H, m, $\underline{CH}$-CO), 2.5-2.8 (6H, m, $\underline{CH}$-CO + NH-$\underline{CH_3}$ + $C_6H_4$-$\underline{CH_2}$), 3.26 (2H, m, $\underline{CH_2}$-NH), 4.28 (2H, s, $C_6H_4$-$\underline{CH_2}$-NH), 4.39 (1H, m, NH-$\underline{CH}$-CO), 7.05 and 7.14 (2H each, m, aromatic-H).

Preparation Example 53

$N^a$-[4-(Hydroxyamino)-2(R)-isobuty-3(R or S)-(m-aminomethylbenzyl)succinyl]-L-alanine N-methylamide • 1 acetate (Compound No. 53)

The procedure of Preparation Example 23 was repeated using 4-(m-benzyloxycarbonylaminomethylphenyl)-3(RS)-tert-butyloxycarbonyl-2(R)-isobutylbutanoic acid and $N^a$-tert-butyloxycarbonyl-L-alanine N-methylamide to provide the title compound as a white solid.

FABMS ($M^+$): 393.

$^1$H-NMR ($CD_3OD$)δ ppm; 0.85-0.95 (6H, m, CH($\underline{CH_3}$)$_2$), 1.08 (1H, m, $\underline{CH}$($CH_3$)$_2$), 1.36 (3H, d, J =7.2Hz, CH-$\underline{CH_3}$), 1.4-1.7 (2H, m, $\underline{CH_2}$-CH($CH_3$)$_2$), 1.91 (3H, s, $\underline{CH_3}CO_2H$), 2.42 (1H, m, $\underline{CH}$-CO), 2.6-2.9 (6H, m, $\underline{CH}$-CO + NH-$\underline{CH_3}$ + $C_6H_4$-$\underline{CH_2}$), 4.03 (2H, s, $C_6H_4$-$\underline{CH_2}$-$NH_2$), 4.40 (1H, m, NH-$\underline{CH}$-CO), 7.1-7.4 (4H, m, aromatic-H).

Preparation Example 54

$N^a$-[4-(Hydroxyamino)-2(R)-isobutyl-3(R or S)-(2-phenoxyethyl)succinyl]-L-arginine N-methylamide • acetate (Compound No. 54)

The procedure of Preparation Example 23 was repeated using 5-phenoxy-3(RS)-tert-butyloxycarbonyl-2(R)-isobutylpentanoic acid and Compound No. 9 to provide the title compound as a white solid.

FABMS ($M^+$): 479.

$^1$H-NMR ($CD_3OD$)δ ppm; 0.8-0.95 (6H, m, CH($\underline{CH_3}$)$_2$), 1.10 (1H, m, $\underline{CH}$($CH_3$)$_2$), 1.4-1.85 (8H, m, $\underline{CH_2}$-$CH_2$-O + CH-($\underline{CH_2}$)$_2$ + $\underline{CH_2}$-CH($CH_3$)$_2$ 1.89 (3H, s, $\underline{CH_3}CO_2H$), 2.45 (1H, m, $\underline{CH}$-CO), 2.65(1H, m, $\underline{CH}$-CO), 2.72 (3H, s, NH-$\underline{CH_3}$), 3.15 (2H, m, $\underline{CH_2}$-NH), 3.8-4.0 (O-$\underline{CH_2}$), 4.34 (1H, m, NH-$\underline{CH}$-CO), 6.88 and 7.23 (2H each, m, aromatic-H).

Preparation Example 55

$N^a$-[4-(Hydroxyamino)-2(R)-isobutyl-3(R or S)-(3-cyclohexylpropyl)succinyl]-L-arginine N-methylamide • 1 acetate (Compound No. 55)

The procedure of Preparation Example 23 was repeated using 6-cyclohexyl-3(RS)-tert-butyloxycarbonyl-2(R)-isobutylhexanoic acid and Compound No. 9 to provide the title compound as a white solid.

FABMS ($M^+$ + 1): 484.

$^1$H-NMR (DMSO-$d_6$)δ ppm; 0.6-1.8 (33H, m, $\underline{CH_2}$-CH($\underline{CH_3}$)$_2$ + CH-($\underline{CH_2}$)$_3$-$\underline{C_5H_{11}}$ + CH-($\underline{CH_2}$)$_2$ + $\underline{CH_3}CO_2H$), 1.99 (1H, m, $\underline{CH}$-CO), 2.49 (1H, m, $\underline{CH}$-CO), 2.55 (3H, d, J =4.4Hz, NH-$\underline{CH_3}$), 3.00 (2H, m, $\underline{CH_2}$-NH), 4.20 (1H, m, NH-$\underline{CH}$-CO), 8.05 and 8.23 (1H each, m, NH× 2).

Preparation Example 56

$N^a$-[4-(Hydroxyamino)-2(R)-(2-naphthylmethyl)-3(R or S)-(3-phenylpropyl)succinyl]-L-arginine N-methylamide • 1 acetate (Compound No. 56)

The procedure of Preparation Example 23 was repeated using 6-phenyl-3(RS)-tert-butyloxycarbonyl-2(R)-(2-naphthylmethyl)hexanoic acid and Compound No. 9 to provide the title compound as a white solid.

FABMS ($M^+$ + 1): 562.

$^1$H-NMR ($CD_3OD$)δ ppm; 1.2-1.8 (8H, m, CH-($\underline{CH_2}$)$_2$× 2), 1.90 (3H, s, $\underline{CH_3}CO_2H$), 2.3-3.1 (11H, m, $\underline{CH}$-CO × 2 + $\underline{CH_2}$-Ph + $\underline{CH_2}$-Naphthyl + $\underline{CH_2}$-NH + N-$\underline{CH_3}$), 4.08 (1H, m, NH-$\underline{CH}$-CO), 7.1-7.9 (12H, m, aromatic-H).

Preparation Example 57

$N^a$-[4-(Hydroxyamino)-2(R)-isobutyl-3(R or S)-(o-aminomethylbenzyl)succinyl]-L-alanine N-methylamide • 1 acetate (Compound No. 57)

The procedure of Preparation Example 23 was repeated using 4-(o-benzyloxycarbonylaminomethylphenyl)-3(RS)-tert-butyloxycarbonyl-2(R)-isobutylbutanoic acid and $N^a$-tert-butyloxycarbonylL-alanine N-methylamide to provide the title compound as a white solid.

FABMS ($M^+$): 393.

$^1$H-NMR ($CD_3OD$)δ ppm; 0.8-1.0 (6H, m, CH($\underline{CH_3}$)$_2$), 1.09 (1H, m, $\underline{CH}$($CH_3$)$_2$), 1.38 (3H, d, J =4.7Hz, CH-$\underline{CH_3}$), 1.60 (2H, m, $\underline{CH_2}$-CH($CH_3$)$_2$), 1.89 (3H, s, $\underline{CH_3}CO_2H$), 2.53 (1H, m, $\underline{CH}$-CO), 2.65-2.95 (6H, m, $\underline{CH}$-CO + NH-$\underline{CH_3}$ + $C_6H_4$-$\underline{CH_2}$), 4.13 (2H, s, $\underline{CH_2}$-$NH_2$), 4.42 (1H, m, NH-$\underline{CH}$-CO), 7.1-7.4 (4H, m, aromatic-H).

Preparation Example 58

$N^a$-[4-(Hydroxyamino)-2(R)-isobuty-3(RS)-[3-(p-aminomethylphenyl)propyl]succinyl]-L-alanine N-methylamide • 1 acetate (Compound No. 58)

The procedure of Preparation Example 23 was repeated using 6-(p-benzyloxycarbonylaminomethylphenyl)-3(RS)-tert-butyloxycarbonyl-2(R)-isobutylhexanoic acid and $N^a$-tert-butyloxycarbonyl-L-alanine N-methylamide to provide the title compound as a white solid.

FABMS ($M^+$): 421.
$^1$H-NMR ($CD_3OD$)δ ppm; 0.7-0.85 (6H, m, CH($\underline{CH_3}$)$_2$), 0.93 (1H, m, $\underline{CH}$($CH_3$)$_2$), 1.17 (3H, d, J =7.0Hz, CH-$\underline{CH_3}$), 1.2-1.6 (6H, m, CH-($\underline{CH_2}$)$_2$ + $\underline{CH_2}$-CH($CH_3$)$_2$), 1.80 (3H, s, $\underline{CH_3}CO_2H$), 2.06 (1H, m, $\underline{CH}$-CO), 2.4-2.55 (3H, m, $\underline{CH}$-CO + $C_6H_4$-$\underline{CH_2}$), 2.62 (3H, s, NH-$\underline{CH_3}$), 3.92 (2H, s, $\underline{CH_2}$-$NH_2$), 4.20 (1H, m, NH-$\underline{CH}$-CO), 7.11 and 7.21 (2H each, m, aromatic-H).

Preparation Example 59

$N^a$-[4-(Hydroxyamino)-2(R)-isobutyl-3(R or S)-(m-isopropyliminomethylbenzyl)succinyl]-L-alanine N-methylamide • 1 acetate (Compound No. 59)

To a mixture of Compound No. 53 (400 mg, 0.884 mmol), acetone (154 mg, 2.65 mmol) and methanol (8 ml) was added sodium cyanoborohydride (67 mg, 1.07 mmol) while cooling in an ice bath, and the mixture was stirred overnight at room temperature. After methanol was evaporated under reduced pressure, the residue was dissolved in 5% aqueous acetic acid, purified by column reversed phase chromatography (25 g of Chromatorex ODS-1020T, Fuji Silysia Chemical, Japan; eluted with a gradient of 1% to 5% methanol/0.1% aqueous acetic acid), and then lyophilized to give the title compound as a white solid.

FABMS ($M^+$) : 435.
$^1$H-NMR ($CD_3OD$)δ ppm; 0.8-1.0 (6H, m, CH($\underline{CH_3}$)$_2$), 1.08 (1H, m, $\underline{CH}$($CH_3$)$_2$), 1.36 (9H, m, CH-$\underline{CH_3}$ + NH-CH($\underline{CH_3}$)$_2$), 1.55 (2H, m, $\underline{CH_2}$-CH($CH_3$)$_2$), 1.89 (3H, s, $\underline{CH_3}CO_2H$), 2.22 (1H, m, $\underline{CH}$-CO), 2.6-2.9 (6H, m, $\underline{CH}$-CO + NH-$\underline{CH_3}$ + $C_6H_4$-$\underline{CH_2}$), 3.37 (1H, m, NH-$\underline{CH}$($CH_3$)$_2$), 4.14 (2H, s, $\underline{CH_2}$-$NH_2$), 4.40 (1H, m, NH-$\underline{CH}$-CO), 7.15-7.4 (4H, m, aromatic-H).

Preparation Example 60

$N^a$-[4-(Hydroxyamino)-2(R)-isobutyl-3(R or S)-[3-(o-aminomethylphenyl)propyl]succinyl]-L-alanine N-methylamide • 1 acetate (Compound No. 60)

The procedure of Preparation Example 23 was repeated using 6-(o-benzyloxycarbonylaminomethylphenyl)-3(RS)-tert-butyloxycarbonyl-2(R)-isobutylhexanoic acid and $N^a$-tert-butyloxycarbonyl-L-alanine N-methylamide to give the title compound as a white solid.

FABMS ($M^+$): 421.
$^1$H-NMR (DMSO-$d_6$)δ ppm; 0.7-1.0 (7H, m, $\underline{CH(CH_3)_2}$), 1.13 (3H, d, J=6.9Hz, CH-$\underline{CH_3}$), 1.1-1.6 (6H, m, CH-($\underline{CH_2}$)$_2$ + $\underline{CH_2}$-CH($CH_3$)$_2$), 1.81 (3H, s, $\underline{CH_3}CO_2H$), 2.06 (1H, m, $\underline{CH}$-CO), 2.4-2.6 (m, $\underline{CH}$-CO + $C_6H_4$-$\underline{CH_2}$ + NH-$\underline{CH_3}$), 3.70 (2H, s, $\underline{CH_2}$-$NH_2$), 4.23 (1H, m, NH-$\underline{CH}$-CO), 7.0-7.2 and 7.32 (4H, m, aromatic-H), 7.63 and 8.16 (1H each, m, NH× 2).

Preparation Example 61

$N^a$-[4-(Hydroxyamino)-2(R)-isobutyl-3(R or S)-[3-(p-aminomethylphenyl)propyl]succinyl]-L-lysine N-methylamide • 2 acetate (Compound No. 61)

The procedure of Preparation Example 23 was repeated using 6-(p-benzyloxycarbonylaminomethylphenyl)-3(RS)-tert-butyloxycarbonyl-2(R)-isobutylhexanoic acid and Compound No. 1 to give the title compound as a white solid.

FABMS ($M^+$): 479.

$^1$H-NMR ($CD_3OD$)δ ppm; 0.8-1.0 (6H, m, CH($CH_3$)$_2$), 1.06 (1H, m, CH($CH_3$)$_2$), 1.2-1.9 (12H, m, CH-($CH_2$)$_3$ + CH-($CH_2$)$_2$ + $CH_2$-CH($CH_3$)$_2$), 1.92 (6H, s, $CH_3CO_2H$× 2), 2.19 (1H, m, CH-CO), 2.45-2.7 (3H, m, CH-CO + $C_6H_4$-$CH_2$), 2.72(3H, s, NH-$CH_3$), 2.91 (2H, m, $CH_2$-$NH_2$), 4.06 (2H, s, $C_6H_4$-$CH_2$-$NH_2$), 4.28 (1H, m, NH-CH-CO), 7.22 and 7.35 (2H each, m, aromatic-H).

Preparation Example 62

N$^a$-[4-(Hydroxyamino)-2(R)-isobutyl-3(R or S)-[5-(acetimidoylimino)pentyl]succinyl]-L-alanine N-methylamide (Compound No. 62)

N$^a$-[4-(Hydroxyamino)-2(R)-isobutyl-3(R or S)-(5-aminopentyl)succinyl]-L-alanine N-methylamide • 1 acetate (200 mg, 0.48 mmol) was dissolved in DMF (6 ml), and then cooled to 0°C, followed by addition of TEA (200 ml, 1.44 mmol) and ethyl acetimidate hydrochloride (124 mg, 1.01 mmol). The mixture was stirred for 2 hours. DMF was evaporated under reduced pressure and the residue was dissolved in 5% aqueous acetic acid, purified by column reversed phase chromatography (25 g of Chromatorex ODS-1020T, Fuji Silysia Chemical, Japan; eluted with distilled water and 0.1% aqueous acetic acid), and then lyophilized to give the title compound as a white solid.

FABMS ($M^+$): 400.
$^1$H-NMR ($CD_3OD$)δ ppm; 0.8-0.95 (6H, m, CH($CH_3$)$_2$), 1.04 (1H, m, CH($CH_3$)$_2$), 1.2-1.7 (13H, m, CH-($CH_2$)$_4$ + CH-$CH_3$ + $CH_2$-CH($CH_3$)$_2$), 2.13 (1H, m, CH-CO), 2.20 (3H, s, C-$CH_3$), 2.54 (1H, m, CH-CO), 2.72 (3H, s, NH-$CH_3$), 3.19 (2H, m, $CH_2$-NH), 4.34 (1H, m, NH-CH-CO).

Preparation Example 63

N$^a$-[4-(Hydroxyamino)-2(R)-isobutyl-3(R or S)-[5-(isopropylimino)pentyl]succinyl]-L-alanine N-methylamide (Compound No. 63)

The procedure of Preparation Example 59 was repeated using N$^a$-[4-(hydroxyamino)-2(R)-isobutyl-3(R or S)-(5-aminopentyl)succinyl]-L-alanine N-methylamide • 1 acetate to give the title compound as a white solid.

FABMS ($M^+$): 401.
$^1$H-NMR ($CD_3OD$)δ ppm; 0.8-0.95 (6H, m, CH($CH_3$)$_2$), 1.06 (1H, m, CH($CH_3$)$_2$), 1.25-1.8 (19H, m, NH-CH($CH_3$)$_2$ + CH-($CH_2$)$_4$ + CH-$CH_3$ + $CH_2$-CH($CH_3$)$_2$), 2.15 (1H, m, CH-CO), 2.56 (1H, m, CH-CO), 2.60 (3H, s, N-$CH_3$), 2.96 (2H, m, $CH_2$-NH), 3.36 (m, NH-CH($CH_3$)$_2$) 4.35 (1H, m, NH-CH-CO).

Preparation Example 64

N$^a$-[4-(Hydroxyamino)-2(R)-isobutyl-3(R or S)-[5-(pyridin-4-ylmethylimino)pentyl]succinyl]-L-alanine N-methylamide • 1 acetate (Compound No. 64)

The prodedure of Preparation Example 59 was repeated using N$^a$-[4-(hydroxyamino)-2(R)-isobutyl-3(R or S)-(5-aminopentyl)succinyl)-L-alanine N-methylamide • 1 acetate, except that acetone was replaced with 4-pyridylaldehyde, to give the title compound as a white solid.

FABMS ($M^+$): 450.
$^1$H-NMR ($CD_3OD$)δ ppm; 0.8-0.95 (6H, m, CH($CH_3$)$_2$), 1.06 (1H, m, CH($CH_3$)$_2$), 1.1-1.7 (13H, m, CH-($CH_2$)$_4$ + CH-$CH_3$ + $CH_2$-CH($CH_3$)$_2$), 1.93 (3H, s, $CH_3CO_2H$), 2.13 (1H, m, CH-CO), 2.54 (1H, m, CH-CO), 2.71 (3H, s, N-$CH_3$), 2.87 (2H, m, $CH_2$-NH), 4.02 (2H, m, NH-$CH_2$-Pyridyl), 4.40 (1H, m, NH-CH-CO), 7.47 and 8.55 (2H each, m, aromatic-H).

Preparation Example 65

N$^a$-[4-(Hydroxyamino)-2(R)-isobutyl-3(R or S)-[3-(p-methoxycarbonylphenyl)propyl]succinyl]-L-lysine N-methylamide • 1 acetate (Compound No. 65)

The procedure of Preparation Example 23 was repeated using 6-(p-methoxycarbonylphenyl)-3(RS)-tert-butyloxycarbonyl-2(R)-isobutylhexanoic acid and Compound No. 1 to give the title compound as a white solid.

FABMS ($M^+$ + 1): 508.

$^1$H-NMR ($CD_3OD$)δ ppm; 0.8-0.9 (6H, m, $CH\underline{(CH_3)_2}$), 1.04 (1H, m, $\underline{CH}(CH_3)_2$), 1.2-1.8 (12H, m, $CH\text{-}\underline{(CH_2)_3}$ + $CH\text{-}\underline{(CH_2)_2}$ + $\underline{CH_2}\text{-}CH(CH_3)_2$), 1.91 (6H, s, $\underline{CH_3}CO_2H$), 2.20 (1H, m, $\underline{CH}$-CO), 2.53 (1H, m, $\underline{CH}$-CO), 2.63 (2H, m, $C_6H_4\text{-}\underline{CH_2}$), 2.71 (3H, s, $\underline{N\text{-}CH_3}$), 2.89 (2H, m, $\underline{CH_2}\text{-}NH_2$), 3.88 (3H, s, $\text{-}CO_2\underline{CH_3}$), 4.24 (1H, m, NH-$\underline{CH}$-CO), 7.26 and 7.90 (2H each, m, aromatic-H).

Preparation Example 66

$N^a$-[4-(Hydroxyamino)-2(R)-isobutyl-3(R or S)-[2-(2-ethoxyethoxy)ethyl]succinyl]-L-lysine N-methylamide (Compound No. 66)

The procedure of Preparation Example 23 was repeated using 5-(2-ethoxyethoxy)-3(RS)-tert-butyloxycarbonyl-2(R)-isobutylpentanoic acid and Compound No. 1 to give the title compound as a white solid.

FABMS ($M^+$ + 1): 448.

$^1$H-NMR ($CD_3OD$)δ ppm; 0.8-0.95 (6H, m, $CH\underline{(CH_3)_2}$), 1.07 (1H, m, $\underline{CH}(CH_3)_2$), 1.20 (3H,t, J = 7.0Hz, $CH_2\text{-}\underline{CH_3}$), 1.3-1.9 (10H, m, $CH\text{-}\underline{(CH_2)_3}$ + $CH\text{-}\underline{CH_2}$ +$\underline{CH_2}\text{-}CH(CH_3)_2$), 2.32 (1H, m, $\underline{CH}$-CO), 2.58 (1H, m, $\underline{CH}$-CO), 2.72 (3H, s, $N\text{-}\underline{CH_3}$), 2.92 (2H, m, $\underline{CH_2}\text{-}NH_2$), 3.2-3.4 (8H, m, $O\text{-}\underline{CH_2} \times 4$), 4.32 (1H, m, NH-$\underline{CH}$-CO).

Preparation Example 67

$N^a$-[4-(Hydroxyamino)-2(R)-isobutyl-3(R or S)-[3-(p-carboxyphenyl)propyl]succinyl]-L-lysine N-methylamide (Compound No. 67)

Alkaline hydrolysis of Compound No. 65 yielded the title compound as a white solid.

FABMS ($M^+$): 493.

$^1$H-NMR ($D_2O$-NaOD) δ ppm; 0.8-0.9 (6H, m, $CH\underline{(CH_3)_2}$), 0.95-1.7 (13H, m, $CH\text{-}\underline{(CH_2)_3}$ + $CH\text{-}\underline{(CH_2)_2}$ + $\underline{CH_2\text{-}CH}(CH_3)_2$), 2.15 (1H, m, $\underline{CH}$-CO), 2.4-2.6 (3H, m, $\underline{CH}$-CO + $\underline{CH_2}\text{-}NH_2$), 2.6-2.8 (5H, m, $N\text{-}\underline{CH_3}$ + $C_6H_4\text{-}\underline{CH_2}$), 4.02 (1H, m, NH-$\underline{CH}$-CO), 7.26 and 7.81 (2H each, m, aromatic-H).

Preparation Example 68

$N^a$-[4-(Hydroxyamino)-2(R)-isobutyl-3(R or S)-(8-hydroxyoctyl)succinyl)-L-lysine N-methylamide • 1 acetate (Compound No. 68)

The procedure of Preparation Example 23 was repeated using 11-(tetrahydropyranyloxy)-3(RS)-tert-butyloxycarbonyl-2(R)-isobutylundecanoic acid and Compound No. 1 to give the title compound as a white solid.

FABMS ($M^+$): 459.

$^1$H-NMR ($CD_3OD$)δ ppm; 0.8-0.95 (6H, m, $CH\underline{(CH_3)_2}$), 1.05 (1H, m, $\underline{CH}(CH_3)_2$), 1.1-1.9 (22H, m, $CH\text{-}\underline{(CH_2)_7}$ + $CH\text{-}\underline{(CH_2)_3}$ + $\underline{CH_2}\text{-}CH(CH_3)_2$) 1.92 (3H, s, $\underline{CH_3}CO_2H$), 2.13 (1H, m, $\underline{CH}$-CO), 2.56 (1H, m, $\underline{CH}$-CO), 2.72 (3H, s, $N\text{-}\underline{CH_3}$), 2.91 (2H, m, $\underline{CH_2}\text{-}NH_2$), 3.53 (2H,t, J=6.5Hz, $\underline{CH_2}$-OH), 4.31 (1H, m, NH-$\underline{CH}$-CO).

Preparation Example 69

$N^a$-[4-(Hydroxyamino)-2(R)-isobutyl-3(R or S)-(2-n-butyloxyethyl)succinyl]-L-lysine N-methylamide • 1 acetate (Compound No. 69)

The procedure of Preparation Example 23 was repeated using 5-(n-butyloxy)-3(RS)-tert-butyloxycarbonyl-2(R)-isobutylpentanoic acid and Compound No. 1 to give the title compound as a white solid.

FABMS ($M^+$ 1): 432.

$^1$H-NMR ($CD_3OD$)δ ppm; 0.8-0.95 (9H, m, $CH\underline{(CH_3)_2}$ + $CH_2\text{-}\underline{CH_3}$), 1.06 (1H, m, $\underline{CH}(CH_3)_2$), 1.2-1.9 (14H, m, $CH\text{-}\underline{CH_2}$ + $CH\text{-}\underline{(CH_2)_3}$ + $\underline{(CH_2)_2}\text{-}CH_3$ + $\underline{CH_2}\text{-}CH(CH_3)_2$), 1.91 (3H, s, $\underline{CH_3}CO_2H$), 2.29 (1H, m, $\underline{CH}$-CO), 2.58 (1H, m, $\underline{CH}$-CO), 2.72 (3H, s, $N\text{-}\underline{CH_3}$), 2.91 (2H, m, $\underline{CH_2}\text{-}NH_2$), 3.2-3.4 (m, $O\text{-}\underline{CH_2} \times 2$), 4.31 (1H, m, NH-$\underline{CH}$-CO).

Preparation Example 70

$N^a$-[4-(Hydroxyamino)-2(R)-isobutyl-3(R or S)-(2-isobutyloxyethyl)succinyl)-L-lysine N-methylamide • 1 acetate (Compound No. 70)

The procedure of Preparation Example 23 was repeated using 5-(isobutyloxy)-3(RS)-tert-butyloxycarbonyl-2(R)-isobutylpentanoic acid and Compound No. 1 to give the title compound as a white solid.

FABMS ($M^+$): 431.
$^1$H-NMR ($CD_3OD$)δ ppm; 0.8-0.95 (12H, m, CH$\underline{(CH_3)_2}$× 2), 1.07 (1H, m, $\underline{CH}(CH_3)_2$), 1.3-1.9 (11H, m, CH-$\underline{CH_2}$ + CH-$\underline{(CH_2)_3}$ + O-$CH_2$-$\underline{CH}$ + $\underline{CH_2}$-$CH(CH_3)_2$), 1.92 (3H, s, $\underline{CH_3}CO_2H$), 2.30 (1H, m, $\underline{CH}$-CO), 2.58 (1H, m, $\underline{CH}$-CO), 2.72 (3H, s, N-$\underline{CH_3}$), 2.92 (2H, m, $\underline{CH_2}$-$NH_2$), 3.13 (2H, d, J=6.6Hz, O-$\underline{CH_2}$-CH), 3.2-3.4 (m, O-$\underline{CH_2}$), 4.32 (1H, m, NH-$\underline{CH}$-CO).

Preparation Example 71

$N^a$-[4-(Hydroxymino)-2(R)-isobutyl-3(R or S)-[3-(m-methoxycarbonylphenyl)propyl]succinyl]-L-lysine N-methylamide • 1 acetate (Compound No. 71)

The procedure of Preparation Example 23 was repeated using 6-(m-methoxycarbonylphenyl)-3(RS)-tert-butyloxycarbonyl-2(R)-isobutylhexanoic acid and Compound No. 1 to give the title compound as a white solid.

FABMS ($M^+$ + 1): 508.
$^1$H-NMR ($CD_3OD$)δ ppm; 0.8-1.0 (6H, m, CH$\underline{(CH_3)_2}$), 1.06 (1H, m, $\underline{CH}(CH_3)_2$), 1.2-1.85(12H, m, CH-$\underline{(CH_2)_3}$ + CH-$\underline{(CH_2)_2}$ + $\underline{CH_2}$-$CH(CH_3)_2$ 1.91 (3H, s, $\underline{CH_3}CO_2H$), 2.20 (1H, m, $\underline{CH}$-CO), 2.5-2.8 (6H, m, $\underline{CH}$-CO + N-$\underline{CH_3}$ + $\underline{CH_2}$-$NH_2$), 2.91 (2H, m, $C_6H_4$-$\underline{CH_2}$), 3.90 (3H, s, -$CO_2\underline{CH_3}$), 4.27 (1H, m, NH-$\underline{CH}$-CO), 7.39 and 7.82 (2H each, m, aromatic-H).

Preparation Example 72

$N^a$-[4-(Hydroxyamino)-2(R)-isobutyl-3(R or S)-[3-(p-hydroxyphenyl)propyl]succinyl]-L-lysine N-methylamide • 1 acetate (Compound No. 72)

The procedure of Preparation Example 23 was repeated using 6-(p-benzyloxyphenyl)-3(RS)-tert-butyloxycarbonyl-2(R)-isobutylhexanoic acid and Compound No. 1 to give the title compound as a white solid.

FABMS ($M^+$): 465.
$^1$H-NMR ($CD_3OD$)δ ppm; 0.8-0.95 (6H, m, CH$\underline{(CH_3)_2}$), 1.04 (1H, m, $\underline{CH}(CH_3)_2$), 1.2-1.85 (12H, m, CH-$\underline{(CH_2)_3}$ + CH-$\underline{(CH_2)_2}$ + $\underline{CH_2}$-$CH(CH_3)_2$), 1.92 (3H, s, $\underline{CH_3}CO_2H$), 2.17 (1H, m, $\underline{CH}$-CO), 2.35-2.6 (3H, m, $\underline{CH}$-CO + $C_6H_4$-$\underline{CH_2}$), 2.71 (3H, m, N-$\underline{CH_3}$), 2.88 (2H, m, $\underline{CH_2}$-$NH_2$), 4.25 (1H, m, NH-$\underline{CH}$-CO), 6.67 and 6.94 (2H each, m, aromatic-H).

Preparation Example 73

$N^a$-[4-(Hydroxyamino)-2(R)-isobutyl-3(R or S)-[3-(morpholin-4-yl)propyl]succinyl]-L-lysine N-methylamide • 1 acetate (Compound No. 73)

The procedure of Preparation Example 23 was repeated using 6-morpholino-3(RS)-tert-butyloxycarbonyl-2(R)-isobutylhexanoic acid and Compound No. 1 to give the title compound as a white solid.

FABMS ($M^+$): 458.
$^1$H-NMR ($CD_3OD$)δ ppm; 0.7-0.9 (6H, m, CH$\underline{(CH_3)_2}$), 1.02 (1H, m, $\underline{CH}(CH_3)_2$), 1.2-1.8 (12H, m, CH-$\underline{(CH_2)_3}$ + CH-$\underline{(CH_2)_2}$ + $\underline{CH_2}$-$CH(CH_3)_2$), 1.89 (3H, s, $\underline{CH_3}CO_2H$), 2.20 (1H, m, $\underline{CH}$-CO), 2.5-3.0 (12H, m, $\underline{CH}$-CO + N-$\underline{CH_2}$ × 3 + N-$\underline{CH_3}$ + $\underline{CH_2}$-$NH_2$), 3.74 (4H, m, O-$\underline{CH_2}$× 2), 4.28 (1H, m, NH-$\underline{CH}$-CO).

Preparation Example 74

$N^a$-[4-(Hydroxyamino)-2(R)-isopropyl-3(R or S)-(3-phenylpropyl)succinyl]-L-lysine N-methylamide • 1 acetate (Compound No. 74)

The procedure of Preparation Example 23 was repeated using 6-phenyl-3(RS)-tert-butyloxycarbonyl-2(R)-isopropylhexanoic acid and Compound No. 1 to give the title compound as a white solid.

FABMS ($M^+$ + 1): 436.
$^1$H-NMR ($CD_3OD$)δ ppm; 0.85-1.0 (6H, m, CH$\underline{(CH_3)_2}$), 1.3-1.9 (11H, m, $\underline{CH}(CH_3)_2$ + CH-$\underline{(CH_2)_3}$ + CH-$\underline{(CH_2)_2}$), 1.90 (3H, s, $\underline{CH_3}CO_2H$), 2. 38 (1H, m, $\underline{CH}$-CO), 2.4-2.65(3H, m, $\underline{CH}$-CO + $\underline{CH_2}$-$C_6H_5$), 2.72 (3H, m, N-$\underline{CH_3}$), 2.89 (2H, m, $\underline{CH_2}$-$NH_2$), 4.27(1H, m, NH-$\underline{CH}$-CO), 7.0-7.3 (5H, m, aromatic-H).

Preparation Example 75

$N^a$-[4-(Hydroxyamino)-2(R)-isobutyl-3(R or S)-[3-(m-carboxyphenyl)propyl]succinyl]-L-lysine N-methylamide • 1 acetate (Compound No. 75)

Alkaline hydrolysis of Compound No. 71 yielded the title compound as a white solid.

FABMS ($M^+$ + 1): 494.
$^1$H-NMR ($CD_3OD$)δ ppm; 0.8-0.9 (6H, m, CH$\underline{(CH_3)_2}$), 1.01 (1H, m, $\underline{CH}(CH_3)_2$), 1.1-1.8 (12H, m, CH-$\underline{(CH_2)_3}$ + CH-$\underline{(CH_2)_2}$ + $\underline{CH_2}$-$CH(CH_3)_2$), 1.96 (3H, s, $\underline{CH_3}CO_2H$), 2.13 (1H, m, $\underline{CH}$-CO), 2.49 (1H, m, $\underline{CH}$-CO), 2.55-2.85 (5H, m, N-$\underline{CH_3}$ + $C_6H_4$-$\underline{CH_2}$), 2.84 (2H, m, $\underline{CH_2}$-$NH_2$), 4.11 (1H, m, NH-$\underline{CH}$-CO), 7.26 and 7.73 (2H each, m, aromatic-H).

Preparation Example 76

$N^a$-[4-(Hydroxyamino)-2(R)-isobuty-3(R or S)-[3-(piperidin-1-yl)propyl]succinyl]-L-lysine N-methylamide • 2 acetate (Compound No. 76)

The procedure of Preparation Example 23 was repeated using 6-(piperidin-1-yl)-3(RS)-tert-butyloxycarbonyl-2(R)-isobutylhexanoic acid and Compound No. 1 to give the title compound as a white solid.

FABMS ($M^+$ + 1): 457.
$^1$H-NMR ($CD_3OD$)δ ppm; 0.8-0.95 (6H, m, CH$\underline{(CH_3)_2}$), 1.07 (1H, m, $\underline{CH}(CH_3)_2$), 1.2-1.85 (18H, m, $\underline{CH_2}$-$CH(CH_3)_2$ + CH-$\underline{(CH_2)_2}$ + CH-$\underline{(CH_2)_3}$-$CH_2$-$NH_2$ + N-$CH_2$-$\underline{(CH_2)_3}$), 1.91 (6H, s, $\underline{CH_3}CO_2H \times 2$), 2.18 (1H, m, $\underline{CH}$-CO), 2.57 (1H, m, $\underline{CH}$-CO), 2.72 (3H, s, N-$\underline{CH_3}$), 2.7-3.1 (8H, m, N-$\underline{CH_2} \times 3$ + $\underline{CH_2}$-$NH_2$), 4.28 (1H, m, NH-$\underline{CH}$-CO).

Preparation Example 77

$N^a$-[4-(Hydroxyamino)-2(R)-isobutyl-3(R or S)-methylsuccinyl]-$N^e$-benzimidoyl-L-lysine N-methylamide • 1 acetate (Compound No. 77)

The procedure of Preparation Example 22 was repeated using Compound No. 18 and Compound No. 22-a, except that ethyl acetimidate hydrochloride was replaced with methyl benzimidate hydrochloride, to give the title compound as a white solid.

FABMS ($M^+$): 448.
$^1$H-NMR ($CD_3OD$)δ ppm; 0.7-0.9 (6H, m, $\underline{CH(CH_3)_2}$), 0.94 (3H, d, J=7.0Hz, CH-$\underline{CH_3}$), 1.01 (1H, m, $\underline{CH}(CH_3)_2$), 1.2-1.7 (8H, m, $\underline{CH_2}$-$CH(CH_3)_2$+ CH-$\underline{(CH_2)_3}$), 1.80 (3H, s, $\underline{CH_3}CO_2H$), 2.15 (1H, m, $\underline{CH}$-CO), 2.48 (1H, m, $\underline{CH}$-CO), 2.64 (3H, s, N-$\underline{CH_3}$), 3.35 (2H, m, $\underline{CH_2}$-$NH_2$), 4.26 (1H, m, NH-$\underline{CH}$-CO), 7.4-7.7 (5H, m, aromatic-H).

Preparation Example 78

$N^4$-[3-Amino-1(S)-methylcarbamoylpropyl]-$N^1$-hydroxy-2(R or S)-[3-(p-aminomethylphenyl)propyl]-3(R)-isobutylsuccinamide • 2 acetate (Compound No. 78)

The procedure of Preparation Example 23 was repeated using 6-(p-benzyloxycarbonylaminomethylphenyl)-3(RS)-tert-butyloxycarbonyl-2(R)-isobutylhexanoic acid and 2(S)-tert-butyloxycarbonylamino-4-benzyloxycarbonylaminobutanoic acid N-methylamide to give the title compound as a white solid.

FABMS ($M^+$): 450.
$^1$H-NMR ($CD_3OD$)δ ppm; 0.75-0.9 (6H, m, CH($CH_3$)$_2$), 1.07 (1H, m, CH($CH_3$)$_2$), 1.2-1.7 (6H, m, $CH_2$-CH($CH_3$)$_2$ + CH-($CH_2$)$_2$), 1.85 (6H, s, $CH_3CO_2H$× 2), 1.8-2.1 (2H, m, CH-$CH_2$), 2.18 (1H, m, CH-CO), 2.52 (3H, m, CH-CO + $C_6H_4$-$CH_2$), 2.69 (3H, s, N-$CH_3$), 2.88 (2H, m, $CH_2$-$NH_2$), 3.99 (2H, s, $C_6H_4$-$CH_2$-$NH_2$), 4.36 (1H, m, NH-CH-CO), 7.17 and 7.29 (2H each, m, aromatic-H).

Preparation Example 79

$N^a$-[4-(Hydroxyamino)-2(R)-isobutyl-3(R or S)-[3-(p-aminomethylphenyl)propyl]succinyl]-L-tert-leucine N-methylamide • 1 acetate (Compound No. 79)

The procedure of Preparation Example 23 was repeated using 6-(p-benzyloxycarbonylaminomethylphenyl)-3(RS)-tert-butyloxycarbonyl-2(R)-isobutylhexanoic acid and $N^a$-tert-butyloxycarbonyl-L-tert-leucine N-methylamide to give the title compound as a white solid.

FABMS ($M^+$ + 1): 464.
$^1$H-NMR ($CD_3OD$)δ ppm; 0.7-0.85 (6H, m, CH($CH_3$)$_2$), 0.94 (9H, s, C($CH_3$)$_3$), 1.02(1H, m, CH($CH_3$)$_2$), 1.2-1.7 (6H, m, $CH_2$-CH($CH_3$)$_2$ + CH-($CH_2$)$_2$, 1.88 (3H, m, $CH_3CO_2H$), 2.14 (1H, m, CH-CO), 2.4-2.6 (3H, m, CH-CO + $C_6H_4$-$CH_2$), 2.65 (3H, s, N-$CH_3$), 4.00 (2H, s, $CH_2$-$NH_2$), 4.18 (1H, m, NH-CH-CO), 7.16 and 7.28 (2H each, m, aromatic-H).

Preparation Example 80

$N^a$-[4-(Hydroxyamino)-2(R)-isobutyl-3(R or S)-[3-(p-aminomethylphenyl)propyl]succinyl]-L-ornithine N-methylamide • 1 acetate (Compound No. 80)

The procedure of Preparation Example 23 was repeated using 6-(p-benzyloxycarbonylaminomethylphenyl)-3(RS)-tert-butyloxycarbonyl-2(R)-isobutylhexanoic acid and Compound No. 7 to give the title compound as a white solid.

FABMS ($M^+$ + 1): 465.
$^1$H-NMR ($CD_3OD$)δ ppm; 0.8-0.9 (6H, m, CH($CH_3$)$_2$), 1.05 (1H, m, CH($CH_3$)$_2$), 1.2-1.85 (12H, m, $CH_2$-CH($CH_3$)$_2$ + CH-($CH_2$)$_3$ + CH-($CH_2$)$_2$ , 1.89 (3H, s, $CH_3CO_2H$), 2.20 (1H, m, CH-CO), 2.5-2.7 (3H, m, CH-CO + $C_6H_4$-$CH_2$), 2.72 (3H, s, N-$CH_3$), 2.89 (2H, s, $CH_2$-$NH_2$), 4.01 (2H, s, $C_6H_4$-$CH_2$-$NH_2$), 4.29 (1H, m, NH-CH-CO), 7.22 and 7.31 (2H each, m, aromatic-H).

Preparation Example 81

$N^a$-[4-(Hydroxyamino)-2(R)-isobutyl-3(R or S)-[3-(p-guanidomethylphenyl)propyl]succinyl]-L-ornithine N-methylamide • 2 acetate (Compound No. 81)

The procedure of Preparation Example 23 was repeated using 6-[p-($N^1$,$N^2$-dibenzyloxycarbonylguanidomethyl)phenyl)-3(RS)-tert-butyloxycarbonyl-2(R)-isobutylhexanoic acid and Compound No. 7 to give the title compound as a white solid.

FABMS ($M^+$): 506.
$^1$H-NMR ($CD_3OD$)δ ppm; 0.7-0.85 (6H, m, CH($CH_3$)$_2$), 0.99 (1H, m, CH($CH_3$)$_2$), 1.15-1.8 (10H, m, $CH_2$-CH($CH_3$)$_2$ + CH-($CH_2$)$_2$ × 2), 1.81 (6H, s, $CH_3CO_2H$× 2), 2.12 (1H, m, CH-CO), 2.4-2.6 (3H, m, CH-CO + $C_6H_4$-$CH_2$), 2.67 (3H, s, N-$CH_3$), 2.82 (2H, s, $CH_2$-$NH_2$), 4.22 (1H, m, NH-CH-CO), 4.26 (2H, s, $C_6H_4$-$CH_2$-NH), 7.0- 7.2 (4H, m, aromatic-H).

Preparation Example 82

$N^a$-[4-(Hydroxyamino)-2(R)-isobutyl-3(R or S)-[3-(3,4,4-trimethyl-2,5-dioxo-imidazolidin-1-yl)propyl]succinyl]-L-lysine N-methylamide (Compound No. 82)

The procedure of Preparation Example 23 was repeated using 6-(3,4,4-trimethyl-2,5-dioxo-imidazolidin-1-yl)-3(RS)-tert-butyloxycarbonyl-2(R)-isobutylhexanoic acid and Compound No. 1 to give the title compound as a white solid.

FABMS ($M^+$ + 1): 514.
$^1$H-NMR ($CD_3OD$)δ ppm; 0.8-0.9 (6H, m, CH$\underline{(CH_3)_2}$), 1.03 (1H, m, $\underline{CH}(CH_3)_2$), 1.2-1.9 (18H, m, $\underline{CH_2}$-$CH(CH_3)_2$ + CH-$\underline{(CH_2)_2}$ + CH-$\underline{(CH_2)_3}$) + C$\underline{(CH_3)_2}$, 2.11 (1H, m, $\underline{CH}$-CO), 2.54 (1H, m, $\underline{CH}$-CO), 2.72 (3H, s, NH-$\underline{CH_3}$), 2.87 (3H, s, N-$\underline{CH_3}$), 3.00 (2H,t, J=7.5Hz, $\underline{CH_2}$-$NH_2$), 3.42 (2H,t, J=6.1Hz, $\underline{CH_2}$-N), 4.30 (1H, m, NH-$\underline{CH}$-CO).

Preparation Example 83

$N^a$-[4-(Hydroxyamino)-3(S)-isobutyl-2(R or S)-(8-hydroxyoctyl)succinyl]-L-lysine N-methylamide • 1 acetate (Compound No. 83)

The procedure of Preparation Example 23 was repeated using 10-tetrahydropyranyloxy-2(RS)-[1-(S)-tert-butyloxycarbonyl-3-methylbutyl]decanoic acid and Compound No. 1 to give the title compound as a white solid.

FABMS ($M^+$ + 1): 460.
$^1$H-NMR ($CD_3OD$)δ ppm; 0.8-0.9 (6H, m, CH$\underline{(CH_3)_2}$), 1.0 (1H, m, $\underline{CH}(CH_3)_2$), 1.1-1.85 (22H, m, $\underline{CH_2}$-$CH(CH_3)_2$ + CH-$\underline{(CH_2)_3}$ + CH-$\underline{(CH_2)_7}$), 1.91 (3H, s, $\underline{CH_3}CO_2H$), 2. 29 (1H, m, $\underline{CH}$-CO), 2.41 (1H, m, $\underline{CH}$-CO), 2.73 (3H, s, N-$\underline{CH_3}$), 2.90 (2H,t, J=7.6Hz, $\underline{CH_2}$-$NH_2$), 3.52 (2H, m, $\underline{CH_2}$-OH), 4.31 (1H, m, NH-$\underline{CH}$-CO).

Preparation Example 84

$N^a$-[4-(Hydroxyamino)-2(R),3(R or S)-diisobutylsuccinyl]-4'-aminomethyl-L-phenylalanine N-(2-carboxyethyl)amide • 1 acetate (Compound No. 84)

The procedure of Preparation Example 23 was repeated using Compound No. 17 and $N^a$-tert-butyloxycarbonyl-L-4'-cyanophenylalanine N-(2-benzyloxycarbonylethyl)amide to give the title compound as a white solid.

FABMS ($M^+$): 493.
$^1$H-NMR ($CD_3OD$)δ ppm; 0.75-1.1 (14H, m, $CH_2$-$\underline{CH(CH_3)_2}$ × 2), 1.2-1.6 (4H, m, $\underline{CH_2}$-$CH(CH_3)_2$× 2), 1.97 (3H, s, $\underline{CH_3}CO_2H$), 1.9-2.2 (3H, m, $\underline{CH}$-CO + $\underline{CH_2}$-$CO_2H$), 2.52 (1H, m, $\underline{CH}$-CO), 2.99 (2H, m, $\underline{CH_2}$-$C_6H_4$), 3.25-3.4 (m, NH-$\underline{CH_2}$), 4.02 (2H, m, $C_6H_4$-$\underline{CH_2}$-$NH_2$), 4.53 (1H, m, NH-$\underline{CH}$-CO), 7.32 (4H, m, aromatic-H).

Preparation Example 85

$N^a$-[4-(Hydroxyamino)-2(R),3(R or S)-diisobutylsuccinyl)-4'-acetimidoyliminomethyl-L-phenylalanine N-(2-carboxyethyl)amide • 1 acetate (Compound No. 85)

The procedure of Preparation Example 23 was repeated using Compound No. 17 and $N^a$-tert-butyloxycarbonyl-L-4'-cyanphenylalanine N-(2-benzyloxycarbonylethyl)amide to give the title compound as a white solid.

FABMS ($M^+$): 534.
$^1$H-NMR ($CD_3OD$)δ ppm; 0.7-0.9 (12H, m, CH$\underline{(CH_3)_2}$× 2), 1.0-1.1 (2H, m, $\underline{CH}(CH_3)_2$× 2), 1.3-1.7 (4H, m, $\underline{CH_2}$-$CH(CH_3)_2$× 2), 1.94 (3H, s, $\underline{CH_3}CO_2H$), 2.0-2.3 (6H, m, $\underline{CH}$-CO + $\underline{CH_2}$-$CO_2H$ + C-$\underline{CH_3}$), 2.52 (1H, m, $\underline{CH}$-CO), 2.9-3.1 (2H, m, $\underline{CH_2}$-$C_6H_4$), 3.2-3.4 (m, NH-$\underline{CH_2}$), 4.38 (2H, s, $C_6H_4$-$\underline{CH_2}$-NH), 4.55 (1H, m, NH-$\underline{CH}$-CO), 7.28 (4H, m, aromatic-H).

Preparation Example 86

$N^4$-[2-Amino-2-methyl-1(RS)-methylcarbamoylpropyl]-$N^1$-hydroxy-2(R or S)-[3-(p-aminomethylphenyl)propyl]-3(R)-isopropylsuccinamide • 2 acetate (Compound No. 86)

The procedure of Preparation Example 23 was repeated using 6-(p-benzyloxycarbonylaminomethylphenyl)-3(RS)-tert-butyloxycarbonyl-2(R)-isopropylpentanoic acid and 2(RS)-tert-butyloxycarbonylamino-3-benzyloxycarbonylamino-3-methylbutanoic acid N-methylamide to give the title compound as a white solid.

FABMS ($M^+$): 450.
$^1$H-NMR ($CD_3OD$)δ ppm; 0.85-1.0 (6H, m, CH($\underline{CH_3}$)$_2$), 1.2-1.8 (11H, m, $\underline{CH}$($CH_3$)$_2$ + C-$\underline{CH_3}$ × 2 + CH-($\underline{CH_2}$)$_2$), 1.91 (6H, s, $\underline{CH_3}CO_2H$× 2), 2.31 (1H, m, $\underline{CH}$-CO), 2.4-2.6 (3H, m, $\underline{CH}$-CO + $C_6H_4$-$\underline{CH_2}$), 2.65 (3H, s, N-$\underline{CH_3}$), 4.01 (2H, s, $\underline{CH_2}$-$NH_2$), 4.72 (1H, m, NH-$\underline{CH}$-CO), 7.17 and 7.29 (2H each, m, aromatic-H).

Preparation Example 87

$N^a$-[4-(Hydroxyamino)-2(R)-isobutyl-3(R or S)-methylsuccinyl]-4'-aminomethyl-L-phenylalanine N-(morpholin-4-yl)amide • 1 acetate (Compound No. 87)

The procedure of Preparation Example 23 was repeated using Compound No. 2 and Compound No. 18 to give the title compound as a white solid.

FABMS ($M^+$): 464.
$^1$H-NMR ($CD_3OD$)δ ppm; 0.62, 0.71 and 0.80 (3H each, m, CH($\underline{CH_3}$)$_2$× 2 + CH-$\underline{CH_3}$), 0.91 (1H, m, $\underline{CH}$($CH_3$)$_2$), 1.2-1.5 (2H, m, $\underline{CH_2}$-CH($CH_3$)$_2$), 1.81 (3H, s, $\underline{CH_3}CO_2H$), 2.01 (1H, m, $\underline{CH}$-CO), 2.39 (1H, m, $\underline{CH}$-CO), 2.5-2.6 (4H, m, N-$\underline{CH_2}$ × 2), 2.8-3.0 (2H, m, $\underline{CH_2}$-$C_6H_4$), 3.61 (4H, m, O-$\underline{CH_2}$ × 2), 3.93 (2H, s, $C_6H_4$-$\underline{CH_2}$-$NH_2$), 4.48 (1H, m, NH-$\underline{CH}$-CO), 7.29 (4H, m, aromatic-H).

Preparation Example 88

$N^a$-[4-(Hydroxyamino)-2(R)-isobuty-3(S)-hydroxysuccinyl]4'-aminomethyl-L-phenylalanine N-methylamide • 1 acetate (Compound No. 88)

The procedures of Preparation Examples 23-b and c except omitting removal of a tert-butyl group with trifluoroacetic acid were repeated using Compound No. 43-b to provide the title compound as a white solid.

FABMS ($M^+$ + 1): 395.
$^1$H-NMR ($CD_3OD$)δ ppm; 0.87 (6H, m, CH($\underline{CH_3}$)$_2$), 1.1-1.6 (3H, m, $\underline{CH_2}$-$\underline{CH}$($CH_3$)$_2$), 1.98 (3H, s, $\underline{CH_3}CO_2H$), 2.6-2.8 (4H, s + m, N-$\underline{CH_3}$ + $\underline{CH}$-CO), 2.9-3.1 (2H, m, $\underline{CH_2}$-$C_6H_4$), 3.93 (2H, m, $\underline{CH_2}$-$NH_2$), 4.4-4.6 (2H, m, NH-$\underline{CH}$-CO + HO-$\underline{CH}$-CO), 7.30(4H, m, aromatic-H).

Preparation Example 89

$N^a$-[4-(Hydroxyamino)-2(R),3(R or S)-diisobutylsuccinyl]-4'-aminomethyl-L-phenylalanine N-(2-N',N'-dimethylaminoethyl)amide • 2 acetate (Compound No. 89)

The procedure of Preparation Example 23 was repeated using Compound No. 17 and $N^a$-tert-butyloxycarbonyl-L-4'-cyanophenylalanine N-(2-N',N'-dimethylaminoethyl)amide to provide the title compound as a white solid.

FABMS ($M^+$ + 1): 493.
$^1$H-NMR ($CD_3OD$)δ ppm; 0.6-0.9 (12H, m, CH($\underline{CH_3}$)$_2$× 2), 1.02 (2H, m, $\underline{CH}$($CH_3$)$_2$ × 2), 1.3-1.6 (4H, m, $\underline{CH_2}$-CH($CH_3$)$_2$× 2), 1.91 (6H, s, $\underline{CH_3}CO_2H$ × 2), 2.16 (1H, m, $\underline{CH}$-CO), 2.4-2.55 (7H, s + m, N-$\underline{CH_3}$× 2 + $\underline{CH}$-CO), 2.71 (2H, m, $\underline{CH_2}$-N), 2.9-3.1 (2H, m, $\underline{CH_2}$-$C_6H_4$), 3.3-3.5 (2H, m, NH-$\underline{CH_2}$), 4.03 (2H, s, $C_6H_4$-$\underline{CH_2}$-$NH_2$), 4.60 (1H, m, NH-$\underline{CH}$-CO), 7.38 (4H, m, aromatic-H).

Preparation Example 90

$N^a$-[4-(Hydroxyamino)-2(R),3(R or S)-diisobutylsuccinyl]-4'-acetimidoyliminomethyl-L-phenylalanine N-(2-N',N'-dimethylaminoethyl)amide • 2 acetate (Compound No. 90)

The procedure of Preparation Example 22 was repeated using Compound No. 17 and $N^a$-tert-butyloxycarbonyl-L-4'-cyanophenylalanine N-(2-N',N'-dimethylaminoethyl)amide to provide the title compound as a white solid.

$^1$H-NMR ($CD_3OD$)δ ppm; 0.6-0.9 (13H, m, CH($\underline{CH_3}$)$_2$ + $\underline{CH}$($CH_3$)$_2$), 0.95-1.1 (1H, m, $\underline{CH}$($CH_3$)$_2$), 1.4-1.6 (4H, m, $\underline{CH_2}$-CH($CH_3$)$_2$× 2), 1.91 (6H, s, $\underline{CH_3}CO_2H$× 2), 2.16 (1H, m, $\underline{CH}$-CO), 2.24 (3H, s, C-$\underline{CH_3}$), 2.37 (6H, s, N-$\underline{CH_3}$× 2), 2.4-2.5 (3H, m, $\underline{CH}$-CO + $\underline{CH_2}$-N), 2.9-3.1 (2H, m, $\underline{CH_2}$-$C_6H_4$), 3.2-3.4 (m, NH-$\underline{CH_2}$), 4.40 (2H, s, $C_6H_4$-$\underline{CH_2}$-NH), 4.60 (1H, m, NH-$\underline{CH}$-CO), 7.31 (4H, m, aromatic-H).

Preparation Example 91

$N^a$-[4-(Hydroxyamino)-2(R),3(RS)-diisobutylsuccinyl]-4'-acetimidoyliminomethyl-L-phenylalanine N-(2-hydroxyethyl)amide • 2 acetate (Compound No. 91)

The procedure of Preparation Example 22 was repeated using Compound No. 14 and Compound No. 17 to provide the title compound as a white solid.

$^1$H-NMR ($CD_3OD$)δ ppm; 0.6-0.9 (13H, m, CH($\underline{CH_3}$)$_2$ + $\underline{CH}$($CH_3$)$_2$), 1.00 (1H, m, $\underline{CH}$($CH_3$)$_2$ ), 1.3-1.6 (4H, m, $\underline{CH_2}$-CH($CH_3$)$_2$ × 2), 1.89 (3H, s, $\underline{CH_3}CO_2H$), 2.15(1H, m, $\underline{CH}$-CO), 2.24 (3H, s, C-$\underline{CH_3}$), 2.46 (1H, m, $\underline{CH}$-CO), 2.9-3.1 (2H, m, $\underline{CH_2}$-$C_6H_4$), 3.2-3.6 (m, NH-$\underline{CH_2}$-$\underline{CH_2}$-OH), 4.40 (2H, s, $C_6H_4$-$\underline{CH_2}$-$NH_2$), 4.65 (1H, m, NH-$\underline{CH}$-CO), 7.31 (4H, m, aromatic-H).

Preparation Example 92

$N^a$-[4-(Hydroxyamino)-2(R)-isobutyl-3(R or S)-[3-(p-aminophenyl)propyl]succinyl]-$N^e$-acetimidoyl-L-lysine N-(2-N',N'-dimethylaminoethyl)amide • 3 acetate (Compound No. 92)

The procedure of Preparation Example 22 was repeated using 6-(p-benzyloxycarbonylaminophenyl)-3(RS)-tert-butyloxycarbonyl-2(R)-isobutylhexanoic acid and Compound No. 3 to provide the title compound as a white solid.

FABMS ($M^+$ + 1): 563.
$^1$H-NMR ($CD_3OD$)δ ppm; 0.7-0.95 (6H, m, CH($\underline{CH_3}$)$_2$), 1.09 (1H, m, $\underline{CH}$($CH_3$)$_2$), 1.1-1.8 (12H, m, $\underline{CH_2}$-CH($CH_3$)$_2$ + CH-($\underline{CH_2}$)$_2$ + CH-($\underline{CH_2}$)$_3$), 1.96 (9H, s, $\underline{CH_3}CO_2H$× 3), 2.21 (3H, s, C-$\underline{CH_3}$), 2.3-2.6 (4H, m, $\underline{CH}$-CO × 2 + $\underline{CH_2}$-$C_6H_4$), 2.76 (6H, m, N-$\underline{CH_3}$ × 2), 2.9-3.5 (m, $\underline{CH_2}$-$NH_2$ + NH-$\underline{CH_2}$-$\underline{CH_2}$-N), 4.13 (1H, m, NH-$\underline{CH}$-CO), 6.64 , 6.89, 7.07 and 7.41 (1H each, m, aromatic-H).

Preparation Example 93

$N^a$-[4-(Hydroxyamino)-2(R),3(RS)-diisobutylsuccinyl]-4'-acetimidoyliminomethyl-L-phenylalanine N-(2-hydroxy-1,1-dimethylethyl)amide • 1 acetate (Compound No. 93)

The procedure of Preparation Example 22 was repeated using Compound No. 17 and $N^a$-tert-butyloxycarbonyl-L-4'-cyanophenylalanine N-(1,1-dimethyl-2-hydroxyethyl)amide to provide the title compound as a white solid.

FABMS ($M^+$ + 1): 535.
$^1$H-NMR ($CD_3OD$)δ ppm; 0.7-0.95 (12H, m, CH($\underline{CH_3}$)$_2$ × 2), 1.0-1.1 (2H, m, $\underline{CH}$($CH_3$)$_2$× 2 ), 1.1-1.8 (10H, m, C($\underline{CH_3}$)$_2$ + $\underline{CH_2}$-CH($CH_3$)$_2$× 2), 1.89 (3H, s, $\underline{CH_3}CO_2H$), 2.15 (1H, m, $\underline{CH}$-CO), 2.24 (3H, s, C-$\underline{CH_3}$), 2.46 (1H, m, $\underline{CH}$-CO), 3.0-3.4 (2H, m, $\underline{CH_2}$-$C_6H_4$), 3.4-3.6 (2H, m, $\underline{CH_2}$-OH), 4.41 (2H, s, $C_6H_4$-$\underline{CH_2}$-NH), 4.62 (1H, m, NH-$\underline{CH}$-CO), 7.31 (4H, m, aromatic-H).

Preparation Example 94

$N^4$-[3-Amino-2,2-dimethyl-1(RS)-methylcarbamoylpropyl]-$N^1$-hydroxy-2(R or S)-[3-(p-aminomethylphenyl)propyl]-3(R)-isopropylsuccinamide • 2 acetate (Compound No. 94)

The procedure of Preparation Example 23 was repeated using 6-(p-benzyloxycarbonylaminomethylphenyl)-3(RS)-tert-butyloxycarbonyl-2(R)-isopropylhexanoic acid and 2(RS)-tert-butyloxycarbonylamino-4-benzyloxycarbonylamino-3,3-dimethylbutanoic acid N-methylamide to provide the title compound as a white solid.

FABMS ($M^+$): 464.
$^1$H-NMR ($CD_3OD$)δ ppm; 0.8-1.0 (6H, m, CH($\underline{CH_3}$)$_2$), 1.2-1.8 (11H, m, $\underline{CH}$($CH_3$)$_2$ + C-$\underline{CH_3}$ × 2 + CH-($\underline{CH_2}$)$_2$), 1.89 (6H, s, $\underline{CH_3}CO_2H$× 2), 2.27 (1H, m, $\underline{CH}$-CO), 2.4-2.6 (3H, m, $\underline{CH}$-CO + $C_6H_4$-$\underline{CH_2}$), 2.6-2.8 (5H, s, $\underline{CH_2}$ -$NH_2$ + N-$\underline{CH_3}$), 4.00(2H, s, $C_6H_4$-$\underline{CH_2}$-$NH_2$), 4.70 (1H, m, NH-$\underline{CH}$-CO), 7.17 and 7.29 (2H each, m, aromatic-H).

Preparation Example 95

$N^4$-[2-Amino-1(S)-methylcarbamoylpropyl]-$N^1$-hydroxy-2(R or S)-[3-(p-aminomethylphenyl)propyl]-3(R)-isopropylsuccinamide • 2 acetate (Compound No. 95)

The procedure of Preparation Example 23 was repeated using 6-(p-benzyloxycarbonylaminomethylphenyl)-3(RS)-tert-butyloxycarbonyl-2(R)-isopropylhexanoic acid and 2(S)-tert-butyloxycarbonylamino-3-benzyloxycarbonylaminobutanoic acid N-methylamide to provide the title compound as a white solid.

FABMS ($M^+$): 436.
$^1$H-NMR ($CD_3OD$)δ ppm; 0.8-1.0 (6H, m, CH($\underline{CH_3}$)$_2$), 1.1-1.2 (4H, m, $\underline{CH}$($CH_3$)$_2$ + CH-$\underline{CH_3}$), 1.2-1.8 (4H, m, CH-($\underline{CH_2}$)$_2$), 1.88 (6H, s, $\underline{CH_3}CO_2H$ × 2), 2.21 (1H, m, $\underline{CH}$-CO), 2.4-2.6 (3H, m, $\underline{CH}$-CO + $C_6H_4$-$\underline{CH_2}$), 2.66 (3H, s, N-$\underline{CH_3}$), 3.2-3.4 (m, $\underline{CH}$-$NH_2$), 4.00 (2H, s, $\underline{CH_2}$-$NH_2$), 4.65 (1H, m, NH-$\underline{CH}$-CO), 7.21 (4H, m, aromatic-H).

Preparation Example 96

$N^a$-[4-(Hydroxyamino)-2(R)-isopropyl-3(R or S)-[3-(p-aminomethylphenyl)propyl]succinyl]-O-(2,3,4,6-tetra-O-acetyl-β -D-glucopyranosyl)-L-serine N-methylamide • 1 acetate (Compound No. 96)

The procedure of Preparation Example 23 was repeated using 6-(p-benzyloxycarbonylaminomethylphenyl)-3(RS)-tert-butyloxycarbonyl-2(R)-isopropylhexanoic acid and $N^a$-tert-butyloxycarbonyl-O-(2,3,4,6-tetra-O-acetyl-β -D-glucopyranosyl)-L-serine N-methylamide to provide the title compound as a white solid.

FABMS ($M^+$): 753.
$^1$H-NMR ($CD_3OD$)δ ppm; 0.8-1.0 (6H, m, CH($\underline{CH_3}$)$_2$), 1.1-1.2 (17H, m, $\underline{CH}$($CH_3$)$_2$ +CH-($\underline{CH_2}$)$_2$ + $CH_3$CO- × 4), 1.90 (3H, s, $\underline{CH_3}CO_2H$), 2.30 (1H, m, $\underline{CH}$-CO), 2.4-2.75 (3H, m, $\underline{CH}$-CO + $C_6H_4$-$\underline{CH_2}$), 2.78 (3H, s, N-$\underline{CH_3}$), 3.78 (2H, m, $\underline{CH_2}$-O), 3.95-4.6 [7H, m, NH-$\underline{CH}$-CO + $\underline{CH_2}$-$NH_2$ + 2-H, 5-H and 6-H (glucopyranosyl)], 4.9-5.35 [3H, m, 1-H, 3-H and 4-H (glucopyranosyl)], 7.20 (4H, m, aromatic-H).

Preparation Example 97

$N^a$-[4-(Hydroxyamino)-2(R)-isopropyl-3(R or S)-[3-(p-aminomethylphenyl)propyl]succinyl]-O-(β -D-glucopyranosyl)-L-serine N-methylamide • 1 acetate (Compound No. 97)

Hydrolysis of Compound No. 96 yielded the title compound as a white solid.

FABMS ($M^+$ + 1): 586.
$^1$H-NMR ($D_2O$)δ ppm; 0.7-0.9 (6H, m, CH($\underline{CH_3}$)$_2$), 1.1-1.7 (5H, m, $\underline{CH}$($CH_3$)$_2$ + CH-($\underline{CH_2}$)$_2$), 1.78 (3H, s, $\underline{CH_3}CO_2H$), 2.15 (1H, m, $\underline{CH}$-CO), 2.4-2.6 (3H, m, $\underline{CH}$-CO + $C_6H_4$-$\underline{CH_2}$), 2.70 (3H, s, N-$\underline{CH_3}$), 3.43 [1H, m, 5-H (glucopyranosyl)], 3.5-4.0 [7H, m, $\underline{CH_2}$-O + 2-H, 3-H, 4-H and 6-H (glucopyranosyl)], 4.00 (2H, m, $\underline{CH_2}$-$NH_2$), 4.32 (1H, m, NH-$\underline{CH}$-CO), 5.25 [1H, m, 1-H (glucopyranosyl)], 7.18 (4H, m, aromatic-H).

Preparation Example 98

$N^4$-[4-(3,4,4-Trimethyl-2,5-dioxo-imidazolidin-1-yl)-1(S)-methylcarbamoylbutyl]-$N^1$-hydroxy-2(R or S)-[3-(p-aminomethylphenyl)propyl]-3(R)-isopropylsuccinamide • 1 acetate (Compound No. 98)

The procedure of Preparation Example 23 was repeated using 6-(p-benzyloxycarbonylaminomethylphenyl)-3(RS)-tert-butyloxycarbonyl-2(R)-isopropylhexanoic acid and 2(S)-tert-butyloxycarbonylamino-5-(3,4,4-trimethyl-2,5-dioxo-imidazolidin-1-yl)pentanoic acid N-methylamide to provide the title compound as a white solid.

FABMS ($M^+$): 575.
$^1$H-NMR ($CD_3OD$)δ ppm; 0.8-1.0 (6H, m, CH$\underline{(CH_3)_2}$), 1.1-1.8 (15H, s + m, $\underline{CH}(CH_3)_2$ + CH-$\underline{(CH_2)_2}$ × 2 + C-$\underline{CH_3}$ × 2), 1.89 (3H, s, $\underline{CH_3}CO_2H$), 2.20 (1H, m, $\underline{CH}$-CO), 2.4-2.6 (3H, m, $\underline{CH}$-CO + $C_6H_4$-$\underline{CH_2}$), 2.67 (3H, s, N-$\underline{CH_3}$), 2.89 (3H, s, N-$CH_3$), 3.56 (2H,t, J=6.3Hz, N-$\underline{CH_2}$), 4.01 (2H, s, $\underline{CH_2}$-$NH_2$), 4.38 (1H, m, NH-$\underline{CH}$-CO), 7.20 (4H, m, aromatic-H).

Preparation Example 99

$N^4$-[2-Amino-2-methyl-1(RS)-methylcarbamoylpropyl]-$N^1$-hydroxy-2(R or S)-[3-(p-guanidomethylphenyl)propyl]-3(R)-isopropylsuccinamide • 2 acetate (Compound No. 99)

The procedure of Preparation Example 23 was repeated using 3(RS)-tert-butyloxycarbonyl 6-[p-($N^1$,$N^2$-dibenzyloxycarbonylguanidomethyl)phenyl]-2(R)-isopropylhexanoic acid and 2(RS)-tert-butyloxycarbonylamino-3-benzyloxycarbonylamino-3-methylbutanoic acid N-methylamide to provide the title compound as a white solid.

FABMS ($M^+$): 492.
$^1$H-NMR ($CD_3OD$)δ ppm; 0.8-1.0 (6H, m, CH$\underline{(CH_3)_2}$), 1.1-1.8 (11H, m, $\underline{CH}(CH_3)_2$ +C-$\underline{CH_3}$× 2 + CH-$\underline{(CH_2)_2}$), 1.89 (6H, s, $\underline{CH_3}CO_2H$× 2), 2.27 (1H, m, $\underline{CH}$-CO), 2.4-2.6 (3H, m, $\underline{CH}$-CO + $C_6H_4$-$\underline{CH_2}$), 2.72 (3H, s, N-$\underline{CH_3}$), 4.21 (1H, m, NH-$\underline{CH}$-CO), 4.26 (2H, s, $C_6H_4$-$\underline{CH_2}$-NH), 7.0-7.2 (4H, m, aromatic-H).

Preparation Example 100

$N^4$-[3-Amino-2,2-dimethyl-1(RS)-methylcarbamoylpropyl]-$N^1$-hydroxy-2(R or S)-[3-(p-guanidomethylphenyl)propyl]-3(R)-isopropylsuccinamide • 2 acetate (Compound No. 100)

The procedure of Preparation Example 23 was repeated using 3(RS)-tert-butyloxycarbonyl 6-[p-($N^1$,$N^2$-dibenzyloxycarbonylguanidomethyl)phenyl]-2(R)-isopropylhexanoic acid and 2(RS)-tert-butyloxycarbonylamino-4-benzyloxycarbonylamino-3,3-dimethylbutanoic acid N-methylamide to provide the title compound as a white solid.

FABMS ($M^+$): 506.
$^1$H-NMR ($CD_3OD$)δ ppm; 0.85-1.0 (6H, m, CH$\underline{(CH_3)_2}$), 1.1-1.8 (11H, m, $\underline{CH}(CH_3)_2$ + C-$\underline{CH_3}$ × 2 + CH-$\underline{(CH_2)_2}$), 1.90 (6H, s, $\underline{CH_3}CO_2H$ × 2), 2.31 (1H, m, $\underline{CH}$-CO), 2.4-2.6 (3H, m, $\underline{CH}$-CO + $C_6H_4$-$\underline{CH_2}$), 2.6-2.8 (5H, s, $\underline{CH_2}$-$NH_2$ + N-$\underline{CH_3}$), 4.22(1H, m, NH-$\underline{CH}$-CO), 4.28 (2H, s, $C_6H_4$-$\underline{CH_2}$-NH), 7.1-7.4 (4H, m, aromatic-H).

Preparation Example 101

$N^4$-[2-Amino-1(S)-methylcarbamoylpropyl]-$N^1$-hydroxy-2(R or S)-[3-(p-guanidomethylphenyl)propyl]-3(R)-isopropylsuccinamide • 2 acetate (Compound No. 101)

The procedure of Preparation Example 23 was repeated using 3(RS)-tert-butyloxycarbonyl 6-[p-($N^1$,$N^2$-dibenzyloxycarbonylguanidomethyl)phenyl]-2(R)-isopropylhexanoic acid and 2(S)-tert-butyloxycarbonylamino-3-benzyloxycarbonylaminobutanoic acid N-methylamide to provide the title compound as a white solid.

FABMS ($M^+$): 478.
$^1$H-NMR ($CD_3OD$)δ ppm; 0.8-1.0 (6H, m, CH$\underline{(CH_3)_2}$), 1.1-1.2 (4H, m, $\underline{CH}(CH_3)_2$ + C-$\underline{CH_3}$), 1.3-1.7 (4H, m, CH-$\underline{(CH_2)_2}$), 1.90 (6H, s, $\underline{CH_3}CO_2H$ × 2), 2.34 (1H, m, $\underline{CH}$-CO), 2.4-2.7 (3H, m, $\underline{CH}$-CO + $C_6H_4$-$\underline{CH_2}$), 2.73 (3H, s, N-$\underline{CH_3}$), 3.2-3.4 (m, $\underline{CH}$-$NH_2$), 4.28 (1H, m, NH-$\underline{CH}$-CO), 4.33 (2H, s, $\underline{CH_2}$-$NH_2$), 7.19 (4H, m, aromatic-H).

Preparation Example 102

$N^a$-[4-(Hydroxyamino)-2(R)-isopropyl-3(R or S)-[3-(p-guanidomethylphenyl)propyl]succinyl]-O-(2,3,4,6-tetra-O-acetyl-β -D-glucopyranosyl)-L-serine N-methylamide • 1 acetate (Compound No. 102)

The procedure of Preparation Example 23 was repeated using 3(RS)-tert-butyloxycarbonyl 6-[p-($N^1$,$N^2$-dibenzyloxycarbonylguanidomethyl)phenyl]-2(R)-isopropylhexanoic acid and $N^a$-tert-butyloxycarbonyl-O-(2,3,4,6-tetra-O-acetyl-β -D-glucopyranosyl)-L-serine N-methylamide to provide the title compound as a white solid.

FABMS ($M^+$): 795.
$^1$H-NMR ($CD_3OD$)δ ppm; 0.8-1.0 (6H, m, CH$\underline{(CH_3)_2}$), 1.1-2.1 (17H, m, $\underline{CH}(CH_3)_2$ + CH-$\underline{(CH_2)_2}$ + $CH_3$CO-× 4), 1.89 (3H, s, $\underline{CH_3}CO_2H$), 2.35 (1H, m, $\underline{CH}$-CO), 2.4-2.7 (3H, m, $\underline{CH}$-CO + $C_6H_4$-$\underline{CH_2}$), 2.80 (3H, s, N-$\underline{CH_3}$), 3.76 (2H, m, $\underline{CH_2}$-O), 4.0-4.6 [7H, m, NH-$\underline{CH}$-CO + $\underline{CH_2}$-$NH_2$ + 2-H, 5-H and 6-H (glucopyranosyl)], 4.9-5.3 [3H, m, 1-H, 3-H and 4-H (glucopyranosyl)], 7.1-7.3 (4H, m, aromatic-H).

Preparation Example 103

$N^a$-[4-(Hydroxyamino)-2(R)-isopropyl-3(R or S)-[3-(p-guanidomethylphenyl)propyl]succinyl]-O-(β -D-glucopyranosyl)-L-serine N-methylamide • 1 acetate (Compound No. 103)

Hydrolysis of Compound No. 102 yielded the title compound as a white solid.

FABMS ($M^+$ + 1): 628.
$^1$H-NMR ($D_2O$)δ ppm; 0.7-0.9 (6H, m, CH$\underline{(CH_3)_2}$), 1.0-1.7 (5H, m, $\underline{CH}(CH_3)_2$ + CH-$\underline{(CH_2)_2}$), 1.80 (3H, s, $\underline{CH_3}CO_2H$), 2.17 (1H, m, $\underline{CH}$-CO), 2.4-2.6 (3H, m, $\underline{CH}$-CO + $C_6H_4$-$\underline{CH_2}$), 2.71 (3H, s, N-$\underline{CH_3}$), 3.41 [1H, m, 5-H (glucopyranosyl)], 3.5-4.0 [7H, m, $\underline{CH_2}$-O + 2-H, 3-H, 4-H and 6-H (glucopyranosyl)], 4.2-4.4 (3H, m, $C_6H_4$-$\underline{CH_2}$-$NH_2$ + NH-$\underline{CH}$-CO), 5.26 [1H, m, 1-H (glucopyranosyl)], 7.20(4H, m, aromatic-H).

Preparation Example 104

$N^4$-[4-(3,4,4-Trimethyl-2,5-dioxo-imidazolidin-1-yl)-1(S)-methylcarbamoylbutyl]-$N^1$-hydroxy-2(R or S)-[3-(p-guanidomethylphenyl)propyl]-3(R)-isopropylsuccinamide • 1 acetate (Compound No. 104)

The procedure of Preparation Example 23 was repeated using 3(RS)-tert-butyloxycarbonyl 6-[p-($N^1$,$N^2$-dibenzyloxycarbonylguanidomethyl)phenyl]-2(R)-isopropylhexanoic acid and 2(S)-tert-butyloxycarbonylamino-5-(3,4,4-trimethyl-2,5-dioxo-imidazolidin-1-yl)pentanoic acid N-methylamide to provide the title compound as a white solid.

FABMS ($M^+$): 617.
$^1$H-NMR ($CD_3OD$)δ ppm; 0.8-1.0 (6H, m, CH$\underline{(CH_3)_2}$), 1.1-1.8 (15H, s + m, $\underline{CH}(CH_3)_2$ + CH-$\underline{(CH_2)_2}$ × 2 + C-$\underline{CH_3}$ × 2), 1.92 (3H, s, $\underline{CH_3}CO_2H$), 2.23 (1H, m, $\underline{CH}$-CO), 2.45-2.6 (3H, m, $\underline{CH}$-CO + $C_6H_4$-$\underline{CH_2}$), 2.70 (3H, s, N-$\underline{CH_3}$), 2.89 (3H, s, N-$\underline{CH_3}$), 3.56 (2H, t, J=6.3Hz, N-$\underline{CH_2}$), 4.01 (2H, s, $\underline{CH_2}$-$NH_2$), 4.3-4.4 (3H, m, $C_6H_4$-$\underline{CH_2}$-NH + NH-$\underline{CH}$-CO), 7.18 (4H, m, aromatic-H).

Preparation Example 105

$N^a$-[4-(Hydroxyamino)-2(R)-isobutyl-3(R or S)-[3-(p-carbamoylphenyl)propyl]succinyl]-L-lysine N-methylamide • 1 acetate (Compound No. 105)

A mixture of Compound No. 65 (300 mg, 0.592 mmol) and 28% aqueous ammonia (30 ml) was stirred for 10 days at room temperature. The reaction mixture was concentrated, dissolved in 0.1N acetic acid, and purified by column reversed phase chromatography (25 g of Chromatorex ODS-1020T, Fuji Silysia Chemical, Japan; eluted with a gradient of 1 to 20% methanol/0.1% aqueous acetic acid), and then lyophilized to give the title compound as a white solid.

FABMS ($M^+$ + 1): 493.
$^1$H-NMR ($CD_3OD$)δ ppm; 0.8-0.9 (6H, m, CH$\underline{(CH_3)_2}$), 1.05 (1H, m, $\underline{CH}(CH_3)_2$), 1.2-1.8 (12H, m, CH-$\underline{(CH_2)_3}$ + CH-$\underline{(CH_2)_2}$ + $\underline{CH_2}$-CH$(CH_3)_2$), 1.92 (6H, s, $\underline{CH_3}CO_2H$), 2.20 (1H, m, $\underline{CH}$-CO), 2.53 (1H, m, $\underline{CH}$-CO), 2.65 (2H, m, $C_6H_4$-$\underline{CH_2}$), 2.71 (3H, s, N-$\underline{CH_3}$), 2.86 (2H,t, J=7.7Hz, $\underline{CH_2}$-$NH_2$), 4.21 (1H, m, NH-$\underline{CH}$-CO), 7.25 and 7.78 (2H each, m, aromatic-H).

Preparation Example 106

$N^a$-[4-(Hydroxyamino)-2(R)-isopropyl-3(RS)-[3-(p-aminomethylphenyl)propyl]succinyl]-L-ornithine N-methylamide • 2 acetate (Compound No. 106)

The procedure of Preparation Example 23 was repeated using 6-(p-benzyloxycarbonylaminomethylphenyl)-(RS)-tert-butyloxycarbonyl-2(R)-isopropylhexanoic acid and Compound No. 7 to provide the title compound as a white solid.

FABMS ($M^+$ + 1): 451.
$^1$H-NMR (DMSO-$d_6$)δ ppm; 0.85-0.86 (6H, m, CH($\underline{CH_3}$)$_2$), 1.1-1.9 (15H, m, $\underline{CH}$($CH_3$)$_2$ + CH-($\underline{CH_2}$)$_2$ × 2 + $\underline{CH_3}$$CO_2$H × 2), 3.83 (2H, s, $\underline{CH_2}$-$NH_2$), 7.0-7.3 (4H, m, aromatic-H).

Preparation Example 107

$N^4$-[2-Amino-1(S)-methylcarbamoylethyl]-$N^1$-hydroxy-2(R or S)-[3-(p-aminomethylphenyl)propyl]-3(R)-isopropylsuccinamide. 2 acetate (Compound No. 107)

The procedure of Preparation Example 23 was repeated using 6-(p-benzyloxycarbonylaminomethylphenyl)-3(RS)-tert-butyloxycarbonyl-2(R)-isopropylhexanoic acid and 2(S)-tert-butyloxycarbonylamino-3-benzyloxycarbonylaminobutanoic acid N-methylamide to provide the title compound as a white solid.

FABMS ($M^+$ + 1): 422.
$^1$H-NMR ($CD_3OD$)δ ppm; 0.8-1.0 (6H, m, CH($\underline{CH_3}$)$_2$), 1.1-1.2 (1H, m, $\underline{CH}$($CH_3$)$_2$), 1.2-1.8 (4H, m, CH-($\underline{CH_2}$)$_2$), 1.9 (6H, s, $\underline{CH_3}$$CO_2$H × 2), 2.28 (1H, m, $\underline{CH}$-CO), 2.4-2.6 (3H, m, $\underline{CH}$-CO + $C_6H_4$-$\underline{CH_2}$), 2.68 (3H, s, N-$\underline{CH_3}$), 4.02 (2H, s, Ph-$\underline{CH_2}$-$NH_2$), 4.3 (1H, m, NH-$\underline{CH}$-CO), 7.21 (4H, m, aromatic-H).

Preparation Example 108

$N^a$-tert-Butyloxycarbonyl-L-4'-cyanophenylalanine methyl ester (Compound No. 108)

To a suspension of $N^a$-tert-butyloxycarbonyl-L-4'-cyanophenylalanine (150 g, 517 mmol) in dichloromethane (2.5 L) was added methanol (62.8 mL, 1.55 mol), N,N-dimethylaminopyridine (326 mg, 5.17 mmol) and EDC (109 g, 569 mmol) sequentially with stirring at -2°C. The mixture was stirred for 3 hours at 0°C and for another 12 hours at room temperature, and evaporated. AcOEt (2 L) was added to the residue and the mixture was partitioned and washed successively with 1N hydrochloric acid, saturated aqueous sodium chloride, saturated aqueous $NaHCO_3$, and saturated aqueous sodium chloride (twice for each). The organic layer was dried over anhydrous $MgSO_4$, and evaporated under reduced pressure to give a yellow solid. Recrystallization of the yellow solid product from methyl tert-butyl ether afforded the title compound as a white solid (137 g, 451 mmol, yield 87%), m.p.; 104-106°C, $[\alpha]_D^{25}$ =- 8.3° (c=1.0, MeOH).

Preparation Example 109

L-4'-Cyanophenylalanine methyl ester • 1 hydrochloride (Compound No. 109)

Compound No. 108 (137 g, 451 mmol) was dissolved in 4N HCl (AcOEt solution, 800 mL) under ice-cooling and the mixture was stirred for 1 hour at room temperature. $Et_2O$ (500 ml) was added to the reaction mixture (white suspension) to yield precipitated crystals which were collected by filtration and dried under reduced pressure, giving the title compound as white crystals (quantitative yield).

Preparation Example 110

$N^a$-[4-tert-Butyloxy-2(R)-isobutyl-3(RS)-(3-phenylpropyl)succinyl]-L-4'-cyanophenylalanine methyl ester (Compound No. 110)

To a suspension of Compound No. 109 (108 g, 451 mmol) in DMF (1400 mL) - $CH_2Cl_2$ (700 mL) was added Compound No. 16 (146 g, 418 mmol), HOBt (67.8 g, 502 mmol), EDC (96.1 g, 502 mmol) and TEA (62 mL, 446 mmol) sequentially with stirring at -15°C. The mixture was stirred for 1 hours at -15°C and for another 15 hours at room temperature, and evaporated under reduced pressure. AcOEt (2 L) was added to the residue and the mixture was partitioned and washed successively with saturated aqueous sodium chloride, 1N hydrochloric acid, saturated aqueous sodium chloride, saturated aqueous $NaHCO_3$, and saturated aqueous sodium chloride (twice for each). The organic layer was dried over anhydrous $MgSO_4$, and evaporated under reduced pressure to give a reaction mixture. Resolidification of the resultant reaction mixture from $Et_2O$-n-hexane afforded the title compound as a white solid (147 g, 273 mmol, yield 65%), m.p.; 97-100°C, $[\alpha]_D^{25}$ =- 19.3° (c=1.0, MeOH).

Preparation Example 111

$N^a$-[Hydroxy-2(R)-isobutyl-3(RS)-(3-phenylpropyl)succinyl]-L-4'-cyanophenylalanine methyl ester (Compound No. 111)

To Compound No. 110 (146 g, 272 mmol) was added ice-cooled 95% trifluoroacetic acid (containing water (5%), 300 ml) and the mixture was stirred for 30 minutes at 5°C and for another 3.5 hours at room temperature. The reaction mixture was concentrated under reduced pressure, and a mixture of $Et_2O$ (100 mL) and n-hexane (600 mL) was added to the residue. The resulting mixture was filtered to remove precipitated solid products which were dried, yielding the title compound as a white solid (118 g, 245 mmol, 90%), m.p.; 166-169°C, $[\alpha]_D^{25}$ =- 16.4° (c = 1.0, MeOH).

Preparation Example 112

$N^a$-[4-(N-Benzyloxyamino)-2(R)-isobutyl-3(RS)-(3-phenylpropyl)succinyl]-L-4'-cyanophenylalanine methyl ester (Compound No. 112)

Compound No. 111 (118 g, 245 mmol) was dissolved in DMF (700 mL), and then cooled to -15°C. To the cooled solution was added HOBT (39.6 g, 293 mmol), O-benzylhydroxylamine hydrochloride (58.4 g, 366 mmol), EDC (56.1 g, 293 mmol) and TEA (51 mL, 366 mmol) sequentially. The reaction mixture was stirred for 15 hours at room temperature, followed by another successive addition of HOBT (16.5 g, 122 mmol), O-benzylhydroxylamine hydrochloride (19.5 g, 122 mmol), EDC (23.4 g, 122 mmol) and TEA (17 mL, 122 mmol). After stirred for 3 hours at room temperature, the reaction mixture was added dropwise to water (7 L) with stirring to precipitate white crystals which were collected by filtration. The resultant crystals were washed successively with water, 1N hydrochloric acid, water, 10% aqueous $Na_2CO_3$, and water (twice for each), and dried under reduced pressure over phosphorous pentoxide to give the title compound as white crystals (126 g, 216 mmol, 88%), m.p.; 195-206°C, $[\alpha]_D^{25}$ = +11.1° (c = 1.0, MeOH).

Preparation Example 113

$N^a$-[4-(N-Hydroxyamino)-2(R)-isobutyl-3(RS)-(3-phenylpropyl)succinyl]-L-4'-aminomethylphenylalanine methyl ester • 1 acetate (Compound No. 113)

To a solution of Compound No. 112 (41.9 g, 71.7 mmol) in acetic acid (700 mL) was added 5% palladium on carbon (50% wet catalyst, 20 g), and the mixture was vigorously stirred under hydrogen atmosphere for 1 hour at 35°C and for another 2 hours at room temperature. The catalyst was filtered off and then acetic acid was evaporated under reduced pressure. A mixture of AcOEt (180 mL) and $Et_2O$ (360 mL) was added to the resulting residue to precipitate crystals which were collected by filtration and dried under reduced pressure. The resultant crude product was purified by column reversed phase chromatography (2.5 kg of Chromatorex ODS-1020T, Fuji Silysia Chemical, Japan; eluted with a gradient of 10% to 15% methanol/0.1% aqueous acetic acid), and then lyophilized to give the title compound as a white solid (23.7 g, 44.4 mmol, 62%), m.p.; 194-197°C, $[\alpha]_D^{25}$ =- 9.82° (c=1.0, MeOH).

Preparation Example 114

$N^a$-[4-(N-Hydroxyamino)-2(R)-isobutyl-3(RS)-(3-phenylpropyl)succinyl]-L-4'-aminomethylphenylalanine • 1 hydrochloride (Compound No. 114)

To a suspension of Compound No. 113 (16 g, 28.7 mmol) in methanol (150 mL) was added aqueous 1N NaOH (115 mL) dropwise with stirring at 0°C. The mixture was stirred for 1 hour at room temperature and then neutralized with 6N hydrochloric acid. Thereafter, methanol was evaporated under reduced pressure. The resulting solution was purified by column reversed phase chromatography (900 g of Chromatorex ODS-1020T, Fuji Silysia Chemical, Japan; eluted with a gradient of 0% to 30% methanol/0.1% aqueous hydrochloric acid), and then lyophilized to give the title compound as a white solid (10.8 g, 20.7 mmol, 72%), m.p.; 156°C, $[\alpha]_D^{25}$ =- 3.4° (c=1.0, MeOH).

Preparation Example 115

$N^a$-[4-(N-Hydroxyamino)-2(R)-isobuty-3(RS)-(3-phenylpropyl)succinyl]-L-4'-acetimidoyliminomethylphenylalanine methyl ester • 1 hydrochloride (Compound No. 115)

Compound No. 113 (500 mg, 0.9 mmol) was dissolved in DMF (10 mL), and then cooled to 0°C. To the cooled solution was added TEA (263 μL, 1.89 mmol) and ethyl acetimidate hydrochloride (126 mg, 0.99 mmol). The mixture was stirred for 1.5 hours at room temperature. The reaction mixture was adjusted to pH 1 by addition of 1N hydrochloric acid and the solvent was evaporated under reduced pressure. The resulting residue was purified by column reversed phase chromatography (25 g of Chromatorex ODS-1020T, Fuji Silysia Chemical, Japan; eluted with a gradient of 0% to 25% methanol), and then lyophilized to give the title compound as a white solid (336 mg, 0.585 mmol, 65%), m.p.; 136-139°C, $[\alpha]_D^{25}$ =- 10.3° (c=1.0, MeOH).

$^1$H-NMR ($CD_3OD$)δ ppm; 0.85 (6H, m, CH($CH_3$)$_2$), 1.0 (2H, m, $CH_2$-CH($CH_3$)$_2$), 1.3-1.6 (5H, m, ($CH_2$)$_2$-$CH_2$-Ph + $CH_2$-CH($CH_3$)$_2$), 2.10 (1H, m, CH-CO), 2.16 (3H, s, C-$CH_3$), 2.3-2.6 (3H, m, CH-CO + $CH_2$-Ph), 2.92 and 3.15 (1H each, m, CH-$CH_2$-$C_6H_4$), 3.67 (3H, m, $OCH_3$), 4.36 (2H, s, $C_6H_4$-$CH_2$-NH), 4.70 (1H, m, CH-$CH_2$-$C_6H_4$), 7.0-7.4 (9H, m, aromatic-H).

Preparation Example 116

$N^a$-[4-(N-Hydroxyamino)-2(R)-isobutyl-3(RS)-(3-phenylpropyl)succinyl]-L-4'-acetimidoyliminomethylphenylalanine • 1 acetate (Compound No. 116)

Compound No. 114 (500 mg, 0.9 mmol) was dissolved in DMF (10 mL), and then cooled to 0°C. To the cooled solution was added TEA (470 μL, 3.36 mmol) and ethyl acetimidate hydrochloride (180 mg, 1.44 mmol). The mixture was stirred at room temperature overnight and concentrated under reduced pressure. The resulting residue was purified by column reversed phase chromatography (50 g of Chromatorex ODS-1020T, Fuji Silysia Chemical, Japan; eluted with a gradient of 0% to 25% methanol/0.1% aqueous hydrochloric acid), and then lyophilized to give the title compound as a white solid (200 mg, 0.35 mmol, 37%).

$^1$H-NMR ($CD_3OD$)δ ppm; 0.85 (6H, m, CH($CH_3$)$_2$), 1.0 (2H, m, $CH_2$-CH( $CH_3$)$_2$), 1.3-1.6 (5H, m, ($CH_2$)$_2$-$CH_2$-Ph + $CH_2$-CH($CH_3$)$_2$), 1.90 (3H, s, $CH_3CO_2H$), 2.10 (1H, m, CH-CO), 2.15 (3H, s, C-$CH_3$), 2.3-2.6 (3H, m, CH-CO + $CH_2$-Ph), 2.93 and 3.18 (1H each, m, CH-$CH_2$-$C_6H_4$) , 4.35 (2H, s, $C_6H_4$-$CH_2$-NH), 4.68 (1H, m, CH-$CH_2$-$C_6H_4$), 7.0-7.4 (9H, m, aromatic-H).

Preparation Example 117

$N^a$-tert-Butyloxycarbonyl-L-4-'-[N,N'-bis(benzyloxycarbonyl)guanido]phenylalanine-N-methylamide (Compound No. 117)

To a solution of $N^a$-tert-butyloxycarbonyl-L-4'-aminophenylalanine-N-methylamide (2.00 g, 6.82 mmol) in $CH_2Cl_2$ (30 mL) was added 1H-pyrazole-N,N'-bis(benzyloxycarbonyl)carboxamidine (2.84 g, 7.51 mmol), and the mixture was stirred for 3 hours at room temperature. After concentrated under reduced pressure, the reaction mixture was dissolved in AcOEt (30 mL) and stirred for 30 minutes to yield a precipitated crystal which was dried, giving the title compound as a white solid (3.95 g, 96%), m.p.; 168°C, $[\alpha]_D$=+14.1° (c=1.0, $CHCl_3$), Rf value; 0.20 ($CHCl_3$:MeOH=50:1).

Preparation Example 118

2(R)-tert-Butyloxycarbonylmethyl-4-methylpentanoic acid (Compound No. 118)

To a solution of Compound No. 16-b (30.0 g, 66 mmol) in ethanol (100 mL) was added 5% palladium on carbon (50% wet catalyst, 10 g), and the mixture was vigorously stirred under hydrogen atmosphere for 2 hours at room temperature. The catalyst was filtered off and then ethanol was evaporated under reduced pressure. To the resulting residue was added N-ethylmorpholine (8.35 ml, 66 mmol) and toluene (725 mL), and the mixture was refluxed for 1 hour. After cooling, the reaction mixture was partitioned and washed successively with 1N hydrochloric acid, and saturated aqueous sodium chloride (twice for each). The resultant layer was dried over anhydrous magnesium sulfate, evaporated under reduced pressure, and purified by column chromatography (silica gel; 50 g, eluted with a mixture of

hexane:AcOEt=3:1) to give the title compound as a colorless oil (11.0 g, 77%).

$^1$H-NMR ($CDCl_3$)δ ppm; 0.85 (6H, m, CH(CH$_3$)$_2$), 1.2-1.7 (12H, s + m, CH$_2$-CH(CH$_3$)$_2$ + C(CH$_3$)$_3$), 2.35, 2.60 and 2.87 ( 1H each, m, CO-CH$_2$-CH-CO).

Preparation Example 119

N$^a$-[4-tert-Butyloxy-2(R)-isobutylsuccinyl]-L-4'-[N,N'-bis(benzyloxycarbonyl)guanido]phenylalanine-N-methylamide (Compound No. 119)

Compound No. 117 (107 g, 177 mmol) was dissolved in a solution of hydrogen chloride in AcOEt (4N, 750 mL) and the mixture was stirred for 45 minutes under ice-cooling. To the reaction mixture was added $Et_2O$ (1,100 mL) and the mixture was stirred to yield precipitated crystals which were collected by filtration, washed five times with $Et_2O$ (100 mL), and dried. To a solution of the resultant crystals (95.6 g, 177 mmol), Compound No. 118 (37.0 g, 161 mmol), and HOBT (26.1 g, 193 mmol) in DMF (500 mL) was added TEA (27 ml, 193 mmol), and EDC (37.0 g, 193 mmol) sequentially with stirring at -15°C. The mixture was stirred for 1 hour at -15°C and further overnight at room temperature, and then evaporated under reduced pressure. The resultant residue was purified by column chromatography (silica gel; 850 g, eluted with a mixture of $CH_2Cl_2$: AcOEt=3:1) to give the title compound as a white solid (99.1 g, 86%).

$^1$H-NMR ($CDCl_3$)δ ppm; 0.7-0.85 (6H, m, CH(CH$_3$)$_2$), 1.0-1.7 (12H, s + m, CH$_2$-CH(CH$_3$)$_2$ + C(CH$_3$)$_3$), 2.3-2.6 (2H, m, CO-CH$_2$-CH-CO), 2.7-2.9 (4H, m, N-CH$_3$ + CO-CH$_2$-CH-CO), 3.02 (2H, m, C$_6$H$_4$-CH$_2$), 4.05 (1H, m, NH-CH-CO), 5.0-5.2 (5H, m + s, OCH$_2$Ph x 2 + NH), 6.01 (1H, m, NH), 7.0-7.7 (14H, m, aromatic-H + NH x 2).

Preparation Example 120

N$^a$-[4-(N-Benzyloxyamino)-2(R)-isobutylsuccinyl]-L-4'-[N,N'-bis(benzyloxycarbonyl)guanido]phenylalanine-N-methylamide (Compound No. 120)

To Compound No. 119 (99.0 g, 138 mmol) was added ice-cooled 95% trifluoroacetic acid hydrate (300 mL) and the mixture was stirred for 30 minutes under ice-cooling and for another 2 hours at room temperature. The reaction mixture was concentrated under reduced pressure, and triturated with $Et_2O$. The precipitated solid was collected by filtration, and dried to give the title compound as a white solid (89.9 g).

Preparation Example 121

N-[4-(N-Hydroxyamino)-2(R)-isobutylsuccinyl]-L-4'-guanidophenylalanine-N-methylamide 1 acetate (Compound No. 121)

To a suspension of Compound No. 120 (93.9 g, 123 mmol) in AcOH (900 mL) was added 5% Pd-C (50% wet catalyst, 45 g), and the mixture was vigorously stirred under hydrogen atmosphere for 4.5 hours at room temperature. The catalyst was filtered off and then AcOH was evaporated under reduced pressure. The resulting crude product was purified by column reversed phase chromatography (2.5 kg of Chromatorex ODS-1020T, Fuji Silysia Chemical, Japan; eluted with 0.1% aqueous acetic acid), and then lyophilized to give the title compound as a white solid (52 g, 91%).

ESIMS (M + 1) 407,
$^1$H-NMR ($CD_3OD$)δ ppm; 0.75-1.0 (6H, m, CH(CH$_3$)$_2$), 1.15 (1H, m, CH(CH$_3$)$_2$), 1.3-1.6 (2H, m, CH$_2$-CH(CH$_3$)$_2$), 1.93 (3H, s, CH$_3$CO$_2$H), 2.15 (2H, m, CO-CH$_2$), 2.71 (4H, s + m, CO-CH + N-CH$_3$) , 2.95 and 3.15 (1H each, d, J = 6.8 Hz, C$_6$H$_4$-CH$_2$), 4.56 (1H, m, NH), 6.00 (1H, m, NH-CH-CO), 7.1-7.4 (4H, m, aromatic-H).

Preparation Example 122

N-[4-(N-Hydroxyamino)-2(R)-isobutylsuccinyl]-L-4'-guanidophenylalanine-N-methylamide 1 sulfate (Compound No. 122)

A solution of Compound No. 121 (2.0 g, 4.29 mmol) in 1N sulfuric acid (23 ml) was adsorbed onto highly porous polymer resins (HP-21, Mitsubishi Chemical Corporation, 160 ml). The resins were washed with water to make pH=7 and then subjected to an elution with 10% aqueous $CH_3CN$. The eluate was concentrated under reduced pressure to give the title compound as a white solid (1.82 g, 3.61 mmol).

Preparation Example 123

$N^a$-[4-tert-Butoxy-2(R)-isobutyl-3(S)-(3-phenylpropyl)succinyl]-L-4'-[N,N'-bis(benzyloxycarbonyl)guanido]phenylalanine-N-methylamide (Compound No. 123)

The procedure of Preparation Example 119 was repeated using Compound No. 117 and Compound No. 16 to provide the title compound as a white solid (yield 37%), m.p.; 191°C, $[\alpha]_D$= - 10.1° (c=1.0, $CHCl_3$), Rf value; 0.28 (n-hexane:AcOEt=1:1).

Preparation Example 124

$N^a$-[4-(N-Benzyloxyamino)-2(R)-isobutyl-3(S)-(3-phenylpropyl)succinyl]-L-4'-[N,N'-bis(benzyloxycarbonyl)guanido]phenylalanine-N-methylamide (Compound No. 124)

The procedure of Preparation Example 120 was repeated using Compound No. 123 to provide the title compound as a white solid (yield 76%), m.p.; 220°C (dec.), Rf value; 0.58 ($CHCl_3$: MeOH=10:1).

Preparation Example 125

N -[4-(N-Hydroxyamino)-2(R or S)-isobutyl-3(S)-(3-phenyltrimethylene)succinyl]-L-4'-guanidophenylalanine-N-methylamide 1 hydrochloride (Compound No. 125)

To a suspension of Compound No. 124 (42.0 g, 47.6 mmol) in MeOH (1,000 mL) was added 5% Pd-C (50% wet catalyst, 10 g), and the mixture was vigorously stirred under hydrogen atmosphere for 3 hours at the internal temperature of 40 to 45°C. The catalyst was filtered off and then MeOH was evaporated under reduced pressure. The resulting crude product was dissolved in 1N hydrochloric acid (143 mL), purified by column reversed phase chromatography (900 g of Chromatorex ODS-1020T, Fuji Silysia Chemical, Japan; eluted with 2 to 7% aqueous MeOH), and then lyophilized to give the title compound as a white solid (24.5 g, 91%), Rf value; 0.48 ($CHCl_3$:MeOH:AcOH=95:5:3).

Preparation Example 126

N-[4-(N-Hydroxyamino)-2(R)-isobutyl-3(S)-(3-phenyltrimethylene)succinyl]-L-4'-acetimidoyliminomethylenephenyl alanine-N-methylamide • 1 acetate (Compound No. 126)

The procedure of Preparation Example 22 was repeated using Compound No. 5 and Compound No. 16 to provide the title compound as a white solid (overall yield 16%), $[\alpha]_D$= - 8.9° (c=1.0, MeOH), Rf value; 0.13 ($CHCl_3$:MeOH:AcOH=5:2:1), 0.50 (n-BuOH:AcOH:$H_2O$=4:1:1).

Preparation Example 127

N-[4-(N-Hydroxyamino)-2(R),3(S)-diisobutylsuccinyl]-L-4'-aminomethylphenylalaninyl-β-alanine • 1 acetate (Compound No. 127)

The procedure of Preparation Example 23 was repeated using Compound No. 17 and $N^a$-tert-butyloxycarbonyl-L-4'-cyanophenylalanine N-(2-benzyloxycarbonylethyl)amide to provide the title compound as a white solid (overall yield 15%).

$^1$H-NMR (MeOH-$d_4$)δ ppm; 0.7-0.9 (12H, m, $\underline{CH(CH_3)_2}$ x 2), 1.0-1.65 (6H, m, $\underline{CH_2}$-$\underline{CH}(CH_3)_2$ x 2), 1.90 (3H, s, $\underline{CH_3}CO_2H$), 2.0-2.3 (2H, m, -$\underline{CH}$-CO x 2 ), 2.9-3.1 (2H, m, $C_6H_4$-$\underline{CH_2}$), 3.2-3.4 (NH-$\underline{CH_2}$-$\underline{CH_2}$-CO), 4.04 (2H, m, $\underline{CH_2}$-$NH_2$), 4.52 (1H, m, NH-$\underline{CH}$-CO), 7.32 (4H, m, aromatic-H).

Preparation Example 128

N-[4-(N-Hydroxyamino)-2(R)-isobutyl-3(R)-(3-phenylpropyl)succinyl)-L-4'-aminomethylphenylalanine-N-methylamide • 1 hydrochloride (Compound No. 128)

The procedure of Preparation Example 23 was repeated using Compound No. 5 and Compound No. 16 to provide the title compound as a white solid (overall yield 17%).

$^1$H-NMR (MeOH-$d_4$)δ ppm; 0.69 (6H, m, CH($\underline{CH_3}$)$_2$), 0.90 (2H, m, $\underline{CH_2}$-CH($CH_3$)$_2$), 1.3-1.7 (5H, $\underline{CH}$($CH_3$)$_2$ + $\underline{CH_2}$-$\underline{CH_2}$-$CH_2$-Ph), 2.14 (1H, m, $\underline{CH}$-CO), 2.4-2.7 (6H, s + m, $\underline{CH_2}$-Ph + -$\underline{CH}$-CO + N-$\underline{CH_3}$), 2.88 and 3.17 (1H each, m, $C_6H_4$-$\underline{CH_2}$), 4.07 (2H, s, $\underline{CH_2}$-$NH_2$), 4.66 (1H, m, NH-$\underline{CH}$-CO), 7.05-7.45 (9H, m, aromatic-H).

Preparation Example 129

N-[4-(N-Hydroxyamino)-2(R)-isobutyl-3(R or S)-[3-(p-methoxycarbonylphenyl)propyl)succinyl]-L-lysine N-methylamide 1 hydrochloride (Compound No. 129)

A solution of Compound No. 65 (2.0 g, 3.53 mmol) in 1N hydrochloric acid (20 mL) was adsorbed onto highly porous polymer resins (HP-21, Mitsubishi Chemical Corporation, 150 ml). The resins were washed with water to make pH=7 and then subjected to an elution with 10% aqueous $CH_3CN$. After $CH_3CN$ was evaporated under reduced pressure, the eluate was lyophilized to give the title compound as a white solid (1.83 g, 3.34 mmol).

ESIMS (M +) 407,
$^1$H-NMR (MeOH-$d_4$)δ ppm; 0.8-0.9 (6H, m, CH($\underline{CH_3}$)$_2$), 1.05 (1H, m, $\underline{CH}$($CH_3$)$_2$), 1.2-1.8 (12H, m, $\underline{CH_2}$-CH($CH_3$)$_2$ + CH-($\underline{CH_2}$)$_3$ + CH-($\underline{CH_2}$)$_2$), 2.20 (1H, m, $\underline{CH}$-CO), 2.53 (1H, m, -$\underline{CH}$-CO), 2.6-2.75 (5H, s + m, $C_6H_4$-$\underline{CH_2}$ + N-$\underline{CH_3}$), 2.90 (2H, m, $\underline{CH_2}$-$NH_2$), 3.89 (3H, s, -$CO_2\underline{CH_3}$), 4.24 (1H, m, NH-$\underline{CH}$-CO), 7.26 and 7.90 (2H each, m, aromatic-H).

Preparation Example 130

N-[4-(N-Hydroxyamino)-2(R)-isobutyl-3(R or S)-(3-phenyltrimethylene)succinyl)-L-4'-guanidophenylalanine-N-methylamide 1 acetate (Compound No. 130)

To a suspension of Compound No. 124 (7.00 g, 7.90 mmol) in AcOH (200 mL) was added 5% Pd-C (50% wet catalyst, 4.0 g), and the mixture was vigorously stirred under hydrogen atmosphere for 3 hours at room temperature. The catalyst was filtered off and then AcOH was evaporated under reduced pressure. The resulting residue was suspended in AcOEt (300 mL) and stirred for 30 min. Insoluble materials were collected by filtration and dried under reduced pressure in a desiccator followed by addition of water (370 mL). The resultant mixture was lyophilized to give the title compound as amorphous white powders (4.30g, 93%).

$[\alpha]_D$= - 10.2° (c=1.0, MeOH), Rf value; 0.48 ($CHCl_3$:MeOH:AcOH=95:5:3).

Preparation Example 131

N-[4-(N-Hydroxyamino)-2(R)-isobutyl-3(S)-hydroxysuccinyl]-L-4'-aminomethylphenylalanine-N-methylamide 1 acetate (Compound No. 131)

To a solution of Compound No. 43-b (375 mg, 1.0 mmol), O-benzylhydroxylamine hydrochloride (239 mg, 1.5 mmol) and HOBT (162 mg, 1.2 mmol) in DMF (30 mL) was added TEA (209 μl, 1.5 mmol), and EDC (230 mg, 1.2 mmol) with stirring at -15°C. The mixture was stirred for 1 hour at -15°C and further at room temperature overnight, and then added to 0.5N hydrochloric acid (100 mL) dropwise. Precipitated solids were collected by filtration, washed successively with water, saturated aqueous sodium bicarbonate and water, and dried over phosphorus pentoxide under reduced pressure. The resultant white solid was dissolved in acetic acid (17 ml). To the solution was added 5% palladium on carbon (50% wet catalyst, 340 mg), and the mixture was vigorously stirred under hydrogen atmosphere for 3 hours at room temperature. The catalyst was filtered off and then acetic acid was evaporated under reduced pressure. The resulting residue was dissolved in water (10 mL) and lyophilized to give the title compound as a white solid (303 mg, 95%).

$^1$H-NMR ($CD_3OD$)δ ppm; 0.83 (6H, m, CH($\underline{CH_3}$)$_2$), 1.1-1.6 (3H, m, $\underline{CH_2}$-$\underline{CH}$($CH_3$)$_2$), 1.97 (3H, s, $\underline{CH_3}CO_2H$), 2.6-

2.8 (4H, m, N-CH3 + CH-CO), 2.9-3.3 (2H, m, $C_6H_4$-$CH_2$), 3.98 (2H, s, $CH_2$-$NH_2$), 4.4-4.6 (2H, m, NH-CH-CO + HO-CH-CO), 7.28 (4H, m, aromatic-H).

Preparation Example 132

N-[4-(N-Hydroxyamino)-2(R)-isobutyl-3(S)-(3-phenylpropyl)succinyl]-L-4'-aminomethylphenylalanine methyl ester 1 hydrochloride (Compound No. 132)

To a suspension of Compound No. 113 (10 g, 18.7 mmol) in water (500 mL) was added 1N hydrochloric acid (37.5 mL) with stirring, and the resultant solution was lyophilized to give the title compound as a white solid (9.99 g, quant. yield).

$^1$H-NMR ($CD_3OD$)δ ppm; 0.78 (6H, m, $CH(CH_3)_2$), 0.93 (1H, m, $CH(CH_3)_2$), 1.2-1.5 (6H, m, $CH_2$-$CH(CH_3)_2$ + $(CH_2)_2$-$CH_2$-Ph), 2.01 (1H, m, CH-CO), 2.2-2.45 ( 3H, m, $CH_2$-Ph + CH-CO), 2.84 and 3.10 (1H each, m, $C_6H_4$-$CH_2$), 3.59 (3H, s, $OCH_3$), 3.91 (2H, s, $CH_2$-$NH_2$), 4.65 (1H, m,NH-CH-CO), 7.0-7.3 (9H, m, aromatic-H).

The following symbols are intended to have the meanings set forth below in the specification and the appended claims:

$N^a = N^\alpha$
$N^e = N^\varepsilon$
$N^d = N^\delta$
$N^g = N^g$
$N^w = N^\omega$

Industrial Applicability

Provided by the present invention are pharmaceutical compositions exerting antiallergic or anti-inflammatory actions, or prophylactic and/or therapeutic efficacy on bronchial asthma or atopic diseases which comprise as an active ingredient at least one member selected from the hydroxamic acid derivatives according to the present invention. The pharmaceutical drugs containing an effective amount of the compounds according to the present invention can be applied to the therapy of allergy, especially to a diseased site of patients afflicted with allergy including type I and/or type IV allergy, etc., or to a predetermined site of allergic persons including type I and/or type IV allergy, etc., thereby leading to an excellent or significant effect thereon. The drugs are effective in treating bronchial asthma, allergic rhinitis, allergic conjunctivitis, pollinosis, atopic diseases (for example, atopic dermatitis, atopic enteritis, etc.), allergy to food substances, urticaria, various contact-hypersensitivities, graft-versus-host disease (GVH disease) occurred in organ transplantation, etc. The pharmaceutical drugs containing an effective amount of the compounds according to the present invention can be applied to the therapy and/or prophylaxis of inflammation, thereby exerting an advantageous effect thereon. The application of such drugs leads to (A) reduction of cells (such as lymphocytes, neutrophils, mast cells, eosinophils, basophils, macrophages and monocytes) at a diseased site, and/or (B) alleviation of inflammatory symptoms caused by migration, infiltration or accumulation of cells (such as lymphocytes, neutrophils, mast cells, eosinophils, basophils, macrophages and monocytes) to the diseased site, (C) inhibition of pathophysiological functions in cells (such as lymphocytes, neutrophils, mast cells, eosinophils, basophils, macrophages, monocytes, Langerhans cell and dendritic cells), and/or (D) reducing the blood level or inhibiting the production, of antibodies, especially IgE. Accordingly, the drugs are useful in the prophylaxis and/or therapy of diseases, disorders or ill conditions at the site.

While the present invention has been described specifically in detail with reference to certain embodiments and examples thereof, it would be apparent that it is possible to practice it in other forms. In light of the disclosure, it will be understood that various modifications and variations are within the spirit and scope of the appended claims.

## Claims

1. A prophylactic and/or therapeutic drug for allergic diseases which comprises an effective amount of at least one member selected from the group consisting of a compound having the following formula (I):

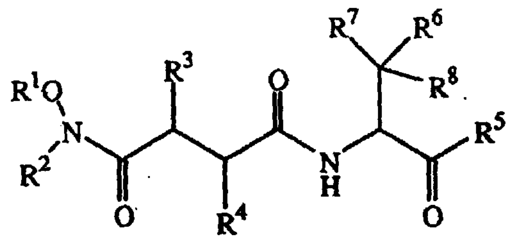

(I)

wherein $R^1$ is selected from the group consisting of hydrogen, unsubstituted or optionally substituted aralkyl, silyl which may optionally have three substituents, tetrahydropyranyl, unsubstituted or optionally substituted aralkyloxycarbonyl, unsubstituted or optionally substituted alkyloxycarbonyl, unsubstituted or optionally substituted alkyl, and a hydroxy-protecting group;

$R^2$ is selected from the group consisting of hydrogen, unsubstituted or optionally substituted aralkyloxycarbonyl, unsubstituted or optionally substituted alkyloxycarbonyl, 9-fluorenylmethyloxycarbonyl, and an amino-protecting group;

$R^3$ is selected from the group consisting of hydrogen, hydroxy, unsubstituted or optionally substituted alkyl, and unsubstituted or optionally substituted aralkyl;

$R^4$ is selected from the group consisting of unsubstituted or optionally substituted alkyl, and unsubstituted or optionally substituted aralkyl;

$R^5$ is $OR^9$ or

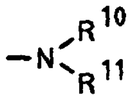

$R^9$ is selected from the group consisting of hydrogen, unsubstituted or optionally substituted alkyl, unsubstituted or optionally substituted aralkyl, and a carboxyl-protecting group;

$R^{10}$ and $R^{11}$, which may be identical or different, are each independently selected from the group consisting of hydrogen, unsubstituted or optionally substituted alkyl, unsubstituted or optionally substituted cycloalkyl, an unsubstituted or optionally substituted heterocyclic group, and an amino-protecting group, or $R^{10}$ and $R^{11}$ taken together with the nitrogen atom to which they are attached form an unsubstituted or optionally substituted heterocyclic group;

$R^6$ is selected from the group consisting of hydrogen, hydroxy, amino, and a group of the formula: -X-Y

wherein X is selected from the group consisting of oxygen, unsubstituted or optionally substituted ($C_1$-$C_6$) alkylene, and unsubstituted or optionally substituted phenylene, and

Y is a group of the formula: -A-B or -B,

wherein A is selected from the group consisting of unsubstituted or optionally substituted ($C_1$-$C_6$) alkylene, oxygen, sulfur, imino, and unsubstituted or optionally substituted ($C_1$-$C_6$) alkyleneimino, and B is selected from the group consisting of hydrogen, amino, amidino, acylimidoyl, unsubstituted or optionally substituted imidazolyl, unsubstituted or optionally substituted hydantoin-1-yl, unprotected or optionally protected bis(phosphono)methyl, unprotected or optionally protected glucopyranosyl, and unprotected or optionally protected bis(phosphono)hydroxymethyl;

$R^7$ is selected from the group consisting of hydrogen, hydroxy, unsubstituted or optionally substituted alkyl, and

unsubstituted or optionally substituted aralkyl;
$R^8$ is selected from the group consisting of hydrogen, hydroxy, unsubstituted or optionally substituted alkyl, and unsubstituted or optionally substituted aralkyl;
and a pharmaceutically acceptable salt or solvate thereof.

**2.** The drug according to claim 1 which exerts a prophylactic and/or therapeutic action on inflammation resulting from an allergic reaction.

**3.** The drug according to claim 1 or 2 for the prophylactic and/or therapeutic treatment of type I allergic disorders.

**4.** The drug according to claim 1 or 2 for the prophylactic and/or therapeutic treatment of type IV allergic disorders.

**5.** The drug according to claim 1 or 2 for reducing a blood antibody titer or inhibiting the production of antibodies to prophylactically and/or therapeutically treat allergic diseases.

**6.** The drug according to claim 1, 2 or 5 for reducing the blood level of IgE or inhibiting the production of IgE to prophylactically and/or therapeutically treat allergic diseases.

**7.** A prophylactic and/or therapeutic drug for bronchial asthma which comprises an effective amount of at least one member selected from the group consisting of a compound having the following formula (I):

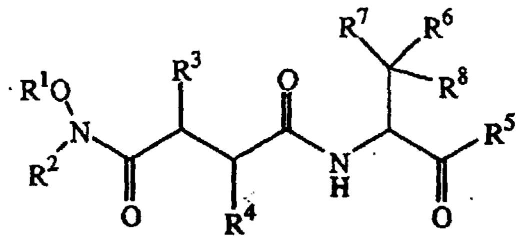

(I)

wherein $R^1$ to $R^8$, all have the same meanings as defined in Claim 1, and a pharmaceutically acceptable salt or solvate thereof.

**8.** The drug according to claim 7 for the prophylactic and/or therapeutic treatment of chronic bronchial asthma.

**9.** A prophylactic and/or therapeutic drug for allergic rhinitis which comprises an effective amount of at least one member selected from the group consisting of a compound having the following formula (I):

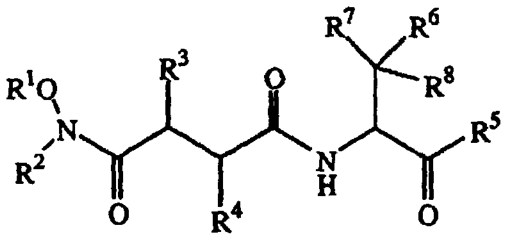

(I)

wherein $R^1$ to $R^8$, all have the same meanings as defined in Claim 1, and a pharmaceutically acceptable salt or solvate thereof.

10. A prophylactic and/or therapeutic drug for atopic diseases which comprises an effective amount of at least one member selected from the group consisting of a compound having the following formula (I):

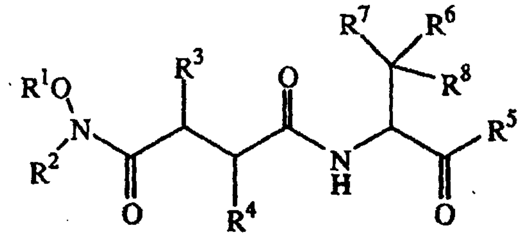

(I)

wherein $R^1$ to $R^8$, all have the same meanings as defined in Claim 1, and a pharmaceutically acceptable salt or solvate thereof.

11. The drug according to claim 10 for the prophylactic and/or therapeutic treatment of atopic dermatitis.

12. A prophylactic and/or therapeutic drug for allergic conjunctivitis which comprises an effective amount of at least one member selected from the group consisting of a compound having the following formula (I):

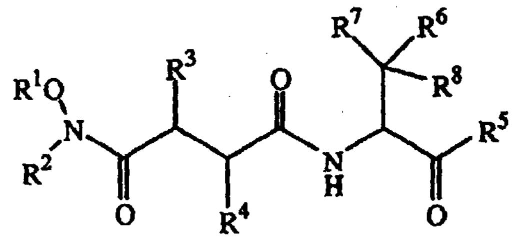

(I)

wherein $R^1$ to $R^8$, all have the same meanings as defined in Claim 1, and a pharmaceutically acceptable salt or solvate thereof.

13. A prophylactic and/or therapeutic drug for allergic responses which comprises an effective amount of at least one member selected from the group consisting of a compound having the following formula (I):

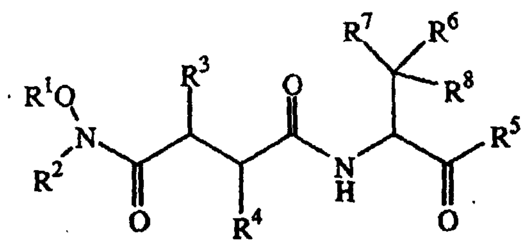

(I)

wherein $R^1$ to $R^8$, all have the same meanings as defined in Claim 1, and a pharmaceutically acceptable salt or

solvate thereof, said drug having the property of alleviating immediate, late and/or very late allergic responses.

**14.** A prophylactic and/or therapeutic drug for allergic gastroenteritis (allergic inflammation in digestive tract) which comprises an effective amount of at least one member selected from the group consisting of a compound having the following formula (I):

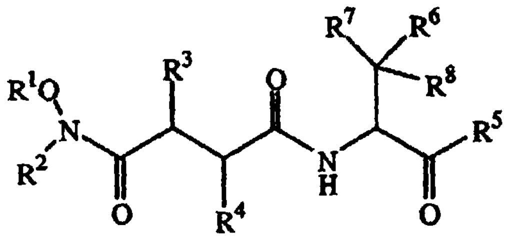

(I)

wherein $R^1$ to $R^8$, all have the same meanings as defined in Claim 1, and a pharmaceutically acceptable salt or solvate thereof.

FIG. 1

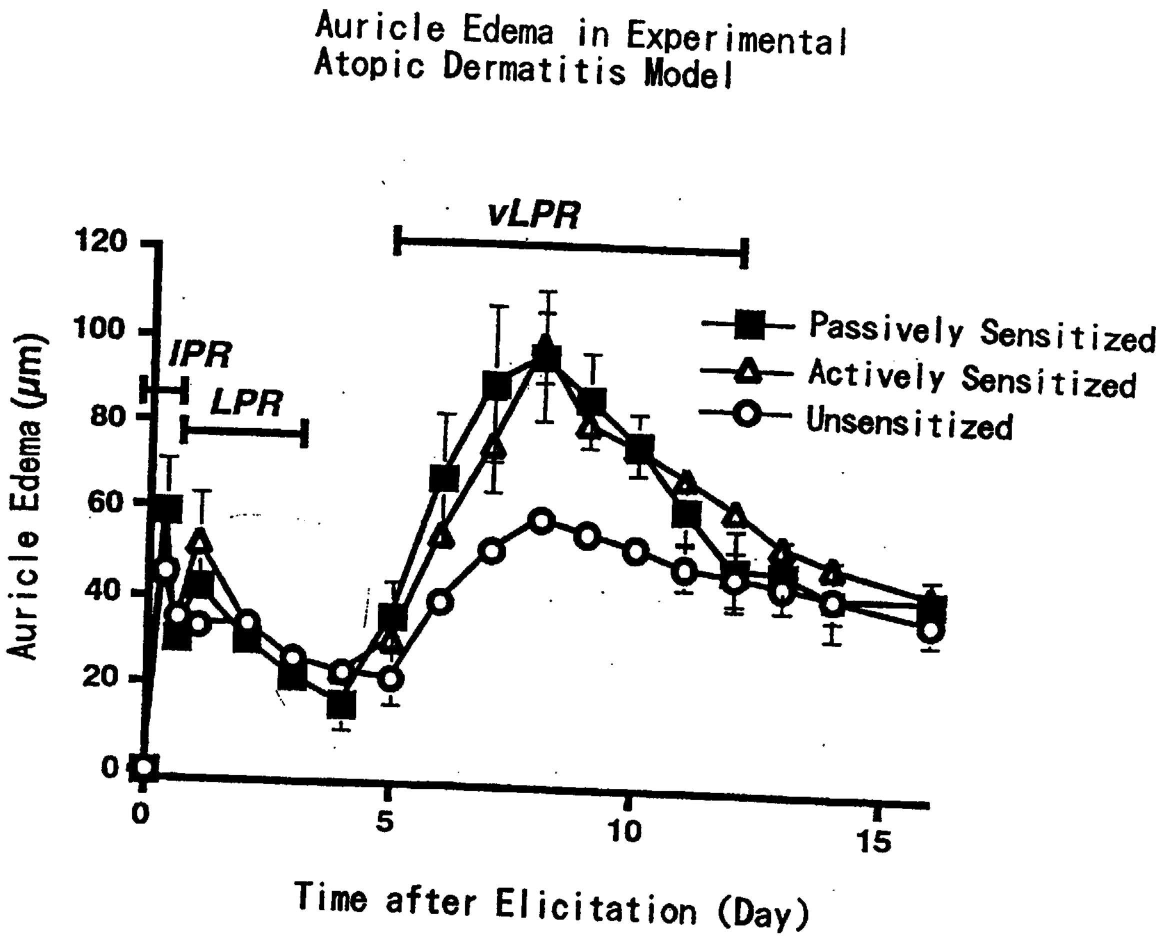
Auricle Edema in Experimental
Atopic Dermatitis Model
vLPR
IPR
LPR
Passively Sensitized
Actively Sensitized
Unsensitized
Auricle Edema (μm)
120
100
80
60
40
20
0
0
5
10
15
Time after Elicitation (Day)

FIG. 2

(1) Immunological Characteristics in Experimental Atopic Dermatitis

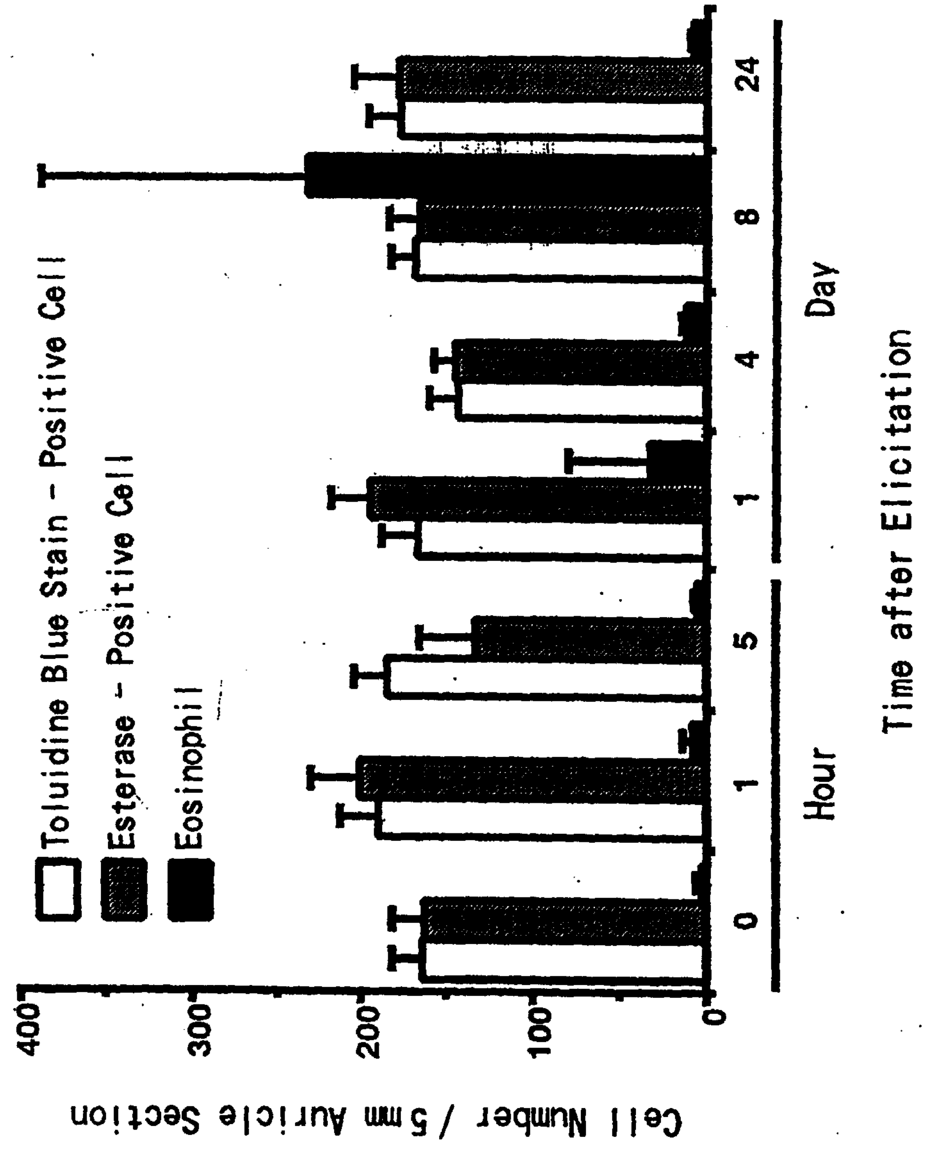

FIG. 3

(2) Immunological Characteristics
in Experimental Atopic Dermatitis

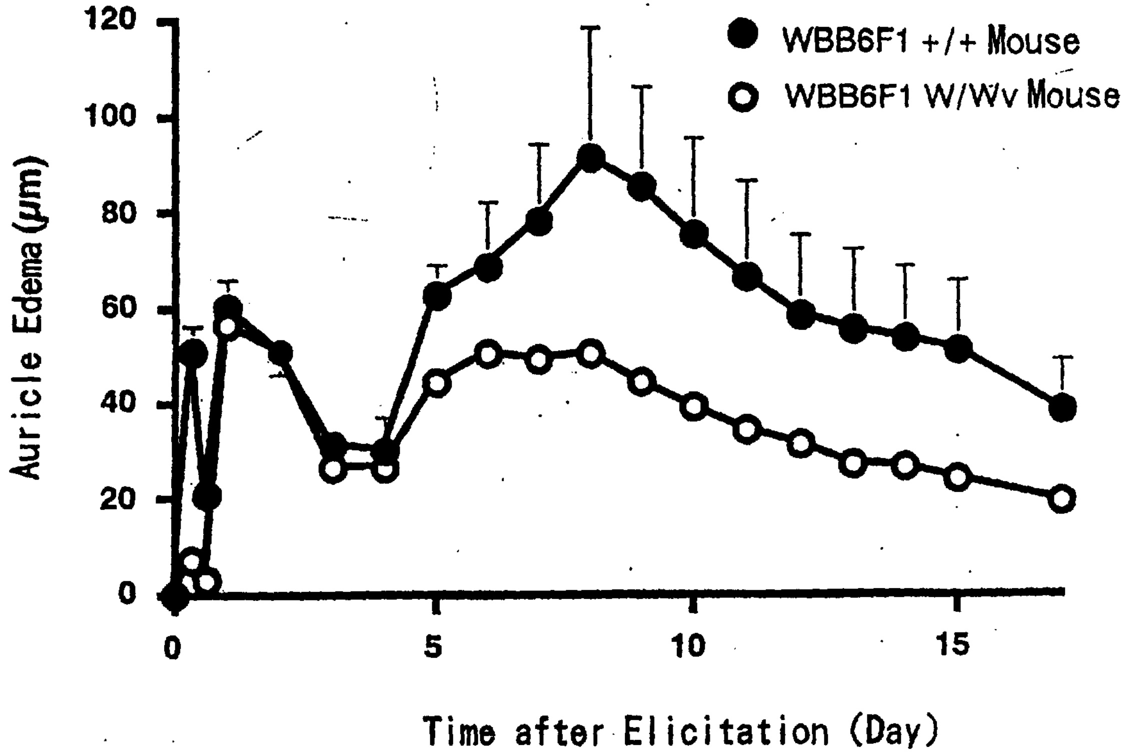

FIG. 4

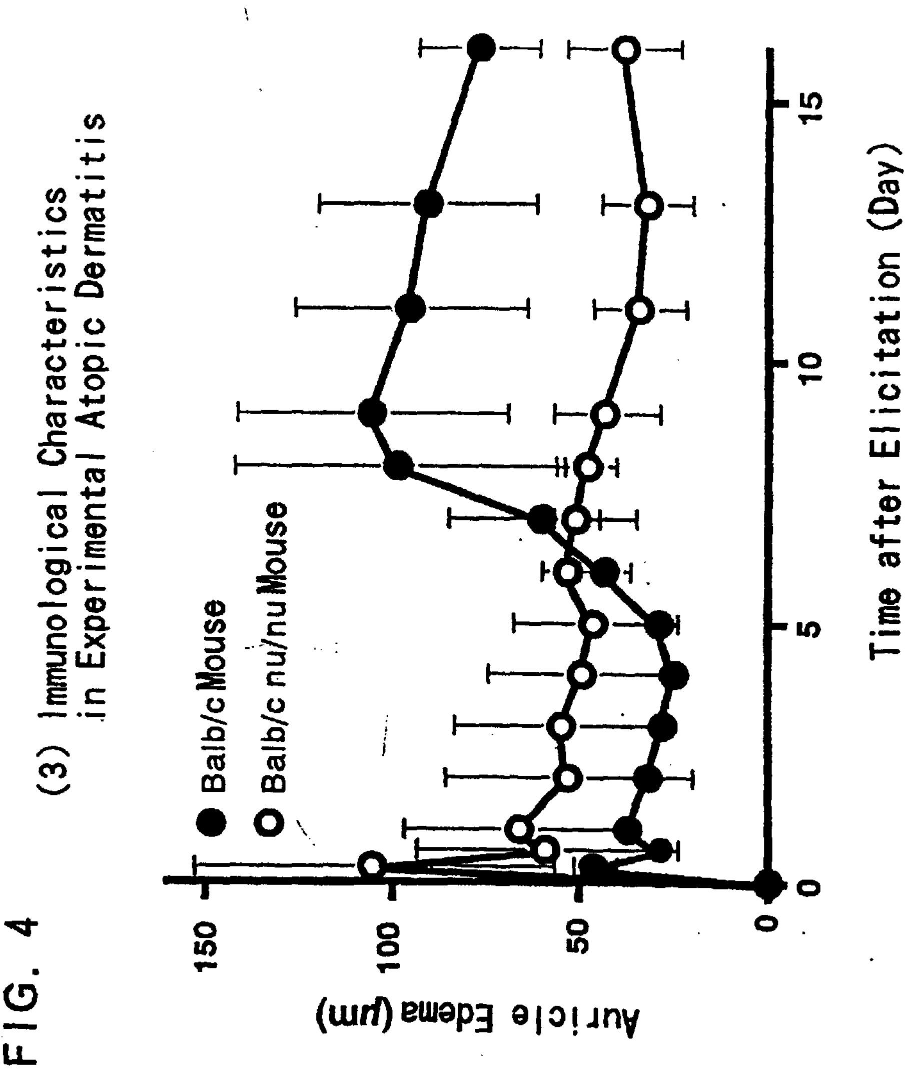
(3) Immunological Characteristics
in Experimental Atopic Dermatitis
● Balb/c Mouse
○ Balb/c nu/nu Mouse
Auricle Edema (μm)
150
100
50
0
0
5
10
15
Time after Elicitation (Day)

FIG. 5

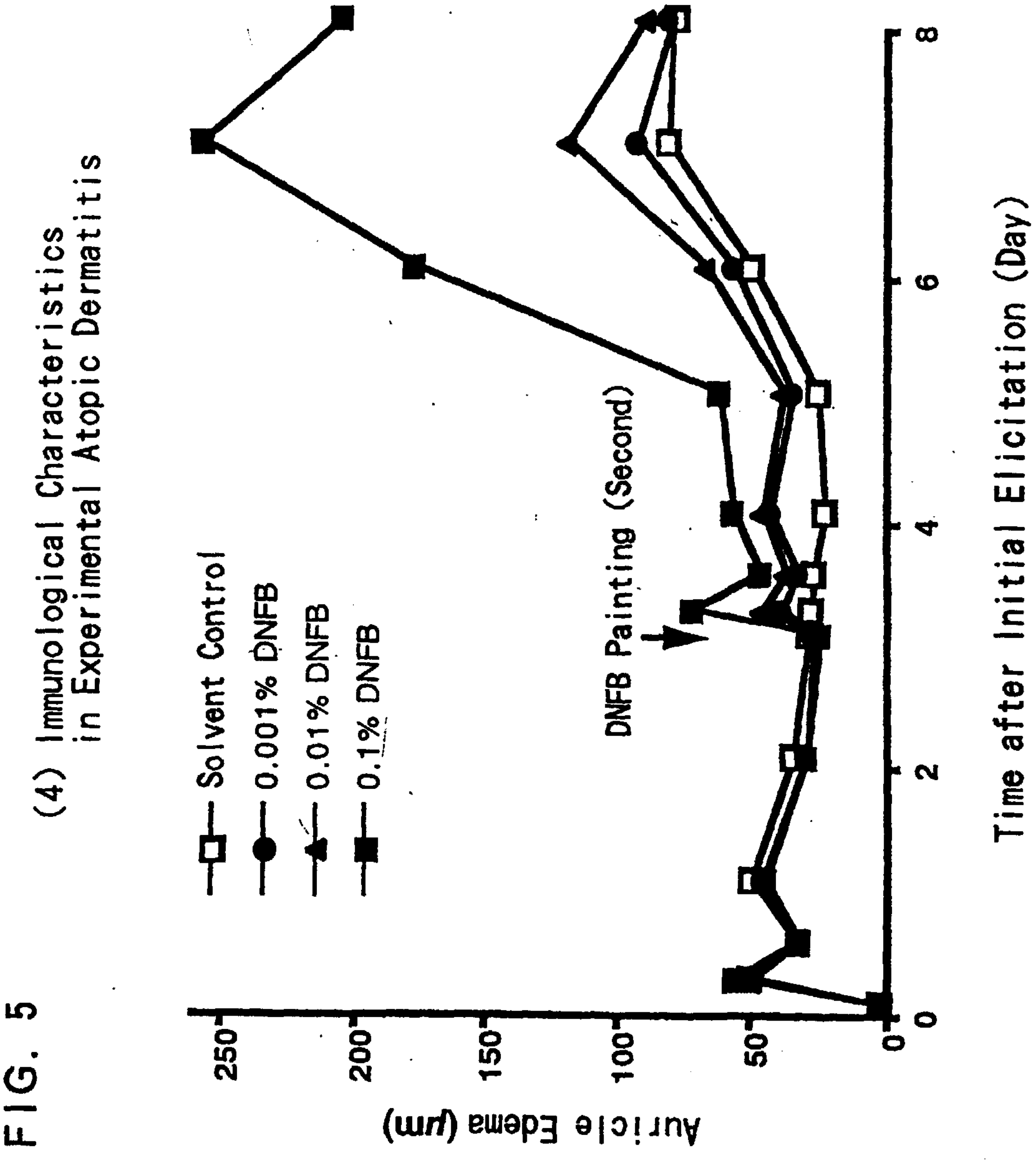
(4) Immunological Characteristics
in Experimental Atopic Dermatitis
Solvent Control
0.001% DNFB
0.01% DNFB
0.1% DNFB
DNFB Painting (Second)
Auricle Edema (μm)
0
50
100
150
200
250
0
2
4
6
8
Time after Initial Elicitation (Day)

FIG. 6

(4) Efficacy on Experimental Atopic Dermatitis

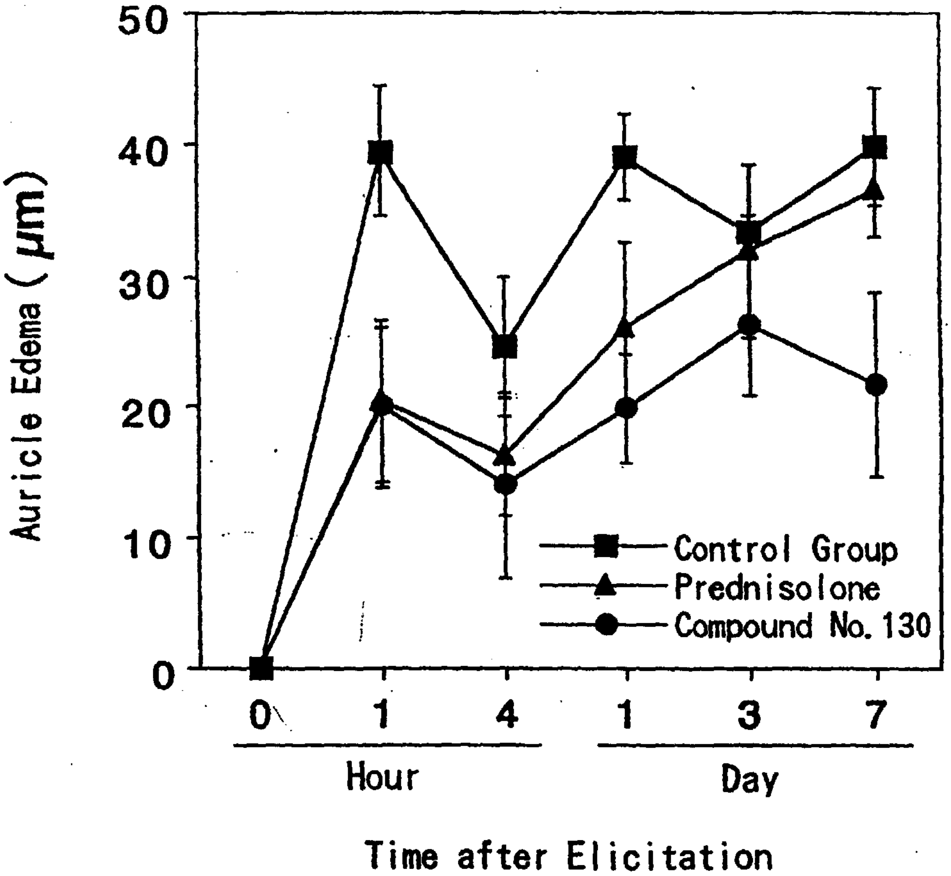

FIG. 7

(5) Efficacy on Atopic Dermatitis

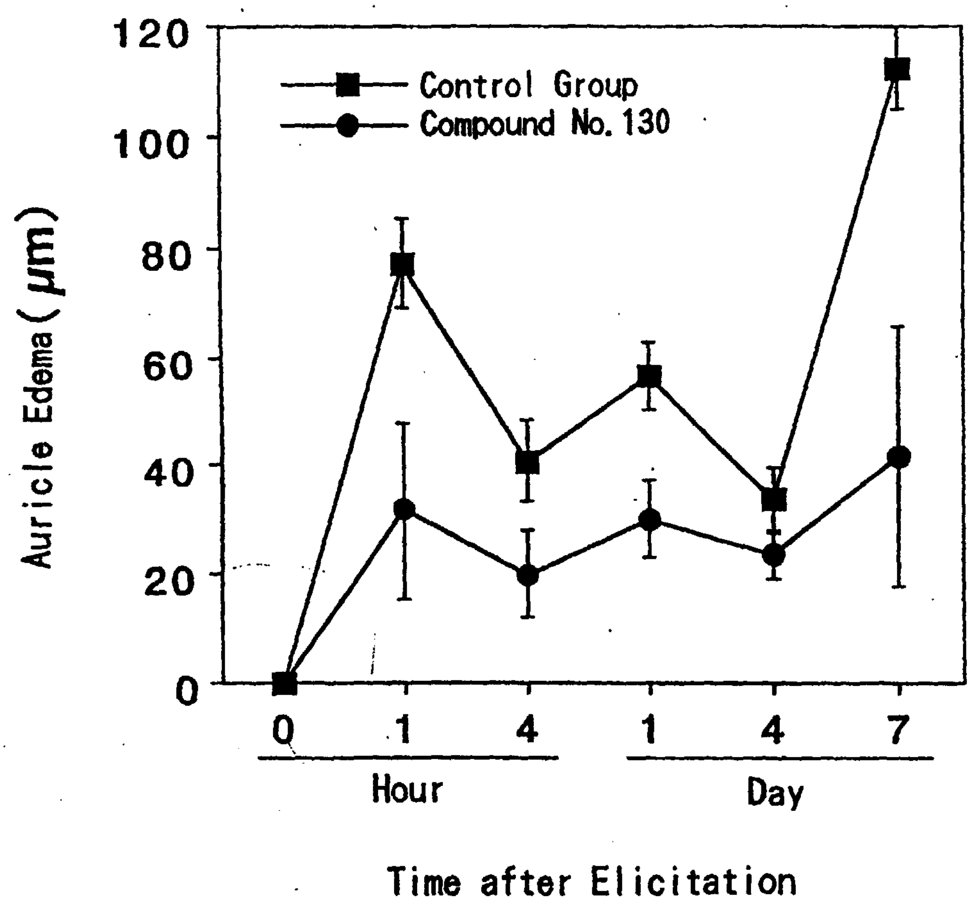

FIG. 8

(6) Efficacy on Experimental Atopic Dermatitis

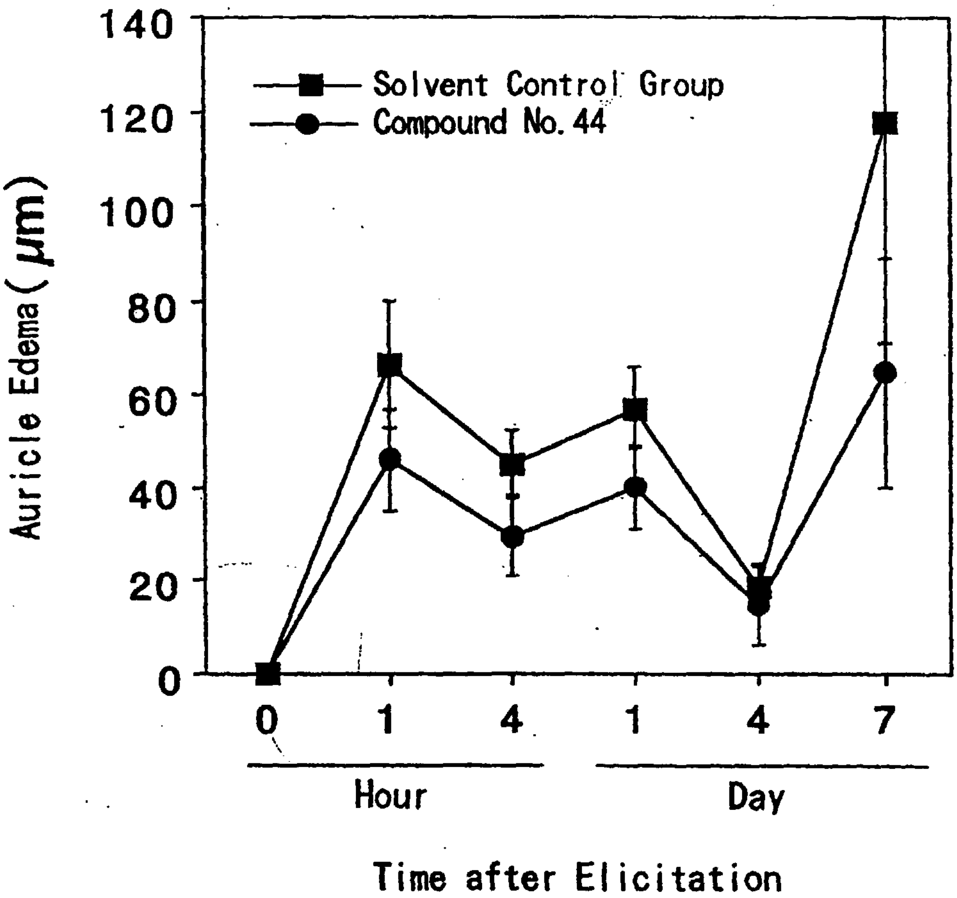

FIG. 9

(1) Efficacy on Scratching Behavior in Experimental Atopic Dermatitis Model

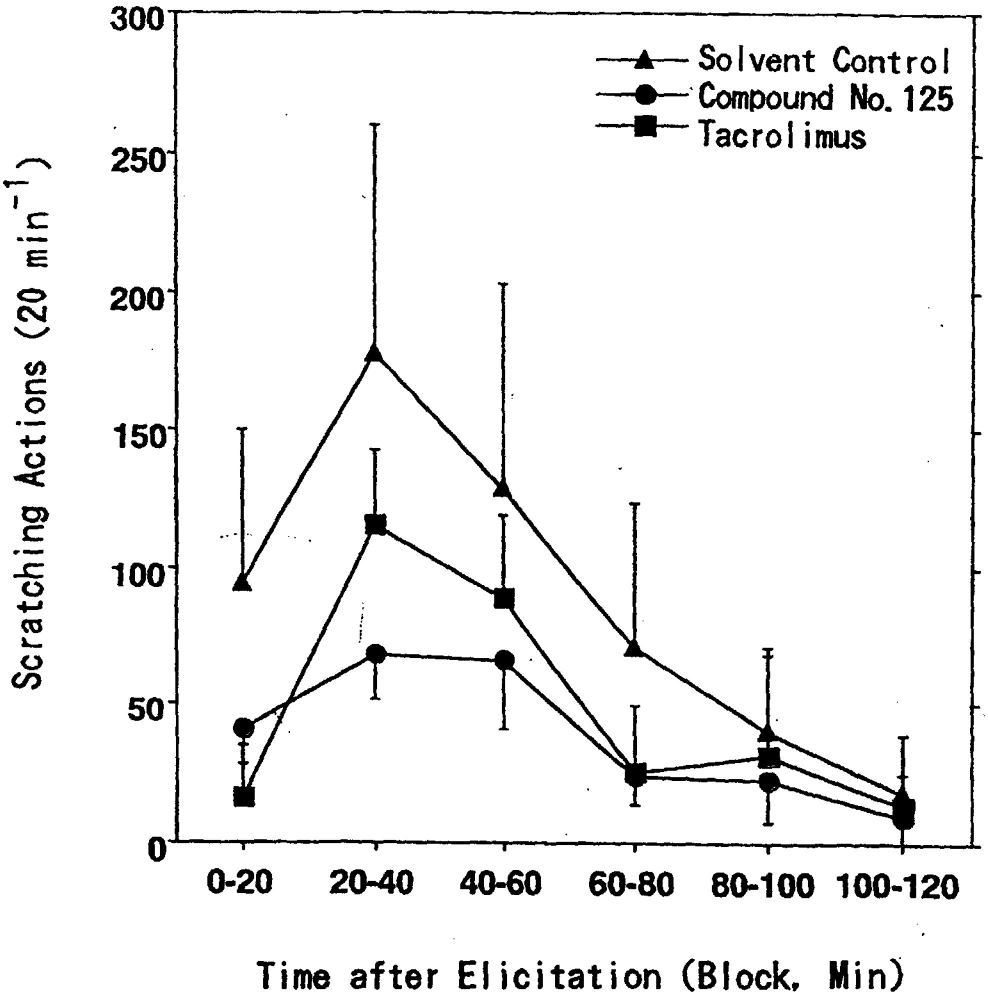

FIG. 10

(2) Efficacy on Body Weight Gain

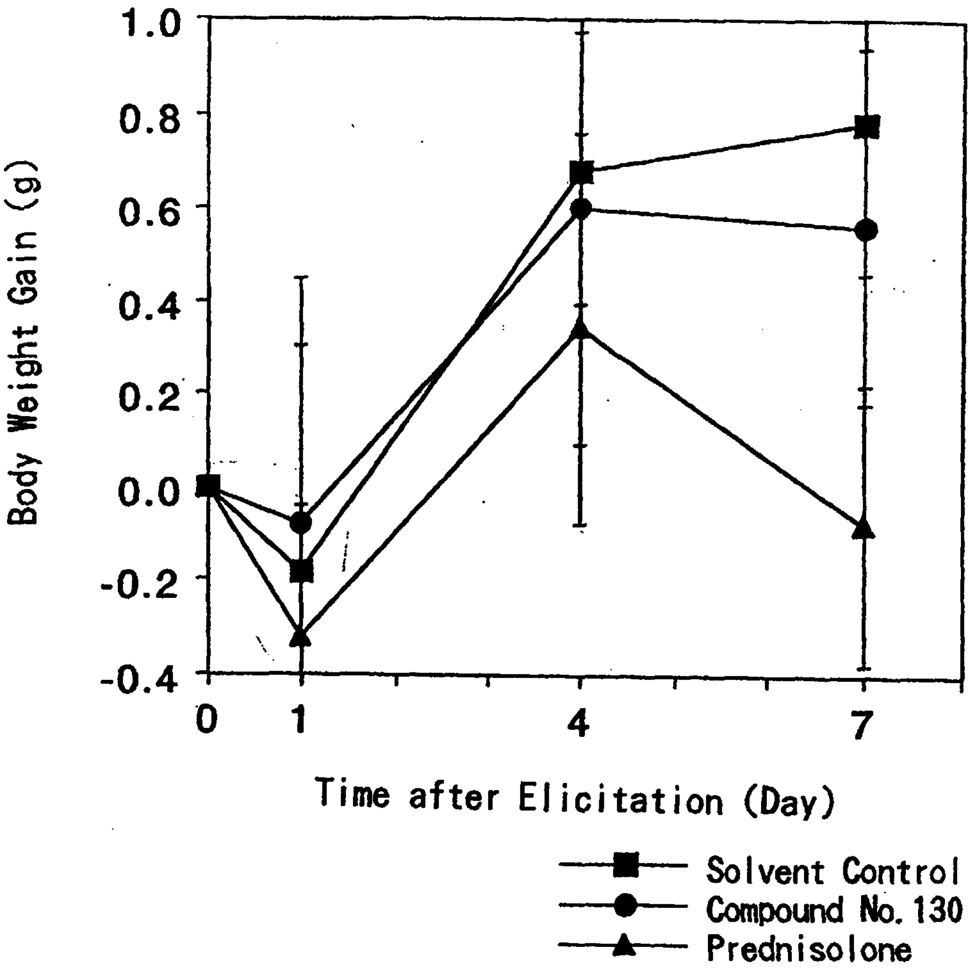

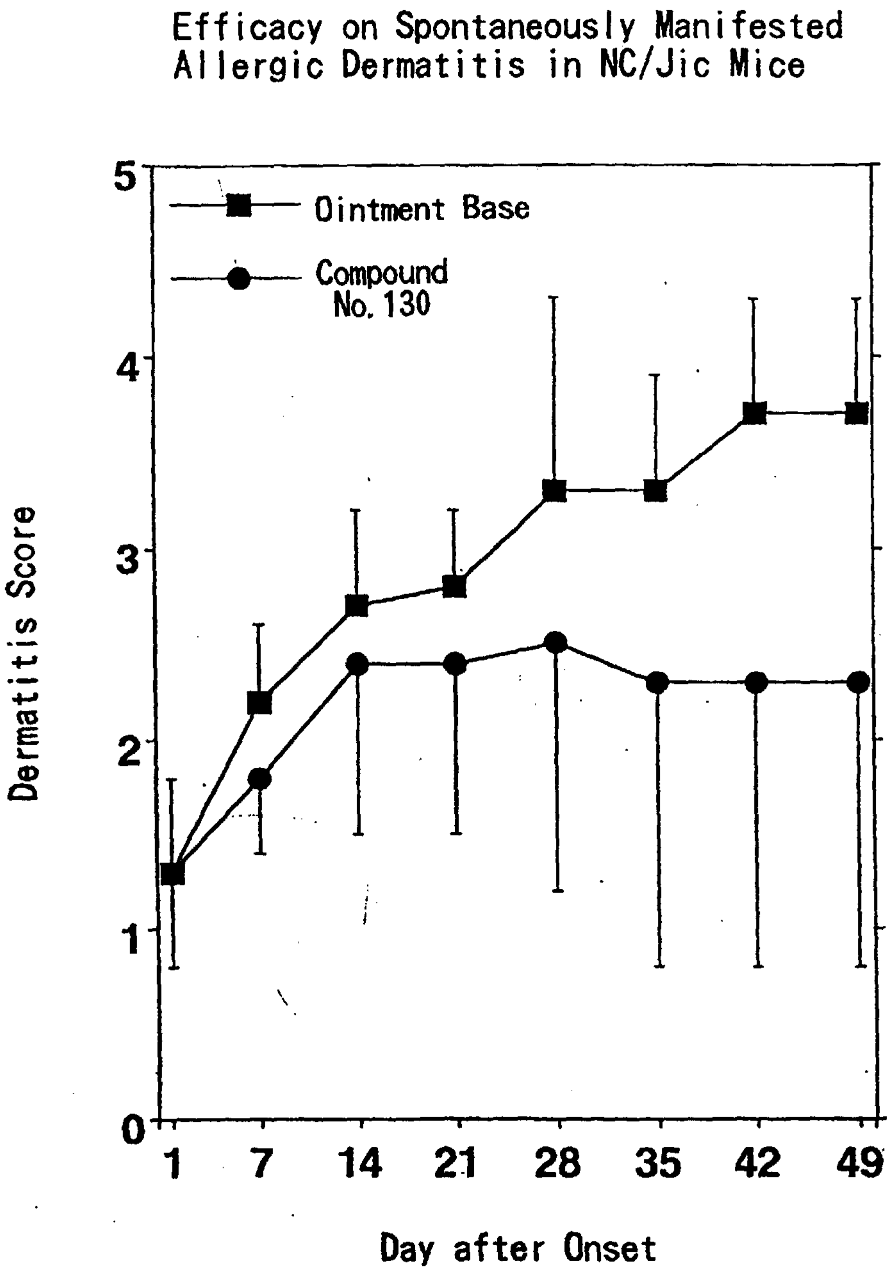
Efficacy on Spontaneously Manifested
Allergic Dermatitis in NC/Jic Mice
Ointment Base
Compound
No. 130
Dermatitis Score
0
1
2
3
4
5
1
7
14
21
28
35
42
49
Day after Onset

FIG. 11

(1) Efficacy on Experimental Bronchial Asthma

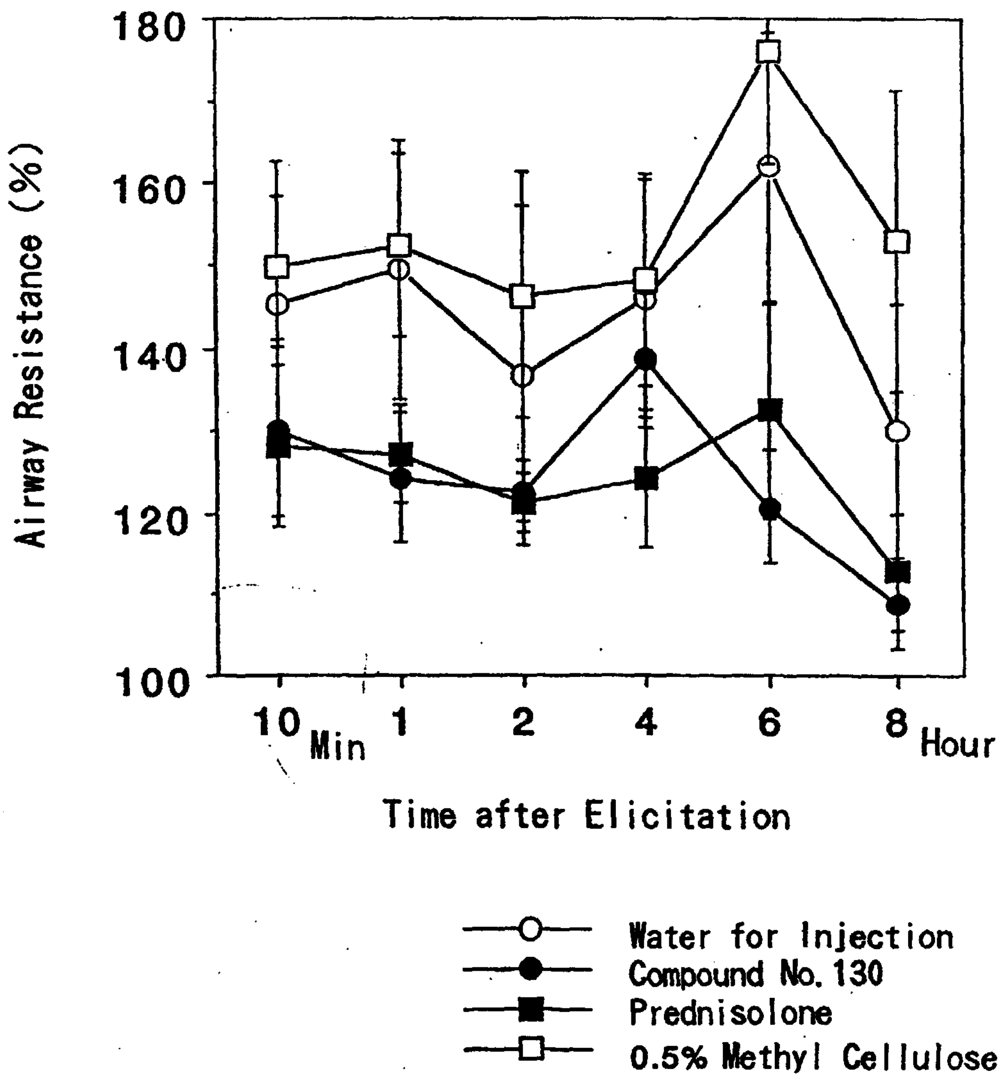


FIG. 12

FIG. 13

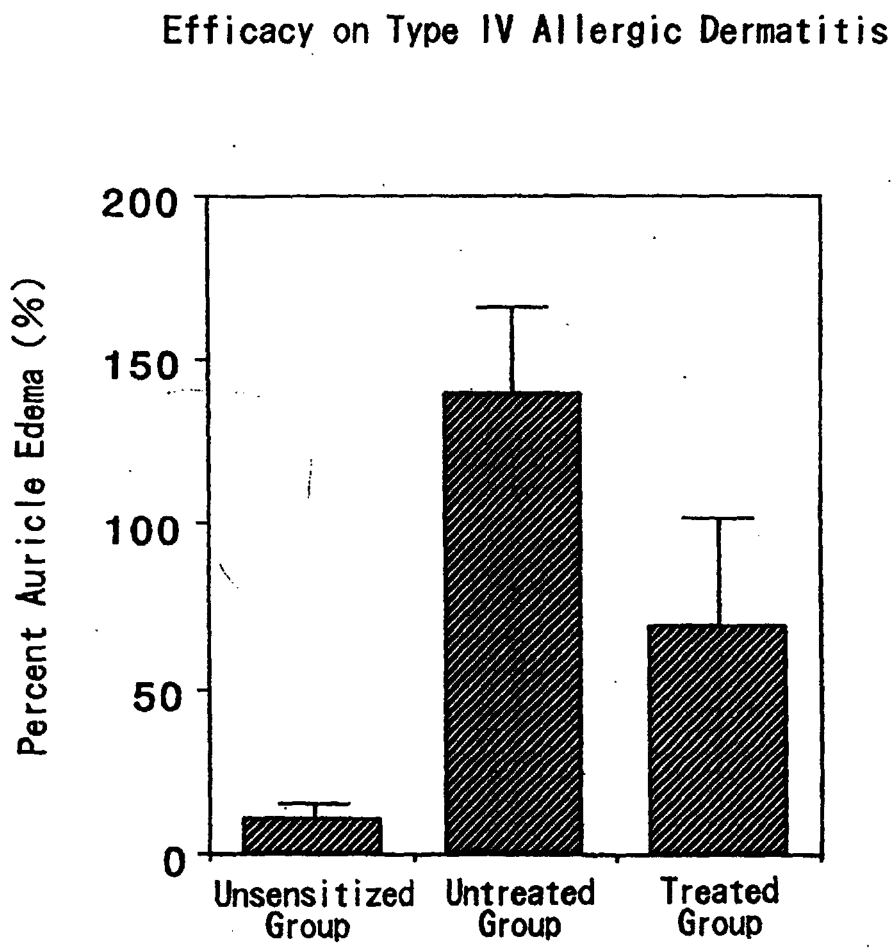
Efficacy on Type IV Allergic Dermatitis
Percent Auricle Edema (%)
200
150
100
50
0
Unsensitized Group
Untreated Group
Treated Group

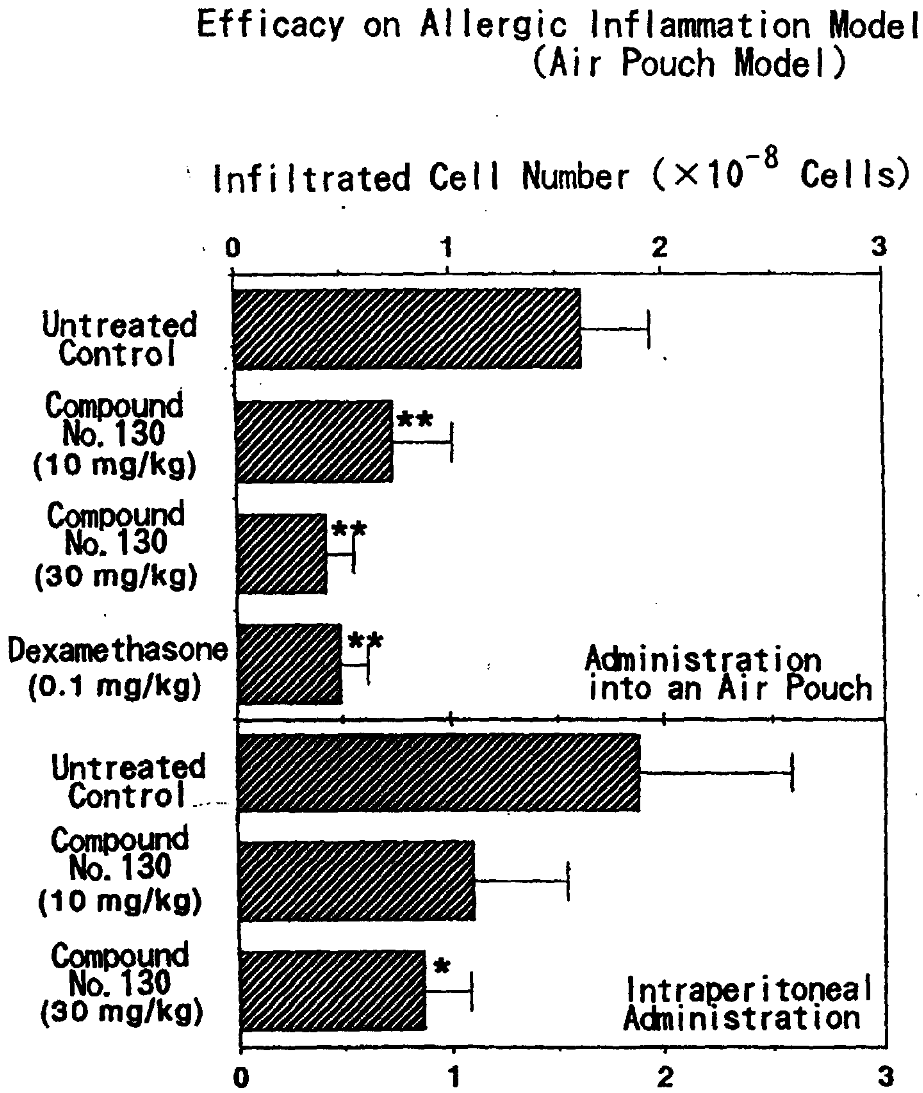
Efficacy on Allergic Inflammation Model
(Air Pouch Model)
Infiltrated Cell Number (×10$^{-8}$ Cells)
0
1
2
3
Untreated Control
Compound No. 130 (10 mg/kg)
**
Compound No. 130 (30 mg/kg)
**
Dexamethasone (0.1 mg/kg)
**
Administration into an Air Pouch
Untreated Control
Compound No. 130 (10 mg/kg)
Compound No. 130 (30 mg/kg)
*
Intraperitoneal Administration
0
1
2
3

FIG. 14

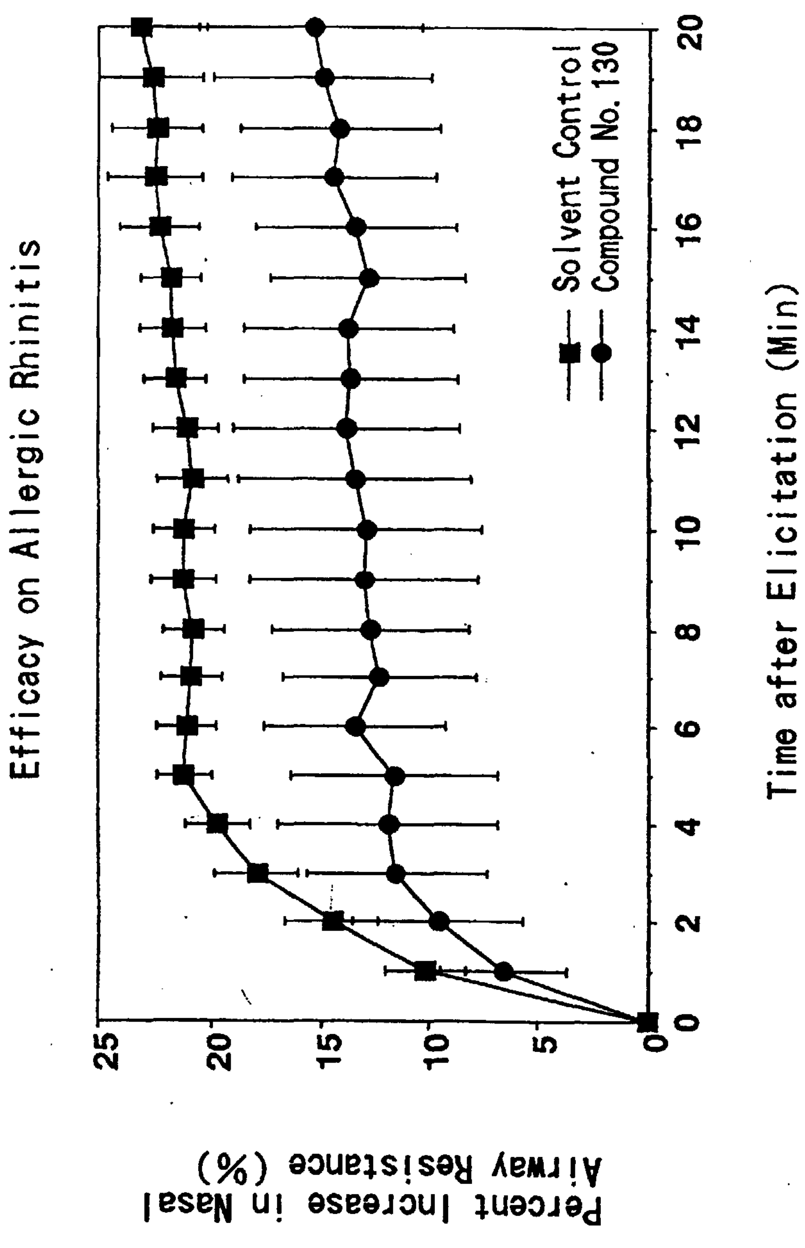
Efficacy on Allergic Rhinitis
Percent Increase in Nasal Airway Resistance (%)
25
20
15
10
5
0
0
2
4
6
8
10
12
14
16
18
20
Time after Elicitation (Min)
Solvent Control
Compound No. 130

FIG. 15

## INTERNATIONAL SEARCH REPORT

International application No.
PCT/JP99/03851

A. CLASSIFICATION OF SUBJECT MATTER
Int.Cl[6] A61K31/16, 31/165, 31/215, 31/35, 31/415, 31/66, 31/70

According to International Patent Classification (IPC) or to both national classification and IPC

B. FIELDS SEARCHED

Minimum documentation searched (classification system followed by classification symbols)
Int.Cl[6] A61K31/16, 31/165, 31/215, 31/35, 31/415, 31/66, 31/70

Documentation searched other than minimum documentation to the extent that such documents are included in the fields searched

Electronic data base consulted during the international search (name of data base and, where practicable, search terms used)
CAPLUS (STN), MEDLINE (STN)

C. DOCUMENTS CONSIDERED TO BE RELEVANT

| Category* | Citation of document, with indication, where appropriate, of the relevant passages | Relevant to claim No. |
|---|---|---|
| X | WO, 96/02240, A2 (Smithkline Beecham P.L.C.), 1 February, 1996 (01. 02. 96), whole document & EP, 769939, A1 & JP, 10-502656, A | 1-14 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 4 October, 1999 (04. 10. 99) | 12 October, 1999 (12. 10. 99) |
| Name and mailing address of the ISA/<br>Japanese Patent Office | Authorized officer |
| Facsimile No. | Telephone No. |

Form PCT/ISA/210 (second sheet) (July 1992)